(19) Europäisches Patentamt / European Patent Office / Office européen des brevets

(11) **EP 3 578 173 B1**

(12) **EUROPEAN PATENT SPECIFICATION**

(45) Date of publication and mention
of the grant of the patent:
**26.10.2022 Bulletin 2022/43**

(21) Application number: **19167974.5**

(22) Date of filing: **28.02.2006**

(51) International Patent Classification (IPC):
**A61K 9/28** *(2006.01)*     **A61K 31/485** *(2006.01)*
**A61P 25/04** *(2006.01)*

(52) Cooperative Patent Classification (CPC):
(C-Sets available)
**A61K 31/485; A61K 9/28; A61P 1/00; A61P 1/10;
A61P 25/00; A61P 25/04; A61P 25/30;
A61P 25/36; A61P 29/00; A61P 43/00**    (Cont.)

(54) **DOSAGE FORM CONTAINING OXYCODONE AND NALOXONE**

DARREICHUNGSFORM MIT OXYCODON UND NALOXON

FORME POSOLOGIQUE CONTENANT DE L'OXYCODONE ET DE LA NALOXONE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **28.02.2005 EP 05004377**

(43) Date of publication of application:
**11.12.2019 Bulletin 2019/50**

(62) Document number(s) of the earlier application(s) in
accordance with Art. 76 EPC:
**15180143.8 / 2 962 686**
**10176716.8 / 2 258 353**
**06708567.0 / 1 855 657**

(73) Proprietor: **Euro-Celtique S.A.**
**2350 Luxembourg (LU)**

(72) Inventors:
• **Leyendecker, Petra**
**35619 Braunfels (DE)**

• **Smith, Kevin**
**Cambridge, Cambridgeshire CB4 9LN (GB)**
• **Hopp, Michael**
**65520 Bad Camberg (DE)**

(74) Representative: **Maiwald Patent- und
Rechtsanwaltsgesellschaft mbH
Elisenhof
Elisenstraße 3
80335 München (DE)**

(56) References cited:
EP-A- 1 604 666     WO-A-02/092060
WO-A-03/007802     WO-A-03/084504
WO-A2-03/084520     DE-A1- 4 325 465

**(Cont. next page)**

(52) Cooperative Patent Classification (CPC): (Cont.)

C-Sets
**A61K 31/485, A61K 2300/00**

**Description**

The invention concerns a sustained release oral dosage form comprising oxycodone or a pharmaceutically acceptable salt thereof and naloxone or a pharmaceutically acceptable salt thereof for use in (A) providing an improvement of bowel function during pain therapy, wherein the improvement of bowel function is compared to administering oxycodone alone or for use in (B) treating moderate to severe pain and constipation as side effect occurring during pain therapy or for use in (C) the prevention and/or treatment of constipation as side effect during pain therapy without reducing the analgesic effect of the oxycodone, wherein the dosage form comprises (i) oxycodone or a pharmaceutically acceptable salt thereof in an amount corresponding to 40 mg anhydrous oxycodone hydrochloride, and naloxone or a pharmaceutically acceptable salt thereof in an amount corresponding to 20 mg anhydrous naloxone hydrochloride; or (ii) oxycodone or a pharmaceutically acceptable salt thereof in an amount corresponding to 20 mg anhydrous oxycodone hydrochloride, and naloxone or a pharmaceutically acceptable salt thereof in an amount corresponding to 10 mg anhydrous naloxone hydrochloride; or (iii) oxycodone or a pharmaceutically acceptable salt thereof in an amount corresponding to 10 mg anhydrous oxycodone hydrochloride, and naloxone or a pharmaceutically acceptable salt thereof in an amount corresponding to 5 mg naloxone hydrochloride.

**BACKGROUND OF THE INVENTION**

The treatment of severe pain resulting from diseases such as cancer, rheumatism and arthritis is central to the treatment of these diseases. The range of pain felt by tumor patients comprises pain of the periosteum and of the bone itself, as well as visceral pain and pain in soft tissues. All such pain forms render the daily life of patients intolerable and often lead to depressive states. Successful pain therapy resulting in a lasting improvement of quality of life for the patients is therefore equally important for the success of a comprehensive therapy, as is the treatment of the actual causes of the disease.

Having regard to the importance of a successful pain therapy, the World Health Organization (WHO) has developed a 4-step model for the treatment of patients with tumor pain. This model has proven to be effective in daily routine practice and can be extended to patients suffering from chronic pain or pain forms resulting from diseases other than cancer. Depending on the intensity, kind and localization of pain, four steps are distinguished during this therapy, with each next step being indicated if the effect of the pain relief agent used until then is no longer sufficient (Ebell, H. J.; Bayer A. (Ed.): Die Schmerzbehandlung von Tumorpatienten, Thieme 1994 (Supportive Maßnahmen in der Onkologie, Band 3) and Zech, D.; Grond, S.; Lynch, J.; Hertel, D.; Lehmann, K.: Validation of World Health Organisation Guidelines for Cancer Pain Relief: a 10-year prospective study, Pain (1995), 63, 65-76).

According to this 4-step model of the WHO, opioid analgesics take a central role in treating pain. The group of opioid analgesics comprises, besides morphine (which represents the prototype of these pharmaceutically active agents), also oxycodone, hydromorphone, nicomorphine, dihydrocodeine, diamorphine, papaveretum, codeine, ethylmorphine, phenylpiperidine and derivatives thereof; methadone, dextropropoxyphene, buprenorphine, pentazocine, tilidine, tramadol and hydrocodone. The ATCC-Classification (Anatomical Therapeutic Chemical Classification) of the WHO indicates whether the pharmaceutically active agent is an opiod analgesic or not. The pronounced pain-relieving effect of opioid analgesics is due to the imitation of the effect of endogenous, morphine-like acting substances ("endogenous opioids"), whose physiological function is to control the reception and processing of pain stimuli.

Opioids repress the propagation of pain stimuli. Besides the immediate inhibition of neuronal excitatory signal transduction in the spinal cord caused by opioids, an activation of those nerve tracts projecting from the brainstem into the spinal cord also plays a role. This activation results in an inhibition of pain propagation in the spinal cord. Moreover, opioids limit the pain reception of the thalamus and, by affecting the limbic system, they influence the affective pain evaluation.

Opioid receptors are found at different sites in the body. Receptors of the intestine and brain are of particular importance for pain therapy by opioids, especially as their occupation results in different side effects.

Opioid analgesics are considered to be strong agonists if they bind with high affinity to opioid receptors and induce a strong inhibition of pain reception. Substances that also bind with high affinity to opioid receptors, but that do not cause a reduction of pain reception and which thereby counteract the opioid agonists, are designated as antagonists. Depending on the binding behaviour and the induced activity, opioids can be classified as pure agonists, mixed agonists/antagonists and pure antagonists. Pure antagonists comprise, for example, naltrexone, naloxone, nalmefene, nalorphine, nalbuphine, naloxoneazinen, methylnaltrexone, ketylcyclazocine, norbinaltorphimine, naltrindol, 6-β-naloxol und 6-β-naltrexol (Forth W.; Henschler, D.; Rummel W.; Starke, K.: Allgemeine und Spezielle Pharmakologie und Toxikologie, 7. Auflage, 1996, Spektrum Akademischer Verlag, Heidelberg Berlin Oxford).

Due to their good analgesic efficiency, compounds such as oxycodone, tilidine, buprenorphine und pentazocine, have been used in the form of medicaments for pain therapy. It has been proven that medicaments such as Oxygesic® having oxycodone as the analgesic active compound und Valoron® having tilidine as the analgesic active compound are valuable for pain therapy.

However, use of opioid analgesics for pain therapy might be accompanied by undesirable side effects. For instance, long-term use of opioid analgesics can lead to psychological and physical dependence.

Especially the physical dependence of patients suffering from pain on opioid analgesics may lead to the development of tolerance, meaning that upon extended intake, increasingly higher doses of the pain-relieving agent have to be taken by the patient, in order to experience pain relief. The euphoregenic effect of opioid analgesics may lead to the abuse of pain-relievers. Drug abuse and psychological dependence are known, especially among teenagers. However, opioid analgesics are legitimately used for medical purposes and medicine cannot do without them.

Besides the mentioned disadvantages, the use of potent opioid analgesics for pain therapy often also lead to undesirable side effects, such as constipation, breath depression, sickness and sedation. Less frequently, urge or inability to pass water are observed.

Different attempts have been made to counteract the habituation processes and the other side effects occurring during pain therapy. This can be done, e.g. by traditional treatment methods. In the case of drug addiction this might be a drug withdrawal treatment, and in the case of constipation, this might be done by administration of laxatives.

Other attempts aim at minimizing the addictive and habituation forming potential of opioid analgesics, as well as their other side effects by the administration of antagonists which counteract the opioid analgesic. Such antagonists might be naltrexone or naloxone.

There have been numerous proposals and suggestions as to how the application of the aforementioned active compounds could be used to avoid undesired habituation and dependence, or even addiction.

US 3,773,955 and US 3,966,940 suggested formulating analgesics in combination with naloxone, purportedly to prevent dependence-promoting effects such as euphoria and the like upon parenteral application. The avoidance of side effects such as constipation was not addressed.

To limit the parenteral abuse of oral application forms, US 4,457,933 suggested using a combination of morphine with naloxone in defined ranges. The avoidance of side effects such as constipation was not mentioned in this patent either.

US Patent No. 4,582,835 describes, again in order to avoid abuse, a preparation comprising a combination of buprenorphine and naloxone to be administered either parenterally or sublingually.

EP 0 352 361 A1 concerns the treatment of constipation during pain therapy by the oral application of an opioid analgesic and one antagonist. Avoidance of abuse of the opioid analgesic is not an issue in this application.

DE 43 25 465 A1 also concerns the treatment of constipation during pain therapy using a preparation comprising an opioid analgesic and an antagonist. According to this disclosure, the antagonist, which can be naloxone, may be present in higher amounts than the opioid analgesic, which is preferably morphine. The avoidance of abuse of the opioid analgesic is not an issue in DE 43 25 465 A1.

In order to avoid abuse of pain medications, preparations have been introduced on the market which can be taken orally and comprise an opioid analgesic and the opioid antagonist, naloxone. The medicament Talwin® of Windrop/Sterling comprises pentazocine and naloxone. The medicament Valoron® of Gödeke comprises a tilidine-naloxone combination.

Besides potent analgesic effect, the reduction of addictive potential and the avoidance of side effects, medicaments suitable for a successful pain therapy should possess additional characteristics.

Generally, medicaments have to be formulated in such a way that the active compounds are stable as long as possible under standard storage conditions. Medicaments have also to be formulated in such a way that the intended release profiles of the active compounds do not change upon long-term storage.

Medicaments suitable for pain therapy should either contain the active compounds in such amounts, or be formulated in such a way, that they have to be taken by the patients only at long intervals. The easier the application scheme for a pain-reliever is, and the clearer it is for the patient why and how often he should take which tablet, the more exactly will he adhere to the physician's orders. The necessity to take the pain-reliever only infrequently will result in increased willingness of the patient to take the pain-reliever (compliance).

The medicament Oxygesic® is a preparation from which the opioid analgesic oxycodone is released in a sustained manner. Oxygesic® does not contain opioid antagonists.

According to EP 0 352 361 A1, neither the opioid analgesic nor the antagonist are formulated to be released in a sustained manner. Accordingly, the time period during which such preparations are effective is limited and preparations have to be taken a number of times a day. The desired compliance of the patient is not achieved. EP 0 352 361 A1 also does not disclose the advantages of formulations of preparations that are characterized by a time-stable and independent release of the active compounds. The storage stability of such preparations is also not addressed by this disclosure.

DE 43 25 465 A1 discloses formulations according to which constipation occurring during pain therapy is prevented by the sustained release of the opioid agonist, while the antagonist, which is present in excess, is not released in a sustained manner. Due to the high first-pass-effect of naloxone, relatively large amounts of this compound have therefore to be used. However, DE 43 25 465 A1 does not disclose preparations, which are characterized by time-stable and independent release of the active compounds. The storage stability of such preparations is also not described therein.

Under the trademark Valoron®, a pain-reliever is marketed which comprises a tilidine naloxone combination. According to the product literature, a formulation is used from which both active compounds are released in a sustained manner. The matrix used comprises a significant amount of water-swellable material, that is HPMC. However, this formulation, given identical mass ratio but different absolute amounts of tilidine and naloxone, shows different release profiles. The release rates of the agonist and the antagonist are not independent from each other. Accordingly, it is necessary for the physician to carry out extensive titration experiments for each individual patient if an increase of the dosage is desired, even though the mass ratio of tilidine:naloxone is not altered, since it cannot be assumed that the release profiles of both components will remain constant. The range of therapeutically suitable amounts of the analgesic is therefore limited.

WO 03/084520 describes a storage-stable pharmaceutical preparation comprising oxycodone and naloxone for use in pain therapy, with the active compounds being released from the preparation in a sustained, invariant and independent manner.

WO 03/084504 discloses a storage stable pharmaceutical formulation comprising preferably two active compounds such as oxycodone and naloxone in a non-swellable diffusion matrix, wherein the compounds are released from the matrix in a sustained, invariant and, if several compounds are present, independent manner. Preferably, the matrix is determined with respect to its substantial release characteristics by ethylcellulose and at least one fatty alcohol.

There is a need for oxycodone naloxone dosage forms characterized by *in vivo* parameters which provide for a fast and long-lasting analgesic effect while preventing and/or treating side effects during pain therapy and also preventing or reducing drug abuse.

## SUMMARY OF THE INVENTION

It is therefore an object of the present invention to provide an oxycodone naloxone dosage form which provides a fast analgesic effect and, at the same time, is suitable in chronic maintenance therapy.

It is a further object of the present invention to provide an oxycodone naloxone dosage form which is suitable for the prevention and/or treatment of side effects during pain therapy such as opioid bowel dysfunction syndromes such as constipation without substantially reducing the analgesic effect of oxycodone.

Further, it is not according to the invention to provide an oxycodone naloxone dosage form which is suitable to prevent habituation and/or addiction-promoting effects during pain therapy without substantially reducing the analgesic effect of oxycodone.

It is not according to the invention to provide an oxycodone naloxone dosage form which is suitable to prevent abuse of the preparation by e.g. drug addicts.

In particular, it is an object of the present invention to provide a dosage form for pain therapy that, besides high analgesic activity, is characterized by reduced side effects, said dosage form also being characterized by reduced administration frequency thus ensuring increased patient compliance, as well as facilitating individual adaptation of the dosage for each patient.

It is another object of the present invention to provide a sustained release oxycodone naloxone formulation which may also be used to titrate a patient receiving oxycodone therapy and, at the same time, is suitable in chronic maintenance therapy after titration of the patient.

Further, it is not according to the invention to provide an oxycodone naloxone dosage form which does not evoke clinically significant opioid withdrawal symptoms in patients or healthy human subjects.

Further, it is not according to the invention to provide an oxycodone naloxone dosage form which evokes opioid withdrawal symptoms in opioid addicted individuals and opioid abusers, if e.g. administered intravenously or by the nasal route.

Further, it is an object of the present invention to provide an oxycodone naloxone dosage form which reduces laxative intake.

Further, it is an object of the present invention to provide an oxycodone naloxone dosage form which is acceptable in terms of occurrence of adverse effects such as diarrhea.

Further, it is not according to the invention to provide an oxycodone naloxone dosage form which during steady state provides a reduction of severity of elicited opioid typical adverse events and but no substantial increase of severity of elicited naloxone typical adverse events. Further, it is an object of the present invention to provide an oxycodone naloxone dosage form which shows good efficacy and tolerability.

Further, it is not according to the invention to provide an oxycodone naloxone dosage form which does not show a clinically relevant food effect after eating a high fat meal with respect to pharmacokinetic parameters such as AUC, $t_{max}$ and $c_{max}$.

Further, it is not according to the invention to provide an oxycodone naloxone dosage form that can be used in patients or individuals in amounts that would not be indicated if oxycodone was to be administered without naloxone

One particular object of the present invention is to provide a sustained release pharmaceutical dosage form comprising oxycodone and naloxone in a ratio that is particularly suitable to ensure analgetic efficacy and tolerability and reduction and/or prevention of side effects.

In one aspect not according to the invention, a dosage form is provided which comprises oxycodone and naloxone and provides a mean $t_{max}$ for oxycodone at about 1 to about 17 hours, at about 2 to about 15 hours, at about 3 to about 8 hours or at about 4 to about 5 hours after administration at steady state or of a single dose to human patients or healthy human subjects. In one preferred embodiment the dosage form provides a mean $t_{max}$ of 3 hours, 3.5 hours or 4.0 hours for oxycodone after single dose or steady state administration to healthy human subjects or human patients. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that with respect to its release characteristics is formed from an ethyl cellulose and at least one fatty alcohol.

In one aspect of the present invention, a dosage form is provided which comprises oxycodone and naloxone and which is for use in providing an improvement of bowel function during pain therapy compared to administering oxycodone alone, wherein the dosage form is the dosage form as defined in claim 1. Such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises 40 mg oxycodone and 20 mg naloxone. The dosage form preferably releases the active agents in a sustained, invariant and independent manner from a substantially non-swellable diffusion matrix that with respect to its release characteristics is formed from an ethyl cellulose and at least one fatty alcohol.

In a further aspect not according to the invention, a dosage form is provided which comprises oxycodone and naloxone and provides an analgesic effect for at least about 12 hours or at least about 24 hours after administration at steady state or of a single dose to human patients or healthy human subjects. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

In a further aspect not according to the invention, a dosage form is provided which comprises oxycodone and naloxone and provides an mean AUCt value for oxycodone of about 100 ng·h/mL to about 600 ng·h/mL, or of about 300 ng·h/mL to about 580 ng·h/mL or of about 400 ng·h/mL to about 550 ng·h/mL, or of about 450 ng·h/mL to about 510 ng·h/mL after administration at steady state or of a single dose to human patients or healthy human subjects. In one embodiment such values are obtained if dosage strengths of 10 mg, 20mg or up to 40 mg oxycodone are administered either as single dose or during steady state. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

In a further aspect not according to the invention, a dosage form is provided which comprises oxycodone and naloxone and which provides a mean $C_{max}$ for oxycodone of about 5 ng/mL to about 50 ng/mL, or of about 20 ng/mL to about 40 ng/mL or of about 30 ng/mL or of about 35 ng/mL after administration at steady state or of a single dose to human patients or healthy human subjects. In one embodiment, such values are obtained if dosage strengths of 10mg, 20 mg or up to 40 mg oxycodone are administered either as single dose or during steady state. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that with respect to its release characteristics is formed from an ethyl cellulose and at least one fatty alcohol.

In a further aspect not according to the invention, a dosage form is provided which comprises oxycodone and naloxone and preferably, or alternatively, in terms of efficacy is ranked good or very good by more than 50% of patients and preferably by more than 70% of patients.

In a further aspect not according to the invention, a dosage form is provided which comprises oxycodone and naloxone and preferably, or alternatively, in terms of tolerability is ranked good or very good by more than 60% of patients and preferably by more than 70% or even 80% of patients.

In a further aspect not according to the invention, a dosage form is provided which comprises oxycodone and naloxone and which provides a reduction of days with laxative intake by at least 10%, preferably by at least 20%, more preferably by at least 25% and even more preferably by at least 30%. Some dosage forms of the present invention even allow a reduction of at least 35% or at least 40%.

In a further aspect not according to the invention, a dosage form is provided which comprises oxycodone and naloxone and preferably, or alternatively, is clinically acceptable in terms of adverse events.

In a further aspect not according to the invention, a dosage form is provided which comprises oxycodone and naloxone and preferably or alternatively provides a reduction of severity of elicited opioid typical adverse events and but no substantial increase of severity of elicited naloxone typical adverse events.

Yet another embodiment not according to the invention relates to oxycodone naloxone dosage forms preparations that preferably, or alternatively, shows no substantial food effect.

Yet another embodiment not according to the invention relates to oxycodone naloxone dosage forms preparations that precipitate withdrawal symptoms in opioid dependent humans, preferably if the preparations are administered intravenously or via the nasal route. In one embodiment the dosage forms in accordance with the disclosure precipitate longer lasting withdrawal effects than naloxone alone. In a preferred embodiment, the above dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

According to a further aspect not according to the invention, a method of treating moderate to severe pain in a patient by administering a dosage form according to the present disclosure is provided. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

According to another aspect of the invention, a dosage form is provided for use in treating moderate to severe pain and constipation as side effect occurring during pain therapy, wherein the dosage form is the dosage form as defined in claim 2. Such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

According to a further aspect not according to the invention, a method of treating moderate to severe pain in a patient while preventing or reducing abuse by administering a dosage form according to the present disclosure is provided. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form may release the active agents in a sustained, invariant and independent manner from a substantially non-swellable diffusion matrix that, with respect to its release characteristics is formed from an ethyl cellulose and at least one fatty alcohol.

According to an embodiment not according to the invention, a method of treating moderate to severe pain in a patient while ensuring tolerability and preventing or reducing abuse and side effects such as opioid bowel dysfunction syndromes such as constipation, diarrhea etc. by administering a dosage form according to the present disclosure is provided. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

According to further aspect not according to the invention, a method of treating moderate to severe pain is provided in which during steady state severity of elicited opioid typical adverse events is reduced while elicited naloxone typical adverse events are not increased and remain substantially the same.

According to further aspect not according to the invention, a method of treating moderate to severe pain in patient goups is provided in which oxycodone amounts can be administered that would be prohibitive if naloxone was not present. In one embodiment these methods are use treat moderate to severe pain in opioid naive patients or elderly patients.

According to a further aspect of the invention, an oxycodone naloxone dosage form is provided for use in the prevention and/or treatment of constipation as side effect during pain therapy without reducing the analgesic effect of oxycodone, wherein the dosage form is the dosage form as defined in claim 3.

## BRIEF DESCRIPTION OF THE DRAWINGS

Figure 1 shows a paper form for assessing the bowel function index (BFI3) which is suitable for use in a method for assessing bowel function.

Figure 2 shows a circular bowel function index (BFI3) meter which is suitable for use in a method for assessing bowel function.

Figures 3 and 4 show the demographics of the patient group that was tested in example 1.

Figure 5 shows the schematic study design for the clinical study of example 1.

Figures 6 to 8 are tables summarizing the values for mean bowel function at each study visit by dose ratio, by absolute dose of naloxone and by absolute dose of naloxone given the same oxycodone/naloxone dose ratio in the ITT population according to example 1.

Figure 9 is a table summarizing the test for difference for each dose of naloxone versus placebo according to example 1.

Figure 10 shows a surface plot of the whole dose range investigated based on the RSREG estimations of the model parameters according to example 1.

Figure 11 shows a contour plot of the bowel function with a granulation of 10 according to example 1.

Figure 12 to 15 show the results for the global assessment of the preparations tested in example 1.

Figures 16 and 17 show the results for laxative intake during the clinical trials described in example 1.

Figures 18 to 21 show the results for adverse events as observed in the clinical trials of example 1.

Figures 22 to 28 show mean observed plasma concentration - time curves for oxycodone, naloxone-3-glucuronide, naloxone, noroxycodone, oxymorphone, noroxymorphone and 6-$\beta$-naloxol according to example 2.

Figure 29 illustrates the study design of the clinical trials of example 3.

Figures 30 to 37 show the results for pharmacokinetic parameters of oxycodone, naloxone-3-glucuronide and naloxone as observed in the clinical trials of example 3.

Figure 38 illustrates the study design of clinical trials of example 4.

Figures 39 and 40 illustrate the experimental pain model of and parameters measured in example 4.

Figures 41 to 43 show the results for pain-related evoked potentials and mean tonic pain scores as measured in example 4.

Figures 44 and 45 show the determination of pharmacokinetic parameters and a dose-response curve for i.v. oxycodone in rats of example 5.

Figure 46 to 48 show the results for occurrence of withdrawal symptoms in example 5.

Figures 49 to 52 show the sum score for elicited opioid typical and elicited naloxone typical adverse events as determined in experiment 1.

## DETAILED DESCRIPTION OF THE INVENTION

Oxycodone is an opioid analgesic that was introduced into the German market as a controlled-release formulation (Oxygesic®) in 1998. Its indication is severe to most severe pain of malignant and non-malignant origin. However, like all opioids, oxycodone has a potential for abuse. The restriction on narcotic drugs worldwide limits the use of opioids in the medical field and impedes the pain therapy of chronic pain patients with strong opioids. According to the present invention, development of habituation and addiction as well as obstipation and breath depression are to be considered as side effects of analgesically effective opioid agonists such as oxycodone.

Naloxone is a commercially available intravenous narcotic antagonist, which is indicated for the blockade of exogenously administered opioids. It acts at all opioid receptor sites ($\mu$, $\kappa$, and $\delta$). Following oral administration, naloxone is rapidly absorbed (within 5-30 minutes) but has a very low oral bioavailability of <3% due to an extensive first-pass-metabolism. In low oral doses, naloxone does not become systemically available but acts mainly on local opioid receptors in the gastrointestinal tract.

According to the present invention, severe to moderate pain can be treated by administering an oxycodone/naloxone dosage form according to the present invention while treating constipation as side effect during pain therapy, wherein the dosage form is the dosage form as defined in claim 2. In particular embodiments, the dosage forms for use according to the present invention eliminate the need to first titrate a patient on an immediate release oxycodone dosage form before switching the patient to a sustained release dosage form for chronic therapy.

Co-administration of oxycodone with naloxone by administering dosage forms for use according to the present invention confers advantages with regard to side effects of the drug as specified in the claims. An oxycodone/naloxone dosage form for use according to the present invention reduces the frequency and intensity of opioid bowel dysfunctions syndromes such as constipation as compared to oxycodone alone, wherein the dosage form is the dosage form as defined in claim 1. Moreover, an oxycodone/naloxone dosage form for use according to the present invention reduces oral, intranasal, and i.v. abuse of oxycodone. Since naloxone is not expected to enter the brain, the dosage forms for use according to the present invention do not inhibit the pain relieving action of the oxycodone. The amount of naloxone in the combination product is preferably high enough to precipitate withdrawal effects or at least strong dislike feelings.

The concentration gradients or blood plasma curves can be described by the parameters such as $C_{max}$, $t_{max}$ and AUC. These parameters are important in describing the pharmacokinetic properties of a specific drug formulation.

The $C_{max}$ value indicates the maximum blood plasma concentration of the active agents, i.e. oxycodone and/or naloxone.

The $t_{max}$ value indicates the time point at which the $C_{max}$ value is reached. In other words, $t_{max}$ is the time point of the maximum observed plasma concentration. Usually, the blood concentration gradients with a late $t_{max}$ were aimed at for sustained release formulations, because it was assumed that only in that way a prolonged effect could be guaranteed. However, a disadvantage of a late $t_{max}$ value may be the long time period needed in order to achieve an analgesic effect. The AUC (Area Under the Curve) value corresponds to the area of the concentration curve. The AUC value is proportional to the amount of active agents, i.e. oxycodone and naloxone absorbed into the blood circulation in total and is hence a measure for the bioavailability.

The AUCt value is the value for the area under the plasma concentration-time curve from the time of administration to the last measurable concentration. AUCt are usually calculated using the linear trapezoidal method. Where possible, LambdaZ, which is the terminal phase rate constant, is estimated using those points determined to be in the terminal lock-linear phase. t1/2Z, which is the apparent terminal phase half-life, is commonly determined from the ratio of ln2 to LambdaZ. The areas under the plasma concentration-time curve between the last measured point and infinity may be calculated from the ratio of the final observed plasma concentration ($C_{last}$) to LambdaZ. This is then added to the AUCt to yield AUCinf, which is the area under the plasma concentration-time curve from the time of administration to infinity.

Parameters describing the blood plasma curve can be obtained in clinical trials, first by once-off administration of the active agent such as oxycodone and naloxone to a number of test persons. The blood plasma values of the individual test persons are then averaged, e.g. a mean AUC, $C_{max}$ and $t_{max}$ value is obtained. In the context of the present invention, pharmacokinetic parameters such as AUC, $C_{max}$ and $t_{max}$ refer to mean values. Further, in the context of the present invention, *in vivo* parameters such as values for AUC, $C_{max}$, $t_{max}$, bowel function or analgesic efficacy refer to parameters or values obtained after administration at steady state or of a single dose to human patients and/or healthy human subjects.

If pharmacokinetic parameters such as mean $t_{max}$, $C_{max}$ and AUC are measured for healthy human subjects, they are typically obtained by measuring the development of blood plasma values over time in a test population of approximately 16 to 24 healthy human subjects. Regulatory bodies such as the European Agency for the Evaluation of Medicinal Products (EMEA) or the Food and Drug Administration (FDA) will usually accept data obtained from e.g. 20 or 24 test persons.

The term "healthy" human subject in this context refers to a typical male or female of usually Caucasian origin with average values as regards height, weight and physiological parameters such as blood pressure etc. Healthy human subjects for the purposes of the present invention are selected according to inclusion and exclusion criteria which are based on and in accordance with recommendations of the International Conference for Harmonization of Clinical Trials (ICH). For the purposes of the present invention, healthy subjects may be identified according to the inclusion and exclusion criteria as outlaid in Examples 2, 3, 4 and 6.

Thus, inclusion criteria comprise an age between $\geq 18$ and $\leq 45$ years; a BMI within the range 19 - 29 kg/m$^2$, and within the weight range 60 - 100 kg for males and 55 - 90 kg for females; that females must be non-nursing, non-pregnant, and provide a negative urine $\beta$-hCG pregnancy test within 24 hours before receiving the study medication; generally good health, evidenced by a lack of significantly abnormal findings on medical history, physical examination, clinical laboratory tests, vital signs, and ECG etc.

Exclusion criteria comprise exposure to any investigational drug or placebo within 3 months of the first dose of study medication; any significant illness within the 30 days before the first dose of study medication; any clinically significant abnormalities identified at prestudy screening for medical history, physical examination or laboratory analyses; use of any prescription medication (except HRT for postmenopausal females and contraceptive medication) in the 21 days, or over the counter medication including acid controllers, vitamins, herbal products and/or mineral supplements in the 7 days, before first dose of study medication; concurrent medical condition known to interfere with gastrointestinal drug absorption (e.g. delayed gastric emptying, mal absorption syndromes), distribution (e.g. obesity), metabolism or excretion (e.g. hepatitis, glomerulonephritis); history of, or concurrent medical condition, which in the opinion of the investigator would compromise the ability of the subject to safely complete the study; history of seizure disorders for which subjects required pharmacologic treatment; current history of smoking more than 5 cigarettes a day; subjects with evidence of active or past history of substance or alcohol abuse, according to DSM-IV criteria; subjects who reported regular consumption of 2 or more alcoholic drinks per day or have blood alcohol levels of $\geq 0.5\%$ at screening; donation of more than 500 mL of blood or blood products or other major blood loss in the 3 months before first dose of study medication; any positive results in the prestudy screen for ethanol, opiates, barbiturates, amphetamines, cocaine metabolites, methadone, propoxyphene, phencyclidine, benzodiazepines, and cannabinoids in the specimen of urine collected at screening; known sensitivity to oxycodone, naloxone, or related compounds etc.

If pharmacokinetic parameters such as mean $t_{max}$, $c_{max}$ and AUC are obtained in patients, the patient group will comprise between 10 to 200 patients. A reasonable number of patients will e.g. be 10, 20, 30, 40, 50, 75, 100, 125 or 150 patients. Patients will be selected according to symptoms of the condition to be treated. For the purposes of the present invention, patients may be selected according to the inclusion and exclusion criteria of Example 1. Thus patients will be $\geq$ 18 years, suffer from severe chronic pain of tumor and non- tumor origin, will show insufficient efficacy and/or tolerability with a WHO II or II analgesic etc. A patient will not be considered for determination of pharmacokinetic parameters if there indications of current alcohol or drug abuse, of current severe cardiovascular and respiratory diseases, of sever liver and renal insufficiency etc.

It is to be understood that values of pharmacokinetic parameters as indicated above and below have been deduced on the basis of the data which were obtained in experiments 2, 3, 4 and 6, all of which relate to single dose studies in healthy human subjects. However, it is assumed that comparable results will be obtained upon steady state administration in healthy human subject or single dose and steady state administration in human patients. The same applies *mutatis mutandis* for parameters such as analgetic efficacy, tolerability, intake of laxatives, occurrence of adverse events etc. which are determined in example 1 by testing preparations in accordance with the invention in patients during steady state.

Pharmacokinetic parameter calculations may be performed with WinNonlin Enterprise Edition, Version 4.1.

The term "bioavailability" is defined for purposes of the present invention as the extent to which active agents such as oxycodone and naloxone are absorbed from the unit dosage forms.

The term "sustained release" is defined for purposes of the present invention as the release of oxycodone and/or naloxone at such a rate that blood levels are maintained within the therapeutic range but below toxic levels over a period of time of about 8 hours or about 12 hours or about 24 hours or even longer. The term "sustained release" differentiates the preparations in accordance with the invention from "immediate release" preparations.

The phrase "(initial) rapid rate of rise" with regard to oxycodone blood plasma concentration is defined for purposes of the present invention as signifying that the minimum effective analgesic concentration is quickly approached in patients who have measurable if not significant pain at the time of dosing. In particular, this might be achieved by administering a dosage form according the present invention which provides a $t_{max}$ of up to 17 hours, preferably of up to 10 hours, more preferably of up 6 hours or even less, e.g. up to 5 hours or up to 4 hours or up to 3 hours.

The term $T_{1/2}$ is defined for purposes of the present invention as the amount of time necessary for one half of the absorbable dose of oxycodone and/or naloxone to be transferred to plasma. This value may be calculated as a "true" value (which would take into account the effect of elimination processes), rather than an "apparent" absorption half-life.

The term "steady state" means that a plasma level for a given drug has been achieved and which is maintained with subsequent doses of the drug at a level which is at or above the minimum effective therapeutic level and is below the minimum toxic plasma level for oxycodone. For opioid analgesics such as oxycodone, the minimum effective therapeutic level will be partially determined by the amount of pain relief achieved in a given patient. It will be well understood by those skilled in the medical art that pain measurement is highly subjective and great individual variations may occur among patients. It is clear that after the administration of each dose the concentration passes through a maximum and then again drops to a minimum.

The steady state may be described as follows: At the time t = 0, the time the first dose is administered, the concentration C is also 0. The concentration then passes through a first maximum and then drops to a first minimum. Before the concentration drops to 0, another dose is administered, so that the second increase in concentration doesn't start at 0. Building on this first concentration minimum, the curve passes through a second maximum after the second dose has been administered, which is above the first maximum, and drops to a second minimum, which is above the first minimum. Thus, the blood plasma curve escalates due to the repeated doses and the associated step-by-step accumulation of active agent, until it levels off to a point where absorption and elimination are in balance. This state, at which absorption and elimination are in equilibrium and the concentration oscillates constantly between a defined minimum and a defined maximum, is called steady state.

The terms "maintenance therapy" and "chronic therapy" are defined for purposes of the present invention as the drug therapy administered to a patient after a patient is titrated with an opioid analgesic to a steady state as define above.

In the context of the present invention, "agonist" or "analgesic" always refers to oxycodone and "antagonist" always refers to naloxone. Active compounds according to the present invention are oxycodone and naloxone or pharmaceutically acceptable salts thereof. Unless expressly indicated otherwise, amounts and ratios of the active compounds as described herein refer to the form actually used, i.e. the free base or a pharmaceutically acceptable salt thereof. Further, unless expressly indicated otherwise, amounts and ratios of the active compounds as described herein refer to the anhydrous form of the compound.

In one aspect not according to the invention, the present disclosure provides a dosage form comprising oxycodone and naloxone which provides a mean $t_{max}$ for oxycodone at about 1 to about 17 hours, at about 2 to about 2 to about 15 hours, at about 3 to about 8 hours or at about 4 to about 5 hours after administration of a single dose or at steady state to healthy human subjects or patients. Mean $t_{max}$ values of oxycodone of about 6, about 7, about 9, about 10, about 11, about 12, about 13, about 15, about 16 hours or more are also preferred. In a preferred embodiment such dosage forms comprise oxycodone and naloxone in a 2:1 weight ratio. These preparations are preferably administered at a total amount of 80 mg oxycodone and 40 mg naloxone per day. It is particularly preferred to administer such 2:1 preparations at an amount of 40 mg oxycodone and 20 mg naloxone per day. The dosage form preferably releases the active agents in a sustained, invariant and independent manner from a substantially non-swellable diffusion matrix that, with respect to its release characteristics is formed from an ethyl cellulose and at least one fatty alcohol.

The oxycodone naloxone dosage forms for use according to the present invention provide an improvement of the bowel function during pain therapy, wherein the dosage form is the dosage form as defined as in claim 1. In the context of the present invention, an improvement of bowel function during pain therapy means that bowel function is improved compared to the administration of oxycodone alone, e.g. in combination with naloxone placebo.

Bowel function is usually assessed by observing parameters which are associated with bowel function. In particular, bowel function may be determined based on parameters selected from ease or difficulty of defecation, feeling of incomplete bowel evacuation, and/or personal judgment of patient regarding constipation. Other parameters which may be observed alternatively or in addition in order to assess the bowel function of a patient include among other things stool frequency, stool consistency, cramping, and painful laxation.

It is preferred to determine bowel function by measuring parameters which are associated with bowel function using numerical analog scales (NAS) for these parameters since this may provide more accurate results. This is particularly advantageous when assessing the bowel function in patients receiving treatment with analgesics, since analgesic efficacy of drugs is usually assessed using a numeric analog scale. Hence, patients receiving treatment with analgesics are used to handle numerical analog scales which provides for obtaining meaningful results.

In a preferred embodiment, the oxycodone/naloxone dosage forms for use according to the present invention as defined in claim 1 provide an improvement of the bowel function characterized by an improvement of the mean bowel function score of at least 5, at least about 8, at least about 10 or at least about 15 after administration at steady state or of a single dose to human patients or healthy human subjects, wherein the mean bowel function score is measured with a numerical analog scale ranging from 0 to 100. Such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises 40 mg oxycodone and 20 mg naloxone. The dosage form may release the active agents in a sustained, invariant and independent manner from a substantially non-swellable diffusion matrix that, with respect to its release characteristics is formed from an ethyl cellulose and at least one fatty alcohol.

According to the invention the bowel function can be assessed by the bowel function index (BFI) which is measured preferably in patients. In this context the inclusion and exclusions criteria of example 1 can be applied for selecting patients. Similarly, the BFI can be measured using a comparable patient number as in example 1.

The terms BFI and BFI3 are used interchangeably for the purposes of the present invention.

The mean bowel function score is in particular determined by a method for assessing bowel function in a patient which comprises the following steps:

- providing the patient with a numeric analog scale for at least one parameter, which parameter is associated with bowel function;
- causing the patient to indicate on the numeric analog scale the amount and/or intensity of the parameter being experienced; and
- observing the amount and/or intensity of the at least one parameter indicated on the numeric analog scale in order to assess bowel function.

The patient usually indicates the amount and/or intensity of parameter being experienced during the last days or weeks, e.g. during the last 1, 2, 3, 4, 5, 6, 7, 10 or 14 days.

The numerical analog scale on which the patient indicates his/her subjective experience of the observed parameter may have any size or form and may range from 0 or any other number to any number, such as from 0 to 10 or from 0 to 50 or from 0 to 300 or from 1 to 10.

If more than one parameter is observed, a mean bowel function may be obtained in form of a numerical value which is the mean of the parameters observed, e.g. the three numeric analog scale values for ease or difficulty of defecation, feeling of incomplete bowel evacuation and judgment of constipation. The mean bowel function is also designated as mean bowel function score, bowel function index or BFI3 (if three parameters are observed).

Parameters which are measures of bowel function or which are associated with bowel function may comprise opioid bowel dysfunctions (OBD) syndromes. OBD is an often severe adverse drug reaction related to strong opioid analgesic therapy such as oxycodone that limits the continuous treatment of pain patients. OBD is primarily associated with constipation but also with abdominal cramping, bloating and gastroesophageal reflux.

In particular, bowel function may be determined based on the following three parameters:

- ease or difficulty of defecation, for example during the last 7 days according to the patient assessment, wherein 0 corresponds to no difficulties and 100 corresponds to severe difficulties;
- feeling of incomplete bowel evacuation, for example during the last 7 days according to the patient assessment, wherein 0 corresponds to no feeling of incomplete bowel evacuation and 100 corresponds to very strong feeling of incomplete bowel evacuation;
- personal judgment of patient regarding constipation, for example during the last 7 days, wherein 0 corresponds to no constipation at all and 100 corresponds to very heavy constipation.

Mean bowel function may be obtained in form of a numerical value which is the mean of the parameters observed, e.g. the three numeric analog scale values for ease or difficulty of defecation, feeling of incomplete bowel evacuation and judgment of constipation.

In particular, the method for assessing bowel function is performed by using devices or analog scales as described in the following.

In one embodiment, the parameter scale or numeric analog scale presented to the patient may be an uninterrupted line that bears no indicators or markings other than at the ends indicating no experience or very strong experience of the parameter to be observed. The patient is then caused to indicate the amount and/or intensity of the parameter experienced by making a dash on the uninterrupted line. Then, the health care provider or medical practitioner may measure the distance from the dash to the end indicating no experience or to the end indicating very strong experience, and divide this measure by the distance between both ends. The result is a numerical value which is a score for the bowel function. If more than one parameter is observed a mean bowel function score is usually determined by averaging the numeric analog scale values for each parameter. If three parameters are observed this mean bowel function score is also designated as Bowel Function Index or BFI3. Rome II-criteria can be detected by this scale.

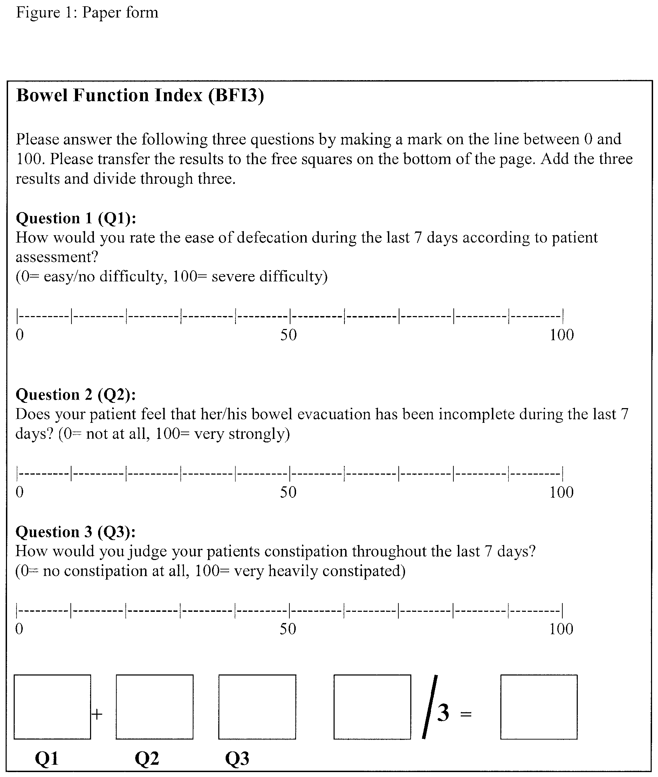

In a further embodiment, Fig. 1 illustrates an example for a paper form which can be used for assessing the bowel function index or mean bowel function score. In particular, the patient or the medical practitioner responsible for this patient may be asked to answer questions rendered on the paper form which concern parameters associated with bowel function such as the ease or difficulty of defecation, for example during the last 1, 3, 7 or 14 days; the feeling of incomplete bowel evacuation, for example during the last 1, 3, 7 or 14 days; and a personal judgment of the patient regarding constipation, again for example during the last 1, 3, 7 or 14 days. In this embodiment, the questions are answered by making a mark on a line between 0 and 100, wherein 0 corresponds to no difficulties and 100 corresponds to severe difficulties of defecation and/or wherein 0 corresponds to no feeling of incomplete bowel evacuation at all and 100 corresponds to very strong feeling of incomplete bowel evacuation and/or wherein 0 corresponds to no constipation at all and 100 corresponds to very heavy constipation. Of course, the scale may range from 0 or any other number to any number, such as from 0 to 10 or 0 to 50 or 0 to 300 or 1 to 10. The three numerical values which, for example, may be obtained by measuring the distance from the mark to the end indicating no experience or to the end indicating very strong experience, and dividing this measure by the distance between both ends, are then preferably added and divided by three in order to obtain the mean bowel function score or mean bowel function index (BFI) or BFI3.

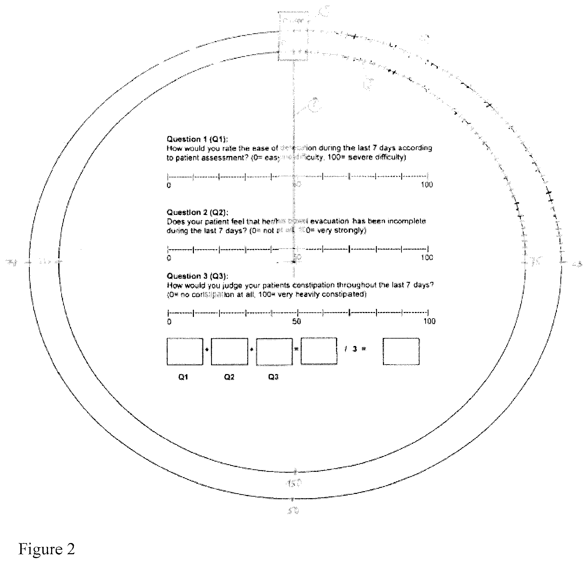

In a further embodiment, Fig. 2 illustrates an example of a circular BFI meter for determining the mean bowel function score. Preferably, a circular BFI meter contains a paper form with questions concerning the patient's assessment on one or more parameters which are associated with bowel function as described above. Further, such a circular BFI meter preferably contains a numerical scale on an inner circle and a numerical scale on an outer scale. The numerical scales are preferably correlated with each other such that a value on one scale is a multiple of the corresponding value on the other scale wherein the factor corresponds to the number of parameters which are observed. For example, if three parameters are observed, a value on one scale shows the corresponding value on the other scale divided or multiplied by three. Moreover, a BFI meter contains a needle or pointer which is attached to the middle of the circle and can be moved around the circle in order to facilitate the correlation of the corresponding values on the numerical scales on the inner and outer circle.

For example, three questions concerning the ease or difficulty of defecation, for example during the last 7 days, wherein 0 corresponds to no difficulties and 100 corresponds to severe difficulties; the feeling of incomplete bowel evacuation, for example during the last 7 days according to the patient assessment, wherein 0 corresponds to not at all and 100 corresponds to very strong; and a personal judgment of the patient regarding constipation, in order to obtain the BFI 3 are given on the inner field of a circle of the BFI meter. On the inner circle (3), a scale going clockwise from 0-300 is arranged. On the outer circle (4), a scale going clockwise from 0-100 is arranged which is in line with the marks of the scale of the inner circle and shows the value of the inner circle divided by 3. To facilitate the calculation, a needle or pointer (1) is attached to the middle of the circle which can be moved around the circle. At the outer end of the needle there is a window (2) which frames the numbers of the inner and outer circle. In order to assess the mean bowel function the needle may be moved to the number in the inner circle which is the result of question 1. Then, the result of question 2 may be added by moving the needle to that point of the inner circle. In a third step, the result of question 3 is added by moving the needle to the resulting point of the inner circle. As a result, the mean bowel function score can be seen on the outer circle.

In other preferred embodiments, the method according to the present invention may be performed with analogs scales as described in US 6,258,042 B1 and WO 03/073937 A1 which have to be adapted to devices or analog scales as described above.

Preferably, the oxycodone naloxone dosage forms for use according to the present invention provide an analgesic effect for at least 8 hours, more preferably for at least 12 hours, or most preferably for at least about 24 hours after administration at steady state or of a single dose to human patients.

Preferably, the oxycodone naloxone dosage forms for use according to the present invention provide a mean $t_{max}$ for oxycodone at about 1 to about 17 hours, at about 2 to about 15 hours, at about 3 to about 8 hours or at about 4 to about 5 hours after administration at steady state or of a single dose to human patients or healthy human subjects. In one preferred embodiment the dosage form provides a mean $t_{max}$ of 3 hours, 3.5 hours or 4.0 hours for oxycodone after administration at steady state or of a single dose to human healthy subjects or human patients. Such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide a mean $t_{max}$ for naloxone-3-glucuronide at about 0.25 to about 15 hours, at about 0.5 to about 12 hours, at about 1 to about 4 hours or at about 1 to about 3 hours after administration at steady state or of a single dose to human patients or healthy human subjects. In one preferred embodiment the dosage form provides a mean $t_{max}$ of 0.5 hour, 1 hour or 2.0 hours for naloxone-3-glucuronide after administration at steady state or of a single dose to human healthy subjects or human patients. Such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide an mean AUCt value for oxycodone of about 100 ng·h/mL or about 200 ng·h/mL or about 300 ng·h/mL to about 600 ng·h/mL, more preferably about 400 ng·h/mL to about 550 ng·h/mL and most preferably from about 450 ng·h/mL to about 510 ng·h/mL. Preferably, these mean AUCt values for oxycodone refer to an oxycodone naloxone dosage forms according to the present invention which comprise 40 mg oxycodone or a pharmaceutically acceptable salt thereof and, e.g., 20 mg naloxone or a pharmaceutically acceptable salt thereof. The above values relate to single dose administration or steady state administration in healthy human subjects or patients. Such dosage forms comprise oxycodone and naloxone in a 2:1 weight ratio. The dosage form preferably releases the active agents in a sustained, invariant and independent manner from a substantially non-swellable diffusion matrix that, with respect to its release characteristics is formed from an ethyl cellulose and at least one fatty alcohol.

For oxycodone naloxone dosage forms for use according to the present invention comprising less than 40 mg oxycodone or a pharmaceutically acceptable salt thereof, the mean AUCt values for oxycodone may be lower such as 50 ng·h/mL or 75 ng·h/mL. This may be the case if 20 mg of oxycodone and 10 mg of naloxone or 10 mg of oxycodone and 5 mg of naloxone are administered (see e.g. examples 3 and 4). These values relate again to single dose administration or steady state administration in healthy human subjects or patients.

Preferably, the oxycodone naloxone dosage forms for use according to the present invention provide an mean AUCt/mg oxycodone value for oxycodone of about 10 ng·h/mL mg to about 15 ng·h/mL mg, preferably about 10 ng·h/mL mg to about 14 ng·h/mL mg and most preferably from about 11.2 ng·h/mL mg to about 14 ng·h/mL. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide an mean AUCt value for naloxone-3-glucuronide of about 100 ng·h/mL or about 200 ng h/mL or about 300 ng h/mL to about 750 ng·h/mL, more preferably about 400 ng h/mL to about 700 ng·h/mL and most preferably from about 500 ng·h/mL to about 600 ng·h/mL. Preferably, these mean AUCt values for naloxone-3-glucuronide refer to an oxycodone naloxone dosage form for use according to the present invention which comprises 40 mg oxycodone or a pharmaceutically acceptable salt thereof and, e.g., 20 mg naloxone or a pharmaceutically acceptable salt thereof. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol. Preferably, the oxycodone naloxone dosage forms for use according to the present invention provide a mean AUCt/mg naloxone value for naloxone-3-glucuronide of about 20 ng·h/mL mg to about 35 ng·h/mL mg, preferably about 25 ng·h/mL mg to about 30 ng·h/mL mg. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone.The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide a mean $C_{max}$ value for oxycodone of about 5 ng/mL to about 50 ng/mL, more preferably of about 20 ng/mL to 40 ng/mL or most preferably of about 30 ng/mL of about 35 ng/mL. Preferably, these mean $C_{max}$ values for oxycodone refer to an oxycodone naloxone dosage forms for use according to the present invention which comprise 40 mg oxycodone or a pharmaceutically acceptable salt thereof and, e.g., 20 mg naloxone or a pharmaceutically acceptable salt thereof. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

For oxycodone naloxone dosage forms for use according to the present invention comprising less than 40 mg oxycodone or a pharmaceutically acceptable salt thereof, the mean $C_{max}$ values for oxycodone may be lower such as 1 ng/mL or 3 ng/mL. This may be the case if 20 mg of oxycodone and 10 mg of naloxone or 10 mg of oxycodone and 5 mg of naloxone are administered (see e.g. examples 3 and 4).

Preferably, the oxycodone naloxone dosage forms for use according to the present invention provide a mean $C_{max}$ value for oxycodone of about 0.125 ng/mL mg oxycodone to about 1.25 ng/mL mg oxycodone, more preferably of about 0.5 ng/mL mg oxycodone to 1 ng/mL mg oxycodone or most preferably of about 0.75 ng/mL mg oxycodone to about 0.875 ng/mL mg oxycodone. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone.The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide a mean $C_{max}$ value for naloxone-3-glucuronide of about 10 pg/mL to about 100 pg/mL, more preferably of about 40 pg/mL to 90 pg/mL or most preferably of about 60 pg/mL of about 90 pg/mL. Preferably, these mean $C_{max}$ values for oxycodone refer to an oxycodone naloxone dosage forms for use according to the present invention which comprise 40 mg oxycodone or a pharmaceutically acceptable salt thereof and, e.g., 20 mg naloxone or a pharmaceutically acceptable salt thereof. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone.The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide a mean $C_{max}$ value for naloxone-3-glucuronide of about 2 pg/mL mg naloxone to about 4.5 pg/mL mg naloxone, more preferably of about 3 pg/mL mg naloxone to 4.5 pg/mL mg naloxone. The above values relate to single dose administration or steady state administration in healthy human subjects or patients. Such dosage forms comprise oxycodone and naloxone in a 2:1 weight ratio. Preferably the dosage form comprises 40 mg oxycodone and 20 mg naloxone.The dosage form preferably releases the active agents in a sustained, invariant and independent manner from a substantially non-swellable diffusion matrix that, with respect to its release characteristics is formed from an ethyl cellulose and at least one fatty alcohol.

The oxycodone naloxone formulations for use according to the present invention, which provide an initial rapid rate of rise in the plasma concentration and/or have a $t_{max}$ value e.g. of up to 8 hours, preferably up to 6 hours or up to 5 hours or even up to 4 hours, are advantageous in that a fast and greater analgesic efficacy is achieved. No substantially flat serum concentration curve is exhibited, but instead a more rapid initial opioid release is provided, so that the minimum effective analgesic concentration can be more quickly attained in many patients. This makes the dosage forms for use according to the present invention also suitable for titrating patients by avoiding the necessity of first titrating on an immediate release oxycodone naloxone dosage form before switching him to a sustained release dosage form for chronic therapy. The above $t_{max}$ values relate to single dose administration or steady state administration in healthy human subjects or patients. Such dosage forms comprise oxycodone and naloxone in a 2:1 weight ratio. These preparations are preferably administered up a total amount of 80 mg oxycodone and 40 mg naloxone per day. It is particularly preferred to administer such 2:1 preparations up to an amount of 40 mg oxycodone and 20 mg naloxone per day. Preferably the dosage form comprises 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention provide an efficacy and tolerability that is judged by patients as equally good as the efficacy and tolerability of preparations that comprise the same amount of oxycodone, but no naloxone.

A global assessment of efficacy can be measured in patients measured using a 0 to 7 numerical analogue scale (1 = very good, 2 = good, 3 = pretty good, 4 = moderate, 5 = slightly poor, 6 = poor, 7 = very poor). Tolerability can be measured in patients using the same 0 to 7 numerical analogue scale. Another parameter that can be considered is preference for maintenance (oxycodone/naloxone combination) or titration/run-in (oxycodone only) regarding efficacy/tolerability of study medication using a 0 to 3 NAS (1 = titration/run-in, 2 = maintenance, 3 = no preference).

For the global assessment of efficacy, tolerability and preference summary statistics can then be performed for the groupings dose ratio of oxycodone and naloxone, absolute dose of naloxone and absolute dose of naloxone given the same oxycodone/naloxone ratio.

In one embodiment not according to the invention, the present disclosure provides dosage forms of oxycodone and naloxone that in terms of efficacy are ranked good or very good by more than 50% of patients and preferably by more than 70% of patients if the above mentioned NAS is used.

Additionally or alternatively dosage forms not according to the invention comprise oxycodone and naloxone and in terms of tolerability are ranked good or very good by more than 60% of patients and preferably by more than 70 or even 80% of patients if the above mentioned NAS is used. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Preferably, the oxycodone naloxone dosage forms for use according to the present invention allow a reduction as regards the dose and frequency of laxative intake compared to a preparation that comprises only oxycodone but not naloxone.

OBD symptoms such as constipation are typical side effects of opioid administration and typically treated by administering laxatives. However, it is not known whether distinct opioid agonist to antagonist ratios exist that ensure not only efficacy and tolerability, but allow also to prevent or at least reduce at the same time OBD symptoms such constipation.

Laxative intake/mean laxative dose development can be calculated in accordance with the invention from the patients' reports. In one embodiment of the invention, an analysis of the mean laxative dose and/or laxation events during the last seven days is performed for patients. In this context laxatives can be identified by the WHO ATC Code A06A. For laxative intake, number of days with laxation during the last 7 days and the percentage of days with laxation during the last 7 days can be calculated for each study visit. In addition, the percentage of days with laxation during the whole maintenance phase and during the follow-up phase can be calculated. An example of determining the need for laxative intake and the influence of the preparations in accordance with the invention is provided by example 1.

One embodiment not according to the invention provides dosage forms of oxycodone and naloxone that provide a reduction of days with laxative intake by at least 10%, preferably by at least 20%, more preferably by at least 25% and even more preferably by at least 30%. Some dosage forms of the present disclosure even allow a reduction of at least 35% or at least 40%. The same should apply also for the dose of laxative intake. In a preferred embodiment such dosage forms comprise oxycodone and naloxone in a 2:1 weight ratio. These preparations are preferably administered at up to total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Yet another embodiment of the present invention relates to oxycodone naloxone dosage forms preparations for the claimed uses that preferably do not induce substantial withdrawal symptoms in patients or healthy human subjects, i.e. groups of opioid users that must not be confused with opioid addicts and drug abusers.

One of the rationales for using naloxone in combination with oxycodone is to deter abuse of the inventive preparations by these opioid dependent individuals or drug abusers. However, withdrawal symptoms should not occur when preparations comprising opioid agonists and antagonists are administered to patients in need of pain therapy. The present invention shows that surprisingly preparations of oxycodone and naloxone with distinct ratios exist that ensure analgetic efficacy, that are very well liked by the patients, that allow to specifically treat side effects such as constipation and laxative intake and that at the same time do not lead to significant withdrawal symptoms.

Subject symptoms of withdrawal (SOWS) in accordance with the invention can be recorded daily by the patient in a diary and can include parameters such as: I am anxious; I have to yawn; I am sweating; My eyes are watering; My nose is running, I have gooseflash; I am shivering; I feel hot; I feel cold; My bones and muscles are aching; I am restless; I feel sick; I have to vomit; My muscles are twitching; I have abdominal cramps; I cannot sit still. These symptoms can be rated by a NAS such as "0 = not at all", "1 = little", "2 = medium", "3 = strong" or "4 = extreme"

In one embodiment SOWS are recorded during the first 7 days of a maintenance phase. The total score (= sum score) of the SOWS items can then be calculated for each patient and day.

In one embodiment not according to the invention, the present disclosure provides sustained release dosage forms of oxycodone and naloxone that do not lead to substantial increases in sum scores of SOWS in a clinically relevant extent and that therefore do not pose safety concerns in patients or healthy human subjects. In a preferred embodiment such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Yet another embodiment not according to the invention relates to oxycodone naloxone dosage forms preparations that preferably, or alternatively, are clinically acceptable in terms of occurrence adverse events such as e.g. diarrhea.

For the purposes of the present invention, an adverse event can be considered as any untoward medical occurrence in a patient or clinical investigation subject administered a pharmaceutical product, including placebo, and which does not necessarily have a causal relationship with treatment. The way that adverse events such as diarrhoea are classified, measured and evaluated is described in detail in example 1 which in this context is not to be construed as limited to the specific preparation tested.

Elicited opioid-typical adverse events are considered to be nausea, emesis, sedation, skin reactions, as identified in the Medical Dictionary for Regulatory Affairs (MeDRA). Elicited naloxone-typical adverse events are considered to be abdominal pain, cramping and diarrhea with the definitions applied as laid out in MeDRA

Severity of such adverse effects can be measured by a sum score which can be calculated by assigning scores each of the above-mentioned adverse events that occure during e.g. the last 7 days. A score of 0 is assigned, if the respective side-effect is not observed during the last 7 days, a score of 1, if the adverse event is mild, a score of 2, if the adverse event is moderate, and a score of 3, if the adverse event is severe. This means that elicited opioid typical adverse events would have a maximum sum score of 12 while elicited naloxone typical adverse would leas to a maximum sum score of 9

It was surpisingly found that the preparations for the claimed use according to the invention during a maintenance phase, i.e. during steady state provide reduced severity of elicited opioid typical adverse events compared to an oxycodone only treatment while severity of elicited naloxone typical adverse events does not substantially increase, i.e.it is the same or reduced compared to an oxycodone only treatment.

An embodiment not according to the invention therefore relates to dosage forms comprising oxycodone and naloxone which provide an improved side effect profile, i.e. during steady state administration, lead to a reduction of severity of elicited opioid typical adverse events without increasing the severity of elicited naloxone typical adverse events as measured by calculating sum scores in comparison to administration of an oxycodone only dosage form.

In a preferred embodiment of the afore-mentioned paragraph such dosage forms comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

Yet another embodiment not according to the invention relates to oxycodone naloxone dosage forms preparations that preferably, or alternatively, show no substantial food effect.

A food effect is determined by measuring pharmacokinetic parameters such as AUC, $c_{max}$ and $t_{max}$ which are determined in healthy human subjects or patients after single dose or steady state administration. It has been observed that the dosage forms for use in the present invention do not lead to increased pharmacokinetic parameters of naloxone. This is important as it shows that food will not have a detrimental effect on the analgetic efficacy of the inventive preparations.

A food effect will be observed if the pharmacokinetic parameters after a FDA high fat meal will be substantially, i.e. to a clinically relevant extent, outside the 90% confidence limits of bioequivalence for AUC, $c_{max}$ and $t_{max}$ One way of determining a food effect is described in experiment 3 which in this context is not to be construed as limited to the specific preparation tested.

In an embodiment not according to the invention dosage forms showing no substantial food effect comprise oxycodone and naloxone in a 2:1 weight ratio. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone. The dosage form preferably releases the active agents in a sustained, invariant and independent manner from a substantially non-swellable diffusion matrix that, with respect to its release characteristics is formed from an ethyl cellulose and at least one fatty alcohol.

An embodiment not according to the invention relates to oxycodone naloxone dosage forms preparations that precipitate withdrawal symptoms in opioid dependent humans. In a preferred embodiment the precipitation of withdrawal effects is more pronounced and longer lasting for the inventive dosage forms than for naloxone, as would be expected. Such dosage forms are particularly suitable to prevent abuse of the dosage forms by e.g. intravenous application or administration via the nasal route.

It is highly desirable to have a preparation of an opioid agonist and antagonist that would provide the above characteristics, i.e. good analgetic efficacy, good tolerability, improvement in BFI, reduction in laxative intake, no withdrawal symptoms in patients, no food effect but at the same time would induce withdrawal symptoms in opioid dependent individuals such as drug addicts. Experiment 5 shows that an i.v. administration of a 2:1 ratio of oxycodone: naloxone precipitates withdrawal symptoms in oxycodone dependent rats. Given the advantages of the 2:1 ratio with respect to the above-described parameters it is assumed that in view of the data of example 5, preparations in accordance with the invention will also precipitate withdrawal symptoms in opioid dependent human individuals. A surprising feature of the 2:1 ratio is that withdrawal symptoms are actually prolongued and more pronounced for the combination product despite the presence of oxycodone.

In an embodiment not according to the invention dosage forms having the capacity of precipitating and prolonging withdrawal effects in opioid-dependent humans comprise oxycodone and naloxone in a 2:1 weight ratio. Preferably these dosage forms can even prolong the precipitated withdrawal effects leading to long lasting withdrawal symptoms in addicts. These preparations are preferably administered up to a total amount of 80 mg oxycodone and 40 mg naloxone per day. It is particularly preferred to administer such 2:1 preparations up to an amount of 40 mg oxycodone and 20 mg naloxone per day. Preferably the dosage form comprises approximately 80 mg of oxycodone and 40 mg of naloxone and more preferably about 40 mg oxycodone and 20 mg naloxone.The dosage form preferably releases the active agents in a sustained, invariant and independent manner from a substantially non-swellable diffusion matrix that, with respect to its release characteristics is formed from an ethyl cellulose and at least one fatty alcohol.

A further aspect not according to the invention relates to the use of preparations in accordance with the disclosure for human individuals and particularly patients which typically would not be treated with higher amounts of oxycodone. For example, the 80 mg and 160 mg dosage strengths of OxyContin are not indicated for treatment of opioid naive patients as breath depression may occur. Similarly, physicians are very reluctant to treat elderly patients with the aforementioned high amounts of oxycodone. However, the preparations of the present disclosure can be used for treatment of opioid naive individuals and/or elderly patients in amounts of 80 mg and up to 160 mg oxycodone if naloxone is present. This particularly applies for the oxycodone: naloxone 2:1 ratio. Thus, not according to the invention to treat moderate to severe pain in patient groups which so far could not be treated with comparatively large dosage amounts of oxycodone are also provided.

A large dosage amount of oxycodone is considered to be more than 80 mg, preferably more than 100 mg, more preferably more than 120 mg, even more preferably more than 140 mg of oxycodone and most preferably more than 160 mg of oxycodone. This is possible because naloxone is present, preferably in an oxycodone:naloxone ratio of 2:1.

An embodiment not according to the invention relates to the use of dosage forms comprising oxycodone and naloxone for providing an improved side effect profile, i.e. for providing during steady state administration, a reduction of severity of elicited opioid typical adverse events without increasing the severity of elicited naloxone typical adverse events.

As has been already mentioned above, it has been surprisingly found that sustained release preparations of oxycodone and naloxone can be obtained that allow for (1) efficient and long lasting pain treatment, i.e. up to 24 hours, (2) show improvements in bowel function, (3) show excellent tolerability, (4) do not show significantly elevated sum scores for opioid withdrawal symptoms in patients and healthy human subjects, (5) allow for reduction of laxative intake, (6) are clinically acceptable in terms of adverse events such as diarrhea, (7) do not show a food effect and (8) are likely to precipitate withdrawal symptoms in opioid addicted individuals. Experiments 1 to 6 clearly show that particularly oxycodone naloxone preparations with a oxycodone: naloxone ratio of 2:1 are suited for these different purposes. The experiments also clearly establish that the 2:1 ratio of oxycodone to naloxone is particularly suitable for achieving the above objections if preferably 80 mg of oxycodone and 40 mg of naloxone are administered per day. In an especially preferred embodiment the 2:1 ratio dosage forms are administered at a daily dose of 40 mg oxycodone and 20 mg naloxone. This ratio seems to be the optimum for achieving the above-described effects in combination. In a further preferred embodiment the preparations for use according to the invention may comprise 40 mg oxycodone or an equivalent amount of a pharmaceutically acceptable salt and 20 mg naloxone or an equivalent amount of a pharmaceutically acceptable salt. Such preparations will preferably comprise the active ingredients embedded in a substantially non-swellable and non-erosive diffusion matrix that is formed with respect to its essential release characteristics by ethyl cellulose and at least one fatty alcohol

Further, there is no significantly greater incidence of side effects such as constipation which would normally be expected as higher peak plasma concentrations as a result of an initial rapid rate of rise in the plasma concentration occur.

Further, particularly if the dosage form for use according to the present invention is a matrix formulation, it is ensured that the agonist, i.e. oxycodone, as well as the antagonist, i.e. naloxone, are always released in predetermined percentages and that their release rates do not influence each other. Thereby, abuse of the medicament, which presupposes that oxycodone can selectively be extracted from the formulation, is prevented. The formulation for use according to the present invention therefore disables selective extraction of oxycodone from the dosage form without the corresponding amount of the antagonist naloxone, regardless of the absolute and relative amounts of agonist and antagonist chosen.

Hence, the dosage forms for use according to the present invention are also suitable for a method for titrating human patients with a sustained release oxycodone naloxone formulation. The first step of this embodiment not according to the invention comprises administering to a human patient, e.g. on a twice-a-day or once-a-day basis, a unit dose of the sustained release oxycodone/naloxone dosage forms as described above and in the following paragraphs. Thereafter, this embodiment includes the further step of monitoring pharmacokinetic and pharmacodynamic parameters elicited by said formulation in said human patient and determining whether said pharmacokinetic and/or pharmacodynamic parameters are appropriate to treat said patient on a repeated basis. The patient is titrated by adjusting the dose of oxycodone and/or naloxone administered to the patient by administering a unit dose of the dosage forms according to the present invention containing a different amount of oxycodone and/or naloxone if it is determined that said pharmacokinetic and/or said pharmacodynamic parameters are not satisfactory or maintaining the dose of oxycodone and/or naloxone in the unit dose at a previously administered amount if said pharmacokinetic and/or pharmacodynamic parameters are deemed appropriate. The titration is continued by further adjusting the dose of oxycodone and/or naloxone until appropriate steady-state pharmacokinetic/pharmacodynamic parameters are achieved in the patient. Thereafter, the administration of the dose of oxycodone and/or naloxone in the sustained release dosage form according to the present invention is continued, e.g. on a twice-a-day or once-a-day basis, until treatment is terminated.

Oxycodone and naloxone are released from the dosage forms for use according to the present invention in a sustained manner and optionally additionally in an invariant and/or independent manner.

This embodiment ensures that, given identical relative amounts, the active compounds show equal release profiles, independent of the absolute amount present. Such an independent release behavior provides a wide range of useable absolute amounts of the analgesic active substance to the physician, given that the optimal agonist/antagonist ratio is known. Thus, it is possible to comfortably adjust the dosage for each individual patient, either by a step-wise dosage increase or, if necessary, a step-wise dosage reduction. This ability to adjust the dosage for the individual patient is extremely useful from a medical point of view.

The sustained, invariant and/or independent release of the active compounds, i.e. oxycodone and naloxone or pharmaceutically acceptable salts thereof, ensures additionally that pharmaceutical preparations for use according to the invention are characterized by a low administration frequency, so that high patient compliance is achieved. Furthermore, preparations for use according to the invention allow the physician to adjust the dosage for individual patients. They enable use over a broad range with respect to the useable absolute amounts of the active compounds and ensure that the active compounds, even after long-term storage, become effective with equal release profiles.

According to the present invention, sustained release of oxycodone or a pharmaceutically acceptable salt thereof and naloxone or a pharmaceutically acceptable salt thereof means that pharmaceutically active substances are released from the medicament over a longer period of time than they are known from formulations for immediate release. Typically, an immediate release preparation will have released substantially all the active ingredients within approximately 30 minutes if measured according to the USP paddle method.

In a specific embodiment of the present invention, the dosage form release is between 25% to 65%, preferably between 30% to 60%, more preferably between 35% to 55% and even more preferred between 40% to 50% of oxycodone or a pharmaceutically acceptable salt thereof and/or naloxone or a pharmaceutically acceptable salt thereof after 4 hours.

Other specific embodiments of the invention relate to dosage forms for the claimed uses that release between 70% to 100%, preferably between 75% to 100%, more preferably between 80% to 95% and even more preferably between 80% to 85%, between 85% to 90% or between 90% to 95% of oxycodone or a pharmaceutically acceptable salt thereof and/or naloxone or a pharmaceutically acceptable salt thereof after 8 hours. Preferred embodiments of the invention also relate to preparations for the claimed uses that release approximately 80%, approximately 85%, approximately 90% or approximately 95% of oxycodone or a pharmaceutically acceptable salt thereof and/or naloxone or a pharmaceutically acceptable salt thereof after 8 hours. According to the invention, dosage forms or formulations of medicaments that ensure such a sustained release of the active compounds from the preparation or dosage form are designated as retard formulations, sustained release formulations or prolonged release formulations. According to the invention, the release of the active compounds preferably occurs in a pH-independent manner.

According to the present invention, the term "substantially pH-independent" means that the difference, at any given time, between the amount of oxycodone released at pH 1.2 and the amount released at pH 6.8 (when measured in-vitro using USP Basket Method at 100 rpm in 900 ml aqueous buffer), is 20%, preferably 15 % and more preferably 10% (by weight based on the total amount of oxycodone or salt thereof in the dosage form) or less. The same applies *mutatis mutandis* for naloxone. A release value at a distinct time point is typically based on the average of five measurements

Further, according to the invention, the term "sustained release" refers to the release of active compounds from a medicament over an extended period of time. It does not imply the controlled release at a defined place; therefore, it does not mean that the active compounds are either released only in the stomach, or only in the intestine.

According to the invention, "independent release" means that, given the presence of at least two active compounds, a change of the absolute amount of one compound does not influence the release profiles of the other compounds so that the release profiles of the other compounds are not changed. For dosage forms or formulations for use according to the invention such an independent release behavior is independent of the pH value, for which the release is measured, or of the production process. The pH independency particularly applies to the acidic range, i.e. for pH values < 7. The release profile or release behavior is defined as the change of the release of the active compound from the formulation with time, with the amount of each active compound released provided in percents of the total amount of the active compound.

The release profile may be determined by known tests. Preferably, the release of the active compounds from a sustained release formulation is determined by the Basket Method according to USP at pH 1.2 or pH 6.5 with HPLC.

E.g., this means that the release profile of oxycodone observed for an oxycodone/naloxone-combination with 12 mg oxycodone and 4 mg naloxone does not differ from that of a corresponding preparation with the same formulation containing 12 mg oxycodone and 6 mg naloxone.

In particular, independent release is of interest if the release profile of preparations having substantially equal compositions is compared. Preparations of substantially equal composition have different amounts of the active compounds but are otherwise basically the same with respect the components of the composition which essentially influence the release behaviour.

E.g., if the above-mentioned preparations are compared (with the first preparation comprising 12 mg oxycodone and 4 mg naloxone and the second preparation comprising 12 mg oxycodone and 6 mg naloxone) both preparations, provided that they have the same total weight, will provide for the same release profile for oxycodone and naloxone if the difference in the naloxone amount is replaced by a component in the formulation that typically does not influence the release behaviour.

The person skilled in the art is well aware that if the amount of the active compound in which two dosage forms differ is replaced by a substance that is essential for the release behaviour of the formulation, such as ethyl cellulose or a fatty alcohol, differences in the release behaviour may occur. Thus, independent release is preferably provided by dosage forms that have different amounts of the active compounds but are otherwise identical or at least highly similar with respect to the components that essentially influence the release behaviour (given that formulations of the same total weight are compared).

According to the invention, "invariant release behavior" or "invariant release profile" is defined so that the percentage of the absolute amount of each active compound released per time unit does not significantly change and remains sufficiently constant if the absolute amounts of an active compound is altered. Sufficiently constant percentages mean that the percentage released per time unit deviates from a mean value by not more than 20%, preferably by not more than 15% and especially preferably by not more than 10%. The mean value may be calculated from six measurements of the release profile. Of course, the amount released per time unit has to satisfy the legal and regulatory requirements.

For example, this means that given an oxycodone/naloxone combination of 12 mg oxycodone and 4 mg naloxone, during the first 4 hours 25% oxycodone and 20% naloxone are released. If the oxycodone/naloxone combination contains 24 mg oxycodone and 8 mg naloxone, again 25% oxycodone and 20% naloxone will be released during the first 4 hours. In both cases the deviation will not be more than 20% from the mean value (which in this example is 25% oxycodone and 20% naloxone).

As outlined for the independent release behavior, invariant release is of particular interest if preparations of substantially equal composition are compared. Such preparation differ with respect to the amount of the active compounds, but are of the same or at least highly similar composition with respect to the release-influencing components of the preparation. Typically, the difference in the amount of an active compound will be replaced by the amount of a pharmaceutical inert excipient which does not substantially influence the release behavior of the preparation. Such a pharmaceutical excipient may be lactose, which is a typical filler in pharmaceutical preparations. The person skilled in the art is well aware that invariant release may not be provided by preparations in which the difference in the amount of an active compound is replaced by substances that are known to essentially influence the release behavior of the preparation, such as ethyl cellulose or fatty alcohols.

"Storage stable" or "storage stability" means that upon storage under standard conditions (at least two years at room temperature and usual humidity) the amounts of the active compounds of a medicament formulation do not deviate from the initial amounts by more than the values given in the specification or the guidelines of the common Pharmacopoeias.

Storage stability also means that a preparation produced according to the invention can be stored under standard conditions (60% relative humidity, 25°C) as it is required for admission to the market.

"Storage stable" or "time stable" also means that after storage under standard conditions the active compounds show release profiles as they would upon immediate use without storage. The admissible fluctuations with respect to the release profile are characterized in that the amount released per time unit fluctuates by no more than 20%, preferably no more than 15% and especially preferably no more than 10 %, with respect to a mean value. The mean value is calculated from six measurements of the release profile. Storage stability is preferably determined by the Paddle Method according to USP at pH 1.2 with HPLC.

A "non-swellable" or "substantially non-swellable" diffusion matrix is a matrix formulation for which the release of the active compounds is not influenced (or at least not to a relevant degree) by swelling of the matrix (particularly in the physiological fluids of the relevant target sites in the patient's body).

The term "substantially non-swellable" diffusion matrix also refers to a matrix whose volume will increase by approximately 300%, preferably by approximately 200%, more preferably by approximately 100%, by approximately 75% or by approximately 50%, even more preferably by approximately 30% or by approximately 20% and most preferably by approximately 15%, by approximately 10%, by approximately 5% or by approximately 1% in aqueous solutions (and particularly in the physiological fluids of the relevant target sites in the patient's body).

Preparations for use according to the invention are formulations for oral application.

In one embodiment, oxycodone and/or naloxone are present in the dosage form in form the free base.

In an alternative preferred embodiment, oxycodone and/or naloxone are present in the dosage form in form a pharmaceutically acceptable salt, derivative, and the like. Preferred salts comprise, inter alia, hydrochloride, sulfate, bisulfate, tartrate, nitrate, citrate, bitratrate, phosphate, malate, maleate, hydrobromide, hydroiodide, fumarate, succinate and the like.

Dosage forms not according to the invention can comprise from 10 mg to 150 mg oxycodone or a pharmaceutically active salt thereof and more preferred from 20 mg to 80 mg oxycodone or a pharmaceutically active salt thereof and/or from 1 mg to 50 mg naloxone or a pharmaceutically active salt thereof and more preferred from 5 mg to 20 mg naloxone or a pharmaceutically active salt thereof per unit dosage. In other embodiments not according to the invention, the dosage forms or preparations may comprise from 5 to 50 mg oxycodone or a pharmaceutically active salt thereof, from 10 to 40 mg oxycodone or a pharmaceutically active salt thereof, from 15 to 30 mg oxycodone or a pharmaceutically active salt thereof or approximately 20 mg oxycodone or a pharmaceutically active salt thereof.

Dosage forms not according to the invention may also comprise from 1 to 40 mg naloxone or a pharmaceutically active salt thereof, 5 to 30 mg naloxone or a pharmaceutically active salt thereof, or 10 to 20 mg naloxone per unit dosage or a pharmaceutically active salt thereof. Matrix-based retardation formulations may preferably be used as dosage forms or formulations for use in accordance with the invention. It is especially preferred that the dosage forms for use are based on a substantially non-swellable diffusion matrix.

Preferably, matrix materials for dosage forms for use according to the present invention comprise polymers based on ethyl cellulose, with ethyl cellulose being an especially preferred polymer. Especially preferred do matrices comprise polymers which are available on the market under the trademark Ethocel Standard 45 Premium® or Surelease®. Particularly preferred is the use of ethyl cellulose N45 or of Surelease®E-7-7050.

It is particularly preferred that dosage forms for use according to the present invention comprise ethyl cellulose and at least one fatty alcohol as the matrix components that essentially influence the release characteristics of the matrix. The amounts of ethyl cellulose and the at least one fatty alcohol may significantly vary so that preparations with different release profiles may be achieved. Even though the preparations for use according to the invention usually will comprise both of the afore-mentioned components, in some cases it may be preferred that the preparations comprise only ethyl cellulose or the fatty alcohol(s) as the release determining components.

Dosage forms for use in accordance with the invention may further comprise fillers and additional substances, such as granulating aids, lubricants, dyes, flowing agents and plasticizers.

Lactose, glucose or saccharose, starches and their hydrolysates, microcrystalline cellulose, cellatose, sugar alcohols such as sorbitol or mannitol, polysoluble calcium salts like calciumhydrogenphosphate, dicalcium- or tricalciumphosphat may be used as fillers.

Povidone may be used as granulating aid.

Highly-disperse silica (Aerosil®), talcum, corn starch, magnesium oxide and magnesium stearate or calcium stearate may preferably be used as flowing agents or lubricants.

Magnesium stearate and/or calcium stearate can preferably be used as lubricants. Fatty acids like stearic acid, or fats like hydrated castor oil can also preferably be used.

Polyethylene glycols and fatty alcohols like cetyl and/or stearyl alcohol and/ or cetostearyl alcohol can also be used as additional substances that influence retardation.

If fillers and additional substances such as dyes and the mentioned lubricants, flowing agents and plasticizers are used, care has to be taken that according to the invention only such combinations together with the matrix forming substance and/or the matrix forming substances are used, which ensure *in vivo* parameters of the active compounds in accordance with the invention.

All these additional components of the formulations will preferably be chosen in such a way that the release matrix receives the character of a substantially non-water- or non-buffer-swellable and non-erosive diffusion matrix.

According to the invention, it is especially preferred that the dosage forms comprise ethylcellulose such as ethyl cellulose N45 or Surelease® E-7-7050 as a matrix-building substance, stearyl alcohol as fatty alcohol, magnesium stearate as lubricant, lactose as filler and povidone as a granulating aid.

A dosage form (i) for use according to the present invention contains oxycodone in an amount corresponding to 20 mg anhydrous oxycodone hydrochloride, and naloxone in an amount corresponding to 10 mg anhydrous naloxone hydrochloride. For those dosage forms containing 20 mg oxycodone hydrochloride and 10 mg naloxone hydrochloride it is especially preferred that the retardant materials are selected from ethyl cellulose and stearyl alcohol. In some specific embodiments, such dosage forms contain at least 29 mg stearyl alcohol or at least 29.5 mg stearyl alcohol, or even at least 30 mg stearyl alcohol. Preferred amounts for ethylcellulose in dosage forms according to this embodiment are at least 8, or at least 10, or at least 12 mg ethylcellulose

A dosage form (ii) for use according to the present invention contains oxycodone in an amount corresponding to 10 mg anhydrous oxycodone hydrochloride and naloxone in an amount corresponding to 5 mg naloxone hydrochloride. In this embodiment, it is also preferred that the retardant materials are selected from ethylcellulose and stearyl alcohol. Preferred amounts for ethylcellulose and stearyl alcohol in dosage forms according to this embodiment are at least 8, or at least 10, or at least 12 mg ethylcellulose and/or at least 20, or at least 25, or at least 27 mg stearyl alcohol.

A dosage form (iii) for use according to the present invention contains oxycodone in an amount corresponding to 40 mg anhydrous oxycodone hydrochloride and naloxone in an amount corresponding to 20 mg anhydrous naloxone hydrochloride. Again, the retardant materials are preferably selected from ethylcellulose and stearyl alcohol. In this embodiment, the dosage forms preferably contain at least 22 mg, or at least 24 mg, or at least 26 mg ethylcellulose and/or at least 55 mg, or at least 59 mg, or at least 61 mg stearyl alcohol. Preferred amounts for ethylcellulose in dosage forms according to this embodiment are at least 8, or at least 10, or at least 12 mg ethylcellulose

Dosage forms for use in accordance with the invention can be produced like all common dosage forms which, in principle, are suitable for retardation formulations and which provide for the *in vivo* parameters of the active compounds, i.e. oxycodone and naloxone, in accordance with the invention. Especially suitable are tablets, multi-layer tablets and capsules. Additional application forms like granules or powders can be used, with only those applications forms being admissible that provide a sufficient retardation and a release behavior in accordance with the invention.

Pharmaceutical preparations may also comprise film coatings. However, it has to be ensured that the film coatings do not negatively influence the release properties of the active compounds from the matrix and the storage stability of the active compounds within the matrix. Such film coatings may be colored or may comprise an initial dosage of active compounds if required. The active compounds of this initial dosage will be immediately released so that the therapeutically effective blood plasma level is reached very quickly.

Pharmaceutical preparations or preliminary stages thereof which are for use in accordance with the invention can be produced by build-up or break-down granulation. A preferred embodiment is the production by spray granulation with subsequent drying of the granules. Another preferred embodiment is the production of granules by build-up granulation in a drum or on a granulating disk. The granules may then be pressed into e.g. tablets using appropriate additional substances and procedures. The person skilled in the art is familiar with granulating technology as applied to pharmaceutical technology.

Production of pharmaceutical preparations or preliminary stages thereof, which are for use in accordance with the invention, by extrusion technology is especially advantageous. In one preferred embodiment, pharmaceutical preparations or preliminary stages thereof are produced by melt extrusion with co- or counter-rotating extruders comprising two screws. Another preferred embodiment is the production by means of extrusion, with extruders comprising one or more screws. These extruders may also comprise kneading elements.

Extrusion is also a well-established production process in pharmaceutical technology and is well known to the person skilled in the art. The person skilled in the art is well aware that during the extrusion process, various parameters, such as the feeding rate, the screw speed, the heating temperature of the different extruder zones (if available), the water content, etc. may be varied in order to produce products of the desired characteristics.

The aforementioned parameters will depend on the specific type of extruder used. During extrusion the temperature of the heating zones, in which the components of the inventive formulation melt, may be between 40 to 120 °C, preferably between 50 to 100 °C, more preferably between 50 to 90 °C, even more preferably between 50 to 85 °C and most preferably between 65 to 80° C, particularly if counter-rotating twin screw extruders (such as a Leistritz Micro 18 GGL or Leistritz Micro 27 GGL) are used. The person skilled in the art is well aware that not every heating zone has to be heated. Particularly behind the feeder where the components are mixed, cooling at around 25 °C may be necessary. The screw speed may vary between 100 to 500 revolutions per minute (rpm), preferably between 100 to 250 rpm, more preferably between 100 to 200 rpm and most preferably around 150 rpm, particularly if counter-rotating twin screw extruders (such as a Leistritz Micro 18 GGL) are used. The geometry and the diameter of the nozzle may be selected as required. The diameter of the nozzle of commonly used extruders typically is between 1 to 10 mm, preferably between 2 to 8 mm and most preferably between 3 to 5 mm. The ratio of length versus diameter of the screw of extruders that may be used for production of inventive preparations is typically around 40:1.

Generally, the temperatures of the heating zones have to be selected such that no temperatures develop that may destroy the pharmaceutically active compounds. The feeding rate und screw speed will be selected such that the pharmaceutically active compounds are released from the preparations produced by extrusion in a sustained, independent and invariant manner and are storage stable in the matrix. If e.g. the feeding rate is increased, the screw speed may have to be increased correspondingly to ensure the same retardation.

The person skilled in the art knows that all the aforementioned parameters depend on the specific production conditions (extruder type, screw geometry, number of components etc.) and may have to be adapted such that the preparations produced by extrusion provide for *in vivo* parameters of oxycodone in accordance with the present invention.

Examples that display highly advantageous embodiments of the invention are set out below.

## EMBODIMENT EXAMPLES

Reference

### Example 1: Optimization of Naloxone - Oxycodone Ratio in Pain Patients

1. Objective

The primary objective of this study was to investigate whether an oxycodone/naloxone combination in accordance with the invention will lead to a comparable analgesia with a decrease in constipation in patients with severe chronic pain of tumour and non-tumour origin, and need for laxatives, when compared with oxycodone alone. A further objective was to investigate which dose ratio of oxycodone to naloxone was the most effective and most suitable for further development with respect to bowel function improvement, analgesic efficacy, and safety. A third objective was to compare the incidence of other side effects between treatment groups.

The method for the assessment of bowel function and analogue scales for use in this method were employed in a clinical Phase II study conducted in Europe.

2. Test Population, Inclusion and Exclusion Criteria

In total 202 patients were randomized and 152 patients were to receive both naloxone and oxycodone; 50 patients were to receive oxycodone and naloxone placebo. The Intent to Trial (ITT) population consisted of 196 (97,0%) patients. The Per Protocol (PP) population consisted of 99 (49%) patients.

Study participants were selected according to inclusion and exclusion criteria. In general, male or female patients, aged $\geq$ 18 years, suffering from severe chronic pain of tumour and non-tumour origin and who required opioid treatment were enrolled in the study. Patients with insufficient efficacy or tolerability to WHO II or III analgesic and patients with stable oxycodone therapy (40 - 80 mg/day) were suitable for screening. Patients included in the double-blind treatment period were on stable oxycodone treatment and had a medical need for the regular intake of laxatives. Patients were selected according to the following inclusion criteria.

*Inclusion Criteria*

- Aged $\geq$ 18 years
- with severe chronic pain of tumour and non-tumour origin that required opioid treatment
- and/or insufficient efficacy with a WHO II or III analgesic
- and/or insufficient tolerability with a WHO II or III analgesic
- or patients under current stable oxycodone therapy (40 - 80 mg/day)
- were capable of voluntary participation and of providing written informed consent
- could understand the requirements of the protocol and were willing and able to fulfil them.

Patients who were to be included in the maintenance treatment period (maintenance face) and titration or run-in were those:

- on stable oxycodone treatment 40 - 80 mg/day with no more than 5 rescue medication intakes (oxycodone) per week
- with the medical need for the regular intake of laxatives to have at least 3 bowel evaluations/week

*Exclusion Criteria*

Patients were to be excluded from the study where those:

- with current alcohol or drug abuse
- with current severe cardiovascular and respiratory disease (e.g. lung cancer and metastases)
- with current severe liver and renal insufficiency (transaminases threefold above normal range) and/or liver/renal carcinoma and/or metastases
- with a history of paralytic ileus
- with current acute pancreatitis
- with a history of psychosis
- with a history of Morbus Parkinson
- in the process of taking early disease-related retirement
- receiving another opioid treatment besides oxycodone
- with a known hypersensitivity to one of the study drugs
- which participated in another clinical study within 30 days of study entry
- were female and pregnant or lactating
- were female of child bearing potential and not adequately protected against conception

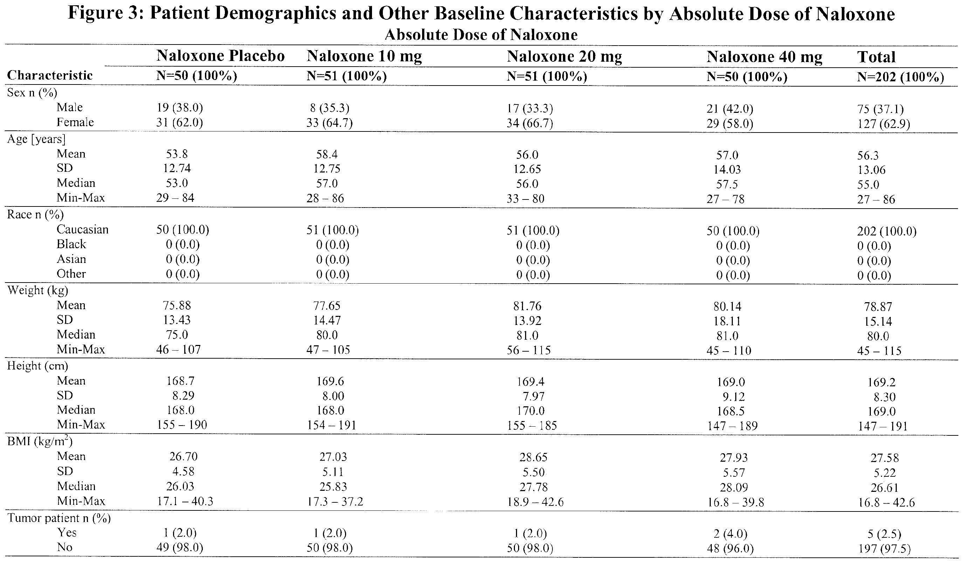

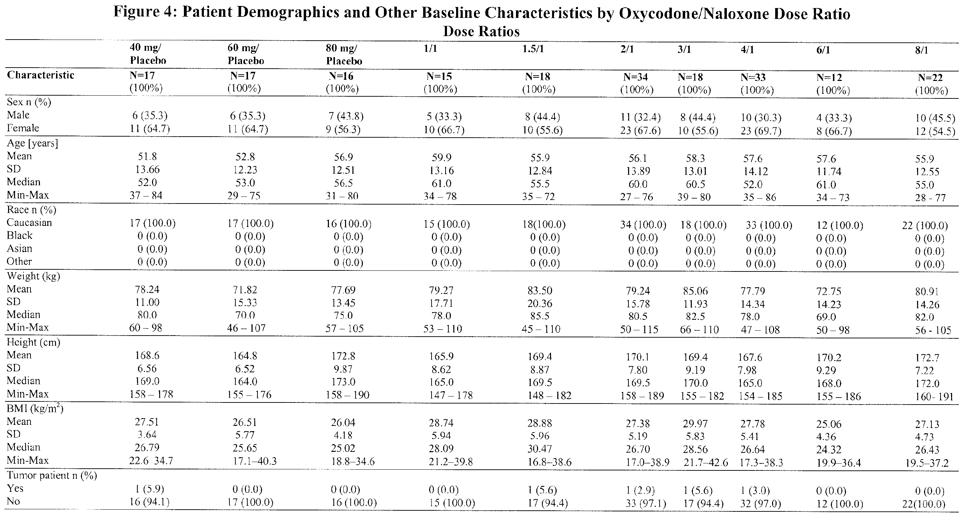

Specifics of the test population can be taken from Figures 3 and 4.

3. Test Treatment, Dose, and Mode of Administration

*Preparations administered*

Tablets of dosage strengths 20 mg oxycodone, 10 mg oxycodone, 5 mg naloxone and 10 mg naloxone were prepared by spray granulation. Oxycodone dosage strengths of 30 mg were administered by using one 10mg dosage strength tablet and one 20 mg dosage strength tablet. Oxycodone dosage strengths of 40 mg were administered by using two 20mg dosage strength tablets.

**Oxycodone Hydrochloride PR Tablets 10 mg**

Oxycodone hydrochloride PR tablets 10 mg are round, biconvex, white film coated tablets with OC on one side and 10 on the other. The composition of oxycodone hydrochloride PR tablets 10 mg is given below:

Composition of Oxycodone Hydrochloride PR Tablets 10 mg

| Constituents | mg/tablet | Function | Reference to Standard |
|---|---|---|---|
| **Tablet Core** | | | |
| Active constituent | | | |
| Oxycodone hydrochloride[1] (Oxycodone base equivalent) | 10.00 (9.00) | Active Ingredient | Ph Eur |
| Other constituents | | | |
| Lactose monohydrate (spray-dried lactose) | 69.25 | Diluent | Ph Eur |
| Povidone (K 30) | 5.00 | Binder | Ph Eur |
| Ammonio methacrylate copolymer dispersion (Eudragit RS 30 D)[2] (solids) | 10.00 | Retardant | USP/NF |
| Triacetin | 2.00 | Plasticiser | Ph Eur |
| Stearyl alcohol | 25.00 | Retardant | Ph Eur |
| Talc | 2.50 | Glidant | Ph Eur |
| Magnesium stearate | 1.25 | Lubricant | Ph Eur |
| Total core weight[3] | 130 | | |

(continued)

| | mg/tablet | Function | Reference to Standard |
|---|---|---|---|
| **Film Coat** | | | |
| Opadry white Y-5R-18024-A[4] | 5.00 | Coating | |
| Purified water[5] | - | Solvent | Ph Eur |
| Total tablet weight | 135 | | |
| **Film Coat Composition** | | | |
| The approximate composition of a 5 mg film coat is as follows:- | | | |
| Component | | | |
| Hypromellose 3 mPa.s (E464) | 1.750 | Film former | Ph Eur |
| Hypromellose 50 mPa.s (E464) | 0.250 | Film former | Ph Eur |
| Hydroxypropylcellulose | 1.500 | Film former | Ph Eur |
| Titanium Dioxide (E171) | 1.000 | Colorant | Ph Eur |
| Macrogol 400 | 0.500 | Plasticiser | Ph Eur |

[1] Anhydrous basis. Batch quantity is adjusted for assay/ moisture content.

[2] Eudragit RS 30 D consists of a 30% dispersion of ammonio methacrylate copolymer NF (Poly [ethylacrylate-co-methylmethacrylate-co-(2-trimethyl ammonio ethyl) methacrylate chloride] {1:2:0.1} NF) in purified water Ph Eur, preserved with 0.25% (E,E)-Hexa-2,4-dienoic acid (sorbic acid) Ph Eur/NF

[3] Includes ~4% residual moisture i.e. 5 mg per tablet core.

[4] Actual quantity of coat is about 5 mg. Coat is applied to the core tablets to obtain a 3-4% weight increase and a uniform appearance.

[5] Removed during processing.

*Oxycodone Hydrochloride PR Tablets 20 mg*

Oxycodone hydrochloride PR tablets 20 mg are round, biconvex, pink film coated tablets with OC on one side and 20 on the other. The composition of oxycodone hydrochloride PR tablets 20 mg is given below.

Composition of Oxycodone Hydrochloride PR Tablets 20 mg

| Constituents | mg/ tablet | Function | Reference to Standard |
|---|---|---|---|
| **Tablet Core** | | | |
| Active constituent | | | |
| Oxycodone hydrochloride[1] (Oxycodone base equivalent) | 20.0 (18.00) | Active Ingredient | Ph Eur |
| Other constituents | | | |
| Lactose monohydrate (spray-dried lactose) | 59.25 | Diluent | Ph Eur |
| Povidone (K 30) | 5.00 | Binder | Ph Eur |
| Ammonio methacrylate copolymer dispersion (Eudragit RS 30 D)[2] (solids) | 10.00 | Retardant | USP/NF |
| Triacetin | 2.00 | Plasticiser | Ph Eur |
| Stearyl alcohol | 25.00 | Retardant | Ph Eur |
| Talc | 2.50 | Glidant | Ph Eur |
| Magnesium stearate | 1.25 | Lubricant | Ph Eur |
| Total core weight[3] | 130 | | |
| **Film Coat** | | | |
| Opadry Pink YS-1R-14518-A[4] | 5.00 | Coating | |
| Purified water[5] | - | Solvent | Ph Eur |
| Total tablet weight | 135 | | |

(continued)

**Film Coat Composition**

The approximate composition of a 5 mg film coat is as follows:-

| Component | | | |
|---|---|---|---|
| Hypromellose 3 mPa.s (E464) | 1.5625 | Film former | Ph Eur |
| Hypromellose 6 mPa.s (E464) | 1.5625 | Film former | Ph Eur |
| Titanium Dioxide (E171) | 1.4155 | Colorant | Ph Eur |
| Macrogol 400 | 0.4000 | Plasticiser | Ph Eur |
| Polysorbate 80 | 0.0500 | Wetting agent | Ph Eur |
| Iron oxide red (E172) | 0.0095 | Colorant | HSE |

[1] Anhydrous basis. Batch quantity is adjusted for assay/ moisture content.

[2] Eudragit RS 30 D consists of a 30% dispersion of ammonio methacrylate copolymer NF (Poly [ethylacrylate-co-methylmethacrylate-co-(2-trimethyl ammonio ethyl) methacrylate chloride] {1:2:0.1} NF) in purified water Ph Eur, preserved with 0.25% (E,E)-Hexa-2,4-dienoic acid (sorbic acid) Ph Eur/NF

[3] Includes ~4% residual moisture i.e. 5 mg per tablet core.

[4] Actual quantity of coat is about 5 mg. Coat is applied to the core tablets to obtain a 3-4% weight increase and a uniform appearance.

[5] Removed during processing.

*Naloxone Tablets*

Naloxone prolonged release tablets, are controlled release tablets using a matrix of stearyl alcohol and ethyl cellulose as the retardant. The tablets contain 10 mg naloxone hydrochloride per tablet. The complete statement of the components and quantitative composition Naloxone prolonged release tablets is given below.

Naloxone prolonged release tablets

| Component | Quantity (mg/tablet) | | | Function | Reference to Standard |
|---|---|---|---|---|---|
| | Nal 5mg | Nal 10mg | Nal 15mg | | |
| Naloxone hydrochloride Dihydrate | 5.45 | 10.90 | 16.35 | Active | Ph. Eur.* |
| *corresponding to* | | | | | |
| *Naloxone hydrochloride anhydrous* | 5.00 | *10.00* | 15.00 | | |
| *Naloxone base* | 4.50 | *9.00* | 13.50 | | |
| Povidone K30 | 5.00 | 5.00 | 5.00 | Binder | Ph. Eur.* |
| Retarding Suspension (Surelease E-7-7050) (dry mass) comprising | 10.00 | 10.00 | 10.00 | | |
| 1. Ethylcellulose | 6.93 | 6.93 | 6.93 | Retardant components of the release controlling matrix | Ph.Eur.* |
| 2. Dibutyl Sebacate | 1.60 | 1.60 | 1.60 | | U.S.N.F * |
| 3. Oleic Acid | 0.77 | 0.77 | 0.77 | | U.S.N.F.* |
| 4. Colloidal anhydrous silica | 0.70 | 0.70 | 0.70 | | Ph.Eur.* |
| Stearyl alcohol | 25.00 | 25.00 | 25.00 | Retardant | Ph. Eur.* |
| Lactose monohydrate | 74.25 | 69.25 | 64.25 | Diluent | Ph. Eur. * |
| Purified talc | 2.50 | 2.50 | 2.50 | Glidant | Ph. Eur. * |
| Magnesium stearate | 1.25 | 1.25 | 1.25 | Lubricant | Ph. Eur. * |
| **TOTAL TABLET WEIGHT** | **123.0** | **123.0** | **123.0** | | * current Edition |

*Study design*

The clinical study was conducted in Germany as a multi-center, prospective, controlled, randomized, double-blind (with placebo-dummy), four group parallel study with oral controlled release (CR) oxycodone, oral controlled-release (CR) naloxone and corresponding naloxone placebo.

The total study duration was up to 10 weeks, including a screening period, a minimum two week titration period (maximum 3 weeks) (or a one week run-in period), a four week treatment period (oxycodone and naloxone/naloxone placebo) and a follow-up phase of two weeks.

Patients with stable pain control, who fulfilled all inclusion/exclusion criteria were randomized to double-blind therapy in one of three naloxone treatment groups or a naloxone placebo treatment group.

The study had three core phases: a pre-randomization phase, a 4-week double-blind treatment period (maintenance phase) and a follow-up phase. The pre-randomization phase consisted of screening and titration/run-in. Following screening, patients entered either a titration or run-in period. Patients with insufficient pain pre-treatment entered a minimum 2-week titration period and were individually titrated and stabilized at an oxycodone dose of 40 mg, 60 mg or 80 mg per day. Patients on stable oxycodone pre-treatment at screening (between 40-80 mg/day) and with concomitant constipation, entered a 1 week run-in period and were eligible for the maintenance phase without prior titration. For all patients, the dose of oxycodone could be adjusted during titration or run-in and investigators maintained compulsory telephone contact every $2^{nd}$ day to assess pain control and make dose changes.

At the end of the titration/run-in period, patients who were receiving a stable maintenance dose of 40 mg, 60 mg or 80 mg oxycodone per day (with no more than 5 rescue medication intakes per week) and had a medical need for the regular intake of laxatives were randomized to one of 3 naloxone treatment groups or a naloxone placebo treatment group. Each patient received their maintenance dose of oxycodone plus either 10 mg, 20 mg, 40 mg or naloxone placebo CR tablets daily (see Table 2).

After the treatment period, patients maintained their maintenance dose of oxycodone only for a further two-week follow-up phase (40 mg, 60 mg, or 80 mg oxycodone per day). Patients maintained a daily diary, and efficacy and safety assessments were performed over the course of the study.

Table 1: Treatment groups for maintenance phase based on naloxone dose per day.

| | Group 1 | Group 2 | Group 3 | Group 4 |
|---|---|---|---|---|
| **Naloxone daily dose (mg)** | Placebo **0** | 5 + 5 **10** | 10 + 10 **20** | 20 + 20 **40** |
| **Oxycodone daily dose** | 2x20, 2x30, 2x40 **40 60 80** | 2x20, 2x30, 2x40 **40 60 80** | 2x20, 2x30, 2x40 **40 60 80** | 2x20, 2x30, 2x40 **40 60 80** |
| **Oxycodone + Naloxone dose (mg)** | **40/pl 60/pl 80/pl** | **40/10, 60/10, 80/10** | **40/20, 60/20, 80/20** | **40/40, 60/40, 80/40** |
| **Ratio** | 40/pl 60/pl 80/pl | 4/1, 6/1, 8/1 | 2/1, 3/1, 4/1 | 1/1, 1.5/1, 2/1 |

Note: Identical dose ratios were obtained for 40/10 mg and 80/20 mg (4/1) and for 40/20 mg and 80/40 mg (2/1)

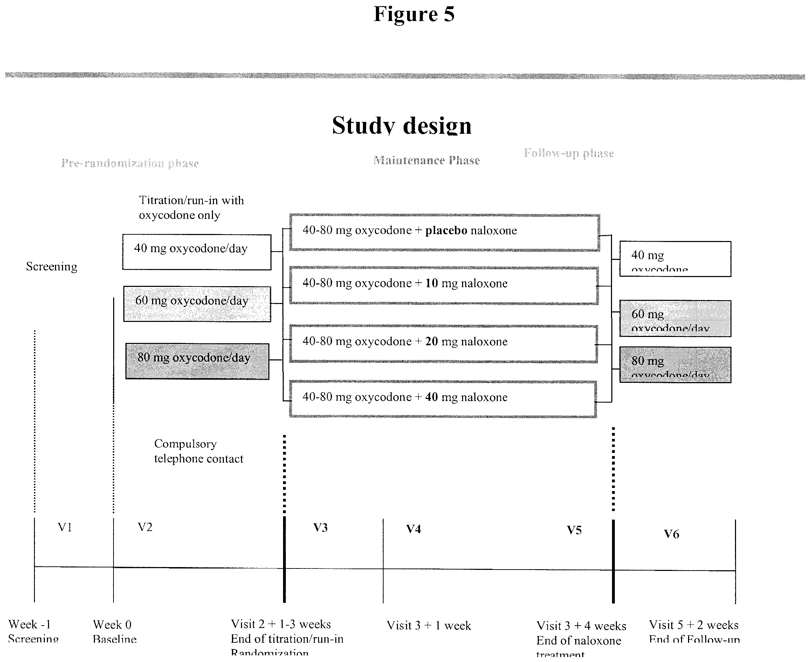

202 subjects were randomized, 196 were in the ITT populations and 166 completed the study. The study design schematic for the clinical study is displayed in Figure 5.

Blinded naloxone CR tablets (5 mg and 10 mg) were supplied in bottles. The dosage regimen was constant for the entire double-blind treatment period and no dose adjustments were allowed. Patients received 5, 10 or 20 mg of oral naloxone each morning and evening.

Open label oxycodone CR tablets (10 mg and 20 mg) were supplied in PP blisters. Dose adjustments could be performed during the titration/run-in period and 10 mg CR oxycodone tablets were available as rescue medication throughout the entire study. The dosage regimen was constant for the entire double-blind treatment period. Patients received 20, 30 or 40 mg of oral oxycodone each morning and evening.

Blinded naloxone placebo tablets were optically identical to naloxone tablets 5 mg and 10 mg. Dose and mode of administration were as for naloxone CR tablets.

The Intent-To-Treat (ITT) population included all randomized patients who received at least one dose of study drug and had at least one post-randomization efficacy assessment. For some analyses, the last observation was carried forward for those ITT subjects who discontinued after Visit 4 (ITT/LOCF). In other instances, only the available data were used (ITT non-missing). The Per Protocol (PP) population included all randomized patients who completed the study (including the follow-up phase) without major protocol violations. Major protocol violations were defined as:

- Patients who received more than 50 mg oxycodone per week as rescue medication during the maintenance phase or did not follow one of the scheduled oxycodone dose regimens (40 mg, 60 mg or 80 mg oxycodone per day).
- Less than 4 morning and 4 evening assessments of mean pain intensity were documented during the last 7 days prior to each visit.
- Very large deviations from the scheduled visits, i.e. the date of visit was outside the respective visit window. Only deviations from the visit window of the maintenance phase visits (visit 4 and 5) were regarded as major protocol violations. Deviations from the other visits were regarded as minor protocol violations. For the identification of a major protocol violation, the visit windows for visit 4 and 5 were slightly increased after a blinded review of the data and were defined as follows:
- visit 4 (during the maintenance phase):
- visit 3 plus 6 to 12 days
- visit 5 (at the end of the maintenance phase):
- visit 3 plus 25 to 31 days.

4. Primary Efficacy Variables

Efficacy assessments were determined based on data recorded in the case report form and in patient diaries.
The primary efficacy variables of interest were pain and bowel function as follows:

a) Mean Pain during the last 7 days prior to each visit, based on the patient's twice-daily assessment of pain intensity using the 0-100 numerical analogue scale (NAS) (0= no pain and 100= worst imaginable pain). Mean Pain was calculated for each study visit as the mean value of the daily mean values of all patient's diary entries from the last 7 days.

b) Mean bowel function: patient's assessment, at each study visit, of bowel function during the last 7 days prior to each visit. Mean bowel function was calculated from the mean of the three 0-100 NAS scores: ease of defecation (0= easy/no difficulty, 100= severe difficulty), feeling of incomplete bowel evacuation (0= not at all, 100= very strong), and judgment of constipation (0= not at all, 100= very strong).

Secondary efficacy variables of interest included among others:
c) Global assessment of efficacy, tolerability and preference. Evaluation for global assessment of efficacy was measured using a 0 to 7 numerical analogue scale (1 = very good, 2 = good, 3 = pretty good, 4 = moderate, 5 = slightly poor, 6 = poor, 7 = very poor). Tolerability was measured using the same 0 to 7 numerical analogue scale. Preference was measured by assessing preference for maintenance (oxycodone/naloxone combination) or titration/run-in (oxycodone only) regarding efficacy/tolerability of study medication using a 0 to 3 NAS (1 = titration/run-in, 2 = maintenance, 3 = no preference).
For the global assessment of efficacy, tolerability and preference summary statistics for the groupings dose ratio of oxycodone and naloxone, absolute dose of naloxone and absolute dose of naloxone given the same oxycodone/naloxone ratio were provided for the ITT population.

d) Laxative intake/mean laxative dose, which was calculated from the respective case report forms (CRF) entries. An analysis of the mean laxative dose during the last seven days was performed for patients who took only one laxative during the entire study. Entries from the medication record CRF page were used for all calculations (laxatives were identified by the WHO ATC Code A06A). For laxative intake number of days with laxation during the last 7 days and the percentage of days with laxation during the last 7 days were calculated for each study visit. In addition, the percentage of days with laxation during the whole maintenance phase and during the follow-up phase was calculated.

e) Subjective symptoms of withdrawal (SOWS), which were recorded daily by the patient in the diary during the first seven days of the maintenance phase included: I am anxious; I have to yawn; I am sweating; My eyes are watering; My nose is running, I have gooseflash; I am shivering; I feel hot; I feel cold; My bones and muscles are aching; I am restless; I feel sick; I have to vomit; My muscles are twitching; I have abdominal cramps; I cannot sit still. All symptoms were rated as "0 = not at all", "1 = little", "2 = medium", "3 = strong" or "4 = extreme".

SOWS were recorded during the first 7 days of the maintenance phase in the patient diary. For the additional post-hoc analysis, the total score (= sum score) of the SOWS items was calculated for each patient and day. Additionally for each patient, the minimum, mean and maximum of the 7 daily dose scores were calculated. These parameters were summarized via simple characteristics for each oxycodone/naloxone ratio and absolute naloxone dose.

Safety assessments were determined based on data recorded in the case report form and in patient diaries.

Safety assessments consisted among others of monitoring and recording all adverse events (AEs).

f) An adverse event was any untoward medical occurrence in a patient or clinical investigation subject administered a pharmaceutical product, including placebo, and which did not necessarily have a causal relationship with treatment. Therefore, an adverse event could be

- an unfavourable and unintended sign (including an abnormal laboratory finding), symptom, or disease temporarily associated with the use of a medicinal product whether or not considered to be related to the medicinal product,
- any new disease or acerbation of an existing disease,
- any detoriation in non-protocol-required measurements of laboratory value or other clinical test that resulted in symptoms, a change in treatment or discontinuation from study drug.

Assessment of causality in suspected adverse events in response to a medicinal product was based on the following considerations: Associated connections (time or place); pharmacological explanations; previous knowledge of the drug; presence of characteristic, clinical or pathological phenomena; exclusion of other causes and/or absence of alternative explanations. The causal relationship to the study drug was assessed using a classification ranging from 0 to 4 (0 = not related: temporal relationship to drug administration is missing or implausible, 1 = improbable: temporal relationship to drug administration makes a causal relationship improbable, and other drugs, chemicals or underlying disease provide plausible explanations; 2 = possible: reasonable time sequence to administration of the drug, but event could also be explained by concurrent disease or other drugs or chemicals; information on drug withdrawal may be lacking or unclear; 3 = probable: reasonable time sequence to administration of the drug, but unlikely to be attributed to concurrent disease or other drugs or chemicals, and which follows the clinically reasonable response on withdrawal (dechallenge), rechallenge information is not required; 4 = definite: plausible time relationship to drug administration; event cannot be explained by concurrent disease or other drugs or chemicals; the response to withdrawal of the drug (dechallenge) should be clinically plausible; the event must be definitive pharmacologically or phenomenologically using a satisfactory rechallenge procedure, if necessary). All adverse events during the course of the study were all collected on the adverse event CRF. Elicited adverse events (nausea, emesis, abdominal pain, cramping, diarrhea, sedation, vertigo, headache, sweating, restlessness, skin reactions (pruritus, urticara and other)) and volunteered adverse events were documented (pain and constipation were not classified as adverse events for the study).

All analysis except the elicited opioid typical and naloxone typical adverse events analysis were performed for the safety population. The elicited opioid typical and naloxone typical adverse events analysis were performed on the ITT population as they were previously considered for be efficacy analysis. Adverse events were summarized by absolute number and percentage of patients, who

- had any adverse events,
- had an adverse event in each defined system organ class,
- experienced each individual adverse event.

The sum score of the severity of elicited opioid typical or elicited naloxone typical adverse events was calculated for each study visit as the sum of the scores assigned to each of the above-mentioned adverse events absolved during the last 7 days. A score of 0 was assigned, if the respective side-effect was not observed during the last 7 days, a score of 1, if the adverse event was mild, a score of 2, if the adverse event was moderate, and a score of 3, if the adverse event was severe. If for one side-effect more than one adverse event with different severities were recorded during the last 7 days, the worst severity was used.

Summary statistics for the sumscore of the severity of elicited opioid typical and elicited naloxone typical adverse events during the last 7 days were provided for each study visit for the groupings dose ratio of oxycodone and naloxone, absolute dose of naloxone and absolute dose of naloxone given the same oxycodone/naloxone ratio. In addition, Wilcocxon tests (modified to handle the Behrens-Fischer problem) of absolute dose of naloxone versus placebo were performed in the ITT population for values at Visit 4 (after 1 week of naloxone treatment) and for values at the end of the maintenance phase (after 4 weeks of naloxone treatment).

Additional summary statistics were provided for the sumscore of the severity of elicited opioid typical and elicited naloxone typical adverse events during the whole maintenance phase for the groupings dose ratio of oxycodone and naloxone, absolute dose of naloxone and absolute dose of naloxone given the same oxycodone/naloxone ratio, and for the sumscore of the severity of elicited opioid typical and elicited naloxone typical adverse events during the follow-up phase by absolute dose of oxycodone. This analysis was performed using the ITT population.

Adverse events, as mentioned above, were identified by the following the Medical Dictionary for Regulatory Affairs (MeDRA). Elicited opioid-typical adverse events were considered to be nausea, emesis, sedation, skin reactions, as identified in the aforementioned MeDRA (leading to a maximum sum scor of 12). Elicited naloxone-typical adverse events were considered to be abdominal pain, cramping and diarrhea with the definitions applied as laid out in MeDRA (leading to a maximum sum scor of 9).

5. Analgesic Efficacy Results

The end of maintenance mean pain results are summarized below:

Table 2: Mean Pain at End of Titration Visit (V3) and End of Maintenance Visit (V5) by Absolute Dose of Naloxone - ITT (with non-missing data) and PP Analysis Populations.

| Population | Statistic | Naloxone Placebo | Naloxone 10 mg | Naloxone 20 mg | Naloxone 40 mg |
|---|---|---|---|---|---|
| ITT non-missing | N | 46 | 42 | 43 | 41 |
| | Mean (SD) V3 | 36.9 (15.9) | 35.9 (16.3) | 39.8 (18.4) | 38.1 (15.8) |
| | Mean (SD) V5 | 37.8 (18.2) | 37.2 (17.3) | 37.5 (20.5) | 38.7 (17.0) |
| | 95% Confidence Interval for Difference vs. Placebo* | | (-5.04, 4.58) | (-2.36, 7.22) | (-4.76, 4.93) |
| | | | | | |
| PP | N | 29 | 26 | 22 | 22 |
| | Mean (SD) V3 | 34.0 (16.0) | 38.0 (17.7) | 40.1 (20.0) | 39.0 (16.1) |
| | Mean (SD) V5 | 32.6 (16.6) | 38.8 (18.4) | 36.1 (19.5) | 38.7 (16.6) |
| | 95% Confidence Interval for Difference vs. Placebo* | | (-9.10, 2.94) | (-5.01, 7.64) | (-8.41, 4.22) |

*95% Confidence Intervals for Difference vs. Placebo at Visit 5 (end of maintenance) are based on an ANCOVA model with treatment and baseline pain intensity as factors in the model.

The differences were small and confidence intervals were fairly narrow relative to the 0-100 pain scale and did not point to a difference in analgesic efficacy between active naloxone and naloxone placebo.

Thus, in the ITT population mean pain scores ($\pm$SD) ranged from 38.3 ($\pm$18.49) to 38.8 ($\pm$16.59) compared to 36.9 ($\pm$15,74) for placebo during the last 7 days prior to visit 4 and 37.2 ($\pm$17.24) to 38.7 ($\pm$17.05) compared to 37.8 ($\pm$18.22) for placebo during the last 7 days at the end of the maintenance phase. Analgesic efficacy did not change at V4 and V5 with oxycodone dose or oxycodone/naloxone ratio in a quadratic response surface model using oxycodone dose and the ratio as factors and baseline mean pain as covariant.

A quadratic response surface model with naloxone and oxycodone dose as factors and baseline pain as covariant shows that the only factor that affects the end of maintenance mean pain is the baseline pain measurement. There was no evidence of changes in mean pain with varying amounts of naloxone. However the study was not designed nor powered as a formal demonstration of non-inferiority of oxycodone/naloxone versus oxycodone/naloxone placebo.

6. Bowel Function Efficacy Results

Mean bowel function was calculated for each study visit from the mean of the three NAS values ease/difficulty of defecation, feeling of incomplete bowel evacuation and judgment of constipation. Summary statistics for mean bowel function during the last 7 days were provided for each study visit for the groupings dose ratio of oxycodone and naloxone, absolute dose of naloxone and absolute dose of naloxone given the same oxycodone/naloxone ratio.

To test for difference of absolute dose of naloxone versus placebo, t-tests were performed for the values obtained during the end of maintenance phase (after 4 weeks of naloxone treatment). In addition, two-sided 95% CIs (CI, confidence interval) for the difference in means between the treatment groups were provided. A response surface analysis was also performed for the end of the maintenance phase (after 4 weeks of naloxone treatment). These analyses were performed for the ITT and PP populations. For the ITT population only, t-tests for difference were also performed to explore mean bowel function at Visit 4 (after 1 week of naloxone treatment).

In addition, summary statistics of mean bowel function during the last 7 days for the end of the follow-up phase were provided for the grouping absolute dose of oxycodone in the ITT population.

To evaluate the effects of the titration/run-in period a paired t-test for difference was conducted for the mean bowel function during the last 7 days before the end of titration/run-in, compared with the mean bowel function during the last 7 days before the baseline visit. This analysis was performed in the titration phase population. In addition, two-sided 95% CIs for the difference in means between the treatment periods were provided.

Figures were provided for the ITT and the PP population. The values obtained for mean bowel function during the last 7 days before the end of the maintenance phase (mean $\pm$ 95% CI) were plotted against the oxycodone/naloxone dose ratio and the absolute dose of naloxone. In addition, surface plots were provided for the results obtained at the end of the maintenance phase.

To investigate if the bowel function depends on the ratio of oxycodone and naloxone or the absolute dose of naloxone additional analysis and figures were provided for the ITT population. A response surface analysis for the total consumed oxycodone dose during the last week of the maintenance phase versus the naloxone dose was performed. The parameter estimates derived were taken to display a surface plot of the whole dose range investigated. Moreover, a contour plot of the bowel function with a granulation of 10 was performed.

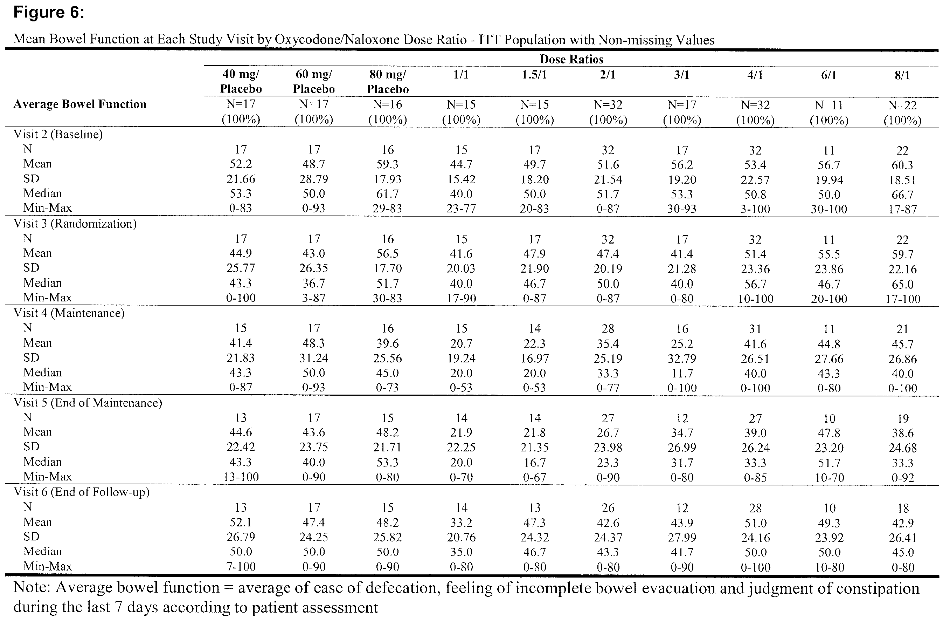

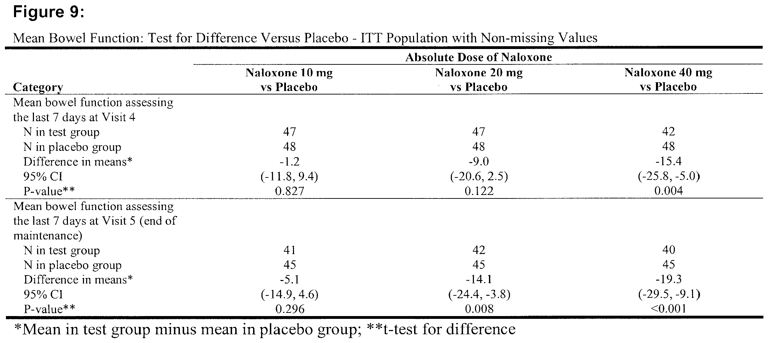

The values for mean bowel function at each study visit by dose ratio, by absolute dose of naloxone and by absolute dose of naloxone given the same oxycodone/naloxone dose ratio in the ITT population are presented in Figures 6 to 8. The test for difference for each dose of naloxone versus placebo is summarized in Figure 9.

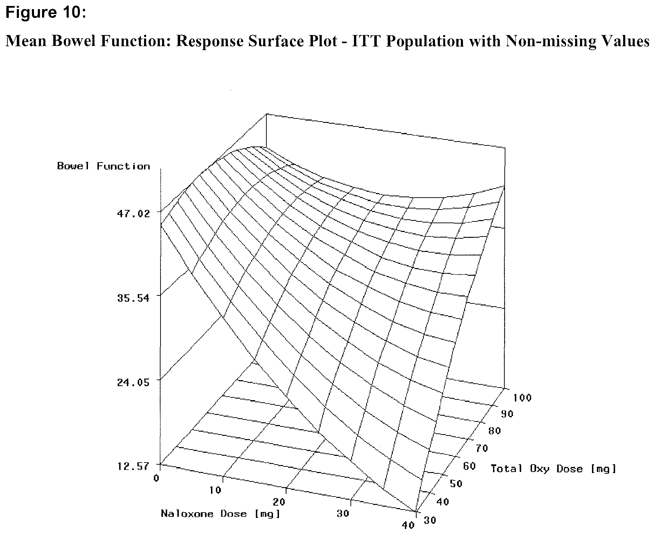

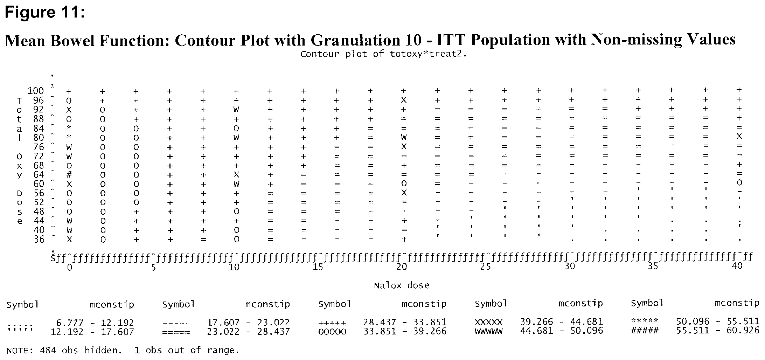

The surface plot of the whole dose range investigated based on the RSREG estimations of the model parameters is displayed in Figure 10. The contour plot of the bowel function with a granulation of 10 is shown in Figure 11.

Within the ITT population, a trend towards improved mean bowel function with increased dose of naloxone was seen. During the last 7 days at the end of the maintenance phase, mean ($\pm$SD) bowel function was lowest in the 1/1, 1.5/1 and 2/1 dose ratios (21.9$\pm$22.25, 21.8$\pm$21.35 and 26.7$\pm$23.98 for the 1/1, 1.5/1 and 2/1 dose ratios, respectively). Furthermore, mean bowel function worsened as the amount of naloxone decreased, to a maximum value of 47.8 ($\pm$23.20) for a dose ratio of 6/1. For the last 7 days prior to Visit 4, mean bowel function ranged from 20.7 ($\pm$19.24) at a ratio of 1/1 to 45.7 ($\pm$26.86) at a ratio of 8/1 (see Figure 6. Values for mean bowel function in the oxycodone/naloxone placebo dose ratios were higher than in the 1/1, 1.5/1 and 2/1 dose ratios at both visits.

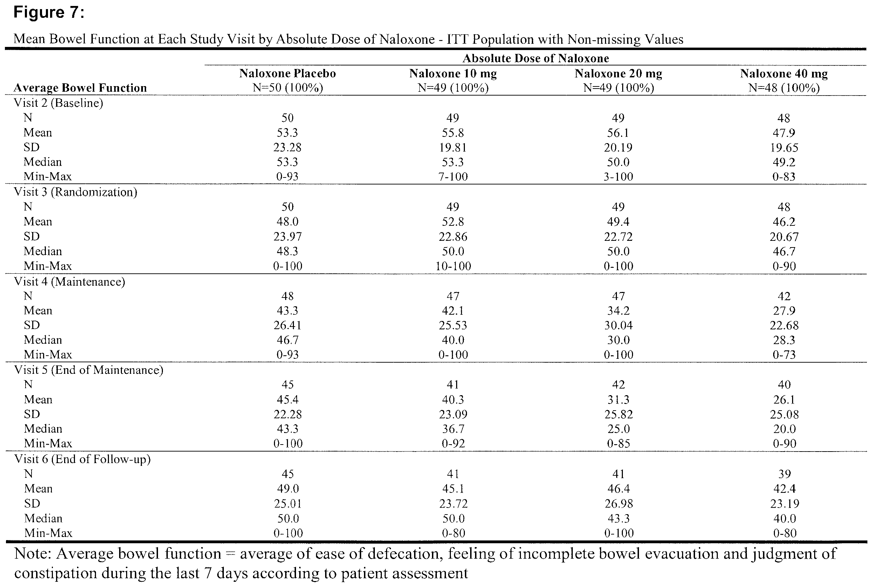

Analysis by absolute dose of naloxone showed values of 45.4 ($\pm$22.28), 40.3 ($\pm$23.09), 31.3 ($\pm$25.82) and 26.1 ($\pm$25.08) for placebo, 10 mg, 20 mg and 40 mg respectively at the end of maintenance (p<0.05 for 20 mg and 40 mg naloxone versus placebo, t-test for difference) and 43.3 ($\pm$26.41), 42.1 ($\pm$25.53), 34.2 ($\pm$30.04) and 27.9 ($\pm$22.68) at Visit 4 (p=0.004 for 40 mg naloxone versus placebo, t-test for difference) (see Figures 7 and 9).

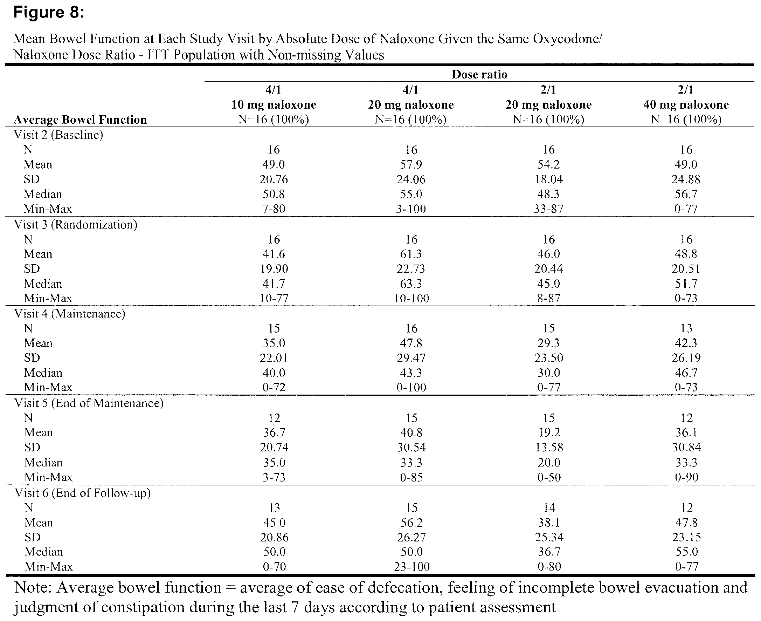

Analysis by absolute dose of naloxone given the same oxycodone/naloxone dose ratio showed that within both dose ratio groups (4/1 and 2/1) patients taking the higher oxycodone dose had higher mean bowel function values at Visits 4 and 5 (see Figure 8).

From the end of the maintenance phase to end of follow-up, mean bowel function worsened (. The range for mean bowel function was 21.8 ($\pm$21.35) to 48.2 ($\pm$21.71) for the dose ratio groups at end of maintenance and 33.2 ($\pm$20.76) to 52.1 ($\pm$26.79) for the dose ratio groups at the end of follow-up. The change was greatest in the 40 mg naloxone group; mean bowel function was 26.1 ($\pm$25.08) at the end of maintenance and 42.4 ($\pm$23.19) at the end of follow-up.

Analysis using the PP population generally mirrored the trends observed in the ITT population with regards to mean bowel function. During the last 7 days at the end of the maintenance phase, mean ($\pm$SD) bowel function was lowest in the 1/1 dose ratio (10.7$\pm$15.35) and worsened to a maximum of 57.3 ($\pm$17.38) for a dose ratio of 6/1. Mean bowel function values were higher than the 1/1, 1.5/1 and 2/1 ratios for all oxycodone/placebo dose ratios. Similar values were seen for the last 7 days prior to Visit 4 with the exception of the 3/1 dose ratio. At the end of the maintenance phase mean bowel function was 42.3 ($\pm$24.03), 39.4 ($\pm$23.44), 29.8 ($\pm$29.29) and 29.6 ($\pm$28.34) for placebo, 10 mg, 20 mg and 40 mg naloxone. The small number of patients in each treatment group in the PP population meant statistically significant p-values were not obtained in the PP analysis for t-tests for difference for mean bowel function.

The end of maintenance mean bowel function results are summarized below:

Table 3: Mean Bowel Function Scores at End of Titration Visit (V3) and End of Maintenance Visit (V5) by Absolute Dose of Naloxone - ITT (non-missing) and ITT/LOCF Analysis Populations.

| Population | Statistic | Naloxone Placebo | Naloxone 10 mg | Naloxone 20 mg | Naloxone 40 mg |
|---|---|---|---|---|---|
| ITT non-missing | N | 45 | 41 | 42 | 40 |
| | Mean (SD) V3 | 48.2 (23.5) | 53.5 (22.2) | 51.3 (21.6) | 48.2 (20.6) |
| | Mean (SD) V5 | 45.4 (22.3) | 40.3 (23.1) | 31.3 (25.8) | 26.1 (25.1) |
| | P-Value* | | 0.1658 | 0.0025 | 0.0002 |
| | | | | | |
| ITT / LOCF | N | 48 | 47 | 47 | 42 |
| | Mean (SD) V3 | 47.7 (24.0) | 53.6 (22.8) | 49.9 (23.1) | 47.7 (20.5) |
| | Mean (SD) V5 | 44.8 (22.9) | 40.1 (24.7) | 33.2 (28.4) | 26.5 (25.7) |
| | P-Value* | | 0.1795 | 0.0140 | 0.0005 |
| *Comparison versus Naloxone Placebo using ANCOVA model with Naloxone dose and baseline bowel function as factors in the model. | | | | | |

As already mentioned above, within the ITT population, improved mean bowel function with increased dose of naloxone was seen, with mean values ($\pm$ SD) of 45.4 ($\pm$22.3), 40.3 ($\pm$23.1), 31.3 ($\pm$25.8) and 26.1 ($\pm$25.1) for placebo, 10 mg, 20 mg and 40 mg respectively at the end of maintenance (p<0.05 for 20 mg and 40 mg naloxone versus placebo). The 95% confidence intervals for the mean bowel function differences from naloxone placebo were (-2.83, 16.69) at 10 mg naloxone, (5.46, 24.82) at 20 mg naloxone, and (9.54, 29.11) at 40 mg naloxone. The results display an increasing improvement in bowel function with increasing dose of naloxone, with the difference of the 20mg and 40mg dose versus naloxone placebo statistically significant at end of maintenance.

The response surface quadratic analysis confirms improving bowel function with increasing dose of naloxone, with the linear effect of naloxone dose statistically significant. The Table 5 displays the estimated improvements in mean bowel function scores versus naloxone placebo for the different oxycodone/naloxone ratios studied; these estimates correspond both to oxycodone/naloxone combinations actually represented in the study design, and some combinations for which quadratic surface interpolation was appropriate.

The estimates indicate that the mean bowel function improvement is in general constant within each ratio, and independent of the varying doses of oxycodone and naloxone. The only possible exception is the 80/40 mg combination, where there is a suggestion of a lower predicted effect than for the 60/30 mg and 40/20 mg combinations; this observation, however, has to be interpreted with the size of the standard error in mind.

Table 4: Response Surface Analysis of Bowel Function Efficacy by Oxycodone Dose and Oxycodone/Naloxone Ratio (Estimated Improvement (SE) vs Naloxone Placebo).

| Oxycodone dose / ratio Oxycodone/Naloxone | 40 mg Oxycodone/day | 60 mg Oxycodone/day | 80 mg Oxycodone/day |
|---|---|---|---|
| 4:1 | 10.2 (3.7) | 11.8 (4.3) | 11.0 (5.6) |
| 3:1 | 13.1 (4.5) | 14.5 (4.8) | 12.5 (6.3) |
| 2:1 | 18.0 (5.7) | 18.2 (4.9) | 12.4 (7.7) |

In addition to estimating the treatment effect for individual oxycodone/naloxone combinations, overall treatment effect estimates were obtained for specific ratios. The estimates were calculated by combining the results from the different oxycodone/naloxone combinations, e.g.; the 2:1 ratio estimate was formed by averaging the predicted results of the 40/20 mg, 60/30 mg, and 80/40 mg oxycodone/naloxone combinations, relative to naloxone placebo. The estimated mean differences (SE) in mean bowel function for various oxycodone/naloxone ratios versus naloxone placebo groups are displayed below.

Table 5: Response Surface Analysis of Bowel Function Efficacy by Oxycodone/Naloxone ratio (Estimated Improvement (SE) vs Naloxone Placebo).

| Oxycodone / Naloxone Ratio | Overall Improvement (SE) vs Placebo |
|---|---|
| 6:1 | 8.0 (3.3) |
| 4:1 | 11.1(4.1) |
| 3:1 | 13.4 (4.6) |
| 2:1 | 16.2 (4.5) |
| 1.5:1 | 16.5 (5.1) |

The estimates indicate that bowel function improvement increases as oxycodone/naloxone ratio decreases, with the estimated improvement at 2:1 approximately 50% higher than at 4:1 ($p < 0.05$) and with a minimal improvement from the 2:1 ratio to the 1.5:1 ratio.

It was thus shown, that the 2/1 and the 1.5/1 ratios demonstrated significant differences compared to the corresponding oxycodone dose plus naloxone placebo at V4 and V5. The oxycodone/naloxone combination provided improvements in ease of defecation, feeling of incomplete bowel evacuation and judgement of constipation. The greatest improvements were seen at dose ratios of 1/1, 1.5/1 and 2/1.

7. Global Assessment-Efficacy, Tolerability and Preference-Results

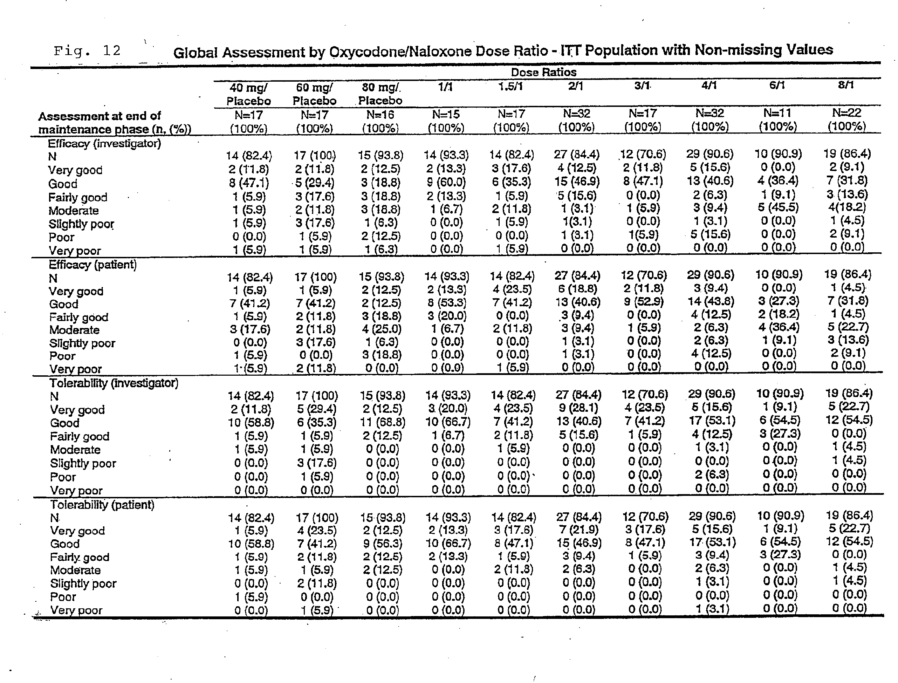

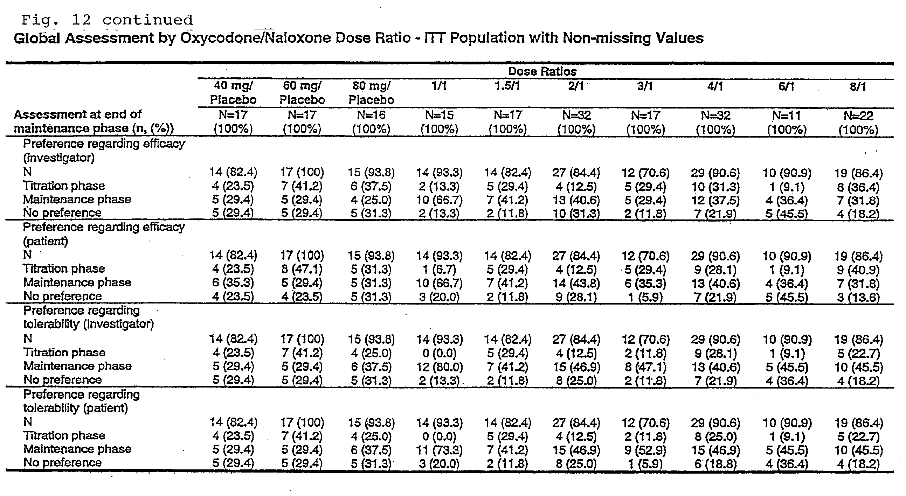

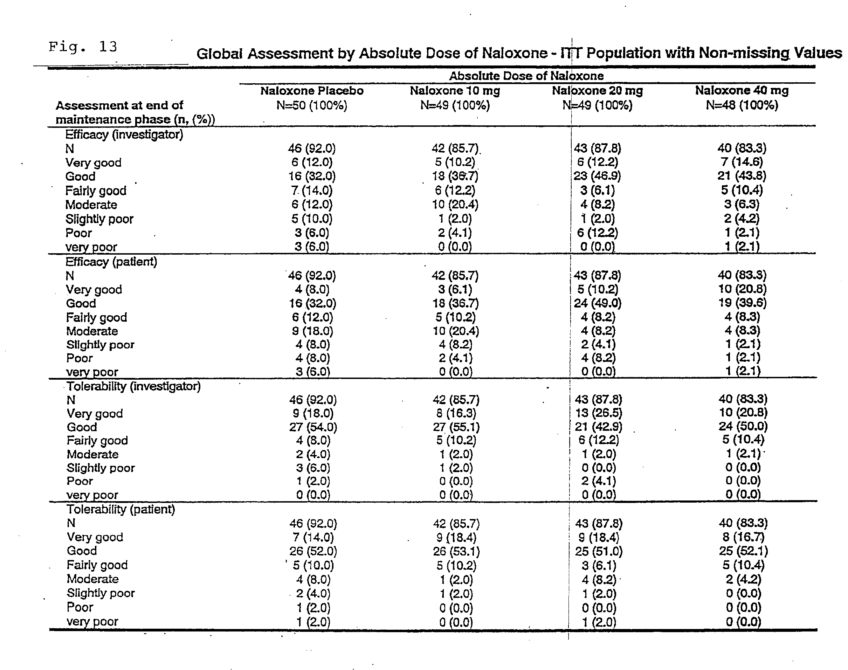

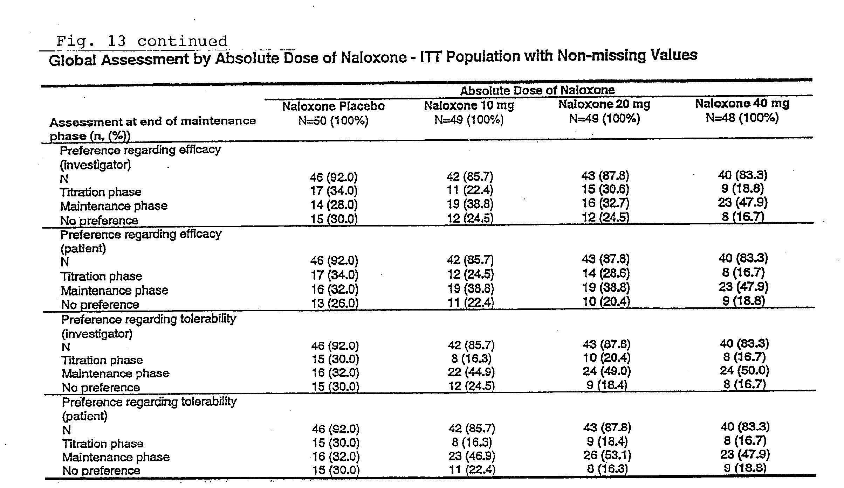

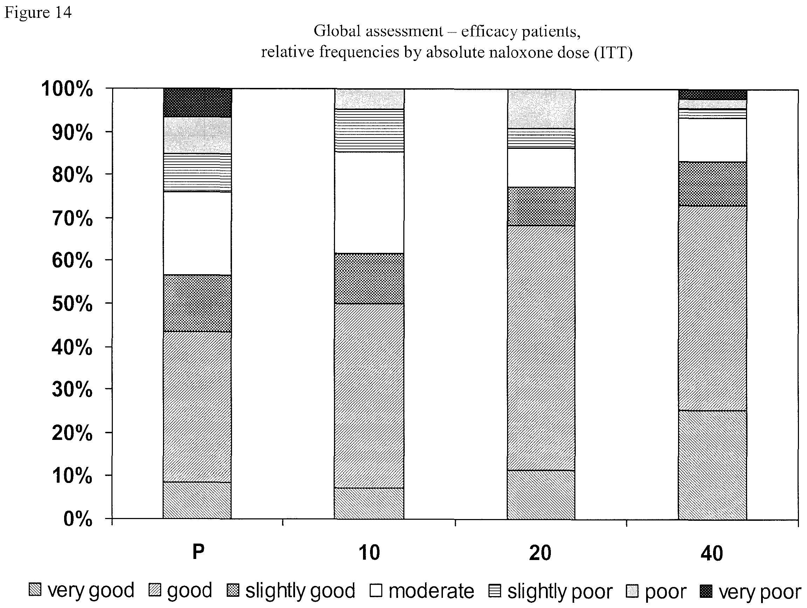

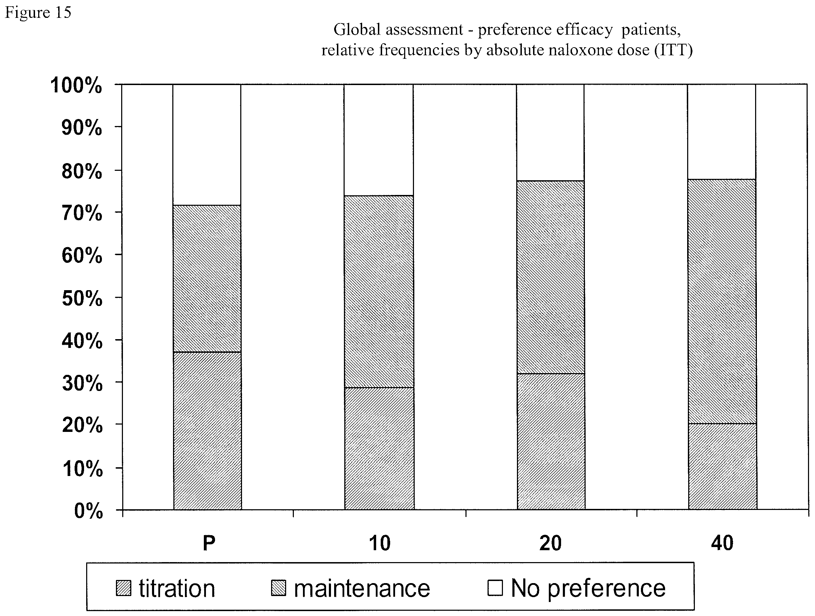

The results for the global assessment of efficacy, tolerability and preference are shown in Figs. 12 to 15. The 1/1 dose ratio was ranked good or very good by more patients and investigators than any other dose ratio. In total, 73.3% of investigators and 66.6% of patients rated the efficacy of the 1/1 dose ratio as good or very good. The 2/1 dose ratio was ranked good or very good by 50.4% of investigators and 59.4% of patients.

A similar trend can be observed for tolerability of medication with 86.7% of investigators and 80% of patients rating the tolerability of the 1/1 dose as good or very good. High ratings were also observed in the 80 mg placebo dose ratio group (81.3% for investigators and 68.8% for patients), 8/1 dose ratio (77.3 for both investigators and patients) and 2/1 dose ratio (68.7% for investigators and 68.8% for patients).

For global preference, the maintenance phase was preferred by the majority of investigators and patients for the 1/1 dose ratio. This was supported by the results obtained in the naloxone 20 mg and 50 mg treatment groups. For naloxone placebo, the distribution of preference between titration, maintenance and no preference was generally even regarding efficacy and tolerability.

8. Subject Opioid Withdrawal Scale Results

Subjects were asked to report the occurrence of opioid withdrawal in their diaries during the first week of treatment with naloxone. These were assessed by rating the above-mentioned 16 symptoms on a scale of 0 (not at all) to 4 (extremely). A total SOWS score ranging from 0 to 64 was computed by summing-up the scores across the 16 symptoms.

The mean sum scores for SOWS are indicated in Table 6 below.

Table 6: Mean sum score for SOWS

| Mean Score | 40 mg Placebo N=17 | 60 mg Placebo N=17 | 80 mg Placebo N=16 | 40 / 20 mg OXN N=16 | 80 / 40 mg OXN N=16 |
|---|---|---|---|---|---|
| Mean | 6.9 | 9.1 | 6.0 | 8.6 | 12.5 |
| Median | 7.3 | 5.3 | 5.5 | 6.6 | 9.2 |
| Minimum | 0.0 | 0.0 | 0.0 | 0.0 | 0.0 |

(continued)

| Mean Score | 40 mg Placebo N=17 | 60 mg Placebo N=17 | 80 mg Placebo N=16 | 40 / 20 mg OXN N=16 | 80 / 40 mg OXN N=16 |
|---|---|---|---|---|---|
| Maximum | 16.9 | 28.9 | 16.7 | 34.5 | 49.5 |

A general trend can be observed that with higher doses of naloxone administered there is a slight increase in the predicted values of maximum total SOWS at a low dose of oxycodone and a moderate increase at higher doses of oxycodone. It is noteworthy that the 2:1 ratio does not indicate additional safety concerns.

9. Laxative Intake/ Laxative Mean Dose Results

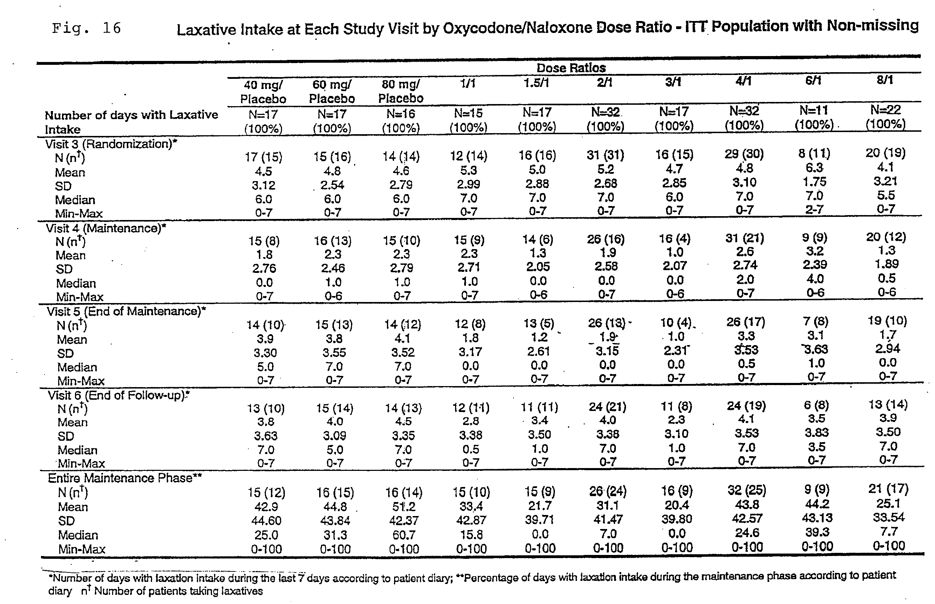

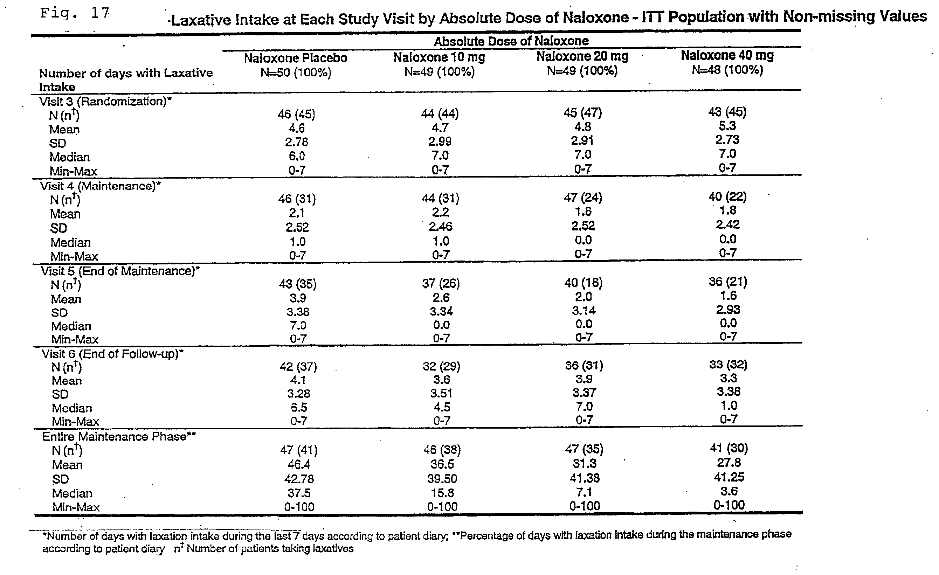

The mean number of days with laxative intake during the last 7 days prior to the end of maintenance decreased with increasing absolute dose of naloxone (3.9 ± 3.38, 2.6 ± 3.34, 2.0 ± 3.14, 1.6 ± 2,93 for placebo, 10 mg, 20 mg and 40 mg naloxone, respectively). The percentage of days (mean ± SD) with laxation during the entire maintenance phase showed a clear decrease for placebo with increasing dose of naloxone. The values being 46.4 ± 42.78, 36.5 ± 33.50, 31.3 ± 41.38 and 27.8 ± 41.25 for placebo, 10 mg, 20 mg and 40 mg naloxone. The mean number of days of laxative intake during the last 7 days prior to the end of maintenance was lowest at the 3/1 ratio and the 1.5/1 ratio. Analysis by absolute dose of naloxone given the same oxycodone/naloxone dose ratio shows no difference between the absolute dose of naloxone within either dose ratio group (4/1 and 2/1). The particulars can be taken from Figs. 16 and 17 and Table 7 below.

Table 7: Laxative Intake (days) by oxycodone/naloxone dose ratio (ITT population)

| Visit Mean (S.D.) | 40 mg Placebo N=17 | 60 mg Placebo N=17 | 80 mg Placebo N=16 | 40 / 20 mg OXN N=16 | 80 / 40 mg OXN N=16 |
|---|---|---|---|---|---|
| Visit 3 - Randomization | 4.5 (3.12) | 4.8 (2.54) | 4.6 (2.79) | 4.8 (2.88) | 5.5 (2.50) |
| Visit 4 - Maintenance 1w | 1.8 (2.76) | 2.3 (2.46) | 2.3 (2.79) | 2.1 (2.71) | 1.6 (26.19) |
| Visit 5 - End maintenance | 3.9 (3.30) | 3.8 (3.55) | 4.1 (3.52) | 1.9 (3.20) | 2.0 (3.22) |
| Visit 6 - End follow up | 3.8 (3.63) | 4.0 (3.09) | 4.5 (3.35) | 4.2 (3.38) | 3.7 (3.53) |

10. Adverse Events - Results

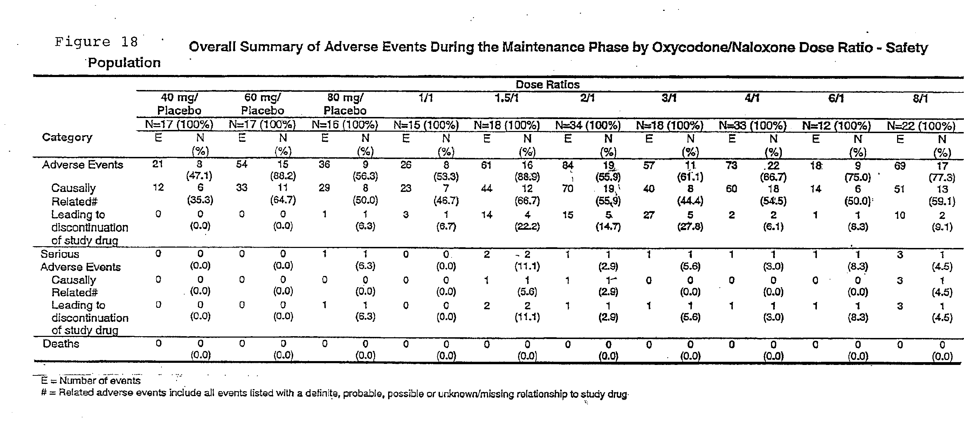

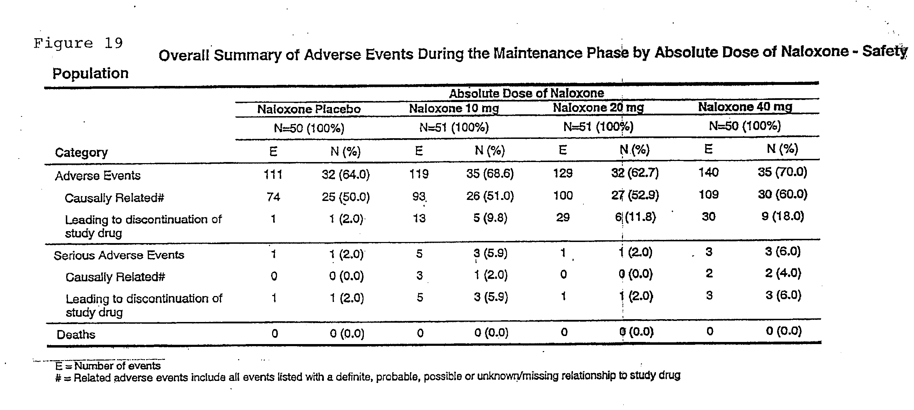

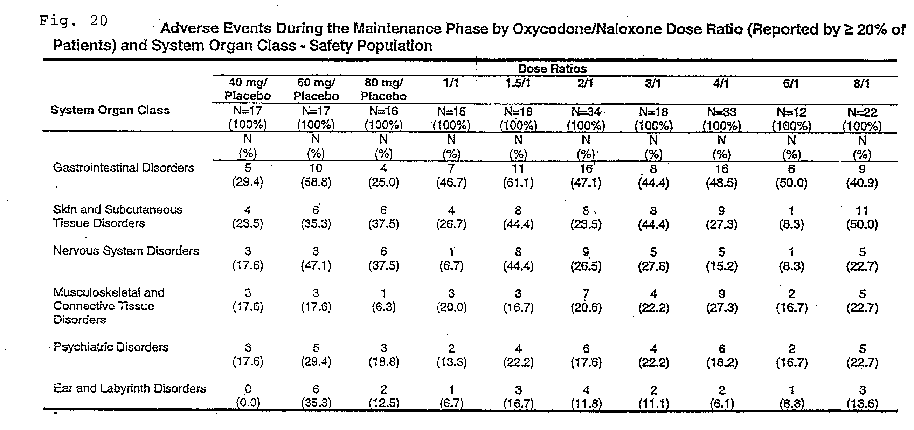

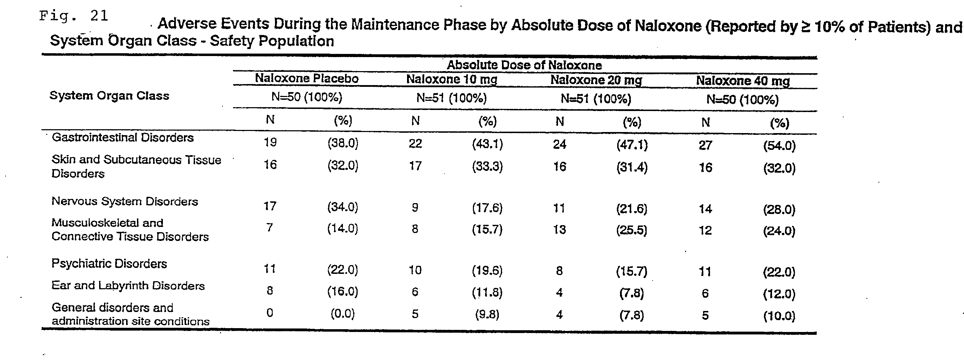

Figs. 18 to 21 provide an overall summary of adverse events during the maintenance phase by oxycodone/naloxone dose ratio and by absolute dose amount of naloxone. The number of patients experiencing any adverse events during the maintenance phase was comparable by absolute dose of naloxone and placebo (range 62.7% - 70%), although the number of events increased with increasing naloxone dose. No relationship to dose ratio could be identified. The incidence of adverse events during the follow-up phase was also comparable between oxycodone dose groups.

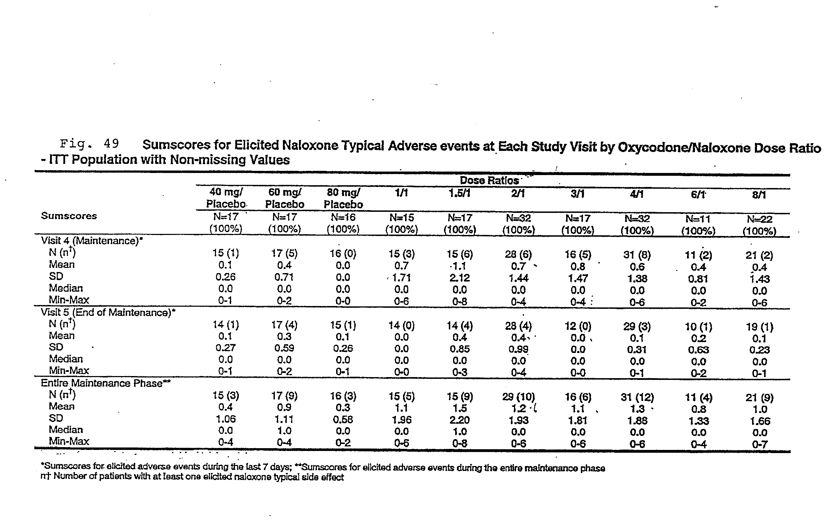

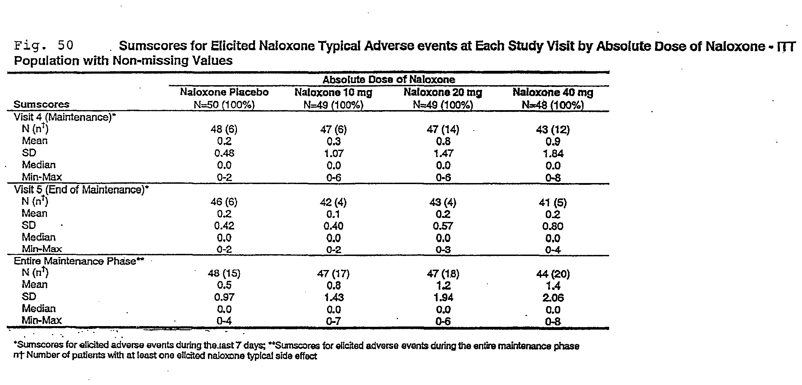

As regards severity of elicited opioid typical adverse events, the mean sum scores were generally low at ech study visit and during the maintenance phase for all treatment groups and dose ratios. During the maintenance phase there was a clear trend for a reduction in mean sum scores for all naloxone treatment groups and naloxone dose ratios when compared to placebo. At the end of the maintenance phase, the mean sum scores were lower in the naloxone treatment groups than in the placebo group wuth a statistically significant difference (p<0.05) for all naloxone treatment groups (see also Figure 49 and 50).

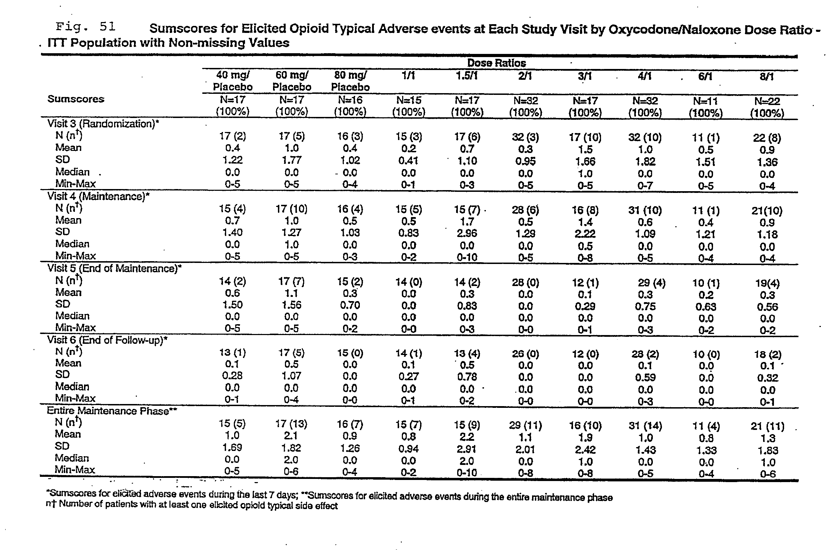

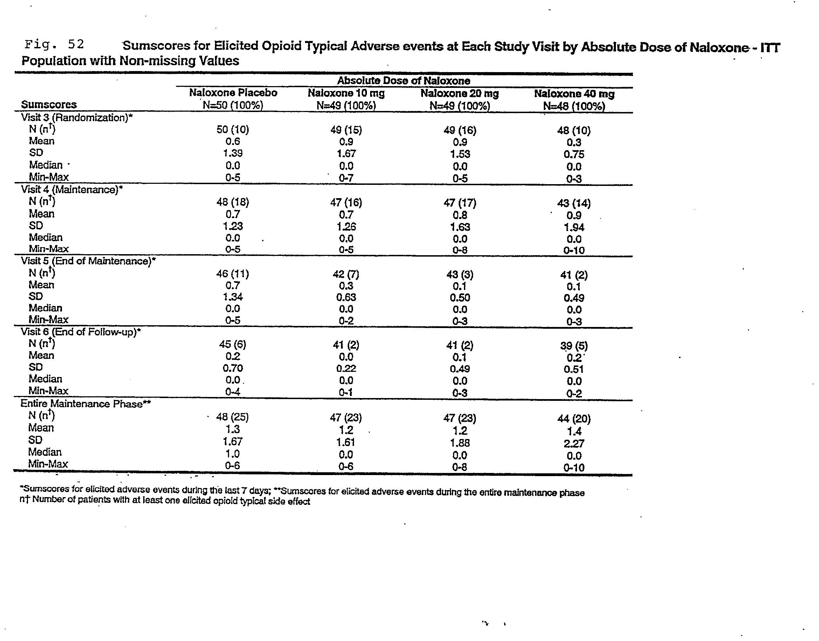

As regards severity of elicited naloxone typical adverse events, there was a trend towards increase mean sumscore with increasing dose of naloxone. However, mean sumscores for naloxone typical adverse events improved during the maintenance phase in allactive naloxone treatment groups and there were no statistically significant differences to placebo for any active naloxone treatment group at the end of the maintenance phase (see Figure 51 and 52).

This could indicate that during steady state elicited opioid typical adverse events are reduced while there is no increase for elicited naloxone typical adverse events if the inventive preparations are used.

11. Incidence of Diarrhea - Results

The number of subjects experiencing diarrhea that began during the maintenance phase was higher in the active naloxone treatment groups with the number of events increasing with higher doses. A trend was observed that with increasing doses of naloxone administered there is an increase in the absolute duration of diarrhea in subjects, who completed the clinical trial.

Nevertheless, comparatively favourable safety data can be detected for the 2:1 ratio of oxycodone and naloxone, whereas the 1.5:1 ratio seems to result in a higher incidence and longer duration of diarrhea.

Table 8 shows that the 2:1 ratio gave comparable results to the placebo.

Table 8: Comparison of days with diarrhea by treatment

| Days of diarrhea | Grouping | | | |
|---|---|---|---|---|
| | OXY/Placebo | OXN 40/20 | OXN 80/40 | OXN total[1] |
| N | 6 (12%) | 5 | 5 | 10 (29%) |
| Mean | 7.3 | 2.0 | 5.6 | 3.8 |
| Median | 5.5 | 1.0 | 2.0 | 2.0 |
| Minimum | 1.0 | 1.0 | 1.0 | 1.0 |
| Maximum | 20.0 | 5.0 | 22.0 | 22.0 |
| [1]2:1 ratio | | | | |

The same can be observed with respect to the incidence of discontinuations from the study due to diarrhea (see Table 9).

Table 9: Incidence of Discontinuations due to Diarrhea

| Total Daily Oxycodone Dose (mg) / Total Daily Naloxone Dose (mg) | 40 | 60 | 80 |
|---|---|---|---|
| 0 | 0/17 (0.0%) | 0/17 (0.0%) | 0/16 (0.0 %) |
| 10 | 0/17 (0.0%) | 0/12 (0.0%) | 1/22 (4.5%) |
| 20 | 1/17 (5.9%) | 3/18 (16.7%) | 0/16 (0.0%) |
| 40 | 1/15 (6.7%) | 3/18 (16.7%) | 2/17 (11.8%) |

12. Study Conclusions

While the study was not designed nor powered as a formal demonstration of non-inferiority of oxycodone/naloxone versus oxycodone/naloxone placebo, the administration of prolonged oxycodone and naloxone in combination was not associated descriptively with differences in the intensity of mean pain whether analyzed by dose ratios or absolute dose of naloxone.

The study demonstrated that addition of controlled release naloxone to controlled release oxycodone results in a statistically significant improvement in mean bowel function at the two higher doses of naloxone (20mg and 40mg). The improvement increases with decreasing oxycodone/naloxone ratio and appears to plateau at the 2:1 ratio, with the overall effect at 2:1 ratio approximately 50% greater than at 4:1. The data indicate that the bowel function improvement is in general a function of the ratio; i.e., the improvement is, in general, constant within each ratio, and independent of the varying doses of oxycodone and naloxone. The only exception is the 80/40 combination, where there is a suggestion of a lower predicted effect than for the 60/30 mg and 40/20 mg combinations.

The greatest improvements were seen at dose ratios of 1/1, 1.5/1 and 2/1 on absolute dose of 40 mg. Model estimates of oral treatment effect for specific ratios show minimal improvement in bowel function between the 2/1 ratio and the 1.5/1 ratio, suggesting that the improvement in bowel function reaches a plateau at the 2/1 ratio.

A global assessment of efficacy and tolerability indicated an overall preference towards the 1/1 dose ratio for both investigators and patients. The 80 mg oxycodone/placebo, 8/1 and 2/1 dose ratios also had a high tolerability. The global assessment of preference also indicated that the majority of patients and investigators preferred the maintenance phase for the 1/1 dose ratio, but formed the 2/1 ratio also as suitable.

The incidence of naloxone- and opioid-typically adverse effects were summarized by sum scores for incidence and severity.

Most reported adverse events were those known to be associated with naloxone or oxycodone and diarrhea was the most frequently reported adverse event that increased with higher doses of naloxone. Diarrhea was the most common causally related adverse event and adverse event. The incidence of diarrhea was substantially reduced from the 1.5/1 to the 2/1 dose ratio. Diarrhea can be regarded as a typical withdrawal symptom for patients with opioid-induced constipation, who receive an opioid antagonist.

In summary, it seems that, if all aspects of treatment are taken into account, i.e. reduction of pain intensity, improvement of BFI, occurrence of adverse effect, avoidance of diarrhea and tolerability and preference, the 2/1 ratio seems to be the best choice. Within the 2/1 ratio, the 40/20 mg dose seems particularly suitable.

**Example 2: Pharmacokinetic and bioavailability characteristics of different strengths of a fixed combination of oxycodone and naloxone and a combination of Oxygesic® plus Naloxone CR**

1. Objective

The objectives of this study were to (i) evaluate the pharmacokinetic and bioavailability parameters of oxycodone and naloxone and their main metabolites when administered as a controlled-release fixed combination tablet formulation; (ii) assess the interchangeability between the 3 different strengths of the fixed combination, OXN 10/5, OXN 20/10 and OXN 40/20; and (iii) compare the pharmacokinetics and bioavailability of the fixed combination formulation with marketed Oxygesic® given together with Naloxone CR tablets;

2. Test population

A total of 28 healthy adult, male and female subjects were randomized to receive the study drugs with the aim that 24 subjects would complete the study and provide valid pharmacokinetic data.

*Inclusion Criteria*

Subjects who were included in the study were those who met all of the following criteria:

- Males or females of any ethnic group;
- Aged between ≥18 and ≤45 years;
- BMI within the range 19 - 29 kg/m$^2$, and within the weight range 60 - 100 kg for males and 55 - 90 kg for females;
- Females must be non-nursing, non-pregnant, and provide a negative urine β-hCG pregnancy test within 24 hours before receiving the study medication. Female subjects of childbearing potential must be using a reliable form of contraception (e.g. intrauterine device, oral contraceptive, barrier method). Female subjects who were postmenopausal must have been postmenopausal for ≥1 year and, in the absence of HRT, have elevated serum FSH;
- Generally good health, evidenced by a lack of significantly abnormal findings on medical history, physical examination, clinical laboratory tests, vital signs, and ECG. Vital signs (after 3 minutes resting in a supine position) must be within the following ranges: oral body temperature between 35.0 - 37.5 °C; systolic blood pressure, 90 - 140 mmHg; diastolic blood pressure, 50 - 90 mmHg; and pulse rate, 40 - 100 bpm. Blood pressure and pulse were taken again after 3 minutes in a standing position. After 3 minutes standing from a supine position, there should be no more than a 20 mmHg drop in systolic blood pressure, 10 mmHg drop in diastolic blood pressure, and no greater than 20 bpm increase in pulse rate; Written informed consent obtained; Willing to eat all the food supplied during the study.

*Exclusion Criteria*

Subjects who were excluded from the study were those who met any of the following criteria:

- Exposure to any investigational drug or placebo within 3 months of their first dose of study medication;
- Any significant illness within the 30 days before their first dose of study medication;
- Any clinically significant abnormalities identified at prestudy screening for medical history, physical examination or laboratory analyses;
- Use of any prescription medication (except HRT for postmenopausal females and contraceptive medication) in the 21 days, or over the counter medication including acid controllers, vitamins, herbal products and/or mineral supplements in the 7 days, before their first dose of study medication;
- Concurrent medical condition known to interfere with gastrointestinal drug absorption (e.g. delayed gastric emptying, mal absorption syndromes), distribution (e.g. obesity), metabolism or excretion (e.g. hepatitis, glomerulonephritis);
- History of, or concurrent medical condition, which in the opinion of the Investigator would compromise the ability of the subject to safely complete the study;
- History of seizure disorders for which subjects required pharmacologic treatment;
- Current history of smoking more than 5 cigarettes a day;
- Subjects with evidence of active or past history of substance or alcohol abuse, according to DSM-IV criteria3, or subjects who, In the investigator's opinion, have demonstrated addictive or substance abuse behaviors;
- Subjects who reported regular consumption of 2 or more alcoholic drinks per day or have blood alcohol levels of ≥0.5% at screening;
- Donation of more than 500 mL of blood or blood products or other major blood loss in the 3 months before their first dose of study medication;
- At risk of transmitting infection via blood samples such as producing a positive HIV test at screening or having participated in a high risk activity for contracting HIV; producing a positive Hepatitis B surface antigen test at screening; producing a positive Hepatitis C antibody test at screening;
- Any positive results in the prestudy screen for ethanol, opiates, barbiturates, amphetamines, cocaine metabolites, methadone, propoxyphene, phencyclidine, benzodiazepines, and cannabinoids in the specimen of urine collected at screening;
- Known sensitivity to oxycodone, naloxone, or related compounds;
- Contraindications and precautions as detailed in the datasheet for Oxygesic@;
- Refusal to allow their primary care physician (if applicable) to be informed;
- The Investigator believed the subject to be unsuitable for a reason not specifically stated in the exclusion criteria.

The demographic data are shown in Table 10.

Table 10: Subject Demographics and Other Baseline Characteristics: Safety Population

|  |  | Male (N = 22) | Female (N = 6) | Overall (N = 28) |
|---|---|---|---|---|
| Characteristics Race, n (%) |  |  |  |  |
|  | Caucasian | 22 (100%) | 6 (100%) | 28 (100%) |
| Age (y) |  |  |  |  |
|  | Mean ± SD | 32.6 ± 5.28 | 31.0 + 6.32 | 32.3 + 5.44 |
|  | Range (min, max) | 25,41 | 24,42 | 24,42 |
| Height (cm) |  |  |  |  |
|  | Mean ± SD | 179.1 ± 4.84 | 168.0 ± 8.72 | 176.7 + 7.33 |
|  | Range (min, max) | 165,187 | 159,181 | 159,187 |
| Weight (kg) |  |  |  |  |
|  | Mean ± SD | 77.8 + 9.04 | 67.0 + 3.03 | 75.5 + 9.25 |
|  | Range (min, max) | 62,97 | 63,71 | 62,97 |
| Body Mass Index (kg/m$^2$) |  |  |  |  |
|  | Mean ± SD | 24.2 ± 2.56 | 23.9 + 2.50 | 24.2 ± 2.50 |
|  | Range (min, max) | 20,29 | 20,27 | 20,29 |

3. Study Design, Test Treatment Dose and Mode of Administration

*Preparation of tested products*

A melt extrusion oxycodone/naloxone controlled-release tablet formulation with an oxycodone:naloxone ratio of 2:1 was produced. There are three dose strengths available, namely OXN 10/5, OXN 20/10, and OXN 40/20, where the first number is the mg amount of oxycodone hydrochloride and the second number is the mg amount of naloxone hydrochloride (see Table 12). OXN 20/10 and OXN 40/20 are from the same granulate, while OXN 10/5 has a slightly different formula in regard to the ratio of active ingredients to excipients.

Oxycodone/naloxone tablets (OXN Tablets) according to this example contain a fixed combination of oxycodone and naloxone in the ratio of 2:1. Tablets formulations are summarized below (see Table 12).

The 20/10 mg and 40/20 mg tablets will be manufactured from the same granulation with these two tablet strengths being compositionally proportional. Oxycodone/Naloxone prolonged release tablets (OXN) tablets according to this example are controlled release tablets using a matrix of stearyl alcohol and ethylcellulose as the retardant. The tablets contain the combination of oxycodone hydrochloride and naloxone hydrochloride in the strengths 10/5 mg, 20/10 mg and 40/20 mg (both as the hydrochloride). The complete statement of the components and quantitative composition of Oxycodone/Naloxone prolonged release tablets is given below in Table 11.

Table 11: Oxycodone/Naloxone prolonged release tablets.

| Component | Quantity (mg/tablet) | | | Function | Reference to Standard |
|---|---|---|---|---|---|
| | OXN 10/5 | OXN 20/10 | OXN 40/20 | | |
| Oxycodone hydrochloride 1) | 10.50 | 21.00 | 42.00 | Active | USP*/ H.S.E. |
| *corresponding to* | | | | | |
| *Oxycodone hydrochloride anhydrous* | *10.00* | *20.00* | *40.00* | | |
| *Oxycodone base* | *9.00* | *18.00* | *36.00* | | |
| Naloxone hydrochloride Dihydrate | 5.45 | 10.90 | 21.80 | Active | Ph. Eur. * |
| *corresponding to* | | | | | |
| *Naloxone hydrochloride anhydrous* | *5.00* | *10.00* | *20.00* | | |
| *Naloxone base* | *4.50* | *9.00* | *18.00* | | |
| Povidone K30 | 5.00 | 7.25 | 14.50 | Binder | Ph. Eur. * |
| Ethylcellulose N 45 | 10.00 | 12.00 | 24.00 | Retardant | Ph. Eur. * |
| Stearyl alcohol | 25.00 | 29.50 | 59.00 | Retardant | Ph. Eur. * |
| Lactose monohydrate | 64.25 | 54.50 | 109.00 | Diluent | Ph. Eur. * |
| Purified talc | 2.50 | 2.50 | 5.00 | Glidant | Ph. Eur. * |
| Magnesium stearate | 1.25 | 1.25 | 2.50 | Lubricant | Ph. Eur. * |
| **Total core** | **123.95** | **138.90** | **277.80** | | |
| **Film Coat °** | | | | | |
| Opadry II HP white - 85F18422 | 3.72 | | | Coating | supplier specificati on |
| Opadry II HP pink - 85F24151 | | 4.17 | | Coating | supplier specificati on |
| Opadry II HP yellow 85F32109 | | | 8.33 | Coating | supplier specificati on |
| Purified talc | 0.12 | 0.14 | 0.28 | Gloss | Ph. Eur. * |
| **Total filmtablet** | **127,79** | **143.21** | **286.41** | | * current Edition |
| 1) calculated based on expected moisture content ° qualitative composition : see Table 12 | | | | | |

Table 12: Qualitative composition of the film coat.

| Opadry II HP | white 85F18422 | pink 85F24151 | yellow 85F32109 | Reference to Standard |
|---|---|---|---|---|
| Polyvinylalcohol part. hydrolized | + | + | + | Ph. Eur. * |
| Titanium dioxide (E 171) | + | + | + | Ph. Eur. * |
| Macrogol 3350 | + | + | + | Ph. Eur. * |
| Talcum | + | + | + | Ph. Eur. * |
| Iron oxide red (E 172) | | + | | NF* /EC Directive |
| Iron oxide yellow (E 172) | | | + | NF* /EC Directive |
| | | | | * current Edition |

*Study Design*

The study was an open-label, single dose, 4-treatment, 4-period, randomized across over study and healthy subjects. The treatments were given orally in the fasted state as follows:

- Treatment A: 4 x tablets of Oxn 10/5
- Treatment B: 2 x tablets of Oxn 20/10
- Treatment C: 1 x tablets of Oxn 40/20

The reference treatment was an Oxygesic® 20 mg tablet. Naloxone was used in the form of Naloxone 10 mg CR spray granulation tablet. Reference treatment was thus

- Treatment D: 2 tablets of Oxygesic® 20 mg and two tablets of Naloxone CR 10 mg.

Duration of treatment included 21 days screening period and four study periods each with a single dose of study drug followed by a seven day wash-out period. There were post study medical 7 to 10 days after dosing of study period 4 and there were 7 to 10 days after discontinuation from the study. The total duration was 49 to 52 days.
The treatment schedule was a single dose of study drug in each of the four study periods. Each dose of study drug was separated by a 7 day wash-out period.
The enrolled population was defined as the subject population that provided the written informed consent to anticipate in the study. The full analysis population for pharmacokinetics was defined as those subjects, who had at least one valid pharmacokinetic parameter calculated on at least one treatment.

4. Pharmacokinetic Assessments

*Drug Concentration Measurements*

Blood samples for determining oxycodone, noroxycodone, oxymorphone, noroxymorphone, naloxone, 6β-naloxol and naloxone-3-glucuronide concentrations were obtained for each subject during each of the 4 study periods immediately before dosing; and at 0.5, 1, 1.5, 2, 2.5, 3, 3.5, 4, 5, 6, 8, 10, 12, 16, 24, 28, 32, 36, 48, 72 and 96 hours (22 blood samples per study period) after dosing. Blood was also drawn where possible at the first report of a serious or severe unexpected adverse event and at its resolution.
At each time of plasma determination, 6 mL venous blood was drawn from a forearm vein into a tube containing K2 EDTA anticoagulant. All samples were processed according to common sample handling procedures.

*Pharmacokinetic Parameters*

The following pharmacokinetic parameters were calculated from the plasma concentrations of oxycodone, noroxycodone, oxymorphone, noroxymorphone, naloxone, 6β-naloxol and naloxone-3 -glucuronide:

- Area under the plasma concentration time curve calculated to the last measurable concentration (AUCt);
- Area under the plasma concentration-time curve, from the time of administration to infinity (AUCINF);

- Maximum observed plasma concentration ($C_{max}$);
- Time point of maximum observed plasma concentration (tmax);
- Terminal phase rate constant (LambdaZ);
- Apparent terminal phase half life (t½Z).

For oxycodone, noroxycodone, oxymorphone, noroxymorphone, and naloxone-3-glucuronide, AUC values were given in ng·h/mL, and $C_{max}$ values in ng/mL. For naloxone and 6β-naloxol, AUC values, due to the low concentrations, were given in pg·h/mL and $C_{max}$ values in pg/mL.

AUCt, AUCINF and $C_{max}$ were regarded as the primary parameters.

AUCt were calculated using the linear trapezoidal method. Where possible, LambdaZ was estimated using those points determined to be in the terminal log-linear phase. t½Z was determined from the ratio of ln 2 to LambdaZ. The areas under the plasma concentration-time curve between the last measured point and infinity were calculated from the ratio of the final observed plasma concentration ($C_{last}$) to LambdaZ. This was then added to the AUCt to yield AUCINF

All pharmacokinetic calculations were performed with WinNonlin Enterprise Edition, Version 4.1.

*Statistical Methods*

$C_{max}$ and AUCINF of oxycodone were important in order to assess the equivalence of the 4 treatments. AUCt was calculated using the linear trapezoidal method. Where possible, LambdaZ was estimated using those points determined to be in the terminal log-linear phase. t½Z were determined from the ratio of ln 2 to LambdaZ. The areas under the plasma concentration-time curve between the last measured point and infinity were calculated from the ratio of the final observed plasma concentration ($C_{last}$) to LambdaZ. This was added to the AUCt to yield the area under the plasma concentration-time curve between the time of administration and infinity (AUCINF).

The dose adjusted relative systemic availabilities (Frelt, and FrelINF) and the $C_{max}$ ratio were obtained from the ratio of AUCt, AUCINF and $C_{max}$ values, respectively, for differences defined in the following comparisons of interest:

fixed combination A vs. open combination D
fixed combination B vs. open combination D
fixed combination C vs. open combination D
fixed combination A vs. fixed combination B
fixed combination A vs. fixed combination C
fixed combination B vs. fixed combination C

The full analysis population for pharmacokinetics were used for these analyses.

The metabolite: parent drug AUCt and AUCINF ratios were estimated for each treatment, where possible.

5. Clinical Pharmacology Results

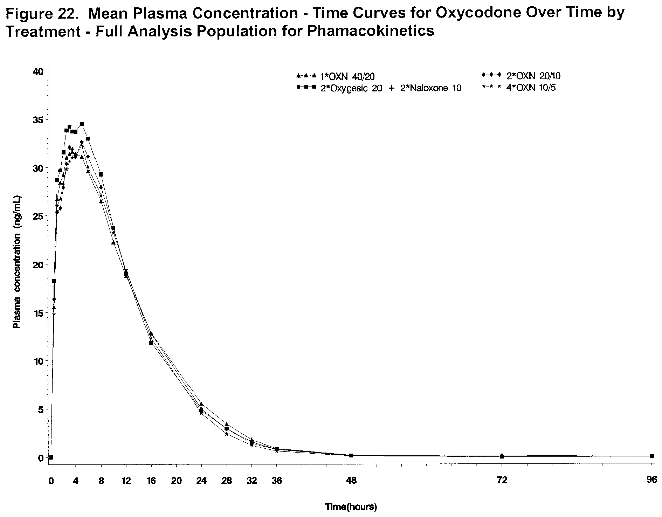

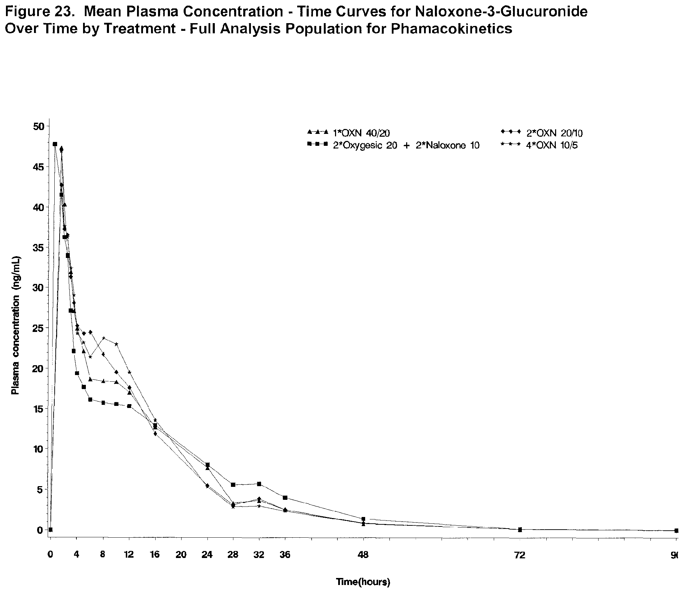

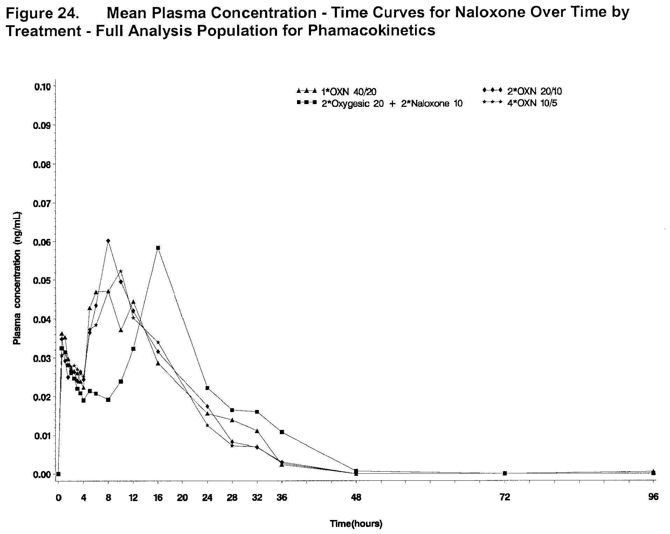

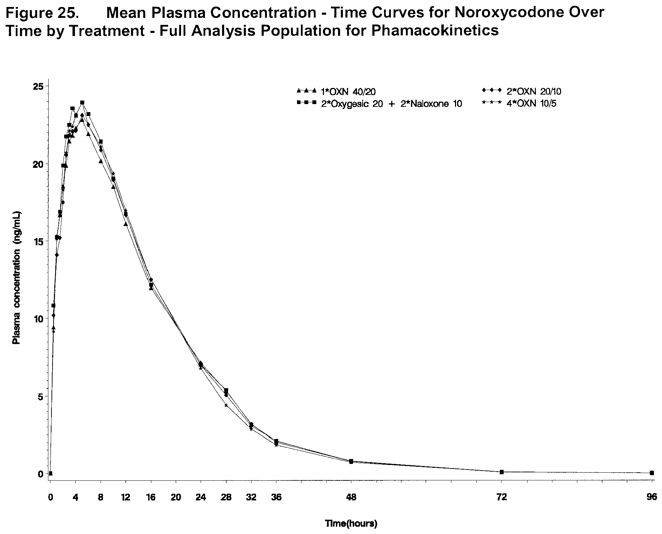

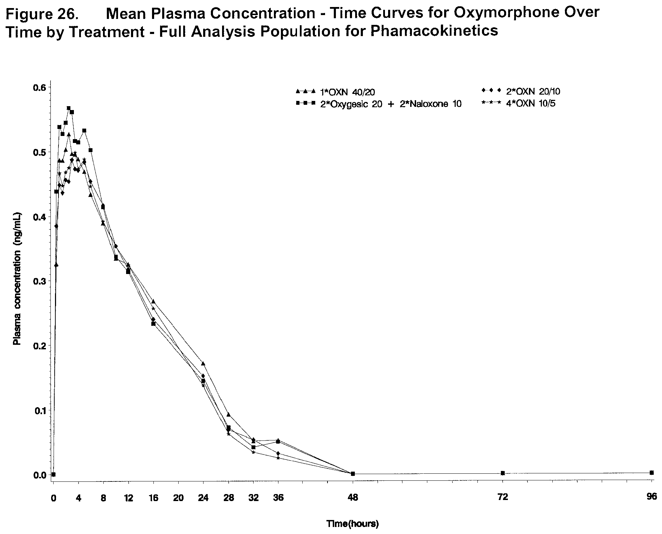

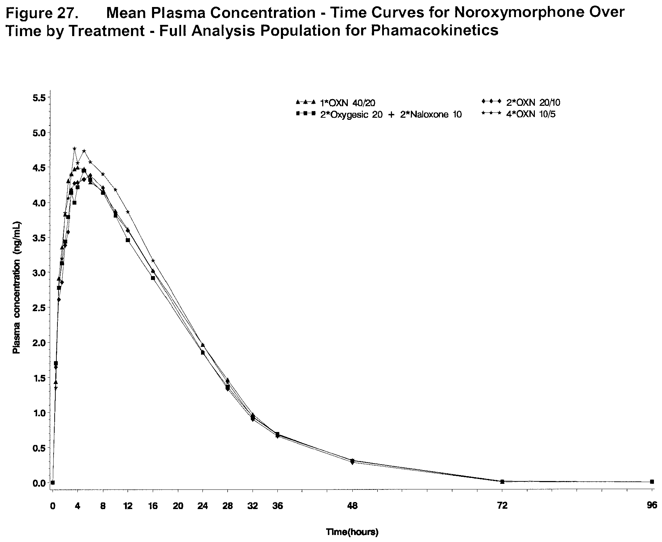

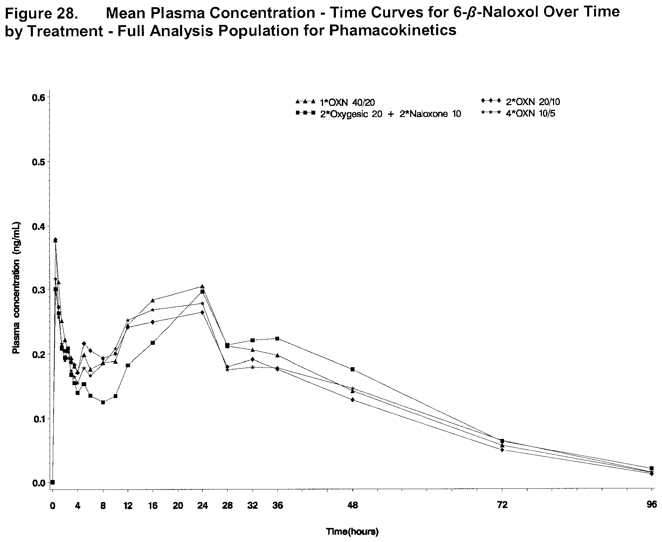

Mean observed plasma concentration - time curves for oxycodone, naloxone-3-glucuronide, naloxone, noroxycodone, oxymorphone, noroxymorphone and 6-β-naloxol are presented in Figures 22 to 28.

Pharmacokinetic parameters for oxycodone, naloxone-3-glucuronide and naloxone are presented in Tables 13 to 26 respectively.

Table 13: Summary of Pharmacokinetic Parameters for Oxycodone by Treatment: Full Analysis Population for Pharmacokinetics

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (ng.h/mL)** | | | | |
| N | 24 | 23 | 23 | 23 |
| Arithmetic Mean | 473.49 | 491.22 | 488.89 | 502.28 |
| (SD) | (72.160) | (82.181) | (91.040) | (84.128) |
| Geometric Mean | 468.29 | 484.58 | 481.08 | 495.72 |
| **AUCINF (ng.h/mL)** | | | | |
| N | 24 | 22 | 22 | 22 |
| Arithmetic Mean | 475.06 | 497.17 | 491.22 | 509.11 |

(continued)

| AUCINF (ng.h/mL) | | | | |
|---|---|---|---|---|
| (SD) | (72.182) | (81.687) | (93.458) | (82.963) |
| Geometric Mean | 469.87 | 490.65 | 483.04 | 502.80 |
| **Cmax (ng/mL)** | | | | |
| N | 24 | 23 | 23 | 23 |
| Arithmetic Mean | 34.91 | 35.73 | 34.46 | 40.45 |
| (SD) | (4.361) | (4.931) | (5.025) | (4.706) |
| Geometric Mean | 34.66 | 35.41 | 34.12 | 40.19 |
| **tmax (h)** | | | | |
| N | 24 | 23 | 23 | 23 |
| Median | 3.5 | 4.0 | 3.0 | 2.5 |
| (Min,Max) | (1.0,6.0) | (2.0,8.0) | (1.0,6.0) | (0.5,8.0) |
| **t1/2Z** | | | | |
| N | 24 | 22 | 22 | 22 |
| Arithmetic Mean | 4.69 | 4.87 | 4.83 | 5.01 |
| (SD) | (0.775) | (0.995) | (0.975) | (0.802) |

Table 14. Oxycodone Summary of Ratios for AUCt, AUCINF, $C_{max}$ and Differences for $t_{max}$ and Half-Life - Full Analysis Population for Pharmacokinetics.

| Pharmacokinetic metric | 4 x OXN 10/5 /2 x Oxygesic 20 + 2 x Naloxone 10 | 2 x OXN 20/10 /2 x Oxygesic 20 + 2 x Naloxone 10 | 1 x OXN 40/20 /2 x Oxygesic 20 + 2 x Naloxone 10 | 4 x OXN 10/5 / 2 x OXN 20/10 | 4 x OXN 10/5/1 x OXN 40/20 | 2 x OXN 20/10 / 1 x OXN 40/20 |
|---|---|---|---|---|---|---|
| **AUCt (ng.h/mL)** | | | | | | |
| Ratio (%) | 94.9 | 98.2 | 98.0 | 96.7 | 96.8 | 100.2 |
| 90%CI | 91.5,98.5 | 94.5, 102.0 | 94.4,101.7 | 93.1,100.4 | 93.3, 100.5 | 96.5, 104.0 |
| **AUCINF(ng.h/mL)** | | | | | | |
| Ratio (%) | 94.5 | 98.2 | 97.8 | 96.2 | 96.5 | 100.4 |
| 90%CI | 90.9,98.1 | 94.5,102.1 | 94.1,101.7 | 92.6,99.9 | 92.9,100.3 | 96.5,104.3 |
| **Cmax (ng/mL)** | | | | | | |
| Ratio (%) | 86.2 | 88.4 | 85.8 | 97.5 | 100.5 | 103.1 |
| 90%CI | 82.2,90.4 | 84.2,92.8 | 81.8,90.0 | 92.9, 102.3 | 95.8,105.4 | 98.2,108.1 |
| **tmax (h)** | | | | | | |
| Difference | 0.49 | 1.11 | 0.14 | -0.63 | 0.35 | 0.97 |
| 90%CI | -0.19,1.16 | 0.42,1.80 | -0.54,0.82 | -1.31,0.05 | -0.33,1.02 | 0.29,1.66 |
| **t1/2Z (h)** | | | | | | |
| Difference | -0.27 | -0.11 | -0.11 | -0.16 | -0.16 | 0.00 |
| 90%CI | -0.60,0.05 | -0.44,0.23 | -0.44,0.22 | -0.49,0.16 | -0.49,0.16 | -0.33,0.33 |

Table 15. Summary of Pharmacokinetic Parameters for Naloxone-3-glucuronide by Treatment: Full Analysis Population for Pharmacokinetics.

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (pg.h/mL)** | | | | |
| N | 24 | 23 | 23 | 23 |

(continued)

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (pg.h/mL)** | | | | |
| Arithmetic Mean | 539.93 | 522.45 | 520.10 | 523.37 |
| (SD) | (142.241) | (128.569) | (133.175) | (119.752) |
| Geometric Mean | 520.14 | 506.63 | 502.26 | 509.38 |
| **AUCINF(pg.h/mL)** | | | | |
| N | 22 | 21 | 22 | 22 |
| Arithmetic Mean | 562.53 | 520.97 | 527.94 | 537.25 |
| (SD) | (130.732) | (133.172) | (135.424) | 110.829 |
| Geometric Mean | 546.73 | 504.34 | 509.62 | 525.91 |
| **Cmax (pg/mL)** | | | | |
| N | 24 | 23 | 23 | 23 |
| Arithmetic Mean | 62.01 | 63.62 | 61.95 | 63.55 |
| (SD) | (15.961) | (19.511) | (18.369) | (16.748) |
| Geometric Mean | 59.93 | 60.70 | 59.34 | 61.55 |
| **tmax (h)** | | | | |
| N | 24 | 23 | 23 | 23 |
| Median | 1.0 | 0.5 | 1.0 | 1.0 |
| (Min,Max) | (0.5, 3.0) | (0.5, 6.0) | (0.5, 3.0) | (0.5, 6.0) |
| **t1/2Z** | | | | |
| N | 22 | 21 | 22 | 22 |
| Arithmetic Mean | 8.48 | 7.93 | 7.81 | 7.66 |
| (SD) | (3.066) | (2.402) | (2.742) | (1.717) |

Table 16. Naloxone-3-Glucuronide Summary of Ratios for AUCt, AUCINF, $C_{max}$ and Differences for $T_{max}$ and Half-Life - Full Analysis Population for Pharmacokinetics.

| Pharmacokinetic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5 / 2 x OXN 20/10 | 4 x OXN 10/5/1 x OXN 40/20 | 2 x OXN 20/10 / 1 x OXN 40/20 |
|---|---|---|---|---|---|---|
| **AUCt (pg.h/mL)** | | | | | | |
| Ratio (%) | 101.0 | 98.8 | 98.6 | 102.2 | 102.4 | 100.2 |
| 90%CI | 95.6, 106.8 | 93.4, 104.5 | 93.3, 104.3 | 96.7, 108.1 | 97.0, 108.2 | 94.8, 105.9 |
| **AUCINF(pg.h/mL)** | | | | | | |
| Ratio (%) | 102.1 | 98.2 | 99.0 | 104.0 | 103.1 | 99.2 |
| 90%CI | 96.3, 108.3 | 92.3, 104.2 | 93.4, 105.0 | 97.9, 110.5 | 97.3, 109.3 | 93.5, 105.2 |
| **Cmax (pg/mL)** | | | | | | |
| Ratio (%) | 95.4 | 96.5 | 95.1 | 98.8 | 100.3 | 101.5 |
| 90%CI | 88.5, 102.8 | 89.4, 104.1 | 88.2, 102.5 | 91.7, 106.6 | 93.1, 108.0 | 94.1, 109.3 |

(continued)

| tmax (h) | | | | | | |
|---|---|---|---|---|---|---|
| Difference | -0.34 | -0.16 | -0.42 | -0.18 | 0.08 | 0.26 |
| 90%CI | -0.84,0.17 | -0.67,0.35 | -0.93,0.10 | -0.69,0.33 | -0.43,0.59 | -0.26, 0.77 |
| t1/2Z (h) | | | | | | |
| Difference | 0.87 | 0.37 | 0.32 | 0.50 | 0.56 | 0.06 |
| 90%CI | -0.02, 1.77 | -0.53, 1.28 | -0.58, 1.21 | -0.41, 1.41 | -0.33, 1.45 | -0.85, 0.96 |

Table 17. Summary of Pharmacokinetic Parameters for Naloxone by Treatment: Full Analysis Population for Pharmacokinetics.

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| AUCt (pg.h/mL) | | | | |
| N | 24 | 23 | 23 | 23 |
| Arithmetic Mean | 0.84 | 0.89 | 0.87 | 0.97 |
| (SD) | (0.656) | (0.749) | (0.718) | (0.976) |
| Geometric Mean | 0.67 | 0.70 | 0.68 | 0.72 |
| AUCINF(pg.h/mL) | | | | |
| N | 2 | 6 | 0 | 1 |
| Arithmetic Mean | - | 1.64 | - | - |
| (SD) | - | (1.043) | - | - |
| Geometric Mean | - | 1.45 | - | - |
| Cmax (pg/mL) | | | | |
| N | 24 | 23 | 23 | 23 |
| Arithmetic Mean | 0.07 | 0.08 | 0.08 | 0.08 |
| (SD) | (0.065) | (0.106) | (0.071) | (0.101) |
| Geometric Mean | 0.06 | 0.06 | 0.06 | 0.06 |
| tmax (h) | | | | |
| N | 24 | 23 | 23 | 23 |
| Median | 4.0 | 5.0 | 2.0 | 1.0 |
| (Min,Max) | (0.5, 12.0) | (0.5, 24.0) | (0.5, 12.0) | (0.5, 24.0) |
| t1/2Z | | | | |
| N | 4 | 9 | 4 | 4 |
| Arithmetic Mean | 9.89 | 12.85 | 13.83 | 11.02 |
| (SD) | (3.137) | (11.924) | (1.879) | (1.075) |

Table 18. Naloxone Summary of Ratios for AUCt, AUCINF, $C_{max}$ and Differences for $T_{max}$ and Half-Life - Full Analysis Population for Pharmacokinetics.

| Pharmacoki netic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5/ 2 x OXN 20/10 | 4 x OXN 10/5/1 x OXN 40/20 | 2 x OXN 20/10 / 1 x OXN 40/20 |
|---|---|---|---|---|---|---|
| AUCt (pg.h/mL) | | | | | | |
| Ratio (%) | 94.2 | 99.4 | 94.1 | 94.7 | 100.1 | 105.7 |

(continued)

| Pharmacokinetic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5/ 2 x OXN 20/10 | 4 x OXN 10/5/1 x OXN 40/20 | 2 x OXN 20/10 / 1 x OXN 40/20 |
|---|---|---|---|---|---|---|
| **AUCt (pg.h/mL)** | | | | | | |
| 90%CI | 82.0, 108.2 | 86.3, 114.5 | 81.8, 108.1 | 82.4, 108.9 | 87.3, 114.9 | 92.0, 121.5 |
| **AUCINF (pg.h/mL)** | | | | | | |
| Ratio (%) | | | | | | |
| 90%CI | | | | | -- | |
| **Cmax (pg/mL)** | | | | | | |
| Ratio (%) | 102.4 | 108.8 | 104.1 | 94.1 | 98.4 | 104.5 |
| 90%CI | 88.0, 119.2 | 93.1, 127.0 | 89.3, 121.2 | 80.8, 109.7 | 84.6, 114.4 | 89.7, 121.8 |
| **tmax (h)** | | | | | | |
| Difference | -0.71 | 0.12 | -2.03 | -0.83 | 1.32 | 2.15 |
| 90%CI | -2.96, 1.54 | -2.17,2.42 | -4.31, 0.24 | -3.10, 1.44 | -0.93,3.57 | -0.12, 4.43 |
| **t1/2Z (h)** | | | | | | |
| Difference | -3.55 | 0.79 | 2.30 | -4.35 | -5.85 | -1.51 |
| 90%CI | -12.92, 5.82 | -23.09, 24.67 | -22.06,26.67 | -28.49, 19.80 | -30.48, 18.77 | -8.80, 5.78 |

Table 19. Summary of Pharmacokinetic Parameters for Noroxycodone by Treatment: Full Analysis Population for Pharmacokinetics.

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (ng.h/mL)** | | | | |
| N | 23 | 23 | 23 | 23 |
| Arithmetic Mean | 439.71 | 442.70 | 436.15 | 451.35 |
| (SD) | (194.093) | (208.868) | (192.795) | (219.059) |
| Geometric Mean | 405.22 | 403.63 | 401.90 | 408.91 |
| **AUCINF (ng.h/mL)** | | | | |
| N | 23 | 22 | 22 | 22 |
| Arithmetic Mean | 447.28 | 453.05 | 440.75 | 462.53 |
| (SD) | (197.697) | (210.830) | (197.780) | (221.201) |
| Geometric Mean | 411.57 | 413.50 | 404.89 | 419.45 |
| **Cmax (ng/mL)** | | | | |
| N | 24 | 23 | 23 | 23 |
| Arithmetic Mean | 24.69 | 25.55 | 24.26 | 26.67 |
| (SD) | (6.507) | (6.986) | (6.415) | (8.428) |
| Geometric Mean | 23.83 | 24.56 | 23.42 | 25.38 |
| **tmax (h)** | | | | |
| N | 24 | 23 | 23 | 23 |
| Median | 5.0 | 5.0 | 3.5 | 4.0 |

(continued)

| | | | | |
|---|---|---|---|---|
| **tmax (h)** | | | | |
| (Min,Max) | (2.0, 8.0) | (2.5, 8.0) | (2.0, 8.0) | (1.0, 8.0) |
| **t1/2Z (h$^{-1}$)** | | | | |
| N | 23 | 22 | 22 | 22 |
| Arithmetic Mean | 7.03 | 7.10 | 7.25 | 6.95 |
| (SD) | (1.679) | (1.598) | (1.587) | (1.539) |
| **Noroxycodone:oxycodone AUCt ratio (ng.h/mL)** | | | | |
| N | 24 | 23 | 23 | 23 |
| Arithmetic Mean | 0.93 | 0.91 | 0.91 | 0.91 |
| (SD) | (0.368) | (0.393) | (0.404) | (0.444) |
| **Noroxycodone:oxycodone AUCINF ratio (ng.h/mL)** | | | | |
| N | 23 | 21 | 21 | 22 |
| Arithmetic Mean | 0.94 | 0.92 | 0.90 | 0.92 |
| (SD) | (0.374) | (0.408) | (0.420) | (0.449) |

Table 20. Noroxycodone Summary of Ratios for AUCt, AUCINF, $C_{max}$ and Differences for $T_{max}$ and Half-Life - Full Analysis Population for Pharmacokinetics.

| Pharmacokinetic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5 / 2 x OXN 20/10 | 4 x OXN 10/5 / 1 x OXN 40/20 | 2 x OXN 20/10 / 1 x OXN 40/20 |
|---|---|---|---|---|---|---|
| **AUCt (ng.h/mL)** | | | | | | |
| Ratio (%) | 98.0 | 97.2 | 97.7 | 100.8 | 100.3 | 99.5 |
| 90%CI | 95.3, 100.8 | 94.4, 100.1 | 95.0, 100.5 | 98.0, 103.7 | 97.5, 103.2 | 96.7, 102.4 |
| **AUCINF(ng.h/mL)** | | | | | | |
| Ratio (%) | 97.2 | 97.3 | 97.7 | 99.8 | 99.5 | 99.6 |
| 90%CI | 94.4, 100.0 | 94.5, 100.3 | 94.9, 100.6 | 97.0, 102.8 | 96.7, 102.3 | 96.8, 102.6 |
| **Cmax (ng/mL)** | | | | | | |
| Ratio (%) | 91.7 | 94.5 | 90.4 | 97.0 | 101.4 | 104.5 |
| 90%CI | 87.7, 95.8 | 90.4, 98.8 | 86.5, 94.5 | 92.8, 101.4 | 97.1, 105.9 | 100.0, 109.2 |
| **tmax (h)** | | | | | | |
| Difference | 0.18 | 0.30 | 0.20 | -0.12 | -0.02 | 0.10 |
| 90%CI | -0.47, 0.84 | -0.37, 0.97 | -0.46, 0.86 | -0.78, 0.54 | -0.67, 0.64 | -0.56, 0.76 |
| **t1/2Z (h)** | | | | | | |
| Difference | 0.13 | 0.25 | 0.33 | -0.12 | -0.20 | -0.08 |
| 90%CI | -0.20, 0.46 | -0.09, 0.59 | -0.00, 0.66 | -0.45, 0.21 | -0.53, 0.12 | -0.41, 0.25 |

Table 21. Summary of Pharmacokinetic Parameters for Oxymorphone by Treatment: Full Analysis Population for Pharmacokinetics.

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (ng.h/mL)** | | | | |
| N | 24 | 23 | 23 | 23 |
| Arithmetic Mean | 8.08 | 8.30 | 8.72 | 8.61 |
| (SD) | (4.028) | (4.276) | (4.586) | (4.463) |
| Geometric Mean | 6.81 | 6.11 | 6.73 | 6.95 |
| **AUCINF (ng.h/mL)** | | | | |
| N | 4 | 5 | 4 | 6 |
| Arithmetic Mean | 13.73 | 12.69 | 17.69 | 11.28 |
| (SD) | (3.538) | (4.176) | (3.200) | (4.400) |
| Geometric Mean | 13.37 | 12.09 | 17.48 | 10.48 |
| **Cmax (ng/mL)** | | | | |
| N | 24 | 23 | 23 | 23 |
| Arithmetic Mean | 0.57 | 0.58 | 0.61 | 0.72 |
| (SD) | (0.223) | (0.248) | (0.234) | (0.328) |
| Geometric Mean | 0.53 | 0.52 | 0.56 | 0.63 |
| tmax (h) | | | | |
| N | 24 | 23 | 23 | 23 |
| Median | 2.0 | 2.0 | 2.0 | 2.0 |
| (Min,Max) | (0.5, 6.0) | (0.5, 8.0) | (0.5, 4.0) | (0.5, 6.0) |
| $t1/2Z$ $(h^{-1})$ | | | | |
| N | 14 | 9 | 13 | 12 |
| Arithmetic Mean | 11.06 | 10.66 | 14.09 | 12.14 |
| (SD) | (3.261) | (1.766) | (8.540) | (4.803) |
| **Oxymorphone:oxycodone AUCt ratio(ng.h/mL)** | | | | |
| N | 24 | 23 | 23 | 23 |
| Arithmetic Mean | 0.02 | 0.02 | 0.02 | 0.02 |
| (SD) | (0.009) | (0.009) | (0.010) | (0.011) |
| **Oxymorphone:oxycodone AUCINF ratio(ng.h/mL)** | | | | |
| N | 4 | 5 | 4 | 5 |
| Arithmetic Mean | 0.03 | 0.02 | 0.03 | 0.03 |
| (SD) | (0.006) | (0.008) | (0.012) | (0.011) |

Table 22. Oxymorphone Summary of Ratios for AUCt, AUCINF, $C_{max}$ and Differences for $T_{max}$ and Half-Life - Full Analysis Population for Pharmacokinetics.

| Pharmacokinetic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5 / 2 x OXN 20/10 | 4 x OXN 10/5 / 1 x OXN 40/20 | 2 x OXN 20/10 / 1 x OXN 40/20 |
|---|---|---|---|---|---|---|
| **AUCt (ng.h/mL)** | | | | | | |
| Ratio (%) | 98.2 | 89.9 | 97.4 | 109.3 | 100.8 | 92.2 |
| 90%CI | 82.4, 117.0 | 75.1, 107.5 | 81.7, 116.2 | 91.6, 130.4 | 84.7, 119.9 | 77.4, 110.0 |

(continued)

| | | | | | | |
|---|---|---|---|---|---|---|
| **AUCINF(ng.h/mL)** | | | | | | |
| Ratio (%) 90%CI | 112.9 | 101.2 | 138.2 | 111.6 | 81.7 | 73.2 |
| **Cmax (ng/mL)** | | | | | | |
| Ratio (%) 90%CI | 82.3 73.3,92.3 | 81.6 72.6,91.8 | 88.3 78.6,99.1 | 100.8 89.7,113.2 | 93.2 83.1,104.5 | 92.5 82.4,103.8 |
| **tmax (h)** | | | | | | |
| Difference 90%CI | 0.48 -0.22,1.18 | 0.51 -0.2,1.23 | -0.05 -0.76,0.66 | -0.03 -0.74,0.68 | 0.53 -0.17,1.23 | 0.56 -0.15,1.27 |
| **t1/2Z (h)** | | | | | | |
| Difference 90%CI | -1.46 -5.33 2.40 | -1.70 -5.72 2.32 | 2.48 -1.266.23 | 0.24 -3.614.08 | -3.94 -7.51 -0.38 | -4.18 -8.07 -0.29 |

Table 23. Summary of Pharmacokinetic Parameters for Noroxymorphone by Treatment: Full Analysis Population for Pharmacokinetics.

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (ng.h/mL)** | | | | |
| N | 24 | 23 | 23 | 23 |
| Arithmetic Mean | 104.26 | 97.58 | 100.69 | 97.36 |
| (SD) | (37.930) | (35.393) | (37.876) | (35.559) |
| Geometric Mean | 94.39 | 88.51 | 91.01 | 87.67 |
| **AUCINF (ng.h/mL)** | | | | |
| N | 24 | 21 | 21 | 22 |
| Arithmetic Mean | 108.47 | 101.03 | 105.73 | 104.77 |
| (SD) | (38.451) | (37.666) | (36.655) | (33.155) |
| Geometric Mean | 98.86 | 91.47 | 97.11 | 97.17 |
| **Cmax (ng/mL)** | | | | |
| N | 24 | 23 | 23 | 23 |
| Arithmetic Mean | 5.36 | 4.97 | 5.16 | 4.90 |
| (SD) | (2.337) | (2.496) | (2.424) | (2.346) |
| Geometric Mean | 4.69 | 4.20 | 4.50 | 4.12 |
| **tmax (h)** | | | | |
| N | 24 | 23 | 23 | 23 |
| Median | 5.0 | 5.0 | 4.0 | 5.0 |
| (Min,Max) | (2.0, 12.0) | (3.0, 16.0) | (2.0, 12.0) | (1.5, 10.0) |
| **t1/2Z** | | | | |
| N | 24 | 21 | 21 | 23 |
| Arithmetic Mean | 10.82 | 10.04 | 10.37 | 10.32 |
| (SD) | (2.626) | (2.056) | (2.533) | (2.791) |
| **Noroxymorphone:Oxycodone AUCt ratio (ng.h/mL)** | | | | |
| N | 24 | 23 | 23 | 23 |
| Arithmetic Mean | 0.23 | 0.21 | 0.22 | 0.20 |
| (SD) | (0.100) | (0.099) | (0.106) | (0.092) |

(continued)

| Noroxymorphone:Oxycodone AUCINF ratio (ng.h/mL) | | | | |
|---|---|---|---|---|
| N | 24 | 20 | 20 | 21 |
| Arithmetic mean | 0.24 | 0.21 | 0.23 | 0.21 |
| (SD) | (0.102) | (0.100) | (0.106) | (0.091) |

Table 24. Noroxymorphone Summary of Ratios for AUCt, AUCINF, $C_{max}$ and Differences for $T_{max}$ and Half-Life - Full Analysis Population for Pharmacokinetics.

| Pharmacokinetic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5 / 2 x OXN 20/10 | 4 x OXN 10/5 / 1 x OXN 40/20 | 2 x OXN 20/10 / 1 x OXN 40/20 |
|---|---|---|---|---|---|---|
| **AUCt (ng.h/mL)** | | | | | | |
| Ratio (%) | 102.9 | 98.4 | 101.2 | 104.5 | 101.6 | 97.2 |
| 90%CI | 99.0, 107.0 | 94.6, 102.4 | 97.4, 105.3 | 100.5,108.7 | 97.8, 105.6 | 93.5, 101.1 |
| **AUCINF(ng.h/mL)** | | | | | | |
| Ratio (%) | 102.7 | 99.3 | 100.7 | 103.4 | 102.0 | 98.6 |
| 90%CI | 98.7, 106.8 | 95.2, 103.5 | 96.6, 104.8 | 99.3, 107.7 | 98.0, 106.1 | 94.6, 102.8 |
| **Cmax (ng/mL)** | | | | | | |
| Ratio (%) | 108.9 | 97.8 | 104.6 | 111.4 | 104.1 | 93.4 |
| 90%CI | 95.3, 124.6 | 85.3, 112.1 | 91.4, 119.7 | 97.3, 127.5 | 91.1, 118.9 | 81.7, 106.9 |
| **tmax (h)** | | | | | | |
| Difference | 0.37 | 0.86 | 0.42 | -0.48 | -0.05 | 0.44 |
| 90%CI | -0.63,1.37 | -0.16,1.88 | -0.59,1.43 | -1.49,0.52 | -1.04,0.95 | -0.57,1.45 |
| **t1/2Z (h)** | | | | | | |
| Difference | 0.38 | -0.42 | -0.07 | 0.80 | 0.46 | -0.35 |
| 90%CI | -0.43, 1.20 | -1.29,0.45 | -0.93,0.78 | -0.05, 1.66 | -0.38, 1.30 | -1.22,0.53 |

Table 25. Summary of Pharmacokinetic Parameters for 6-$\beta$ Naloxol by Treatment: Full Analysis Population for Pharmacokinetics.

| Pharmacokinetic parameter | 4 x OXN 10/5 | 2 x OXN 20/10 | 1 x OXN 40/20 | 2 x Oxygesic 20 + 2 x Naloxone 10 |
|---|---|---|---|---|
| **AUCt (ng.h/mL)** | | | | |
| N | 24 | 23 | 23 | 23 |
| Arithmetic Mean | 13.16 | 12.39 | 13.55 | 13.77 |
| (SD) | (4.375) | (5.330) | (5.285) | (5.121) |
| Geometric Mean | 12.48 | 11.55 | 12.57 | 12.91 |
| **AUCINF (ng.h/mL)** | | | | |
| N | 13 | 15 | 16 | 19 |
| Arithmetic Mean | 13.38 | 13.85 | 14.24 | 15.07 |

(continued)

| AUCINF (ng.h/mL) | | | | |
|---|---|---|---|---|
| (SD) | (2.870) | (6.057) | (5.750) | (5.261) |
| Geometric Mean | 13.10 | 12.84 | 13.22 | 14.31 |
| **Cmax (ng/mL)** | | | | |
| N | 24 | 23 | 23 | 23 |
| Arithmetic Mean | 0.39 | 0.44 | 0.47 | 0.40 |
| (SD) | (0.175) | (0.352) | (0.238) | (0.206) |
| Geometric Mean | 0.37 | 0.38 | 0.43 | 0.37 |
| **tmax (h)** | | | | |
| N | 24 | 23 | 23 | 23 |
| Median | 1.0 | 0.5 | 8.0 | 2.5 |
| (Min,Max) | (0.5, 32.0) | (0.5, 32.0) | (0.5, 24.0) | (0.5, 36.0) |
| **t1/2Z** | | | | |
| N | 13 | 15 | 16 | 19 |
| Arithmetic Mean | 15.16 | 14.37 | 15.87 | 15.39 |
| (SD) | (1.906) | (3.459) | (5.607) | (5.340) |
| **6-β-Naloxol: Naloxone AUCt ratio (ng.h/mL)** | | | | |
| N | 24 | 23 | 23 | 23 |
| Arithmetic Mean | 22.49 | 21.60 | 24.73 | 24.72 |
| (SD) | (14.103) | (18.348) | (24.359) | (25.824) |
| **6-β-Naloxol: Naloxone AUCINF ratio (ng.h/mL)** | | | | |
| N | 2 | 5 | 0 | 1 |
| Arithmetic mean | - | 9.79 | - | - |
| (SD) | - | (5.010) | - | - |

Table 26. 6-β Naloxol Summary of Ratios for AUCt, AUCINF, $C_{max}$ and Differences for $T_{max}$ and Half-Life - Full Analysis Population for Pharmacokinetics.

| Pharmacokinetic metric | 4 x OXN 10/5/2 x Oxygesic 20 + 2 x Naloxone 10 | 2 x OXN 20/10 / 2 x Oxygesic 20 + 2 x Naloxone 10 | 1 x OXN 40/20 / 2 x Oxygesic 20 + 2 x Naloxone 10 | 4 x OXN 10/5 / 2 x OXN 20/10 | 4 x OXN 10/5 / 1 x OXN 40/20 | 2 x OXN 20/10 / 1 x OXN 40/20 |
|---|---|---|---|---|---|---|
| **AUCt (ng.h/mL)** | | | | | | |
| Ratio (%) | 93.6 | 88.1 | 94.0 | 106.2 | 99.6 | 93.8 |
| 90%CI | 88.7,98.7 | 83.5,93.1 | 89.1,99.1 | 100.6,112.1 | 94.5, 105.0 | 88.9,99.0 |
| **AUCINF(ng.h/mL)** | | | | | | |
| Ratio (%) | 89.3 | 89.1 | 93.0 | 100.3 | 96.1 | 95.8 |
| 90%CI | 84.1,94.9 | 84.1,94.4 | 88.0,98.3 | 93.8,107.2 | 90.2, 102.3 | 90.3,101.6 |
| **Cmax (ng/mL)** | | | | | | |
| Ratio (%) | 97.8 | 103.0, | 113.8 | 95.0 | 85.9 | 90.5 |
| 90%CI | 86.4, 110.7 | 90.8, 116.9 | 100.5,128.9 | 83.8, 107.6 | 76.0,97.2 | 79.9, 102.5 |
| **tmax (h)** | | | | | | |
| Difference | -3.84 | -5.07 | -2.71 | 1.23 | -1.13 | -2.36 |

(continued)

| tmax (h) | | | | | | |
|---|---|---|---|---|---|---|
| 90%CI | -8.41,0.74 | -9.73, -0.41 | -7.32, 1.91 | -3.38,5.84 | -5.70,3.43 | -6.97,2.24 |
| t1/2Z (h) | | | | | | |
| Difference | -0.56 | -0.97 | 0.94 | 0.41 | -1.51 | -1.91 |
| 90%CI | -2.55, 1.43 | -2.90,0.96 | -0.90,2.79 | -1.79,2.60 | -3.59,0.58 | -3.89,0.06 |

6. Data Analysis

a) Oxycodone Results

- AUCt

The AUCt values obtained for oxycodone were very consistent between the treatments. Each of the treatments had a mean AUCt value of between 473 ng.h/mL (4 x OXN 10/5) and 502 ng.h/mL (2 x Oxygesic 20 mg & 2 x naloxone CR 10 mg).

In terms of AUCt, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

- t1/2Z

The t1/2Z values obtained for oxycodone were consistent between the treatments. Each of the treatments had a mean t1/2Z value of between 4.69 h (4 x OXN 10/5), and 5.01 h (2 x Oxygesic 20 mg & 2 x naloxone CR 10 mg). There were no statistical differences between the t1/2Z values for the treatments for any of the comparisons that were made.

- AUCINF

The AUCINF values obtained for oxycodone were very consistent between the treatments. Each of the treatments had a mean AUCINF value of between 475 ng.h/mL (4 x OXN 10/5) and 509 ng.h/mL (2 x Oxygesic 20 mg & 2 x naloxone CR 10 mg).

In terms of AUCINF, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

- $C_{max}$

The $C_{max}$ values obtained for oxycodone were consistent between the fixed combination treatments, and ranged from 34.46 ng/mL (1 x OXN 40/20) to 35.73 ng/mL (2 x OXN 20/10). The mean $C_{max}$ value for 2 x Oxygesic 20 mg & 2 x naloxone CR 10 mg was slightly higher at 40.45 ng/mL.

The $C_{max}$ ratios comparing the fixed combination tablets with each other ranged from 97.5% to 103.1%, and each had 90% confidence intervals within 80 - 125%. The higher mean $C_{max}$ value for 2 x Oxygesic 20 mg & 2 x naloxone CR 10 mg meant that the $C_{max}$ ratios comparing the fixed combination tablet with the reference product were lower, ranging from 85.8% to 88.4%. However, these $C_{max}$ ratios were still associated with 90% confidence intervals that were within 80 - 125%.

- $t_{max}$

The median $t_{max}$ values for the fixed combination tablets ranged from 3 h (1 x OXN 40/20) to 4 h (2 x OXN 20/10). The difference between these two treatments, although apparently small, was statistically significant. The median $t_{max}$ for 2 x Oxygesic 20 mg & 2 x naloxone CR 10 mg was 2.5 h, and there was a statistically significant difference between this reference treatment and 2 x OXN 20/10.

b) Naloxone-3-Glucuronide Results

- AUCt

The AUCt values obtained for naloxone-3-glucuronide were very consistent between the treatments. Each treatment had a mean AUCt value of between 520 ng.h/mL (1 x OXN 40/20) and 540 ng.h/mL (4 x OXN 10/5).
In terms of AUCt, each of the fixed combination tablets provided an equivalent availability of naloxone-3-glucuronide to the reference treatment, and to each other. All of the relative bioavailability calculations had 90% confidence intervals that were within the 80 - 125% limits of acceptability for bioequivalence.

- t1/2Z

The t1/2Z values obtained for naloxone-3-glucuronide were consistent between the treatments. Each of the treatments had a mean t1/2Z value of between 7.66 h (2 x Oxygesic 20 mg & 2 x naloxone CR 10 mg) and 8.48 h (4 x OXN 10/5). There were no statistical differences between the t1/2Z values for the treatments for any of the comparisons that were made.

- AUCINF

The AUCINF values obtained for naloxone-3-glucuronide were very consistent between the treatments. Each of the treatments had a mean AUCINF value of between 521 ng.h/mL (2 x OXN 20/10) and 563 ng.h/mL (4 x OXN 10/5).
In terms of AUCINF, each of the fixed combination tablets provided an equivalent availability of naloxone-3-glucuronide to the reference treatment, and to each other. All of the bioavailability calculations had 90% confidence intervals that were within the 80 - 125% limits of acceptability for bioequivalence.

- $C_{max}$

The $C_{max}$ values obtained for naloxone-3-glucuronide were consistent between the treatments. Each of the treatments had a mean $C_{max}$ value that range from 61.95 ng.mL (1 x OXN 40/20) to 63.62 ng.mL (2 x OXN 20/10).
Each of the fixed combination tablets provided an equivalent naloxone-3-glucuronide $C_{max}$ to the reference treatment, and to each other. All of the $C_{max}$ ratio calculations had 90% confidence intervals that were within the 80 - 125% limits of acceptability for bioequivalence.

- $t_{max}$

The median $t_{max}$ values for all the treatments ranged from 0.5 h (2 x OXN 20/10) to 1 h (4 x OXN 10/5, 1 x OXN 40/20 and 2 x Oxygesic 20 mg & 2 x naloxone CR 10 mg). There were no significant differences between the median $t_{max}$ values for any of the treatments.

- Naloxone-3-glucuronide : naloxone AUCt ratios

The mean naloxone-3-glucuronide : naloxone AUCt ratios ranged from 852.25 (2 x Oxygesic 20 mg & 2 x naloxone CR 10 mg) to 933.46 (4 x OXN 10/5).

- Naloxone-3-glucuronide : naloxone AUCINF ratios

The lack of AUCINF estimates for naloxone meant that mean naloxone-3-glucuronide : naloxone AUCINF ratios were only able to be calculated for 2 x OXN 20/10 tablets. These provided a mean naloxone-3-glucuronide : naloxone AUCINF ratio of 414.56, based on 5 subjects' data.

d) Naloxone Results

Naloxone concentrations were low, as was anticipated; therefore these results did not support a full pharmacokinetic assessment.

- AUCt

The AUCt values obtained for naloxone were consistent between the treatments. Each of the treatments had a mean AUCt value of between 0.84 ng.h/mL (2 x OXN 20/10) and 0.97 ng.h/mL (2 x Oxygesic 20 mg & 2 x naloxone CR 10 mg).

In terms of AUCt, each of the fixed combination tablets provided an equivalent availability of naloxone to the reference treatment, and to each other. All of the bioavailability calculations had 90% confidence intervals that were within the 80 - 125% limits of acceptability for bioequivalence.

- t1/2Z

It was not possible to calculate t1/2Z values for naloxone for all of the subjects with confidence, because the plasma concentrations in the terminal part of the profile did not always approximate to a straight line when plotted on a semi-logarithmic scale. The mean values were based on numbers of subjects ranging from 4 to 9.

The mean t1/2Z values obtained for naloxone ranged from between 9.89 h (4 x OXN 10/5) to 13.83 h (1 x OXN 40/20). There were a wide range of t1/2Z values contributing to the means, however, there were no statistical differences between the t1/2Z values for the treatments for any of the comparisons that were made.

- AUCINF

AUCINF values were calculated for those subjects with an estimable t1/2Z value. Some of the AUCINF values were not reportable because the extrapolated portion of the AUC accounted for more than 20% of the AUCINF value. A mean AUCINF value, of 1.64 ng.h/mL, was reportable for 2 x OXN 20/10 tablets only. None of the other treatments had sufficient data to report a mean AUCINF value. There were insufficient data to make comparisons between the treatments.

- Cmax

Each of the treatments had a mean $C_{max}$ value of between 0.07 ng/mL (4 x OXN 10/5) and 0.08 ng/mL (2 x OXN 20/10, 1 x OXN 40/20 and 2 x Oxygesic 20 mg & 2 x naloxone CR 10 mg).

Each of the fixed combination tablets provided an equivalent naloxone $C_{max}$ to each other. All of the $C_{max}$ ratios comparing the fixed combination tablets had 90% confidence intervals that were within the 80 - 125% limits of acceptability for bioequivalence.

When the fixed combination tablets were compared with the reference product, the 2 x OXN 20/10 tablets versus 2 x Oxygesic 20 mg & 2 x naloxone CR 10 mg had a 90% confidence interval that was above the 80 - 125% limit of acceptability for bioequivalence. The remaining fixed combination tablets provided an equivalent naloxone $C_{max}$ to the reference product.

- $t_{max}$

The median $t_{max}$ values for the treatments ranged from 1 h (2 x Oxygesic 20 mg & 2 x naloxone CR 10 mg) to 5 h (2 x OXN 20/10). There were a wide range of $t_{max}$ values for each of the treatments. There were no significant differences between the median $t_{max}$ values for any of the treatments.

e) Noroxycodone Results

- AUCt

The AUCt values obtained for noroxycodone were very consistent between the treatments. Each of the treatments had a mean AUCt value of between 436 ng.h/mL (1 x OXN 40/20) and 451 ng.h/mL (2 x Oxygesic 20 mg & 2 x naloxone CR 10 mg).

In terms of AUCt, each of the AUCt, each of the fixed combination tablets provided an equivalent availability of noroxycodone to the reference treatment, and to each other. All of the relative bioavailability calculations had 90% confidence intervals that were within the 80 - 125% limits of acceptability for bioequivalence.

- t1/2Z

The t1/2Z values obtained for noroxycodone were consistent between the treatments. Each of the treatments had a mean t1/2Z value of between 6.95 h (2 x Oxygesic 20 mg & 2 x naloxone CR 10 mg) and 7.25 h (1 x OXN 40/20). There were no statistical differences between the t1/2Z values for the treatments for any of the comparisons that were made.

- AUCINF

The AUCINF values obtained for noroxycodone were very consistent between the treatments. Each of the treatments had a mean AUCINF value of between 441 ng.h/mL (1 x OXN 40/20) and 463 ng.h/mL (2 x Oxygesic 20 mg & 2 x naloxone CR 10 mg).

In terms of AUCINF, each of the fixed combination tablets provided an equivalent availability of oxycodone to the reference treatment, and to each other. All of the relative bioavailability calculations had 90% confidence intervals that were within the 80 - 125% limits of acceptability for bioequivalence.

- $C_{max}$

The $C_{max}$ values obtained for noroxycodone were consistent between treatments. Each of the treatments had a mean $C_{max}$ value of between 24.26 ng/mL (1 x OXN 40/20) and 26.67 ng/mL (2 x Oxygesic 20 mg & 2 x naloxone CR 10 mg).

Each of the fixed combination tablets provided an equivalent noroxycodone $C_{max}$ to the reference treatment, and to each other. All of the $C_{max}$ ratio calculations had 90% confidence intervals that were within the 80 - 125% limits of acceptability for bioequivalence.

- $t_{max}$

The median $t_{max}$ values for the all the treatments ranged from 3.5 h to 5 h. There were no significant differences between the median $t_{max}$ values for any of the treatments.

- Noroxycodone : oxycodone AUCt ratios

The mean noroxycodone : oxycodone AUCt ratios ranged from 0.91 (2 x OXN 20/10, 1 x OXN 40/20 and 2 x Oxygesic 20 mg & 2 x naloxone CR 10 mg) to 0.93 (4 x OXN 10/5).

- Noroxycodone : oxycodone AUCINF ratios

The mean noroxycodone : oxycodone AUCt ratios ranged from 0.90 (1 x OXN 40/20) to 0.94 (4 x OXN 10/5).

f) Oxymorphone Results

- AUCt

The AUCt values obtained for oxymorphone were very consistent between treatments. Each of the treatments had a mean AUCt value of between 8 ng.h/mL (4 x OXN 10/5) and 9 ng.h/mL (1 x OXN 40/20).

In terms of AUCt, 4 x OXN 10/5 tablets and 1 x OXN 40/20 tablet provided an equivalent availability of oxymorphone to the reference treatment. 2 x OXN 20/10 tablets versus 2 x Oxygesic 20 mg & 2 x naloxone CR 10 mg had a 90% confidence interval that was outside the lower limit of acceptability for bioequivalence. When the fixed combination tablets were compared with each other, the 2 x OXN 20/10 tablets versus 1 x OXN 40/20 tablets had a 90% confidence interval outside the lower limit of acceptability for bioequivalence. The other comparisons between the fixed combination tablets had 90% confidence intervals that were within the 80 - 125% limits of acceptability for bioequivalence.

- t1/2Z

It was not possible to calculate t1/2Z values for oxymorphone for all of the subjects with confidence, because the plasma concentrations in the terminal part of the profiles did not always approximate to a straight line when plotted on a semi-logarithmic scale. The mean values were based on numbers of subjects ranging from 9 for 2 x OXN 20/10 tablets to 14 for 4 x OXN 10/5 tablets. The mean t1/2Z values obtained for oxymorphone ranged between 10.66 h (2 x OXN 20/10) and 14.09 h (1 x OXN 40/20). There were no statistical differences between the half-life values for the fixed combination tablets and the reference product, however, the half-life value for 1 x OXN 40/20 was statistically longer than for the other two strengths of fixed combination tablets.

- AUCINF

The mean AUCINF values were based on a small number of subjects for each of the treatments. AUCINF values could only be calculated for those subjects with an estimable t1/2Z value, and some AUCINF values were not reportable because the extrapolated portion of the AUC accounted for more than 20% of the AUCINF value. The numbers of subjects with reportable AUCINF values ranged from 4 for 4 x OXN 10/5 tablets and 1 x OXN 40/20 tablet, to 6 for 2 x Oxygesic 20 mg & 2 x naloxone CR 10 mg.

The mean AUCINF values ranged between 11 ng.h/mL (2 x Oxygesic 20 mg & 2 x naloxone CR 10 mg) and 18 ng.h/mL (1 x OXN 40/20). There were insufficient data to make comparisons between the treatments or calculate 90% confidence intervals.

- $C_{max}$

Each of the treatments had a mean $C_{max}$ value of between 0.57 ng/mL (4 x OXN 10/5) and 0.72 ng/mL (2 x Oxygesic 20 mg & 2 x naloxone CR 10 mg).

Each of the fixed combination tablets provided a lower oxymorphone $C_{max}$ than the reference treatment. The 90% confidence intervals associated with the $C_{max}$ ratios comparing the fixed combination tablets with the reference product were all below the lower limit of acceptability for bioequivalence.

Each of the fixed combination tablets provided an equivalent oxymorphone $C_{max}$ to each other. All of the $C_{max}$ ratios comparing the fixed combination tablets had 90% confidence intervals that were within the 80 - 125% limits of acceptability for bioequivalence.

- $t_{max}$

The median $t_{max}$ value for all of the treatments was 2 hours. There were no significant differences between the median $t_{max}$ values for any of the treatments.

- Oxymorphone : oxycodone AUCt ratios

The mean oxymorphone : oxycodone AUCt ratios were 0.02 for all of the treatments.

- Oxymorphone : oxycodone AUCINF ratios

The mean oxymorphone : oxycodone AUCINF ratios ranged from 0.02 (2 x OXN 20/10) to 0.03 (4 x OXN 10/5, 1 x OXN 40/20 and 2 x Oxygesic 20 mg & 2 x naloxone CR 10 mg).

g) Noroxymorphone Results

- AUCt

The AUCt values obtained for noroxymorphone were very consistent between treatments. Each of the treatments had a mean AUCt value of between 97 ng.h/mL (2 x Oxygesic 20 mg & 2 x naloxone CR 10 mg) and 104 ng.h/mL (4 x OXN 10/5).

In terms of AUCt, each of the fixed combination tablets provided an equivalent availability of noroxymorphone to the reference treatment, and to each other. Each of the bioavailability calculations had 90% confidence intervals that were within the 80 - 125% limits of acceptability for bioequivalence.

- t1/2Z

The t1/2Z values obtained for noroxymorphone were consistent between the treatments. Each of the treatments had a mean t1/2Z value of between 10.04 h (2 x OXN 20/10) and 10.82 h (4 x OXN 10/5). There were no statistical differences between the t1/2Z values for the treatments for any of the comparisons that were made.

- AUCINF

The AUCINF values obtained for noroxymorphone were very consistent between the treatments. Each of the treatments had a mean AUCINF value of between 101 ng.h/mL (2 x OXN 20/10) and 108 ng.h/mL (4 x OXN 10/5).

In terms of AUCINF, each of the fixed combination tablets provided an equivalent availability of noroxymorphone to the reference treatment, and to each other. All of the relative bioavailability calculations had 90% confidence intervals that were within the 80 - 125% limits of acceptability for bioequivalence.

- $C_{max}$

The $C_{max}$ values obtained for noroxymorphone were consistent between the treatments. Each of the treatments had a mean $C_{max}$ value that ranged from 4.90 ng/mL (2 x Oxygesic 20 mg & 2 x naloxone CR 10 mg) to 5.36 ng/mL (4 x OXN 10/5).

The $C_{max}$ ratios comparing the fixed combination tablets with the reference product ranged from 97.8% to 108.9%, and each had 90% confidence intervals within 80 - 125%. When the fixed combination tablets were compared with each other, the 4 x OXN 10/5 tablets versus 2 x OXN 20/10 tablets had a 90% confidence interval outside the upper limit of acceptability for bioequivalence. The other comparisons between the fixed combination tablets had 90% confidence intervals that were within the 80 - 125% limits of acceptability for bioequivalence.

- $t_{max}$

The median $t_{max}$ values for the treatments ranged from 4 h to 5 h. There were no significant differences between the median $t_{max}$ values for any of the treatments.

- Noroxymorphone : oxycodone AUCt ratios

The mean noroxymorphone : oxycodone AUCt ratios ranged from 0.20 (2 x Oxygesic 20 mg & 2 x naloxone CR 10 mg) to 0.23 (4 x OXN 10/5).

- Noroxymorphone : oxycodone AUCINF ratios

The mean noroxymorphone : oxycodone AUCINF ratios ranged from 0.21 (2 x OXN 20/10 and 2 x Oxygesic 20 mg & 2 x naloxone CR 10 mg) to 0.24 (4 x OXN 10/5).

h) 6β-Naloxol Results

- AUCt

The AUCt values obtained for 6β-naloxol were very consistent between treatments. Each of the treatments had a mean AUCt value of between 12 ng.h/mL (2 x OXN 20/10) and 14 ng.h/mL (2 x Oxygesic 20 mg & 2 x naloxone CR 10 mg).

In terms of AUCt, each of the fixed combination tablets provided an equivalent availability of 6β-naloxol to the reference treatment, and to each other. Each of the bioavailability calculations had 90% confidence intervals that were within the 80 - 125% limits of acceptability for bioequivalence.

- t1/2Z

The t1/2Z values obtained for 6β-naloxol were consistent between the treatments. Each of the mean treatments had a mean t1/2Z value of between 14.37 h (2 x OXN 20/10) and 15.87 h (1 x OXN 40/20). There were no statistical differences between the t1/2Z values for the treatments for any of the comparisons that were made.

- AUCINF

The AUCINF values obtained for 6β-naloxol were very consistent between treatments. Each of the treatments had a mean AUCINF value of between 13 ng.mL (4 x OXN 10/5) and 15 ng.mL (2 x Oxygesic 20 mg & 2 x naloxone CR 10 mg).

In terms of AUCINF, each of the fixed combination tablets provided an equivalent availability of 6β-naloxol to the reference treatment and to each other. All of the relative bioavailability calculations had 90% confidence intervals that were within the 80 - 125% limits of acceptability for bioequivalence.

- $C_{max}$

The mean $C_{max}$ values obtained for 6β-naloxol for each of the treatments ranged from 0.39 ng/mL (4 x OXN 10/5) to 0.47 ng/mL (1 x OXN 40/20).

When the fixed combination tablets were compared with the reference product, 1 x OXN 40/20 tablet versus 2 x Oxygesic 20 mg & 2 x naloxone CR 10 mg had a 90% confidence interval that was above the upper limit of acceptability for bioequivalence. When the fixed combination tablets were compared with each other, the 4 x OXN 10/5 tablets versus 1 x OXN 40/20 tablet, and 2 x OXN 20/10 tablets versus 1 x OXN 40/20 tablet, both had 90% confidence intervals that were slightly below the lower limit of acceptability for bioequivalence. All remaining comparisons had 90% confidence intervals that were within the 80 - 125% limits of acceptability for bioequivalence.

- $t_{max}$

The median $t_{max}$ values for the treatments ranged from 0.5 h (2 x OXN 20/10) to 8 h (1 x OXN 40/20), and for each treatment, consisted of a wide range of individual $t_{max}$ values making up the median values. The median $t_{max}$ value for 2 x OXN 20/10 tablets was significantly lower than for 2 x Oxygesic 20 mg & 2 x naloxone CR 10 mg. There were no other significant differences between the median $t_{max}$ values for the remaining treatments.

- 6β-naloxol : naloxone AUCt ratios

The mean 6β-naloxol : naloxone AUCt ratios ranged from 21.60 (2 x OXN 20/10) to 24.73 (1 x OXN 40/20).

- 6β-naloxol : naloxone AUCINF ratios

The lack of AUCINF estimates for naloxone meant that mean 6β-naloxol :naloxone AUCINF ratios were only able to be calculated for 2 x OXN 20/10 tablets. These provided a mean 6β-naloxol : naloxone AUCINF ratio of 9.79, based on 5 subjects' data.

7. Clinical Pharmacology Discussion and Conclusions

Low oral bioavailability prevents the complete pharmacokinetic assessment of naloxone. This was confirmed as the low plasma concentrations meant that it was not possible to estimate AUCINF values for naloxone for most of the subjects. Naloxone-3-glucuronide was present in the plasma in much higher concentrations, and AUCINF estimates were obtained for naloxone-3-glucuronide for the majority of subjects. The conclusions for the naloxone component of the fixed combination tablets were based on naloxone-3-glucuronide parameters.

a) Oxycodone

The mean plasma oxycodone concentration-time curves for 2 x Oxygesic 20 mg & 2 x naloxone CR 10 mg and the fixed combination tablets were almost superimposable.

A bioequivalence assessment was made for oxycodone. Each of the bioequivalence comparisons had 90% confidence intervals that were within the limits of acceptability for bioequivalence for Frelt, FrelINF and $C_{max}$ ratio. The oxycodone results indicate that each of the fixed combination tablet strengths were bioequivalent, both to each other and also to Oxygesic given together with naloxone CR tablet. There were no statistical differences between any of the $t_{max}$ or t1/2Z values for any of the treatments, further confirming the similarity of the products.

The plasma oxycodone concentrations achieved after administration of the reference product were similar to dose-adjusted oxycodone concentrations seen after administration of OxyContin in a previous study. The mean $C_{max}$ values for the fixed combination tablets were slightly lower, but when these were compared with the reference product, the $C_{max}$ ratios had confidence intervals that were within the limits of acceptability for bioequivalence.

b) Metabolite: parent AUCINF ratios

As expected, the levels of noroxycodone seen in the plasma after administration of the fixed combination tablets and Oxygesic plus naloxone, were similar to the levels of oxycodone that were achieved, resulting in noroxycodone : oxycodone AUCINF ratios of around 0.9. The levels of oxymorphone and noroxymorphone compared with oxycodone were much lower, with AUCINF ratios of around 0.02. These metabolite : parent AUCINF ratios were consistent across the fixed combination tablets and the reference treatment.

c) Noroxycodone, oxymorphone and noroxymorphone

The noroxycodone data confirmed the oxycodone results. Each of the bioequivalence comparisons had 90% confidence intervals that were within the limits of acceptability for bioequivalence for Frelt, FrelINF and $C_{max}$ ratio.

There were differences observed between the AUCt values for oxymorphone for 2 x OXN 20/10 versus 2 x Oxygesic 20 mg & 2 x naloxone CR 10 mg and 2 x OXN 20/10 versus 1 x OXN 40/20, however these differences were small, with only the lower limit of the 90% confidence interval being outside the limits of acceptability for bioequivalence. The fixed combination tablets were bioequivalent to each other in terms of $C_{max}$, but each provided a mean $C_{max}$ value that was between 80% and 90% of the reference product $C_{max}$.

The noroxymorphone data also confirmed the oxycodone results. All but one of the bioequivalence comparisons had 90% confidence intervals that were within the limits of acceptability for bioequivalence for FreIt, FreIINF and $C_{max}$ ratio.

d) Naloxone

The mean plasma naloxone concentrations were low, less than 0.1 ng/mL, and appeared to be biphasic, with a second peak occurring at between 8 to 16 hours.

Even though all of the subjects did have quantifiable plasma naloxone concentrations, individual subjects' plasma naloxone concentrations were low and highly variable. The maximum observed plasma naloxone concentrations were 0.07 to 0.08 ng/mL.

The pharmacokinetic profiles of naloxone from earlier studies were examined. On average, the mean $C_{max}$ values from these studies, dose-adjusted to a single dose of 1 mg, ranged between 4 and 15 pg/mL, confirming that the low plasma naloxone concentrations observed here were consistent with those levels measured in earlier studies.

A bioequivalence assessment was made for naloxone. The variability of the plasma naloxone concentrations did not allow for an estimate of AUCINF, or therefore FreIINF values. The bioavailability estimate was based on FreIt values. Each of the bioavailability comparisons had 90% confidence intervals that were within the limits of acceptability for bioequivalence. The mean $C_{max}$ values for naloxone were comparable, and five out of the six bioavailability comparisons had 90% confidence intervals that met the criteria for bioequivalence.

The $t_{max}$ and t1/2Z values for the treatments were variable, however there were no significant differences between any of the treatments for these two parameters.

As expected, the levels of naloxone-3-glucuronide seen in the plasma after administration of the fixed combination tablets and Oxygesic plus naloxone, were much higher than the levels of naloxone that were achieved, resulting in naloxone-3-glucuronide : naloxone AUCt ratios of around 900. 6β-naloxol was also measured in higher quantities than naloxone, resulting in 6β-naloxol : naloxone AUCt ratios of around 22. These metabolite : parent AUCt ratios were consistent across the fixed combination tablets and the reference treatment.

e) Naloxone-3-glucuronide

The mean plasma naloxone-3-glucuronide levels were higher than naloxone, and it was possible to make a bioavailability assessment based on FreIINF values.

A bioequivalence assessment was made for naloxone-3-glucuronide. Each of the bioequivalence comparisons had 90% confidence intervals that were within the limits of acceptability for bioequivalence for FreIt, FreIINF and $C_{max}$ ratio. The naloxone-3-glucuronide results indicate that each of the fixed combination tablet strengths were bioequivalent to each other, and to Oxygesic plus naloxone. There were no statistical differences between any of the $t_{max}$ or t1/2Z values for any of the treatments, further confirming the similarity of the products.

f) 6β-naloxol

The 6β-naloxol data confirmed the naloxone and naloxone-3-glucuronide results. For most of the comparisons, there were no significant differences observed between the treatments and for the bioequivalence comparisons, most of the 90% confidence intervals were within the limits of acceptability for bioequivalence. There were small differences between the $C_{max}$ values for the fixed combination products and the variability of the $t_{max}$ data led to a significant difference between the 2 x OXN 20/10 tablets and 2 x Oxygesic 20 mg & 2 x naloxone CR 10 mg.

8. <u>Conclusion</u>

These results confirm the interchangeability of the fixed combination tablets across the range of doses administered. This is supported by the bioavailability comparisons made between the treatments; each of the 90% confidence intervals for the ratio of population geometric means (test vs reference) for AUCINF and $C_{max}$ of oxycodone and naloxone, fell within 80% - 125%. The fixed combination tablets were also shown to be bioequivalent to Oxygesic given together with naloxone CR tablet.

These data have also shown that the availability of oxycodone from the fixed combination tablets is similar to what we would expect from oxycodone given alone, indicating that the bioavailability of oxycodone is not influenced by the co-administration of naloxone.

Hence, the results may be summarized as follows:

- In terms of oxycodone and naloxone-3-glucuronide, each of the fixed combination tablet strengths are interchangeable.
- The fixed combination tablets were also shown to be bioequivalent to Oxygesic® + naloxone CR.
- There was no difference in the incidence of treatment-emergent adverse events between oxycodone and naloxone administered as a fixed OXN combination, and oxycodone and naloxone administered as an open combination.

**Experiment 3: Effect of food on pharmacokinetics of oxycodone and naloxone**

1. Objective:

The objective of this study was to investigate the effect of a high-fat breakfast on the bioavailability of oxycodone and naloxone (providing that naloxone concentrations and pharmacokinetic metrics can be adequately quantified) when administered as a fixed combination prolonged release tablet. For this purpose tablets comprising 40 mg oxycodone and 20 mg naloxone (OXN 40/20) 20 mg oxycodone and 10 mg naloxone (OXN 20/10) were investigated.

2. Test population

A total of 28 healthy subjects were randomized to receive the study drug with the aim that 24 subjects would complete the study and provide valid pharmacokinetic data.

*Inclusion Criteria*

Subjects who were included in the study were those who met all of the following criteria:

- Males or females of any ethnic group. Aged between 18 - 45 years.
- BMI within the range 19-29 kg/m$^2$, and within the weight range 60-100 kg for males and 55-90 kg for females.
- Female subjects of childbearing potential must have been using a reliable form of contraception (e.g. Intra-uterine contraceptive device [IUD], oral contraceptive, barrier method). Female subjects who were postmenopausal must have been postmenopausal for 1 year and, in the absence of hormone replacement therapy (HRT), have elevated serum follicle-stimulating hormone (FSH).
- Generally good health, evidenced by a lack of significantly abnormal findings on medical history, physical examination, clinical laboratory tests, vital signs, and electrocardiogram (ECG). Vital signs (after 3 minutes resting in a supine position) had to be within the following ranges: oral body temperature between 35.0 - 38.0°C; systolic blood pressure, 90 - 140 mm Hg; diastolic blood pressure, 50 - 90 mm Hg; and pulse rate, 40 - 100 bpm. Blood pressure and pulse were taken again after 3 minutes in a standing position. After 3 minutes standing from a supine position, there was to be no more than a 20 mm Hg drop in systolic blood pressure, 10 mm Hg drop in diastolic blood pressure, and no greater than 20 bpm increase in pulse rate.
- Willing to eat all the food supplied during the study.
- If applicable, the subject's primary care physician confirmed within the last 12 months that the subject was suitable for taking part in clinical studies.

*Exclusion Criteria*

Subjects who were excluded from the study were those that met any of the following criteria:

- Female subjects who were pregnant (providing a positive β-hCG pregnancy test) or breastfeeding.
- Exposure to any investigational drug or placebo within 3 months of their first dose of study drug.
- Any significant illness within the 30 days before their first dose of study drug.
- Any clinically significant abnormalities identified at prestudy screening for medical history, physical examination or laboratory analyses.
- Use of any prescription medication (except HRT for postmenopausal females and contraceptive medication) in the 21 days, or over the counter medication including acid controllers, vitamins, herbal products and/or mineral supplements in the 7 days before their first dose of study drug.

The safety population included all subjects who received study drug and have at least one postdose safety assessment.
The full analysis population was the group of subjects who have a valid pharmacokinetic parameter metric. To have a valid pharmacokinetic parameter, subjects must not have experienced emesis within 12 hours after dosing.

The demographic data can be taken from the Table 27 below.

Table 27: Subject Demographics and Other Baseline Characteristics: Full Analysis Population

| | | Male (N = 18) | Female (N = 10) | Overall (N = 28) |
|---|---|---|---|---|
| Age (Years)N | | 18 | 10 | 28 |
| | Mean (SD) | 32.7 (6.04) | 30.7 (6.29) | 32.0 (6.09) |
| | Median | 32 | 31 | 32 |
| | Min, Max | 25, 45 | 22, 39 | 22, 45 |
| Sex, n (%) | | | | |
| | Male | | | 18 (64) |
| | Female | | | 10 (36) |
| Race, n (%) | | | | |
| | Caucasian | 18 (100) | 10 (100) | 28 (100) |
| Body Weight (kg) n | | 18 | 10 | 28 |
| | Mean (SD) | 78.7 (8.27) | 64.2(6.41) | 73.5 (10.33) |
| | Median | 78 | 66 | 73 |
| | Min, Max | 68, 98 | 55, 74 | 55, 98 |
| Height (cm) n | | 18 | 10 | 28 |
| | Mean (SD) | 179,8 (5.36) | 170.8 (4.87) | 176.6 (6.72) |
| | Median | 180 | 170 | 178 |
| | Min, Max | 169, 191 | 163, 178 | 163, 191 |
| Body Mass Index (kg/sq m) n | | 18 | 10 | 28 |
| | Mean (SD) | 24.3 (1.90) | 22.0 (1.36) | 23.5 (2.05) |
| | Median | 24 | 23 | 23 |
| | Min, Max | 22,29 | 19,23 | 19,29 |

3. Study Design, Test Treatment, Dose and Mode of Administration

*Preparations used*

The same tablets as in Example 2 were used.

*Study Design*

This was a single-dose, open-label, 4-treatment, 4-period, randomized crossover study in healthy adult male and female subjects.

Subjects were allocated each of the four treatments in accordance with a random allocation schedule (RAS). There was at least a 7-day washout period between dosing in each study period. Subjects attended a screening visit within ~1 days before the first dosing day (Day 1). During each study period, subjects checked in to the study site on the day before dosing (Day-1 ).The appropriate study drug was administered the following morning (Day 1) after an overnight fast of at least 10 hours. Subjects randomized to receive treatment in the fed state consumed a FDA standardized high-fat breakfast before dosing. No additional food was allowed until 4 hours after dosing. Subjects allocated to receive treatment in the fasted state did not have any food until 4 hours after dosing.

Pharmacokinetic blood samples (6 ml) were taken up until 96 hours after dosing. After dosing subjects remained in the study site for 48 hours. The subjects returned to the study site to provide the 72- and 96-hour blood samples.

Adverse events (AEs) were recorded throughout the study. Subjects attended a post study evaluation 7-10 days after dosing at study period 4 or 7-10 days after their last dose in the case of discontinuation from the study.

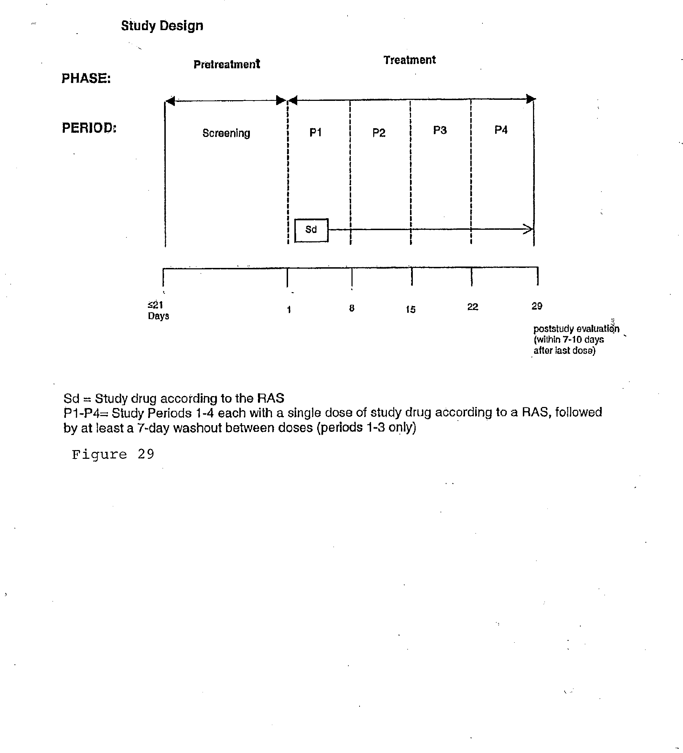

An overview over the treatment schedule is given in Figure 29.

*Treatments Administered*

The treatments administered in the study are presented below:

A = 1 tablet of OXN *40/20,* fed.

B = 1 tablet of OXN *10/5,* fed.
C = 1 tablet of OXN *40/20,* fasted.
D = 1 tablet of OXN *10/5,* fasted.

4. <u>Parameters tested</u>

The primary parameters considered were pharmacokinetic parameters and safety parameters.

4.1 Pharmacokinetic parameters

*Drug Concentration Measurements*

Blood samples (6 mL) for determining oxycodone, noroxycodone, oxymorphone, noroxymorphone, naloxone, 6$\beta$-naloxol, naloxone-3-glucuronide and 6$\beta$-naloxol-3-glucuronide concentrations were obtained from each subject during each of the four study periods as follows:
Immediately before dosing and then at 0.5, 1, 1.5, 2, 2.5, 3, 3.5, 4, 5, 6, 8, 10, 12, 16,24, 28, 32, 36, 48, 72 and 96 hours postdose (22 blood samples per study period).

*Pharmacokinetic Parameters*

The following pharmacokinetic parameters were calculated from the plasma concentrations of oxycodone, noroxycodone, oxymorphone, noroxymorphone, naloxone, 6$\beta$-naloxol, naloxone-3-glucuronide and 6$\beta$-naloxol-3-glucuronide:

- Area under the plasma concentration-time curve calculated from the time of dosing to the last measurable concentration (AUCt);
- Area under the plasma concentration-time curve calculated from the time of dosing to infinity (AUCINF);
- Maximum observed plasma concentration (Cmax);
- Time point of maximum observed plasma concentration (tmax);
- Terminal phase rate constant (LambdaZ);
- Apparent terminal phase half life (t1/2Z);
- Metabolite:parent ratios for both oxycodone and metabolites and naloxone and metabolites.

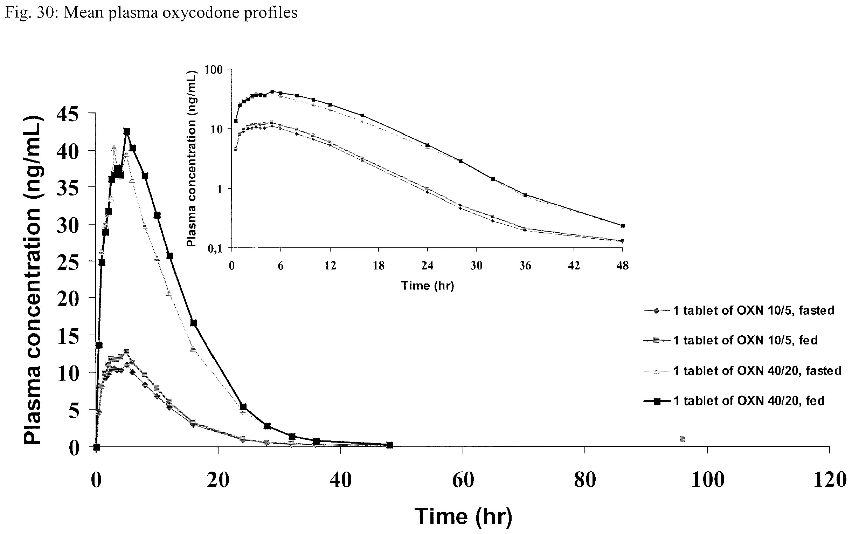

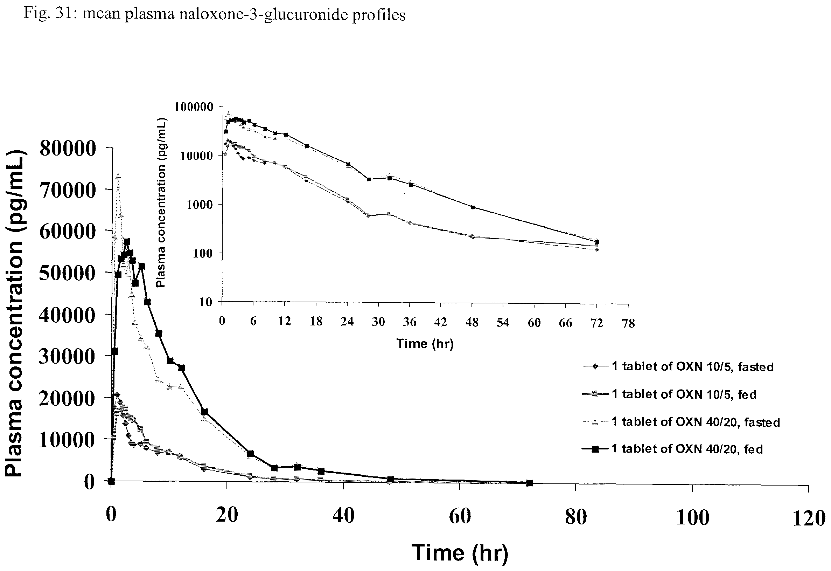

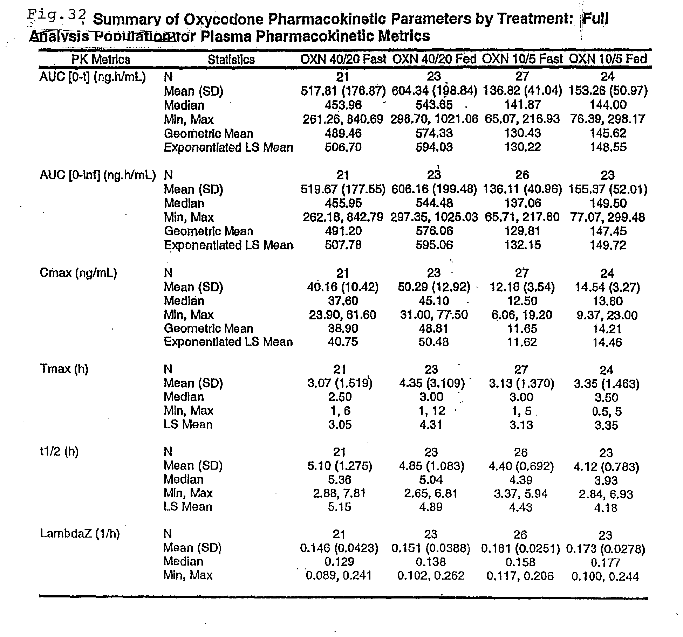

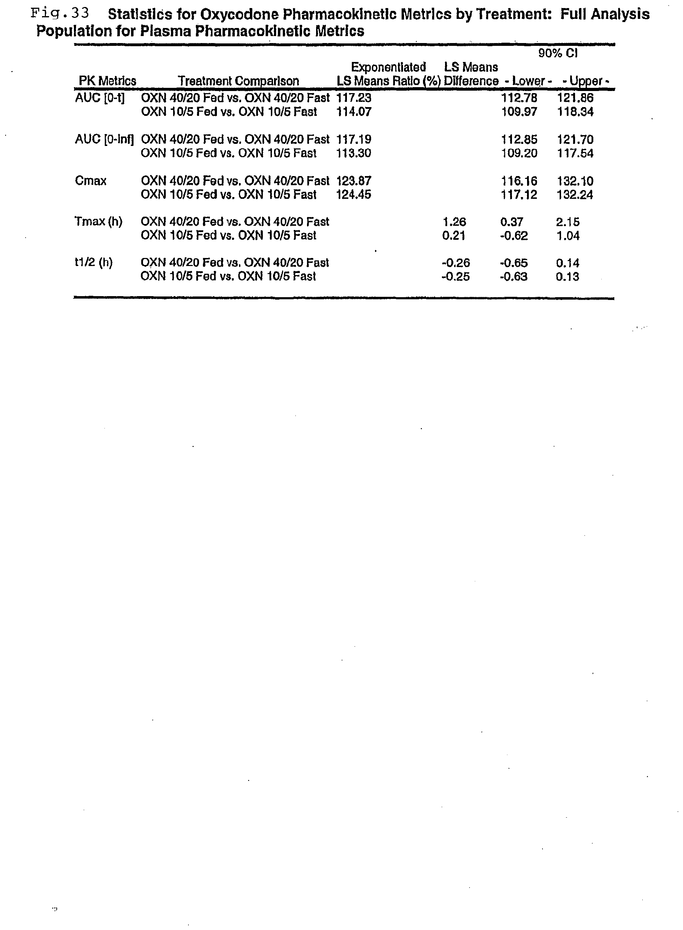

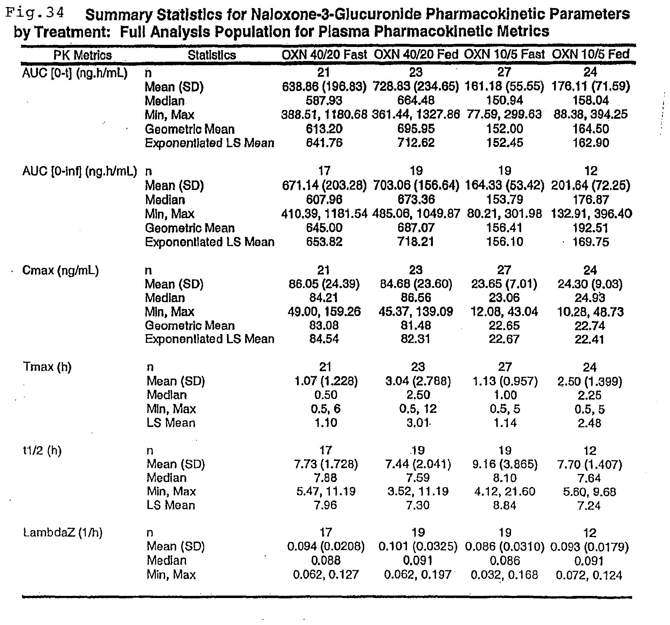

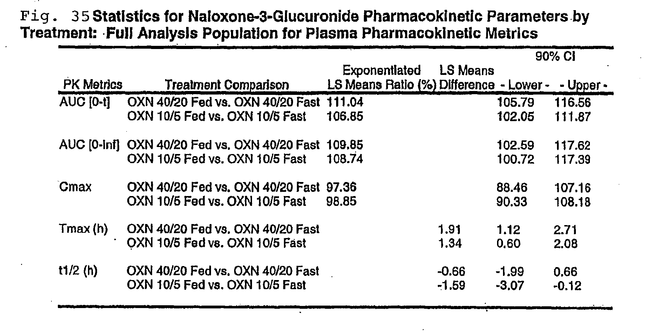

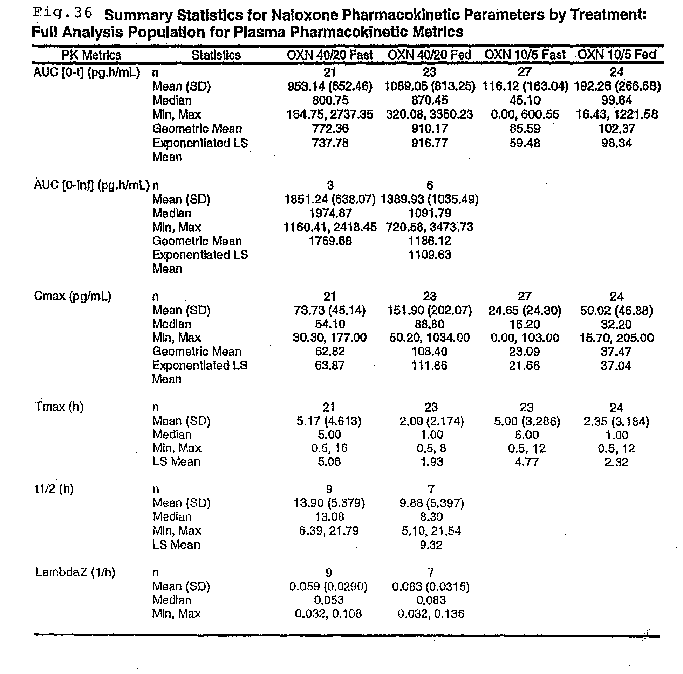

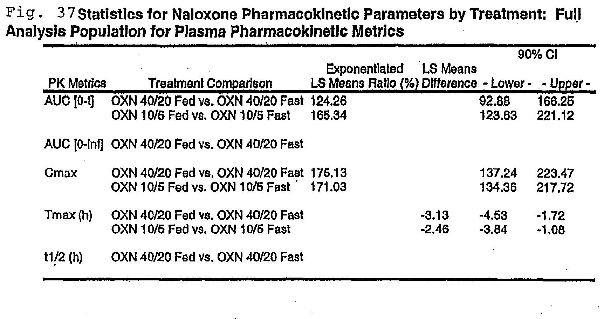

In Figures 30 to 37, for oxycodone, noroxycodone, oxymorphone and naloxone-3-glucuronide, AUC values were given in ng.h/mL, and Cmax values in ng/mL. For naloxone, 6-$\beta$ -naloxol and 6-$\beta$ -naloxol-3-glucuronide, the AUC values were given in pg.h/mL and Cmax values in pg/mL.

*Pharmacokinetic Analyses*

AUCt values were calculated using the linear trapezoidal method. Where possible, LambdaZ values were estimated using those points determined to be in the terminal log-linear phase. t1/2Z values were determined from the ratio of ln 2 to LambdaZ. The areas under the plasma concentration-time curve between the last measured point and infinity were calculated from the ratio of the final observed plasma concentration (Clast) to LambdaZ. These were then added to the AUCt to yield AUCINF

All calculations were performed with WinNolin Enterprise Edition, Version 4.1.

The safety population was used to summarize and graphically display the plasma concentration data. Plasma concentration data for each analyte (oxycodone, noroxycodone, oxymorphone, noroxymorphone, naloxone, 6$\beta$-naloxol, naloxone-3-glucuronide and 6$\beta$-naloxol-3-glucuronide) was summarized as continuous data by time point and treatment, and by gender. Individual and mean plasma concentrations for each analyte were also plotted over time for each treatment.

The full analysis population for pharmacokinetic metrics was used to summarize the pharmacokinetic metrics. Pharmacokinetic metrics (AUCt, t1/2Z, LambdaZ, AUCINF, Cmax and tmax) for each analyte were summarized as continuous data by treatment and gender wherever there was a minimum of 5 subjects for each gender. Pharmacokinetic samples obtained from subjects who did not experience emesis within 12 hours after dosing were used to determine these metrics.

Log transformed data for AUCt, AUCINF (if available), and Cmax were analyzed using a mixed effect linear model, with fixed terms for treatment, sequence and period and a random term for subject. Compound symmetry was assumed. Treatment population geometric means were estimated from treatment LS Means. Ratios of treatment population geometric means were estimated by exponentiating the difference (test-reference) between treatment least square means, and 90% confidence intervals for the ratios were calculated.

The data for tmax, Lambaz and t½Z were analyzed using a mixed effect linear model, with fixed terms for treatment, sequence and period and a random term for subject. Compound symmetry was assumed. Treatment population means were estimated by treatment LS Means. Treatment differences and their associated 90% confidence intervals were calculated from the least square means.

The following comparisons were of interest:

Treatment A vs. C:
From which the relative bioavailability (Freit, FreIINF) and Cmax ratio of all analytes from fixed combination prolonged release tablet OXN 40/20 in the fed vs. fasted state (i.e., the effect of food on OXN 40/20) were estimated.

Treatment B vs. D:
From which the relative bioavailability (Freit, FreIINF) and Cmax ratio of all analytes from fixed combination prolonged release tablet OXN 10/5 in the fed vs. fasted state (i.e., the effect of food on OXN 10/5) were estimated.

In addition, metabolite: parent ratios of AUCt, and where possible AUCINF were summarized using number, mean, standard deviation, minimum and maximum.

4.2 Safety assessments

Assessment of safety was performed for all subjects who received study drug and had at least one postdose safety assessment (the safety population). All safety data was listed for subjects in the enrolled population. Safety assessments consisted of monitoring and recording all adverse events and serious adverse events, the regular monitoring of hematology, blood chemistry, and urine values, regular measurement of vital signs and the performance of physical examinations, ECG and pulse goniometry.

*Adverse Events*

An adverse event (AE) was any untoward medical occurrence in a subject administered a pharmaceutical product, including placebo, occurring during the study that did not necessarily have a causal relationship with the study drug.

An adverse event could be:

- Any unfavorable and unintended sign (including an abnormal laboratory finding), symptom, or disease temporally associated with the use of a medicinal product, whether or not considered related to the medicinal product
- Any new disease or exacerbation of an existing disease
- Any deterioration in non-protocol-required measurements of laboratory value or other clinical test (e.g., ECG or X-ray) that resulted in symptoms, a change in treatment, or discontinuation from study drug

All AEs occurring during the study for subjects who received study drug (starting from signing informed consent to 7 days after the subject's last study visit) were collected on the AEs page of the CRF. For each AE, the following information was recorded:

- AE (e.g. headache).
- Start time and date.
- Stop time and date.
- Severity.
- Study drug action taken.
- Other action taken.
- Relationship to study drug.
- Outcome.
- Seriousness.

A cluster of signs and symptoms that resulted from a single cause was to be reported as a single adverse event (e.g., fever, elevated WBC, cough, abnormal chest x-ray, etc. could all be reported as "pneumonia.").

*Serious Adverse Events*

A serious adverse event (SAE) was any untoward medical occurrence that at any dose:

- resulted in death;
- was life-threatening;
- required inpatient hospitalization or prolongation of existing hospitalization;
- resulted in persistent or significant disability/incapacity; or
- was a congenital anomaly/birth defect.

*Adverse Events Analyses*

Adverse events that occurred after signing of informed consent through all phases of the study to study completion were collected on CRFs. Adverse events that occurred from immediately after study drug administration to 7 days after the last dose of study drug were also included.
Adverse events were classified into standardized terminology from the verbatim description (Investigator term) according to the MedDRA Coding Dictionary. AEs are presented by preferred term nested within System Organ Class.
AEs were summarized by presenting, for each treatment group, the incidence of AEs. The incidence of AEs was based on the numbers and percentages of subjects with AEs. Although a MedDRA term may have been reported more than once for a subject, that subject was counted only once in the incidence count for that MedDRA term.
Data for adverse events were analyzed using the treatment-emergent signs and symptoms (TESS) philosophy. Treatment-emergent signs and symptoms are defined as adverse events that emerge during treatment, having been absent at pre-treatment, or reemerge during treatment, having been present at baseline but stopped prior to treatment or that worsen in severity or frequency relative to the pre-treatment state. Only treatment-emergent adverse events from the study were summarized for this report.

5. Results

*Pharmacokinetic Parameters*

Pharmacokinetic parameters for oxycodone, naloxone-3-glucuronide and naloxone are presented in Figures 30 to 37.

*Oxycodone Results*

- AUCt

The AUCt values obtained for oxycodone were consistent, both between the two OXN 10/5 treatments and between the two OXN 40/20 treatments. Giving OXN of either strength after a high fat meal provided an equivalent availability of oxycodone to OXN given after an overnight fast. The bioavailability calculations each had 90% confidence intervals that were within the 80 - 125% limits of acceptability for bioequivalence.

- t1/2Z

The t1/2Z values obtained for oxycodone appeared consistent between the treatments. Each of the treatments had a mean t1/2Z value of between 4.12 h (OXN 10/5 fasted) and 5.10 h (OXN 40/20 fasted).

- AUCINF

The AUCINF values obtained for oxycodone were very consistent between both the OXN 10/5 treatments and the OXN 40/20 treatments. OXN given after a high fat meal provided an equivalent bioavailability of oxycodone to OXN given after an overnight fast, for both the OXN 10/5 and OXN 40/20 strengths. The bioavailability calculations had 90% confidence intervals that were within the 80 - 125% limits of acceptability for bioequivalence.

- Cmax

Food increased the mean oxycodone Cmax values that were observed, by approximately 24% for OXN 10/5 and OXN 40/20.

- tmax

The median tmax values for each of the treatments ranged from 2.5 h (OXN 40/20 fasted) to 3.5 h (OXN 10/5 fed). The median tmax for OXN 40/20 fasted was numerically lower than the median tmax for OXN 40/20 fed, the 90% confidence interval for the difference between OXN 40/20 fed and OXN 40/20 fasted was 0.35 to 2.17. The 90% confidence interval for the difference between OXN 10/5 fed and OXN 10/5 fasted was -0.61 to 1.11.

*Noroxycodone, oxymorphone and noroxymorphone results*

The noroxycodone and noroxymorphone data supported those observations made for the oxycodone data.
The oxymorphone data were variable for the AUC and Cmax comparisons.

- Noroxycodone:oxycodone AUCt ratios

The mean noroxycodone:oxycodone AUCt ratios ranged from 0.66 (OXN 10/5 fed) to 0.91 (OXN 40/20 fasted).

- Noroxycodone:oxycodone AUCINF ratios

The mean noroxycodone:oxycodone AUCINF ratios ranged from 0.66 (OXN 10/5 fed) to 0.91 (OXN 40/20 fasted).

- Oxymorphone:oxycodone AUCt ratios

The mean oxymorphone:oxycodone AUCt ratios ranged from 0.01 (OXN 10/5 fasted and fed) to 0.02 (OXN 40/20 fasted and fed).

- Oxymorphone:oxycodone AUCINF ratios

The lack of AUCINF estimates for oxymorphone meant that mean oxymorphone:oxycodone ratios were only able to be calculated for OXN 40/20 fed. This treatment provided a mean oxymorphone:oxycodone ratio of 0.02, based on 10 subjects' data.

- Noroxymorphone:oxycodone AUCt ratios

The mean noroxymorphone:oxycodone AUCt ratios ranged from 0.20 (OXN 10/5 fed) to 0.28 (OXN 40/20 fasted).

- Noroxymorphone:oxycodone AUCINF ratios

The mean noroxymorphone:oxycodone AUCINF ratios ranged from 0.22 (OXN 10/5 fed and OXN 40/20 fed) to 0.29 (OXN 20/40 fasted).

*Naloxone-3-glucuronide results*

- AUCt

The AUCt values obtained for naloxone-3-glucuronide were consistent, both between the two OXN 10/5 treatments and between the two OXN 40/20 treatments. Giving OXN of either strength after a high fat meal provided an equivalent availability of naloxone-3-glucuronide to OXN given after an overnight fast. The bioavailability calculations each had 90% confidence intervals that were within the 80 - 125% limits of acceptability for bioequivalence.

- t1/2Z

The t1/2Z values obtained for naloxone-3-glucuronide appeared consistent between OXN 40/20 fasted and OXN 40/20 fed (7.7 hand 7.4 h respectively). The mean naloxone-3-glucuronide t1/2Z value for OXN 10/5 fasted (9.1 h) appeared higher than for the other treatments. OXN 10/5 fed had a mean naloxone-3-glucuronide t1/2Z value that was similar to OXN 40/20.

- AUCINF

The AUCINF values obtained for naloxone-3-glucuronide were consistent, both between the two OXN 10/5 treatments and between the two OXN 40/20 treatments. Giving OXN of either strength after a high fat meal provided an equivalent availability of naloxone-3-glucuronide to OXN given after an overnight fast. The bioavailability calculations each had 90% confidence intervals that were within the 80 - 125% limits of acceptability for bioequivalence.

- Cmax

Food did not increase the mean naloxone-3-glucuronide Cmax values observed for either OXN 10/5 or OXN 40/20. The Cmax ratios comparing OXN fed with OXN fasted had 90% confidence intervals that were within the 80 - 125% limits of acceptability for bioequivalence.

- tmax

The median tmax values for each of the treatments ranged from 0.5 h (OXN 40/20 fasted) to 2.5 h (OXN 40/20 fed). As for oxycodone, food appeared to increase the median tmax values, both for OXN 10/5 and OXN 40/20. The 90% confidence interval for the difference between OXN 10/5 fed and OXN 10/5 fasted was 0.52 - 2.02. The 90% confidence interval for the difference between OXN 40/20 fed and OXN 40/20 fasted was 1.13 - 2.70.

*Naloxone, 6$\beta$-naloxol, and 6$\beta$-naloxol-3/6-glucuronide results*

Naloxone concentrations were low, as anticipated, therefore the naloxone results did not support a full pharmacokinetic assessment. The variability in the plasma concentration data led to bioavailability calculations with 90% confidence intervals that were very wide.
The plasma naloxone data did not support the estimate of lambdaZ values for most of the subjects. Therefore it was not possible to extrapolate the plasma naloxone curves in order to obtain AUCINF values. The lack of AUCINF estimates for naloxone meant that the metabolite: parent AUCINF ratios could not be calculated for OXN 10/5 fasted or fed.
The 6$\beta$-naloxol data were also variable, the 90% confidence intervals for most of the comparisons of interest were outside the 80 - 125% limits of acceptability for bioequivalence.
The 6$\beta$-naloxol-3-glucuronide data supported those observations made for the naloxone-3-glucuronide data for the AUCt and AUCINF comparisons. Food caused an increase in the mean Cmax values for 6$\beta$-naloxol-3-glucuronide, with the mean 6$\beta$-naloxol-3-glucuronide Cmax values being 35 to 42% higher in the presence of food.

- Naloxone-3-glucuronide:naloxone AUCt ratios

The mean naloxone-3-glucuronide:naloxone AUCt ratios ranged from 910 (OXN 40/20 fed) to 5091 (OXN 10/5 fasted).

- Naloxone-3-glucuronide:naloxone AUCINF ratios

The mean naloxone-3-glucuronide:naloxone AUCINF ratios were 360 for OXN 40/20 fasted, based on 3 subjects' data, and 614 for OXN 40/20 fasted, based on 6 subjects' data.

- 6$\beta$-naloxol:naloxone AUCt ratios

The mean 6$\beta$-naloxol:naloxone AUCt ratios ranged from 17.9 (OXN 40/20 fed) to 99.7 (OXN 10/5 fasted).

- 6$\beta$-naloxol:naloxone AUCINF ratios

The mean 6$\beta$-naloxol:naloxone AUCINF ratios were 7.4 for OXN 40/20 fasted, based on 3 subjects' data, and 13.5 for OXN 40/20 fed, based on 5 subjects' data.

- 6$\beta$-naloxol-3/6-glucuronide:naloxone AUCt ratios

The mean 6$\beta$-naloxol-3/6-glucuronide:naloxone AUCt ratios ranged from 790 (OXN 40/20 fed) to 5091 (OXN 20/5 fasted).

- 6β-naloxol-3/6-glucuronide:naloxone AUCINF ratios

The mean 6β-naloxol-3/6-glucuronide:naloxone AUCINF ratios were 302 for OXN 40/20 fasted, based on 3 subjects' data, and 623 for OXN 40/20 fed, based on 5 subjects' data.

*Safety*

One subject experienced SAE of acute laryngitis and dysponea during OXN 10/5 fasted period. Study drug was stopped and the subject was discontinued but fully revovered from the events which were not considered to be related to study drug.

Nausea, fatigue and headache were the most frequently reported AEs events across treatments.

6. Conclusions

*Clinical Pharmacology Discussion*

It was anticipated that low oral bioavailability would prevent the complete pharmacokinetic assessment of naloxone. This was confirmed as the low plasma concentrations meant that it was not possible to estimate AUCINF values for naloxone for most of the subjects. Naloxone-3-glucuronide was present in the plasma in much higher concentrations, and AUCINF estimates were obtained for naloxone-3-glucuronlde for the majority of subjects. The conclusions for the naloxone component of the fixed combination tablets were based on naloxone-3-glucuronide parameters.

Food did not appear to influence the availability of oxycodone from either strength of OXN, as equivalent amounts of oxycodone were available from OXN when given either after an overnight fast, or after a high fat breakfast.

Administering OXN after a high fat breakfast slightly increased the mean observed Cmax values of both strengths of OXN. Examination of the mean plasma profiles shows however, that this difference was numerically small and unlikely to be clinically significant for either strength of OXN

Food did not have an effect on the half-life of oxycodone. The mean half-life of oxycodone was similar for OXN administered after an overnight fast or a high fat breakfast, and was consistent with oxycodone half-lives that have been recorded previously.

The noroxycodone and noroxymorphone data supported those observations made for the oxycodone data.

Food did not appear to influence the bioavailability of naloxone-3-glucuronide from either strength of OXN, as equivalent amounts of naloxone-3-glucuronide were available from OXN when given either after an overnight fast or after a high fat breakfast.

Administering OXN after a high fat breakfast did not affect the mean naloxone-3-glucuronide Cmax value of either strength of OXN. The 90% confidence intervals associated with the Cmax ratios were within the 80 -125% limits of acceptability for bioequivalence.

There was some variability in the naloxone-3-glucuronide $t1/22$ and tmax values for OXN fed compared with OXN fasted, however, the differences that were observed were small and unlikely to be clinically significant.

The plasma naloxone and 6β-naloxol data were variable, and did not support the observations made for naloxone-3-glucuronide. The data recorded for 6β-naloxol-3-glucuronide were more consistent with naloxone-3-glucuronide, except that administration of OXN after a high fat breakfast significantly increased the mean observed Cmax compared with administration after an overnight fast.

*Safety*

Food did not seem to have any influence on the occurrence of AE and was not a safety issue.

7. Summary

- Administering OXN 40/20 and OXN 10/5 after a high fat breakfast had no effect on the bioavailability of oxycodone or naloxone-3-glucuronide, compared with administering OXN 40/20 and OXN 10/15 in a fasted state.

- The presence of food did not alter the mean Cmax value for naloxone-3-glucuronide, and slightly increased the mean Cmax value for oxycodone, though this is not considered to be of clinical significance.

**Reference Experiment 4: Influence of naloxone on analgetic efficacy**

1. Objective

The objective of this study was to assess whether and to what extent naloxone sustained release tablets (5 mg, 15 mg and 45 mg) will block the opioid agonist properties of oxycodone 20 mg in healthy (normal) volunteers.
This study was thus designed to provide evidence for a dose-ratio of naloxone and oxycodone that exerts sufficient analgesic activity. The data should support the development of a combination product of oxycodone and naloxone prolonged release tablets.

2. Test population

*Selection of Study Population*

A total of 21 healthy adult, male and female subjects were randomized. Drop outs were replaced with the aim that 20 subjects (10 male, 10 female) would complete the study and provide valid pharmacodynamic and pharmacokinetic data.

*Inclusion Criteria*

Subjects who were included in the study were those who met all of the following criteria:

- Subjects ranging in age from 21 to 45 years;
- Female subjects of childbearing potential must have a negative urine pregnancy test at screening;
- Normal body weight in relation to height according to Broca: Weight [kg] / (Height [cm] - 100) = 0.8 to 1.2;
- Free of significant abnormal findings as determined by baseline history, physical examination, vital signs (blood pressure, heart rate), hematology, blood chemistries, urine analysis and ECG;
- Willingness to follow the protocol requirements as evidenced by written informed consent

*Exclusion Criteria*

Subjects who were excluded from the study were those who met any of the following criteria:

- Any history of hypersensitivity to oxycodone, naloxone, psychotropic or hypnotic drugs;
- A history of drug or alcohol abuse, positive pre-study urine drug screen;
- History of opioid use in the previous 3 months;
- Any medical or surgical conditions which might significantly interfere with the gastrointestinal absorption, distribution, metabolism or excretion of the reference or test drug. This includes any history of serious disease of the gastrointestinal tract, liver, kidneys, and/or blood forming organs;
- History of cardiovascular, pulmonary, neurology, endocrine or psychiatry disease;
- A history of frequent nausea or emesis regardless of etiology;
- Participation in a clinical drug study during the preceding 60 days;
- Any significant illness during the 4 weeks preceding entry into this study;
- Use of any medication (except oral contraceptives) during the 7 days preceding study initiation or during the course of this study;
- Refusal to abstain from food 6 hours preceding and 7 hours following study drug administration;
- Excessive intake of alcohol (> 21 units per week of beer or hard liquor or equivalent in other forms);
- Consumption of alcoholic beverages within 24 hours of first dosing;
- Blood or blood products donated in the past 90 days prior to study drug administration; any contraindication to blood sampling.

Table 28 below summarizes the demographic characteristics by gender.

Table 28: Subject Demographics and Other Baseline Characteristics: Safety Population

| | Male (N = 10) | Female (N = 11) | Overall (N = 21) |
|---|---|---|---|
| Characteristics Age (y) | | | |
| Mean± SD | 25.7 ±2.41 | 28.9 ±4.97 | 27.4 ± 4.20 |
| Range (min, max) | 22,29 | 23,37 | 22,37 |
| Height (cm) | | | |
| Mean±SD | 182.4 ±5.38 | 170.1 ±3.73 | 176.0 ±7.72 |
| Range (min, max) | 170,189 | 162,174 | 162,189 |
| Weight (kg) | | | |
| Mean± SD | 78.8 ±4.57 | 63.2 ± 5.00 | 70.4 ± 9.04 |
| Range (min, max) | 73,86 | 56,75 | 56,86 |
| Body Mass Index (kg/m$^2$) | | | |
| Mean±SD | 23.6 ± 2.14 | 21.9 ± 1.89 | 22.7 ± 2.16 |
| Range (min, max) | 21,26 | 19,27 | 19,27 |

There were no significant demographic or baseline characteristic differences between male and female subjects in the safety population at baseline. Female subjects were generally shorter and lighter than male subjects, and had a lower BMI. As this study had a crossover design, there were no demographic differences between the treatment groups at baseline.

3. Study Design, Test Treatment, Dose and Mode of Administration

*Preparations used*

The same preparations as in Example 1 were used.

*Study Design*

This was a single site, single dose, double blind, placebo-controlled, 5-treatment, 5-period, randomized, balanced crossover study in healthy adult male and female subjects. It was conducted to evaluate the dose-ratio of naloxone and oxycodone in which oxycodone still exerts sufficient analgesic activity. Subjects were allocated each of the 5 treatments described in the synopsis according to a random allocation schedule (RAS). There was a 7-day washout period

Subjects attended a screening visit within 3 weeks of the first dosing day. During each study period, subjects were checked in to the study site at least 1 hour before dosing. They were administered the study medication and then remained at the study site for 12 hours unless they exhibited any opioid effects or other findings, which in the opinion of the Principal investigator required a prolonged stay of the subjects at the study site. Subjects were discharged after the 12-hour blood sample was taken and returned to the study site to provide the 24-hour blood sample. Dosing of test medications occurred after a 6-hour overnight fast, and patients remained fasted until 7 hours post-dose.

Pharmacodynamic measurements including pain-related evoked potentials (EEG), phasic / tonic pain intensity estimates, EEG background activity, acoustic evoked potentials, and tracking performance during phasic / tonic pain were conducted within 40 minutes pre-dose and at 1, 3 and 6 hours post-dose. Sought symptoms (tiredness, nausea, dizziness and drowsiness) were assessed pre-dose and at 1,2, 3, 4, 6, 8 and 12 hours post-dose.

Subjects also attended a post-study evaluation after discontinuation from the study or after dosing of Study period 5.

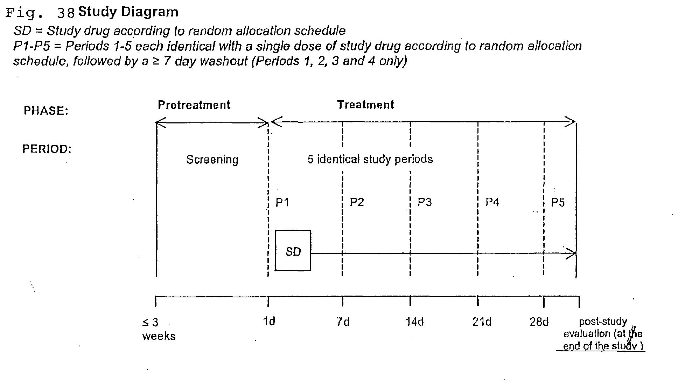

Figure 38 presents the design for this study.

*Treatments*

The following treatment schemes were administered according to a defined Random Allocation Schedule (RAS):

A =    1 tablet of Oxycodone PR 20 mg + 1 tablet of Naloxone PR 5 mg + 2 tablets of Naloxone placebo (Oxynal 20/5)

B =    1 tablet of Oxycodone PR 20 mg + 1 tablet of Naloxone PR 15 mg + 2 tablets of Naloxone placebo (Oxynal 20/15)

C =    1 tablet of Oxycodone PR 20 mg + 3 tablets of Naloxone PR 15 mg (Oxynal 20/45)

D =    1 tablet of Oxycodone PR 20 mg + 3 tablets of Naloxone placebo (Oxycodone PR)

E =    1 tablet of Oxycodone placebo + 3 tablets of Naloxone placebo (Placebo)

*Plasma Concentration Data*

Pharmacokinetic blood samples (9 mL) were taken for 24 hours after administration of study drug in each period.
Blood samples for determining oxycodone, noroxycodone, oxymorphone, noroxymorphone, naloxone, 6-β-naloxol, naloxone-3-glucuronide, and naloxol-glucuronide concentrations were obtained for each subject during each of the 5 study periods immediately before dosing; and at 1, 2, 3, 4, 5, 6, 8, 12, and 24 hours after dosing (10 blood samples per study period).

4. Efficacy Parameters

4.1 Experimental Pain Model

Analgesic effects were assessed by means of an experimental human pain model based on the chemosomatosensory pain-related cortical potentials (CSSEPs) and pain-ratings after specific phasic nociceptive stimulation of the nasal mucosa with gaseous $CO_2$. In addition, intensity estimates of tonic pain produced by stimulation of the nasal mucosa with dry air at controlled flow and temperature were employed.
Within the present pain model, the following were used as indicators of analgesia:

- post-treatment decrease in pain-ratings and/or
- post-treatment decrease in amplitudes of pain-related evoked potentials and/or
- post-treatment increase in latencies of pain-related evoked potentials, relative to the pretreatment values.

Each CO2 concentration was evaluated separately.
Primary target parameters were pain-related evoked cerebral potentials:

1. Base-to-peak amplitudes PI, N1 and P2, peak-to-peak amplitudes PI N1 and N1P2 of pain related evoked potentials
2. Latencies PI, N1 and P2 of pain-related evoked potentials
3. Intensity estimates of phasic (C02-) pain
4. Intensity estimates of tonic pain

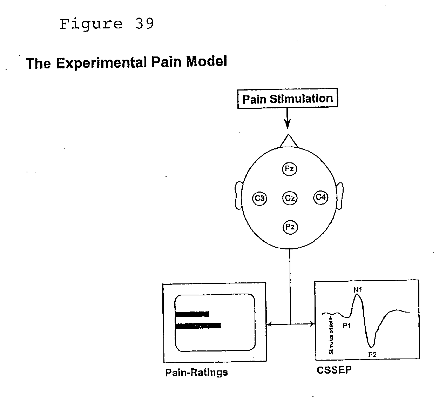

A schematic presentation of the experimental pain model is presented in Figure 39. During the experiments, subjects were comfortably seated in an air-conditioned room. To mask switching clicks of the chemical stimulator, white noise of approximately 50 dB SPL was used.
After painful stimulation of the nasal mucosa, subjects rated the intensity of the perceived pain by means of a visual analog scale. Concomitantly to the stimuli, the EEG was recorded from 5 positions (Fz, Cz, Pz, C3, C4) and pain-related evoked potentials were obtained

*Time Schedule of an Experimental Session*

In a training session taking place within 2 weeks prior to the actual experiments the subjects became acquainted with the experimental conditions and procedures. Especially, a breathing technique was trained by means of which it was possible to avoid respiratory flow inside the nasal cavity during stimulation (velopharyngeal closure). Otherwise the respiratory flow could have influenced the measurement of the evoked potentials and an investigation of the temporal characteristics would have been impossible.
Analgesimetric measurements were taken over a period of 6 hours after drug administration. On each study day, 4 analgesimetric sessions were carried out:

> session 0 : Baseline, immediately before administration of the study drug
> sessions 1-3 : 1, 3 and 6 hours after administration of the study drug

One session lasted for 36 minutes.

In the first 20 minutes, 40 phasic $CO_2$-stimuli were applied (20 stimuli at a concentration of 70% and 20 at a concentration of 60%, interstimulus interval 30 s). In response to these stimuli, pain-related potentials and subjective intensity estimates were recorded. Subsequently, tonic pain was induced for 16 minutes and subjects had to rate the intensity of the dull, burning pain.

*Phasic Painful Stimulation of Nasal Mucosa*

$CO_2$-stimuli were mixed in a constantly flowing air stream with controlled temperature (36.5°C) and humidity (80 % relative humidity) presented to the left nostril (stimulus duration 200 ms, interstimulus interval 30 s). As demonstrated in previous publications, presentation of $CO_2$-stimuli did not simultaneously activate mechano- or thermoreceptors in the nasal mucosa. During intervals between phasic stimuli subjects performed a simple tracking task on a video screen. Using a joystick, they had to keep a small square inside a larger one that randomly moved around.

*Tonic Painful Stimulation of Nasal Mucosa*

Following the period of phasic stimulation, tonic painful stimulation was induced into the right nostril by means of a dry air stream of controlled temperature (32°C), flow (8 L *$min^{-1}$) and humidity (20 % relative humidity) for 16 min.

4.2 Pharmacokinetic Parameters

The following pharmacokinetic parameters were calculated from the plasma concentrations of oxycodone, noroxycodone, oxymorphone, naloxone, 6-$\beta$-naloxol, naloxone-3-glucuronide, and naloxol-glucuronide:

- Area under the plasma concentration time curve measured from the time of dosing to the last measurable concentration (AUCt)
- Area under the plasma concentration time curve measured from the time of dosing to infinity (AUCINF)
- Maximum observed plasma concentration (Cmax)
- Time to maximum observed plasma concentration (tmax)
- Terminal phase rate constant (LambdaZ); Terminal phase half-life (t1/2Z).

AUCt was calculated using the linear trapezoidal method. Where possible, the terminal phase rate constants were estimated using those points determined to be in the terminal log-linear phase.

Half-life values (t1/2Z) were determined from the ratio of ln2 to LambdaZ. The areas under the plasma concentration-time curve between the last measured point and infinity were calculated from the ratio of the final observed plasma concentration (Clast) to LambdaZ. This was added to the AUCt to yield the area under the plasma concentration-time curve between the time of administration and infinity (AUCINF).

Log transformed data for AUCt, AUCINF (if available), and Cmax for each analyte were analyzed using a mixed effect linear model, with fixed terms for treatment, sequence and period and a random term for subjects. Compound symmetry was assumed. Treatment population geometric means were estimated from the exponential of the treatment LS Means. Ratios of treatment population geometric means were estimated by exponentiating the difference (test-reference) between treatment least square means for the comparisons of interest, and 90% confidence intervals for the ratios were calculated.

The data for tmax, LambaZ and t1/2Z were also analyzed using a mixed effect linear model, with fixed terms for treatment, sequence and period and a random term for subject. Compound symmetry was assumed. Treatment population means were estimated by treatment LS Means. Treatment differences for the comparisons of interest and their associated 90% confidence intervals were calculated from the least square means.

The relative systemic availabilities (Freit, and FrelINF) and the Cmax ratio were obtained from the ratio of AUCt, AUCINF and Cmax values respectively for differences defined in the following comparisons of interest for oxycodone, noroxycodone, and oxymorphone:

- Oxynal 20/5 A vs. Oxycodone PR D
- Oxynal 20/15 B vs. Oxycodone PR D

- Oxynal 20/45 C vs. Oxycodone PR D

The relative systemic availabilities (Freit, and FreIINF) and the Cmax ratio were obtained from the dose adjusted ratio of AUCt, AUCINF and Cmax values respectively for differences defined in the following comparisons of interest for naloxone, 6-β-naloxol, naloxone-3-glucuronide, and naloxol-glucuronide:

- Oxynal 20/15 B vs. Oxynal 20/5 A
- Oxynal 20/45 C vs. Oxynal 20/5 A

As there should not be any oxycodone or naloxone present when the placebo treatment was given, there were only four treatments included in the analysis.

All pharmacokinetic calculations were performed with WinNonlin Enterprise Version 4.1.

4.3 Efficacy Assessments/Pharmacodynamic Measurements

*Pain-related evoked Potentials*

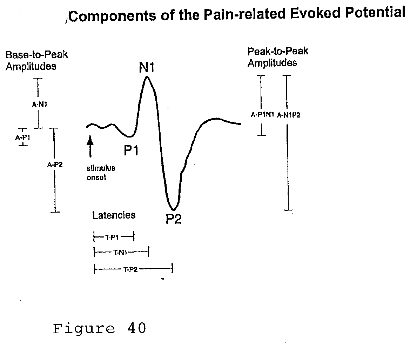

The EEG was recorded from 5 positions of the international 10/20 system (Cz, C3, C4, Fz and Pz; see Figure 40) referenced to linked earlobes (A1 +A2). Possible eye blink artifacts were monitored from an additional site (Fp2/A 1 +A2). Stimulus linked EEG segments of 2040 ms duration were sampled with a frequency of 250 Hz (band pass 0.2 - 30 Hz, pre-stimulus period 512 ms). The recorded analog EEG segments were then converted to digital and filed electronically. The average value for each recording position was separately calculated, discarding all eye blink contaminated records. By this procedure pain-related evoked potentials were obtained in response to the painful CO2 stimuli. Base to peak amplitudes PI, N1 and P2, the peak to peak amplitudes PI N1 and N1 P2 and the latencies of PI, N2 and P2 were measured. Wherever the time of measurement was used in the data analysis, the mid-time of a session was taken. Figure 40 presents the components of the pain-related evoked potentials.

*Intensity Estimates of Phasic Pain*

Within 3 - 4 seconds after presentation of each $CO_2$ stimulus, subjects compared the perceived intensity to a standard stimulus (70% v/v $CO_2$) presented at the beginning of the first session of each trial day. The intensity of the pain was rated by means of a visual analog scale displayed on a computer monitor (see Figure 39). The intensity of the standard stimulus was defined as 100 Estimation Units (EU). The mid-time of a session was regarded as time of measurement. Intensity estimates of the $CO_2$ stimuli (60% and 70%) were evaluated separately for each concentration. On a trial day, the ratings of each post-treatment session were evaluated relative to base line values. The mid-time of a session was regarded as time of measurement.

*Intensity Estimates of Tonic Pain*

The intensity of pain evoked by the tonic stimuli was estimated as described for the phasic stimuli. Subjects rated the pain intensity every 30 seconds during the 16 minutes stimulation period. Since in previous studies the tonic pain reached its steady state after 8 minutes of stimulation, only estimates of the second half of the 16 minutes stimulation period were analyzed. For further statistical evaluation, the average of single estimates was calculated for each session. The mid-time of the second half of a stimulation period was regarded as time of measurement.

4.4 Safety Assessments

Safety assessments consisted of recording of all adverse events and serious adverse events, pre-study and post-study hematology, biochemistry, urine values, ECGs, and physical examinations, and regular measurement of vital signs (including blood oxygen saturation).

*Adverse Events*

An adverse event (AE) was any unfavorable and unintended sign (including an abnormal laboratory finding), symptom, or disease temporally associated with the use of a medicinal (investigative) product, whether or not related to the medicinal (investigative) product.

A non-leading question was asked at each pharmacodynamic assessment time, i.e. "How do you feel?" If an AE occurred the investigator decided about the subject's further participation in the study. In case of discontinuation, the subject stopped receiving study medication and was followed-up until health status was back to baseline values. End of study physical examination, 12-lead ECG, hematology, biochemistry, and urine analysis were performed at this point.

All adverse events occurring during the study for subjects who received study drug were recorded. For each adverse event, the following information was recorded:

- Description (e.g. headache);
- Date of onset;
- Duration (minutes, several hours, one day, several days, > 1 week, ongoing);
- Intensity (slight, moderate, severe);
- Actions (none, intensified observation);
- Causality (likely, unlikely, not assessable);
- Frequency (once, occasionally, often);
- Seriousness (not serious, serious).

The Investigator carefully evaluated the comments of the subject and the response to treatment in order to judge the true nature and severity of the adverse The Investigator assessed the causal relationship of the AE to study medication on the grounds of all available information.

*Serious and/or unexpected Adverse Events*

If evidence of serious adverse drug events were encountered, appropriate supportive and/or definitive therapy was to be given by the responsible investigator. Clinical, laboratory and diagnostic measures were employed as required in an attempt to elucidate the etiology of the adverse event. Subjects were closely followed-up by the study staff until the complete recovery of the SAE could be justified by data obtained through Le. laboratory examinations. Appropriate remedial measures were taken and the response recorded.

A serious adverse event (SAE) was any unfavorable medical occurrence that at any dose:

- Resulted in death;
- Was life-threatening;
- Required in-patient or prolonged hospitalization; Resulted in persistent or significant disability/incapacity.

According to the definition described in the study protocol an unexpected adverse event was an adverse event which nature or severity was not consistent with the applicable product information (i.e. Investigator's Brochure for a pre-approved product or package insert/summary of product characteristics for an approved product).

5. Efficacy/Pharmacodynamic Results

*Primary Efficacy Results*

The primary end points of this study were:

- Pain-related evoked potentials (EEG)
- Intensity estimates of phasic pain
- Intensity estimates of tonic pain

*Pain-related Evoked Potentials*

A statistically significant overall effect of active treatments could be shown for the following parameters:

- Amplitude PI was reduced after stimulation with 70 % $CO_2$ at recording position - Cz:

  - all active treatments reduced the amplitude significantly compared to placebo no significant naloxone
  - no significant naloxone effect could be observed

- Latency PI was increased after stimulation with 70 % C02 at recording positions

  - C3:

- all active treatments increased the latency compared to placebo
- after administration of Oxynal (oxycodone/naloxone) 20/5,20/45, and oxycodone alone the increase was significant compared to placebo
- no significant naloxone effect could be observed

  - C4:

    - all active treatments increased the latency significantly compared to placebo
    - no significant naloxone effect could be observed

  - Fz:

    - all active treatments increased the latency compared to placebo
    - after administration of Oxynal 20/5, 20/45, and oxycodone alone the increase was significant compared to placebo
    - no significant naloxone effect could be observed

  - Pz:

    - all active treatments increased the latency compared to placebo
    - after administration of Oxynal 20/5 and oxycodone alone the increase was significant compared to placebo
    - no significant naloxone effect could be observed

  - Cz:

    - all active treatments increased the latency compared to placebo
    - after administration of Oxynal 20/5 and oxycodone alone the increase was significant compared to placebo
    - a naloxone effect could be observed
    - after administration of Oxynal 20/15 the increase was significantly less compared to oxycodone alone

- Latency P2 was increased after stimulation with 70 % C02 at recording positions

  - Cz:

    - all active treatments increased the latency compared to placebo
    - after administration of Oxynal 20/5, 20/15, and oxycodone alone the increase was significant compared to placebo
    - no significant naloxone effect could be observed

  - Pz:

    - all active treatments increased the latency compared to placebo
    - after administration of Oxynal 20/5, 20/15, and oxycodone alone the increase was significant compared to placebo
    - no significant naloxone effect could be observed

- Amplitude P1 N 1 was reduced after stimulation with 60 % $CO_2$ at recording position

  - C4:

    - all active treatments reduced the amplitude compared to placebo
    - after administration of oxycodone alone the reduction was significant compared to placebo
    - a dose-dependent naloxone effect could be observed
    - after administration of Oxynal 20/15 and Oxynal 20/45 the reduction was significantly less than from oxycodone alone

- Latency PI was increased after stimulation with 60 % $CO_2$ at recording positions

- C3:

  - all active treatments increased the latency significantly compared to placebo
  - a dose-dependent naloxone effect could be observed
  - after administration of Oxynal 20/45 the increase was significantly less compared to oxycodone alone

- C4:

  - all active treatments increased the latency compared to placebo
  - after administration of Oxynal 20/5 and oxycodone alone the increase was significant compared to placebo
  - a dose-dependent naloxone effect could be observed
  - after administration of Oxynal 20/15 and 20/45 the increase was significantly less compared to oxycodone alone

- Fz:

  - all active treatments increased the latency significantly compared to placebo
  - a naloxone effect could be observed
  - after administration of Oxynal 20/15 and 20/45 the increase was significantly less compared to oxycodone alone

- Pz:

  - all active treatments increased the latency compared to placebo
  - after administration of Oxynal 20/5 and oxycodone alone the increase was significant compared to placebo
  - a naloxone effect could be observed
  - after administration of Oxynal 20/15 the increase was significantly less compared to oxycodone alone

- Cz:

  - all active treatments increased the latency significantly compared to placebo
  - a dose-dependent naloxone effect could be observed
  - after administration of Oxynal 20/15 and 20/45 the increase was significantly less compared to oxycodone alone

- Latency P2 was increased after stimulation with 60 % C02 at recording positions - Fz

  - all active treatments increased the latency compared to placebo
  - after administration of Oxynal 20/5 and Oxynal 20/15 the increase was significant compared to placebo
  - no significant naloxone effect could be observed

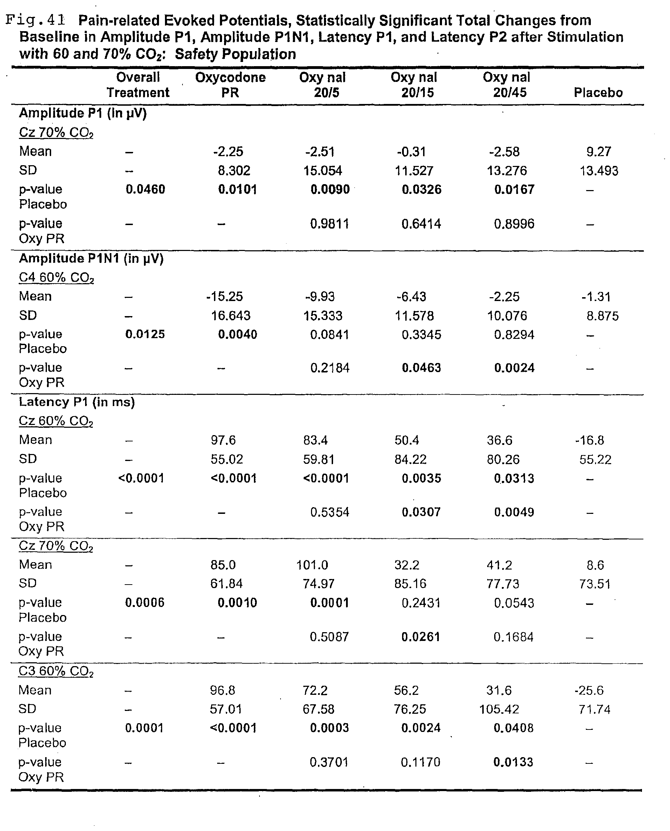

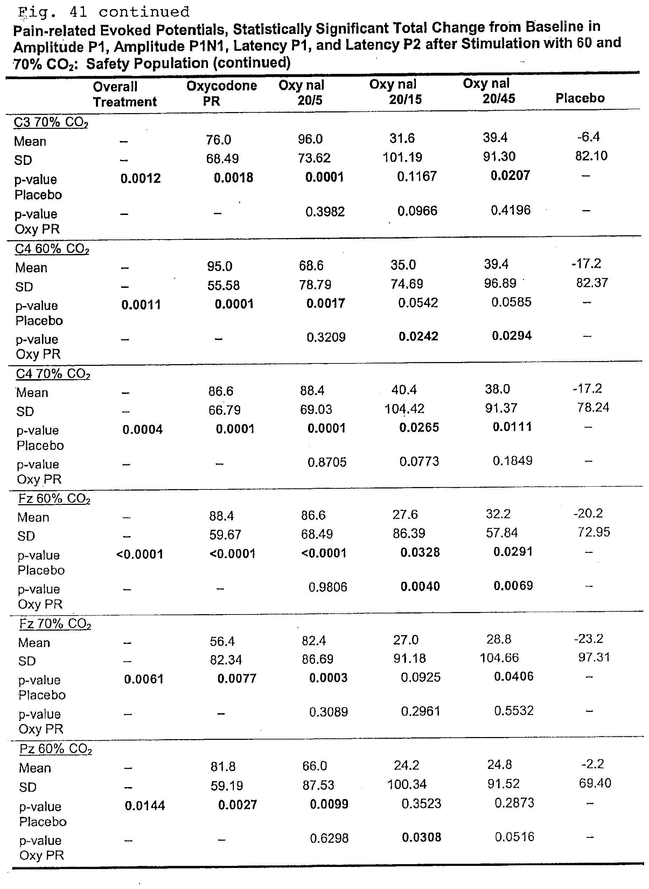

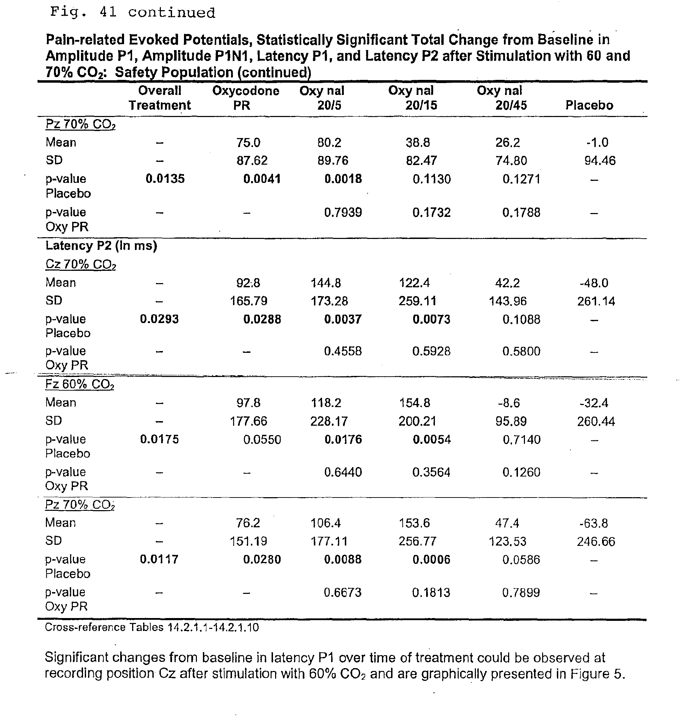

Figure 41 presents statistically significant total changes from baseline in pain-related evoked potentials after stimulation with 60 and 70% $CO_2$ for safety population.

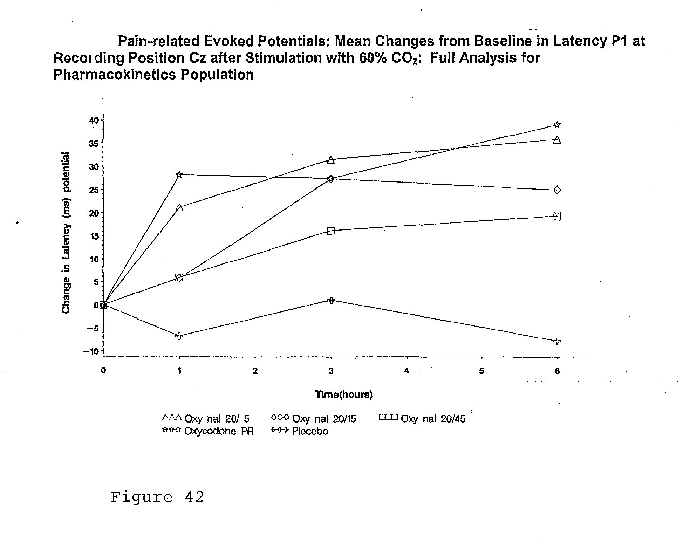

Figure 42 shows pain-related Evoked Potentials and Mean Changes from Baseline in Latency P1 at Recording Position Cz after Stimulation with 60% $CO_2$ for full analysis population.

*Intensity Estimates of Phasic Pain*

A decrease in intensity estimates of phasic pain stimuli with 70% $CO_2$ was observed after administration of active treatments. A dose of 45mg naloxone seemed to antagonize partly the oxycodone effect. However, compared to placebo, these effects just failed to reach statistical significance.

Table 29 presents intensity estimates of phasic pain stimuli with 70% CO2, total change from baseline by treatment group.

Table 29: Intensity Estimates of Phasic Pain Stimuli with 70% $CO_2$ in Estimation Units, Total Change from Baseline: Safety Population

| Treatment | Overall treatment | Oxy PR | Oxynal 20/5 | Oxynal 20/15 | Oxynal 20/45 | Placebo |
|---|---|---|---|---|---|---|
| Mean | - | -21.6 | -36.1 | -28.1 | -8.1 | 2.1 |
| SD | - | 72,3 | 68.99 | 54.72 | 55.26 | 55.60 |
| p-value Placebo | 0.0735 | n.d. | n.d. | n.d. | n.d. | - |
| p-value Oxy PR | - | - | n.d. | n.d. | n.d. | - |

n.d. = not determined due to non-significant overall treatment effect

*Intensity Estimates of Tonic Pain*

All treatments containing oxycodone showed a reduction in the intensity estimates of tonic pain (2nd half of the stimulation period). The results of all 4 active treatments showed statistically significant differences to baseline. It was not possible to distinguish between the effects of the different naloxone doses.

Table 30 presents intensity estimates of tonic pain, total change from baseline measured in the 2nd half of the stimulation period by treatment group.

Table 30. Intensity Estimates of Tonic Pain in Estimation Units, Total Change from Baseline Measured in the 2nd Half of the Stimulation Period: Safety Population

| Treatment | Overall treatment | Oxy PR | Oxynal 20/5 | Oxynal 20/15 | Oxynal 20/45 | Placebo |
|---|---|---|---|---|---|---|
| **Mean** | **-** | **-41.1** | **-57.6** | **-58.0** | **-57.0** | **4.9** |
| **SD** | **-** | **52.04** | **62.47** | **60.38** | **56.87** | **47.23** |
| **p-value Placebo** | **0.0005** | **0.0055** | **0.0002** | **0.0001** | **0.0005** | **-** |
| **p-value Oxy PR** | **-** | **-** | **0.2822** | **0.2307** | **0.4017** | **-** |

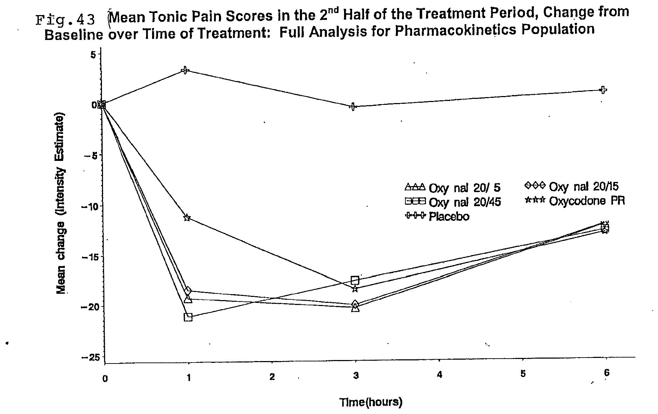

The change from baseline in the mean tonic pain scores (2nd half of treatment period) over time of treatment is graphically presented in Figure 43.

*Clinical Pharmacology Results*

Analyses of pharmacokinetic parameters were performed using data from all the subjects in the pharmacokinetic population.

*Oxycodone Results*

- AUCt

The mean AUCt values for oxycodone were very consistent between treatments, ranging from 213.6 ng.h./ml for the Oxynal 20/45 treatment to 239.6 ng.h./ml for the Oxynal 20/5 treatment.

In terms of AUCt, each of the Oxynal combined treatments provided an equivalent availability of oxycodone to the reference treatment, oxycodone PR tablets 20 mg. All of the relative bioavailability calculations based on AUCt had 90% confidence intervals that were within the 80 - 125% limits of acceptability for bioequivalence.

- t1/2Z

The mean t1/2Z values obtained for oxycodone ranged from 7.1 h for Oxynal 20/15 to 9.0 h for Oxynal I 20/5.

- AUCINF

The mean AUCINF values for oxycodone differed between treatments, ranging from 221.1 ng.h.ml-1 for Oxynal 20/45 to 291.1 ng.h.mr1 for Oxynal 20/5.

In terms of AUCINF, the Oxynal 20/5 combined treatment provided an equivalent availability of oxycodone to the reference treatment, oxycodone PR tablets 20 mg. The Oxynal 20/15 and OXN 20/45 combined treatments provided a slightly reduced availability of oxycodone compared with oxycodone PR tablet 20 mg, and had associated 90% confidence intervals that were outside the lower limits of acceptability for bioequivalence.

- Cmax

The mean Cmax values for oxycodone were consistent between treatments, ranging from 19.7 ng./ml for the Oxynal 20/45 combined treatment to 23.9 ng./ml for the Oxynal 20/5 treatment.

Each of the Oxynal combined treatments provided an equivalent Cmax of oxycodone to the reference treatment, oxycodone PR tablet 20 mg. All of the Cmax ratio calculations had 90% confidence intervals that were within the 80 - 125% limits of acceptability for bioequivalence.

- tmax

The median tmax values appeared consistent between all the treatments and ranged from 2.4 h for Oxynal 20/15 and oxycodone PR tablets, to 3.1 h for Oxynal 20/5 and Oxynal 20/45.

Tables 31 and 32 show summaries of the pharmacokinetic parameters of oxycodone.

Table.31 Summary of Pharmacokinetic Parameters for Oxycodone by Treatment: Full Analysis Population for Pharmacokinetics

| Pharmacokinetic Parameter | Oxynal 20/5 | Oxynal 20/15 | Oxynal 20/45 | Oxycodone PR |
|---|---|---|---|---|
| **AUCINF (ng.h/m)** | | | | |
| N | 11 | 12 | 13 | 13 |
| Arithmetic mean (SD) | 291.1 (93.08) | 249.2 (53.55) | 221.1 (36.36) | 264.3 (58.13) |
| Geometric mean | 280.2 | 243.9 | 218.2 | 258.4 |
| **AUCt (ng.h/ml)** | | | | |
| N | 16 | 18 | 17 | 19 |
| Arithmetic mean (SD) | 239.6 (79.29) | 223.7 (55.35) | 213.6 (40.55) | 223.0 (48.26) |
| Geometric mean | 229.1 | 217.1 | 209.8 | 218.1 |
| **Cmax (ng/ml)** | | | | |
| N | 16 | 18 | 17 | 19 |
| Arithmetic mean (SD) | 23.9 (9.94) | 21.3 (4.52) | 19.7 (3.37) | 21.4 (3.60) |
| Geometric mean | 22.6 | 20.9 | 19.4 | 21.2 |
| **tmax (h)** | | | | |
| N | 16 | 18 | 17 | 19 |
| Arithmetic mean (SD) | 2.50 (0.966) | 2.44 (1.149) | 3.06 (1.919) | 2.84 (1.740) |
| Median (Min, Max) | 3.0 (1.00, 4.00) | 2.0 (1.00, 5.00) | 3.0 (1.00, 8.00) | 2.0 (1.00, 6.00) |
| **tl/2Z** | | | | |
| N | 13 | 13 | 15 | 15 |
| Arithmetic mean (SD) | 8.99 (3.434) | 7.12 (1.580) | 7.84 (2.449) | 8.66 (3.440) |
| (Min, Max) | (5.57, 17.31) | (3.90, 10.25) | (4.69, 13.75) | (4.75, 17.32) |

Table 32. Oxycodone Summary of Ratios for AUCt, AUCINF, Cmax and differences for tmax and t1/2Z: Full Analysis Population for Pharmacokinetics

| Pharmacokinetic Parameter AUCINF (ng.h/m) | Oxynal 20/5 Oxycodone PR | Oxynal 20/15 Oxycodone PR | Oxynal 20/45 Oxycodone PR |
|---|---|---|---|
| Ratio (%) | 100.6 | 85.0 | 83.1 |
| 90% CI | 89.2, 113.5 | 75.1, 96.3 | 73.4, 94.2 |
| **AUCt (ng.h/ml)** | | | |
| Ratio (%) | 104.9 | 99.1 | 98.3 |
| 90% CI | 94.0, 117.0 | 89.3, 109.9 | 88.3, 109.5 |
| **Cmax (ng/ml)** | | | |
| Ratio (%) | 106.3 | 96.5 | 94.9 |
| 90% CI | 95.0, 119.0 | 86.8, 107.4 | 85.0, 106.0 |
| **tmax (h)** | | | |
| Difference (%) | -0.08 | -0.37 | 0.30 |
| 90% CI | -0.88, 0.72 | -1.13, 0.39 | -0.49, 1.09 |
| **tI/2Z** | | | |
| Difference (%) | 0.02 | -2.49 | -1.28 |
| 90% CI | -1.85, 1.90 | -4.43, -0.55 | -3.20, 0.64 |

6. Conclusions

*Primary Efficacy Results*

In this study a pain model was employed as a pain assessment system. This model permitted a quantitative measurement of pain-related evoked potentials (EEG) and pain ratings. Administration of the active treatments in this study resulted in significant reductions of amplitudes PI and P1N1 and in significant prolongations of latencies PI and P2 of pain-related evoked potentials (EEG) in response to painful stimulation of the nasal mucosa. This can be clearly regarded as an indicator of opioid analgesic effects and has been demonstrated in various studies of non-opioid and opioid analgesics with this experimental pain model.

In this study a significant decrease in pain-related evoked potential amplitudes (PI, P1N1) induced by oxycodone could be seen at central recording sites C4 and Cz. Similar results have been obtained in prior investigations of opioids with agonistic activity at $\mu$-receptors. The increase in pain-related evoked potential latencies induced by oxycodone could be seen at all recording sites and was most pronounced in latency P1 indicating analgesic effects that are typically observed in opioids.

The magnitude of amplitude reduction after stimulation with 60% $CO_2$ was 35.3% in amplitude P1 N1 at C4 after 20 mg oxycodone, 24.5% after combination with 5 mg naloxone, 23.7% after combination with 15 mg naloxone, and 12.8% after combination with 45 mg naloxone compared to baseline. Compared to other investigations with the same model, the magnitude of the analgesic effects of oxycodone is similar to other analgesics.

In this study, naloxone did not produce a significant reversal of oxycodone effects in amplitude P1 (Cz) after administration of a strong stimulus of 70% $CO_2$. After administration of a weak stimulus of 60% $CO_2$, naloxone produced a significant dose-dependent reversal of oxycodone effects in amplitude P1N1 (Cz). A dose-dependent effect of naloxone on latencies was most evident on latency P1 (C4) after stimulation with 60%$CO_2$ indicating a reduction of the effects of oxycodone. No clear indication for a naloxone-induced reversal of the oxycodone effect could be observed on latency P1 after stimulation with 70%$CO_2$ and on latency P2.

In this study, the dose-dependent opioid antagonizing effects of naloxone (reversal of reduction in amplitudes and prolongation of latencies) were indicated to be more pronounced in response to weaker stimuli (60% $CO_2$) than in response to stronger stimuli (70% $CO_2$).

In conclusion, taking into account the results of the pain-related evoked potentials at all recording positions, measured in healthy volunteers, there is an indication of a dose-dependent influence of naloxone on typical amplitude and latency changes, caused by oxycodone as an opioid. The data from this pain model seems to indicate that, based on 20 mg oxycodone PR, a dose of naloxone PR that does not significantly influence the analgesic effect (EEG) of oxycodone would be below 15 mg.

A decrease in intensity estimates of phasic pain stimuli with 70% $CO_2$ was observed after administration of active treatments. A dose of 45mg naloxone seemed to antagonize partly the oxycodone effect. However, compared to placebo, these effects just failed to reach statistical significance.

Intensity estimates of tonic pain significantly decreased after administration of active treatments compared to placebo. However, there was no evidence of antagonism of the effect of naloxone. Response bias could have played a role in this situation. As soon as the subjects experienced any opioid effect, they seemed to cluster estimates to the same level.

*Pharmacokinetic results*

It was anticipated that low oral bioavailability would prevent the complete pharmacokinetic assessment of naloxone. This was confirmed as low naloxone concentrations meant that it was not possible to estimate AUCt values for most of the subjects receiving Oxynal 20/5, or AUCINF values for any of the dose strengths. Naloxone-3-glucuronide was present in the plasma in much higher concentrations. As for other pharmacokinetic studies on OXN, the conclusions for the naloxone component of the open combination treatments were based on naloxone-3 glucuronide parameters.

Similar amounts of oxycodone were available from each of the treatments. The AUCt values were not affected by increasing doses of naloxone. AUCINF values decreased slightly with increasing doses of naloxone; the bioavailability assessments showed that Oxynal 20/5 provided an equivalent availability of oxycodone to oxycodone PR, whilst both Oxynal 20/15 and 20/45 had bioavailability assessments that had 90% confidence intervals below the lower limit of acceptability for bioequivalence. The increasing doses of naloxone did not have an affect on the mean dose-adjusted Cmax values for oxycodone.

7. Summary

*Conclusions on Primary Efficacy Results*

- The analgesic effect of oxycodone PR with different dosages of the opioid antagonist naloxone PR could be demonstrated in an experimental pain model based on evoked potentials after stimulation of the nasal mucosa with $CO_2$. The decreases in amplitudes were in the range of other opioids that have been studied with this model before. The dose dependent opioid antagonizing effects of naloxone (reversal of reduction in amplitudes and reversal of prolongation in latencies of pain-related evoked potentials) were more pronounced in response to weaker stimuli (60% $CO_2$) than in response to stronger stimuli 70% $CO_2$.
- A decrease in intensity estimates of phasic pain stimuli with 70% $CO_2$ was observed after administration of active treatments. A dose of 45 mg naloxone seemed to antagonize partly the oxycodone effect. Compared to placebo, these effects failed to reach statistical significance. Moreover, this only applied if low amounts of oxycodone were present. In a 2:1 ration of oxycodone to naloxone this should not be observed.
- Intensity estimates of tonic pain significantly decreased after administration of active treatments compared to placebo. There was no evidence of antagonism of the effect of naloxone.

*Pharmacokinetic conclusion*

- The availability of oxycodone was similar from each of the active treatments suggesting that the co-administration of naloxone PR tablets did not affect the pharmacokinetics of oxycodone.

**Reference Example 5: Precipitated Withdrawal**

1. Objective

The overall goal of this study was to determine whether intravenous oxycodone co-administered with naloxone in a 2: 1 ratio would precipitate signs of opioid withdrawal in rats physically dependent on oxycodone and consequently confirm the OXN combination as a parenteral abuse deterrent product.

2. Test animals

Male Sprague Dawley rats were obtained from Harlan Sprague Dawley (Indianapolis, Indiana) and acclimated for one week. Prior to randomization, the animals were weighed and examined in detail for signs of physical disorder. Animals determined to be acceptable were assigned randomly to groups using a random number generator (University of Dublin, Trinity College). The acceptable range of body weights were: $\pm$ 10 % of the mean. The animal weights were recorded. Disposition of animals not selected for the study was documented in the study data records. The rats were identified using ear-clip identification numbers starting at 1, 2, 3... for this protocol. The notebook identified these rats as VCU Animal Number (VAN) 1,2,3.

3. Study Design, Test Treatment, Dose and Mode of Administration

Sprague Dawley rats (8/group) were rendered physically dependent on oxycodone by surgically implanted osmotic pumps that infused oxycodone subcutaneously at 1.5 mg/kg/h for 7 days. Since analgesic tolerance develops simultaneously during the development of physical dependence, the analgesic $ED_{80}$ value of oxycodone in tolerant rats (4.8 mg/kg) provided a quantifiable oxycodone dose on which to base the 2:1 oxycodone/naloxone ratio. A separate group of rats was dosed with vehicle:naloxone intravenously and compared to the group administered OXN. Oxycodone and naloxone plasma levels were measured in dependent animals throughout the 60-min observation period.

*Dose Preparation and Verification*

Oxycodone hydrochloride was dissolved in isotonic saline. One 2 - 5 mL sample from each dosing solution was taken within 60 min post dosing.

*Time Course of Intravenous Oxycodone Antinociceptive Effects in Opioid Naive Rats*

Baseline tail-withdrawal latencies were obtained in groups of 8 male Sprague-Dawley rats using the 51°C warm-water tail withdrawal test by immersing the tail to the 7 cm point and measuring the latency in seconds before the rat withdrew its tail from the water. Two groups were then administered either isotonic saline or oxycodone i.v. and tested repeatedly at 2.5, 5, 10, 15, 20, 30, 40, 50 and 60 minutes post dose. A cut-off latency of 15-seconds was used to prevent the development of any tissue damage. Tail-withdrawal latencies were recorded and the data was converted into the percentage of maximum possible effect (%MPE).

*Intravenous Oxycodone Dose Response in Naive Animals*

Dose-response curves were constructed to determine the ED80 value of intravenously administered oxycodone. Baseline tail-withdrawal latencies were obtained in groups of 8 male Sprague-Dawley rats in the 51°C warm-water tail withdrawal test. Individual groups of rats were administered incremental doses of oxycodone (i.e., 0.15, 0.25, 0.35, 0.45 and 0.6 mg/kg) and tested 10-minutes later at the peak time of oxycodone antinociception. Tail-withdrawal latencies were recorded, and the data was converted into the percentage of maximum possible effect (%MPE). The dose response curve was analyzed using least squares linear regression analysis followed by calculation of ED80 value (i.e. the dose of oxycodone to elicit 80% MPE in the warm water tail-withdrawal test). These values are calculated using least squares linear regression analysis followed by calculation of 95% confidence limits.

*Surgical procedures*

Animals were randomized and acclimated for one week as described in Section 3.1. Vehicle control pumps contained sterile filtered isotonic saline. Alzet 2ML 1 osmotic mini pumps were loaded with oxycodone solution as described in "Alzet Osmotic Minipumps: Technical Information Manual" from DURECT Corp., Cupertino, CA. The loaded pumps were primed by placing them in sterile isotonic saline at 37 °G for 3-h before implanting them in the rats. The rats were briefly anesthetized with isoflurane USP (Henry Schein, Inc. Melville, NY, U.S.A.) for implantation of 2ML 1 osmotic minipumps that deliver at a rate 10 mL/h. After induction of anesthesia (as noted by the absence of the righting reflex and foot pinch response). Sterile scissors were used to make a 1.5 cm incision that was expanded under the skin with hemostats in a caudal direction to open the subcutaneous space for the pump. A sterile 2ML 1 pump was then inserted under the skin and moved to the dorsum. The rats were returned to their home cages and monitored until they completely recovered from anesthesia. Pump delivery began at 4-h (DU REGT Corp.) allowing the rats 1-h to recover from the anesthesia. Therefore, time zero began 1-h after implantation of the pumps. The rats were monitored daily for signs of distress, drug toxicity, or problems with the surgical site.

*Implantation Trial (Oxycodone Infusion)*

An implantation trial was conducted in which rats were infused with oxycodone at a rate of 1.25, 1.5, 1.75 and 2.0 mg/kg/h for 7-days. The rats were then challenged with a dose of oxycodone that was predicted to yield a 50% MPE analgesic effect that was 10-fold higher than the ED50 value of oxycodone in the vehicle-pump implanted rats (e.g., vehicle-P ED50 value = 0.32 mg/kg therefore 10-fold = 3.2 mg/kg). If the challenge dose yielded a %MPE value above 50% then the predicted level of tolerance was less than 10-fold. If the value was below 50% then the predicted level of tolerance was greater than 10-fold. The infusion dose that elicited approximately a 50% MPE with the challenge was selected as the 10-fold model of tolerance. Based on our studies, the 1.5 mg/kg/h yielded nearly a 50% MPE when the rats were challenged with 3.2 mg/kg oxycodone, which is 10-fold higher than the ED50 value of the vehicle-P group.

*Development of Oxycodone Tolerance*

Several groups of rats were implanted with 2ML 1 pumps that infused oxycodone at 1.5 mg/kg/h for 7-days. After this, the individual groups (8/group) were challenged with increasing doses of oxycodone for construction of a dose-response curve for calculation of the $ED_{80}$ value. Potency-ratio determinations were made between the oxycodone-pump and vehicle-pump groups. The calculated $ED_{80}$ value was used to calculate the 2: 1 ratio of oxycodone:naloxone to precipitate withdrawal in oxycodone-dependent rats as described above.

*Precipitation of Withdrawal in Oxycodone-Dependent Rats*

The goal of this experiment was to determine the degree of naloxone-precipitated withdrawal resulting from the intravenous administration of oxycodone:naloxone in a 2:1 ratio. In this model, the analgesic $ED_{80}$ dose obtained from the oxycodone tolerant rats as determined above served as the test dose, while naloxone will be tested at one-half the $ED_{80}$ dose of oxycodone to maintain the 2:1 ratio. Rats were implanted with Alzet 2ML 1 osmotic minipumps infusing either saline vehicle or oxycodone at 1.5 mg/kg/h for 7 days as described above. After 7 days, the rats were injected intravenously with oxycodone:naloxone in a 2:1 ratio or with vehicle-naloxone and immediately placed in the observation chambers to assess for signs of naloxone-precipitated withdrawal. The complete parametric design for oxycodone-pump implanted rats required testing rats with vehicle:vehicle and oxycodone:vehicle. In addition, the parametric design required testing vehicle-pump rats with oxycodone:naloxone 2:1, vehicle:naloxone, oxycodone:vehicle and vehicle:vehicle. (see table 33).

Table 33: Parametric Study Design

| Group | N number of animals | Alzet Pump 2ML1 | Challenge Dose (mg/kg, i.v.) | Time (min) |
|---|---|---|---|---|
| 1 | 8 | Vehicle | Veh:Veh | Time-course (1 to 60 min) |
| 2 | 8 | Vehicle | Veh:Naloxone | " |
| 3 | 8 | Vehicle | $ED_{80}$ Oxy:Veh | " |
| 4 | 8 | Vehicle | $ED_{80}$ Oxy: ½ Naloxone | " |
| 5 | 8 | Oxy (1.5 mg/kg/h) | Veh:Veh | Time-course (1 to 60 min) |
| 6 | 8 | Oxy (1.5 mg/kg/h) | Veh:Naloxone | " |
| 7 | 8 | Oxy (1.5 mg/kg/h) | $ED_{80}$ Oxy:Veh | " |
| 8 | 8 | Oxy (1.5 mg/kg/h) | $ED_{80}$ Oxy: ½ Naloxone | " |

Signs of physical dependence were evaluated in rats intravenously administered the drugs, and then immediately placed back in their home cages for a 60-min observation period. The rats were evaluated for the signs of naloxone-precipitated withdrawal using the Gellert-Holtzman scale as described in Table 37 below. The table is divided into Graded Signs and Checked Signs, and assigned a weighted factor. The rats were evaluated for these signs during the 60 min observation period, the scores were collected, and a combined Global Score was assigned to each rat. Data were analyzed by combining the graded signs of escape attempts and wet-dog shakes into a single score of Grades Signs during each 15-min interval for 60-min. Checked signs were analyzed during each 15-min interval for 60-min.

Table 34. Gellert-Holtzmann Scale of Precipitated withdrawal Signs and Weighting Factors

| Sign | Weighting Factor |
|---|---|
| Graded Signs | |
| Weight loss in 2.5-h (each 1.0 % above the weight lost by control rats) | 1 |
| Number of escape attempts | |
| 2-4 | 1 |
| 5-9 | 2 |
| 10 or more | 3 |
| Number of abdominal constrictions (each one) | 2 |
| Number of wet dog shakes | |
| 1-2 | 2 |
| 3 or more | 4 |
| Checked Signs | |
| Diarrhea | 2 |
| Facial fasciculations or teeth chatter | 2 |
| Swallowing movements | 2 |
| Profuse salivation | 7 |
| Chromodacryorrhea | 5 |
| Ptosis | 2 |
| Abnormal posture | 3 |
| Erection or ejaculation | 3 |
| Irritability | 3 |

*Time-Course and Dose Response*

Tail-withdrawal latencies were recorded for the time course of intravenous oxycodone administered to naive animals. The data was converted into the percentage of maximum possible effect %MPE which is calculated as: %MPE = [(Test-Baseline)/(15-Baseline)] X 100. Time-course data was analyzed using two-factor repeated measures ANOVA followed by *post hoc* analysis using the Turkey's test (Sigma Stat Statistical Software, SPSS, Inc.). The data was analyzed to determine which oxycodone time-points were significantly different from the baseline (i.e., before drug response), and significantly different from the respective saline control at each respective time-point. The dose response curves were analyzed using least squares linear regression analysis. The calculation of $ED_{80}$ value with 95% confidence limit was completed using the PharmTools V1.1.27 software used to input the data.

*Global Rating Scores:*

The rats were assessed in 15-min intervals for both graded and checked signs for a total of 60 min. The graded signs of escape attempts and wet-dog shakes were tallied, whereas, checked signs such as diarrhea, profuse salivation, chromodactorrhea, etc. were noted as being either absent or present during the 15-min period. Both graded and checked signs were assigned a numeric score based on studies by Gellert and Holtzman (1978), and the total value for each animal was added to provide a global rating. These data were analyzed with two factor ANOVA followed *by post hoc* analysis using the Turkey's test (Sigma Stat Statistical Software, SPSS, Inc.) to determine whether the oxycodone-pump rats acutely administered vehicle:naloxone and oxycodone:naloxone elicited significant Global Rating Scores compared to vehicle-pump rats administered the same treatment. In addition, analysis determined whether the Global Rating Scores in the oxycodone-pump rats were significantly different between the groups administered vehicle:naloxone and oxycodone:naloxone.

*Graded Withdrawal Signs*

The graded signs of escape attempts and wet-dog shakes were tallied and final statistical analysis was conducted on these data using two-factor ANOVA followed *by post hoc* analysis using the Turkey's test to determine whether the oxycodone-pump rats acutely administered vehicle:naloxone and oxycodone:naloxone elicited significant Graded Withdrawal Signs compared to vehicle-pump rats administered the same treatment. In addition, analysis determined whether the Graded Withdrawal Sign in the oxycodone-pump rats were significantly different between the groups administered vehicle:naloxone and oxycodone:naloxone..

*Weight Loss*

Weight before administration of drug and 2.5-h after drug administration was obtained in order to calculate the percentage of weight loss resulting from the drug treatment (i.e.., [Baseline-2.5 h later)/Baseline] * 100 = % Weight Loss). The % Weight Loss data was analyzed using two-factor ANOVA followed *by post hoc* analysis using the Turkey's test to determine whether the oxycodone pump rats acutely administered vehicle:naloxone and oxycodone:naloxone elicited significant decreases in weight loss compared to vehicle-pump rats administered the same treatment. In addition, analysis determined whether the % Weight Loss values in the oxycodone-pump rats were significantly different between the groups administered vehicle:naloxone and oxycodone:naloxone.

*Checked Signs*

The incidence of checked signs during opioid withdrawal was also analyzed statistically within each time interval at 0-15, 15-30, 30-45 and 45-60 minutes. The data was analyzed within each time interval using contingency table Person's Chi Square analysis (Sigma Stat Statistical Software, SPSS, Inc.) to evaluate the X2 value. X2 values exceeding the critical value for 7 of 14.1 were considered statistically significant intervals for that checked behavioral sign.

*Pharmacokinetics*

A separate set of jugular-vein cannulated rats (8/group) was used for pharmacokinetic analysis. Jugular vein cannulated Sprague Dawley rats (Taconic, Germantown, NY). were randomized into two groups and acclimated for one week as described in Section 3.1. Similar to all other groups of animals in the main study, the PK animals were implanted with 2ML 1 osmotic minipumps as described in Section 3.5 and infused with oxycodone at the rate of 1.5 mg/kg/h for 7 -days. On day 7, one group received vehicle i.v., to determine the plasma concentration of oxycodone provided by the 2ML 1 osmotic minipump. The second group was intravenously administered oxycodone:naloxone at the 2: 1 ratio.

*Blood Collection*

Approximately 1 mL of blood was collected *via* the jugular vein cannula from each rat at pre dose, 5, 15, 30, 45, 60 and 75 minutes post dose.

*Summary of Sample Analysis Procedures*

Plasma samples were obtained and analyzed for oxycodone and naloxone using two liquid chromatography in tandem with mass spectroscopy (LC-MS/MS) methods. The first method was used to quantify oxycodone with a concentration curve ranging from 0.500 to 50.0 ng/mL, using 0.100 mL sample volume. The second method was used to quantify naloxone with a concentration curve ranging from 0.050 to 25.0 ng/mL using 0.100 mL plasma volume.

*Pharmacokinetic Analysis*

Noncompartmental pharmacokinetic metrics were determined using WinNonlin Version 4.1 (Pharsight Corporation) from the individual plasma concentration data obtained after dosing. This program analyzes data using the standard methods described by Gibaldi and Perrier (Reference 7.2). Any value that was below the lower limit of quantitation (LLOQ) of the assay was excluded from pharmacokinetic analyses. The area under the plasma concentration-time curve (AUC) was estimated by the linear trapezoidal rule. Mean calculations, descriptive statistics and statistical analyses were conducted using Microsoft Excel 2003; statistical significance was considered when $p \leq 0.05$.

4. Results

4.1 Pharmacology

*Intravenous Oxycodone Antinociception Time-Course Study and Dose Response in Naive Animals*

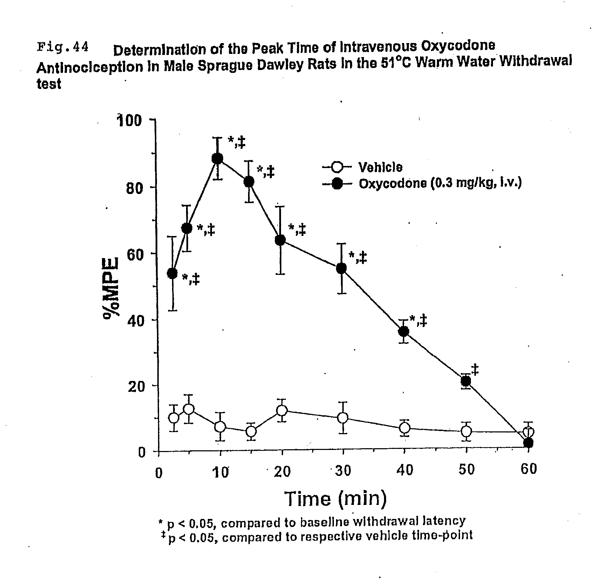

As seen in Figure 44, intravenous administration of 0.3 mg/kg oxycodone free-base (0.35 mg/kg HCl salt) to

male Sprague-Dawley rats resulted in significant antinociception in the 51°C warm water tail withdrawal test compared to rats administered isotonic saline vehicle i.v.. Two-factor repeated measures ANOVA demonstrated a significant drug treatment X repeated measure interaction $F(1,9) = 16.12$, $P < 0.001$. *Post hoc* analysis using the Turkey's test revealed that antinociception was present at the first test point of 2.5-min, and significantly above baseline latencies for 40-min. However, antinociception was significantly above the vehicle group at the 50min time-point. The peak time of antinociception was determined to be 10-min. Finally, no obvious signs were noted in the rats such as sedation, effects on motor control, respiration, or toxicity.

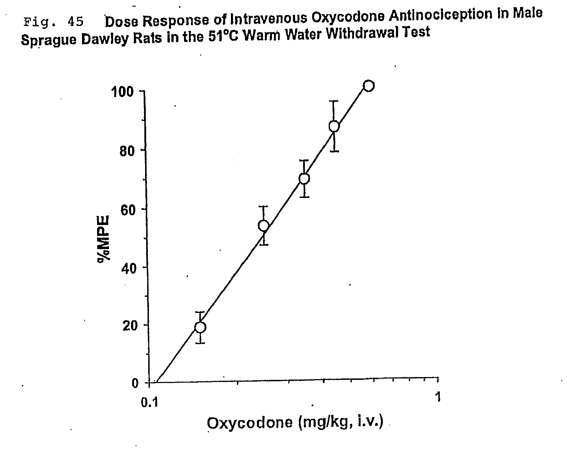

A dose-response curve was generated to determine the $ED_{80}$ value of intravenously administered oxycodone. As seen in Figure 45, an oxycodone dose-response curve was then constructed by intravenously administering groups of rats with increasing doses of oxycodone and testing them at 10-min. As seen in the Figure 45, oxycodone administered i.v. resulted in dose-dependent antinociception in the 51°C tail-withdrawal assay. The dose response curve was analyzed using least squares linear regression analysis. The calculation of $ED_{80}$ value with 95% confidence limits was completed using the method that is contained in the PharmTools V1.1.27 software used to input the data. The $ED_{80}$ value of the oxycodone free-base was 0.41 mg/kg (95% CL 0.38 to 46).

*Dose-Response in surgery animals-Implantation Trial*

Rats were surgically implanted with Alzet 2ML 1 pumps that infused isotonic saline at a rate of 10 $\mu$L/h for 7 days. These rats were designated as vehicle pump-implanted rats in order to serve as control rats. The potency of oxycodone was slightly decreased compared to naive rats following a 7 day Alzet pump implantation. The slight decrease in potency, is typically seen in most Alzet pump implantation studies due to variables such as the effects of surgery, the constant infusion, and even the physical presence of the pump on the tail-withdrawal response. Therefore, statistical comparisons of tolerance were made between the oxycodone pump-implanted rats VS and the vehicle pump-implanted rats, since the influence of the surgically implanted pump was factored out as a potential confound.

An implantation trial was conducted to estimate the oxycodone infusion dose that would elicit an 8- to 10-fold rightward shift in the dose-response curve of oxycodone. It was found that 1.5 mg/kg/h provided the closest infusion dose that approximated the line estimated to elicit a 10-fold level of antinociceptive tolerance. Infusion doses of 1.75 and 2.0 mg/kg/h would have resulted in much higher levels of tolerance, while 1.25 would have resulted in lower levels of tolerance.

*Dose-Response in tolerant animals*

The 1.5 mg/kg/h oxycodone infusion dose was selected since an 8- to 10-fold level of tolerance was expected to occur following a 7-day infusion period. As seen in Table 36 below, a 7-day oxycodone infusion resulted in tolerance, indicated by a significant 8.5-fold rightward shift in the dose-response curve for oxycodone. Rats were surgically implanted with 2ML 1 pumps that infused saline or oxycodone at 1.5 mg/kg/h for 7 days. The rats were then tested in the 51° C warm-water tail-withdrawal test after intravenous administration of oxycodone for construction of dose-response curves. The oxycodone $ED_{80}$ was found to be 4.82 mg/kg. Consequently, the corresponding naloxone dose was selected to be 2.4 mg/kg to maintain the OXN 2:1 ratio

*Naloxone-Precipitated Withdrawal in Oxycodone-Dependent Rats Using the 2:1 Ratio of Oxycodone:Naloxone*

Experiments were conducted to measure the signs of opioid abstinence (i.e., withdrawal signs) after the i.v. administration of oxycodone:naloxone in a 2:1 ratio in oxycodone-dependent rats. The intention of this model was to replicate the potential abuse of oxycodone:naloxone by the i.v. route, and to demonstrate that physically dependent rats would exhibit significant abstinence. Sprague Dawley rats were rendered physically dependent on oxycodone by surgically implanted 2ML1 osmotic pumps that infused oxycodone at 1.5 mg/kg/h for 7 days. On the test day, rats were intravenously administered the antinociceptive $ED_{80}$ dose of oxycodone (4.8 mg/kg) and 2.4 mg/kg naloxone in the 2:1 ratio, and assessed for signs of withdrawal for 60 min. Another group of 8 rats was administered "vehicle:naloxone" which was 2.4 mg/kg naloxone in isotonic saline. This group served to demonstrate the full extent of physical dependence in case the oxycodone in the presence of naloxone suppressed withdrawal.

*Global Rating Scores*

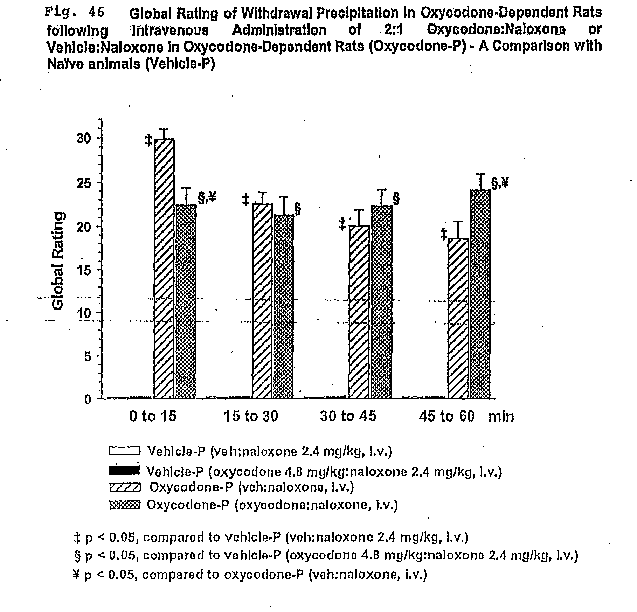

Figure 46 represents the average global rating for the main groups of interest in this study. Several observations were notable from this study. First, no signs of withdrawal were observed in the vehicle-pump groups administered oxycodone:naloxone or vehicle:naloxone, thereby demonstrating that neither the surgery nor the presence of the pump resulted in the stressful release of endogenous opioid peptides.

Second, administration veh:naloxone (2.4 mg/kg) to the oxycodone pump group resulted in a robust withdrawal that was long lasting. Withdrawal was intense in the first 15-min, and then declined incrementally, but remained significantly elevated throughout the 60-min observation. In rats injected with 2:1 oxycodone:naloxone, withdrawal was clearly evident within the first 15-min, however, the global rating score was significantly less than the veh:naloxone group. Yet, by 60-min the global rating scores in the 2:1 oxycodone:naloxone group increased so that withdrawal was significantly higher than the veh:naloxone group. Thus, rather than oxycodone suppressing withdrawal, oxycodone appeared to enhance the later stages of naloxone-precipitated withdrawal.

*Graded Withdrawal Signs*

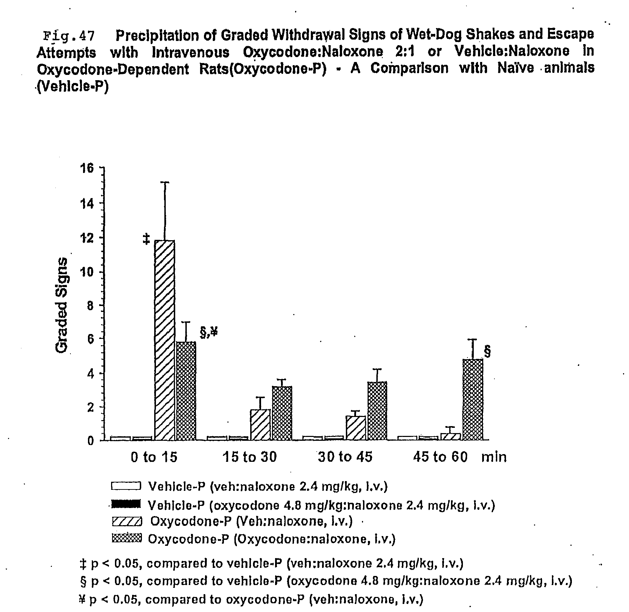

The graded signs of escape attempts and wet-dog shakes were tallied and final statistical analysis was conducted on these data using two-factor ANOVA followed *by post hoc* analysis using the Turkey's test. Figure 47 represents the average graded signs for the main groups of interest in this study. Administration veh:naloxone (2.4 mg/kg) to the oxycodone-pump group resulted in a robust withdrawal that was short-lasting that ended within the first 15-min. Withdrawal was no longer significantly present throughout the remainder of the experiment. This effect is typical of the short lasting effects of naloxone on graded signs in rodents. Similarly, administration of oxycodone:naloxone also resulted in withdrawal within the first 15-min. Withdrawal was present at low, but non-significant levels from 30- to 45-min, but then increased to statistically significant levels during the 45- to 60-min observation. The graded signs demonstrate that the co-administration of oxycodone with naloxone enhanced the later stages of withdrawal. Under these conditions, naloxone may act more potently as a competitive antagonist at the mu-opioid receptor with acutely administered oxycodone (see Figure 47).

*Weight Loss*

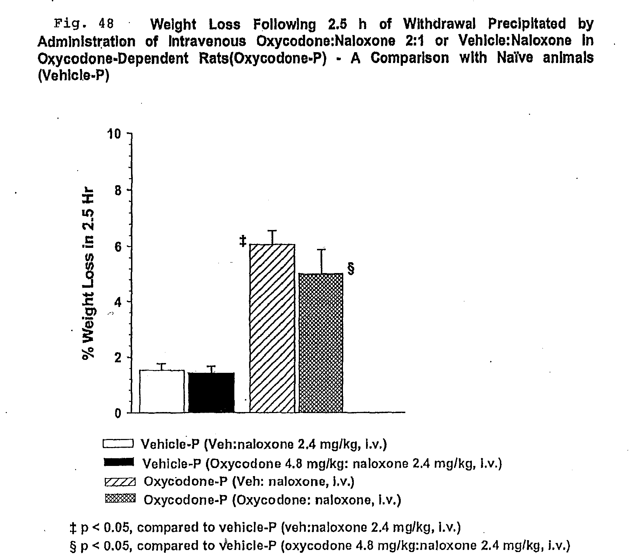

In addition, the rats infused chronically with oxycodone for 7 days experienced significant weight loss over the 2.5 hr period of withdrawal as seen in Figure 48. Weight loss is a classic withdrawal sign indicating the presence of physical dependence. Statistical analysis indicates that the percent weight loss did not differ significantly between the vehicle:naloxone and oxycodone:naloxone groups.

*Checked Signs of Withdrawal*

The incidence of checked signs during opioid withdrawal was also analyzed statistically within each time interval as seen in Tables 35 to 37 (below). Several items were notable from this study that should be described further. First, naloxone precipitated no withdrawal in any of the vehicle-pump groups, demonstrating that neither the surgery nor the presence of the pump caused the stressful release of endogenous opioid peptides. Second, regarding the vehicle:naloxone group, the rats underwent robust withdrawal with two of the most severe signs of dependence-profuse salivation and chromodacorrhea-present in many rats at one time or another. In addition, the checked signs of withdrawal were still present at 60-min. These results indicate that much lower doses of naloxone would have also been highly effective in precipitating withdrawal via the intravenous route of administration. Third, regarding the oxycodone:naloxone group, the presence of oxycodone did not blunt the manifestation of checked signs throughout the 60-min observation period.

Table 35. Comparison of Checked Signs of Precipitated Withdrawal Between Naïve and Oxycodone-Dependent Rats at the 0-15 min Interval.

| Naïve Rats | | | | |
|---|---|---|---|---|
| Time Interval: | 0-15 min | 0-15 min | 0-15 min | 0-15 min |
| Treatment Challenge: | Veh-P Veh:Veh | Veh-P Oxy:Veh | Veh-P Veh:Nx | Veh-P Oxy:Nx |
| Checked Signs | | | | |
| Diarrhea | 0/8 | 0/8 | 0/8 | 0/8 |
| Facial fasciculations or teeth chatter | 0/8 | 0/8 | 0/8 | 0/8 |
| Swallowing movements | 0/8 | 0/8 | 0/8 | 0/8 |
| Profuse salivation | 0/8 | 0/8 | 0/8 | 0/8 |

(continued)

| Naïve Rats | | | | |
|---|---|---|---|---|
| Time Interval: | 0-15 min | 0-15 min | 0-15 min | 0-15 min |
| Treatment Challenge: | Veh-P Veh:Veh | Veh-P Oxy:Veh | Veh-P Veh:Nx | Veh-P Oxy:Nx |
| Chromodacryorrhea | 0/8 | 0/8 | 0/8 | 0/8 |
| Ptosis | 0/8 | 0/8 | 0/8 | 0/8 |
| Abnormal posture | 0/8 | 0/8 | 0/8 | 0/8 |
| Erection or ejaculation | 0/8 | 0/8 | 0/8 | 0/8 |
| Irritability | 0/8 | 0/8 | 0/8 | 0/8 |

| Dependent Rats | | | | | |
|---|---|---|---|---|---|
| Time interval: | 0-15 min | 0-15 min | 0-15 min | 0-15 min | |
| Treatment Challenge: | Oxy-P Veh:Veh | Oxy-P Oxy:Veh | Oxy-P Veh:Nx | Oxy-P Oxy:Nx | $\chi^2$ value |
| Checked Signs | | | | | |
| Diarrhea | 0/8 | 0/8 | 1/8 | 1/8 | 0.34 |
| Facial fasciculations or teeth chatter | 0/8 | 0/8 | 6/8* | 7/8* | 25.9 |
| Swallowing movements | 0/8 | 0/8 | 6/8* | 7/8* | 25.9 |
| Profuse salivation | 0/8 | 0/8 | 8/8* | 4/8* | 25.8 |
| Chromodacryorrhea | 0/8 | 0/8 | 8/8* | 5/8* | 26.6 |
| Ptosis | 0/8 | 0/8 | 8/8* | 7/8* | 28.7 |
| Abnormal posture | 0/8 | 0/8 | 7/8* | 5/8* | 24.8 |
| Erection or ejaculation | 0/8 | 0/8 | 6/8* | 5/8* | 23.0 |
| Irritability | 0/8 | 0/8 | 8/8* | 8/8* | 30.0 |
| e.g., 6/8, number of rats expressing the sign during 15 min interval / number of rats in group. * p< 0.05, contingency table Pearson's Chi Square analysis (critical value for 7 df = 14.1) | | | | | |

Table 36. Comparison of Checked Signs of Precipitated Withdrawal Between Naïve and Oxycodone-Dependent Rats at the 15-30 min Interval.

| Naïve Rats | | | | |
|---|---|---|---|---|
| Time Interval: | 15-30 min | 15-30 min | 15-30 min | 15-30 min |
| Treatment Challenge: | Veh-P Veh:Veh | Veh-P Oxy:Veh | Veh-P Veh:Nx | Veh-P Oxy:Nx |
| Checked Signs | | | | |
| Diarrhea | 0/8 | 0/8 | 0/8 | 0/8 |
| Facial fasciculations or teeth chatter | 0/8 | 0/8 | 0/8 | 0/8 |
| Swallowing movements | 0/8 | 0/8 | 0/8 | 0/8 |
| Profuse salivation | 0/8 | 0/8 | 0/8 | 0/8 |
| Chromodacryorrhea | 0/8 | 0/8 | 0/8 | 0/8 |
| Ptosis | 0/8 | 0/8 | 0/8 | 0/8 |

(continued)

| Naïve Rats | | | | |
|---|---|---|---|---|
| Time Interval: | 15-30 min | 15-30 min | 15-30 min | 15-30 min |
| Treatment Challenge: | Veh-P Veh:Veh | Veh-P Oxy:Veh | Veh-P Veh:Nx | Veh-P Oxy:Nx |
| Abnormal posture | 0/8 | 0/8 | 0/8 | 0/8 |
| Erection or ejaculation | 0/8 | 0/8 | 0/8 | 0/8 |
| Irritability | 0/8 | 0/8 | 0/8 | 0/8 |

| Dependent Rats | | | | | |
|---|---|---|---|---|---|
| Time interval: | 15-30 min | 15-30 min | 15-30 min | 15-30 min | |
| Treatment Challenge: | Oxy-P Veh:Veh | Oxy-P Oxy:Veh | Oxy-P Veh:Nx | Oxy-P Oxy:Nx | $\chi^2$ value |
| Checked Signs | | | | | |
| Diarrhea | 0/8 | 0/8 | 5/8* | 4/8* | 19.9 |
| Facial fasciculations or teeth chatter | 0/8 | 0/8 | 7/8* | 7/8* | 27.3 |
| Swallowing movements | 0/8 | 0/8 | 7/8* | 8/8* | 28.7 |
| Profuse salivation | 0/8 | 0/8 | 8/8* | 6/8* | 27.6 |
| Chromodacryorrhea | 0/8 | 0/8 | 2/8 | 2/8 | 0.62 |
| Ptosis | 0/8 | 0/8 | 8/8* | 8/8* | 30.0 |
| Abnormal posture | 0/8 | 0/8 | 6/8* | 6/8* | 24.4 |
| Erection or ejaculation | 0/8 | 0/8 | 3/8* | 0/8 | 15.9 |
| Irritability | 0/8 | 0/8 | 8/8* | 8/8* | 30.0 |
| e.g., 6/8, number of rats expressing the sign during 15 min interval / number of rats in group. * $p < 0.05$, contingency table Pearson's Chi Square analysis (critical value for 7 df = 14.1) | | | | | |

Table 37. Comparison of Checked Signs of Precipitated Withdrawal Between Naïve and Oxycodone-Dependent Rats at the 30-45 min Interval.

| Naïve Rats | | | | |
|---|---|---|---|---|
| Time Interval: | 30-45 min | 30-45 min | 30-45 min | 30-45 min |
| Treatment Challenge: | Veh-P Veh:Veh | Veh-P Oxy:Veh | Veh-P Veh:Nx | Veh-P Oxy:Nx |
| Checked Signs | | | | |
| Diarrhea | 0/8 | 0/8 | 0/8 | 0/8 |
| Facial fasciculations or teeth chatter | 0/8 | 0/8 | 0/8 | 0/8 |
| Swallowing movements | 0/8 | 0/8 | 0/8 | 0/8 |
| Profuse salivation | 0/8 | 0/8 | 0/8 | 0/8 |
| Chromodacryorrhea | 0/8 | 0/8 | 0/8 | 0/8 |
| Ptosis | 0/8 | 0/8 | 0/8 | 0/8 |
| Abnormal posture | 0/8 | 0/8 | 0/8 | 0/8 |
| Erection or ejaculation | 0/8 | 0/8 | 0/8 | 0/8 |

(continued)

| Naïve Rats | | | | |
|---|---|---|---|---|
| Time Interval: | 30-45 min | 30-45 min | 30-45 min | 30-45 min |
| Treatment Challenge: | Veh-P Veh:Veh | Veh-P Oxy:Veh | Veh-P Veh:Nx | Veh-P Oxy:Nx |
| Irritability | 0/8 | 0/8 | 0/8 | 0/8 |

| Dependent Rats | | | | | |
|---|---|---|---|---|---|
| Time interval: | 30-45 min | 30-45 min | 30-45 min | 30-45 min | |
| Treatment Challenge: | Oxy-P Veh:Veh | Oxy-P Oxy:Veh | Oxy-P Veh:Nx | Oxy-P Oxy:Nx | $\chi^2$ value |
| Checked Signs | | | | | |
| Diarrhea | 0/8 | 0/8 | 5/8* | 5/8* | 21.3 |
| Facial fasciculations or teeth chatter | 0/8 | 0/8 | 7/8* | 7/8* | 27.3 |
| Swallowing movements | 0/8 | 0/8 | 8/8* | 8/8* | 30.0 |
| Profuse salivation | 0/8 | 0/8 | 4/8* | 5/8* | 19.9 |
| Chromodacryorrhea | 0/8 | 0/8 | 3/8* | 4/8* | 16.4 |
| Ptosis | 0/8 | 0/8 | 7/8* | 8/8* | 28.7 |
| Abnormal posture | 0/8 | 0/8 | 7/8* | 6/8* | 25.9 |
| Erection or ejaculation | 0/8 | 0/8 | 2/8 | 1/8 | 0.5 |
| Irritability | 0/8 | 0/8 | 8/8* | 8/8* | 30.0 |
| e.g., 6/8, number of rats expressing the sign during 15 min interval / number of rats in group. * p< 0.05, contingency table Pearson's Chi Square analysis (critical value for 7 df = 14.1) | | | | | |

4.2 Pharmacokinetics

*In life Events*

The jugular vein-cannulated animals successfully underwent the surgical procedures and were implanted with the 2ML 1 osmotic mini pumps. Similar to noncannulated animals used for withdrawal observations, they were infused with oxycodone at the rate of 1.5 mg/kg/h for 7-days. They were divided into two groups. On the test day (day 7) group 1 received the OXN 4.8/2.4 mg/kg intravenously while animals in group 2 were administered the vehicle only to determine the plasma concentration of oxycodone provided by the 2ML 1 osmotic minipumps over 7 days.

*Pharmacokinetics of OXN in Oxycodone Dependent Animals*

Following a 7-day oxycodone-pump infusion, the oxycodone mean (n=6) Cmax value was 429 ng/mL and the mean AUC value at steady state was 23621 ng.min/mL. After intravenous administration *of OXN* 4.8:2.4 mg/kg to dependent animals, oxycodone mean (n=7) Cmax value was 517 ng/mL and the mean AUCo-75min value was 26443 ng.min/mL. Statistical analysis (t-Tests: Two-Sample Assuming Equal Variances and Paired Two Sam pie for Means) indicated that the Cmax and the AUC values in oxycodone dependent rats did not differ significantly following intravenous administration of either vehicle or oxycodone:naloxone at 4.8:2.4 mg/kg. This may be due to the short sampling period of 75 minutes which was not sufficient to detect any PK differences between the two groups, particularly when both groups had relatively high levels of oxycodone at the end of the infusion.

After intravenous administration of OXN to dependent animals, the mean (n=7) Cmax values associated with withdrawal observations were 517 ng/mL for oxycodone and 124 ng/mL for naloxone leading to a corresponding oxycodone:naloxone plasma ratio of 4.2:1. The mean $AUC_{0-75min}$ values were 26443 ng.min/mL for oxycodone and 5889 ng.min/mL for naloxone leading to an oxycodone:naloxone plasma ratio of 4.5:1. Consistent with the pharmacology observations, the oxycodone:naloxone plasma individual ratios in animals administered OXN intravenously remained low at the later stages of withdrawal, e.g.; the 75-min time point exhibited an oxycodone:naloxone plasma ratio ranging from 3:1 to 7:1.

5. Conclusions

Intravenous administration of OXN resulted in significant naloxone-withdrawal as measured by both graded and checked signs of withdrawal throughout the 60-min observation period. In fact, the oxycodone:naloxone 2:1 ratio appeared to enhance the later stages of withdrawal compared to rats administered naloxone alone. Thus, rather than suppressing withdrawal, oxycodone appeared to maintain the later stages of naloxone-precipitated withdrawal. A low oxycodone:naloxone plasma ratio appeared to be associated with the withdrawal throughout the 60-min observation period. This is consistent with the pharmacology observations, where the oxycodone:naloxone plasma mean individual ratios in animals administered OXN remained low at the later stages of withdrawal.

**Reference Experiment 6: Effect of production upscale on pharmacokinetics of oxycodone and naloxone**

1. Objective:

The objective of this study was to establish the bioequivalence of both oxycodone and naloxone (or surrogate) from a fixed combination PR tablet OXN 10/5 (containing 10 mg oxycodone HCl and 5 mg naloxone HCl) manufactured as a small-scale batch with OXN 10/5 manufactured as a large-scale batch, by comparing the AUC ratio and Cmax ratio as primary measures.
A further objective was to establish the bioequivalence of both oxycodone and naloxone (or surrogate) from a fixed combination PR tablet OXN 40/20 (containing 40 mg oxycodone HCl) and 20 mg naloxone HCl) manufactured as a small-scale batch with OXN 40/20 manufactured as a large-scale batch, by comparing the AUC ratio and

2. Test population

The total number of subjects that enrolled was 40. The criteria for inclusion were healthy males and females, 18 - 50 years of age, with no clinically significant medical history, and whose general practitioners (if applicable) confirmed that they were suitable to take part in clinical studies.

3. Study Design, Test Treatment, Dose and Mode of Administration

*Preparations administered*

The same preparations as in Example 2 were administered.

*Study Design*

The study was an open-label, single-dose, randomized, 4-treatment, 4-period crossover.

*Test treatment and mode of administration*

Oxycodone/Naloxone PR tablets *10/5* (OXN 10/5), a PR combination tablet containing 10 mg of oxycodone HCl and 5 mg of naloxone HCl, and Oxycodone/Naloxone PR tablets *40/20* (OXN 40/20), a PR combination tablet containing 40 mg oxycodone HCl and 20 mg naloxone HCl were used. Both test treatments were extruded formulations and were manufactured as large scale batches.

Treatment A: 4 tablets of OXN 10/5 (large-scale batch) taken orally after a 10-hour overnight fast

Treatment B: 1 tablet of OXN 40/20 (large-scale batch) taken orally after a 10-hour overnight fast

The reference treatment was Oxycodone/Naloxone PR tablets 10/5 (OXN 10/5), a PR combination tablet containing 10 mg of oxycodone HCl and 5 mg of naloxone HCl, and Oxycodone/Naloxone PR tablets 40/20 (OXN 40/20), a PR combination tablet containing 40 mg oxycodone HCl and 20 mg naloxone HCl. The reference treatments were in an extruded formulation and were manufactured as small-scale batches.

Treatment C: 4 tablets *of OXN* 10/5 (small-scale batch) taken orally after a 10-hour overnight fast

Treatment D: 1 tablet of OXN 40/20 (small-scale batch) taken orally after a 10-hour overnight fast

*Duration of Treatment and Study Duration:*

Screening period ≤21 days, Pharmacokinetic sampling took place for 96 hours for each of 4 treatment periods, with a 7-day washout between dosing each treatment period, and a post study evaluation 7-10 days after dosing of the last treatment period, for a total of 49-52 days.

*Drug Concentration Measurements*

Predose on Day 1 of the respective study period. and at 0.5,1, 1.5, 2, 2.5, 3, 3.5, 4, 5, 6, 8, 10, 12, 16, 24, 28, 32, 36, 48, 72, and 96 hours postdose (22 blood samples per dosing period).

If subjects experienced emesis within 12 hours after dosing, no further pharmacokinetic blood sampling was to be undertaken for the rest of the study period.

*Bioanalytical Methods*

The plasma samples were analyzed for oxycodone. noroxycodone, oxymorphone, and noroxymorphone, and for naloxone, 6β-naloxol, naloxone-3-glucuronide. and 6β-naloxol glucuronide by validated bioanalytical assays.

*Pharmacokinetic Analyses:*

Pharmacokinetic parameters for all analyses were summarized descriptively by treatment. No further pharmacokinetic analyses were performed as data were gathered for one treatment period only.

4. Results

Plasma concentration-time data were gathered for one treatment period only, therefore it was not possible to make any crossover comparison between the treatments. Consequently, no formal statistical assessment was made comparing any of the treatments, but was limited to descriptive statistics for the derived pharmacokinetic parameters.
The mean parameters summarized in Table 38 below indicate that there were no apparent differences between the treatment groups of the same strength, and are supportive of there being no relevant differences between the small laboratory scale and large production scale batches.

Table 38: Pharmacokinetic parameters

| Mean Pharmacokinetic Parameters for Oxycodone | | | | |
|---|---|---|---|---|
| | OXN 40/20 large | OXN 40/20 small | 4 x OXN 10/5 large | 4 x OXN 10/5 small |
| AUCt (ng.h/mL)* (SD) n | 501.8 (100.90) 7 | 502.4 (144.44) 8 | 485.0 (80.88) 5 | 423.5 (106.19) 5 |
| AUCINF (ng. H/mL)* (SD) n | 503.6 (100.69) 7 | 504.0 (144.52) 8 | 486.4 (81.34) 5 | 424.4 (106.36) 5 |
| Cmax (ng./mL)* (SD) n | 37.40 (6.44) 7 | 39.23 (7.20) 8 | 38.22 (8.52) 5 | 35.36 (6.56) 5 |
| tmax (h)** (Range) n | 3.5 (1.5-6) 7 | 3.5 (2-5) 8 | 3.5 (2.5-5) 5 | 4 (1.5-5) 5 |
| t1/2Z (h)* (SD) n | 4.55 (0.77) 7 | 4.02 (0.89) 8 | 4.36 (0.83) 5 | 3.96 (0.67) 5 |
| * Arithmetic mean, standard deviation **Median, range | | | | |
| Mean Pharmacokinetic Parameters for Naloxone-3-glucuronide | | | | |
| | OXN 40/20 large | OXN 40/20 small | 4 x OXN 10/5 large | 4 x OXN 10/5 small |
| AUCt (ng.h/mL)* (SD) n | 670.6 (159.39) 7 | 662.5 (108.45) 8 | 681.2 (73.89) 5 | 607.2 (217.09) 5 |
| AUCINF (ng. H/mL)* (SD) n | 679.2 (154.94) 7 | 658.5 (116.00) 7 | 660.8 (55.88) 4 | 617.6 (208.54) 5 |
| Cmax (ng./mL)* (SD) n | 78.55 (18.03) 7 | 81.71 (25.76) 8 | 84.66 (15.83) 5 | 86.66 (39.43) 5 |
| tmax (h)** (Range) n | 1 (0.5-2.5) 7 | 0.75 (0.5 - 4) 8 | 0.5 (0.5 - 5) 5 | 1 (0.5 - 1.5) 5 |
| t1/2Z (h)* (SD) n | 11.56 (3.86) 7 | 8.37 (2.21) 7 | 9.50 (1.43) 4 | 9.36 (3.41) 5 |
| * Arithmetic mean, standard deviation **Median, range | | | | |

The above experiments clearly establish that a 2:1 ratio of ocycone to naloxone is particularly suitable to provide analgetic efficicacy, good tolerability, improved bowel function, reduced side effects, no increase in adverse effects, no food effect and effects withdrawal symptoms in opioid dependent subjects.

**Claims**

1. A sustained release oral dosage form comprising oxycodone or a pharmaceutically acceptable salt thereof and naloxone or a pharmaceutically acceptable salt thereof for use in providing an improvement of bowel function during pain therapy, wherein the improvement of bowel function is compared to administering oxycodone alone; and wherein the dosage form comprises (i) oxycodone or a pharmaceutically acceptable salt thereof in an amount corresponding to 40 mg anhydrous oxycodone hydrochloride, and naloxone or a pharmaceutically acceptable salt thereof in an amount corresponding to 20 mg anhydrous naloxone hydrochloride; or (ii) oxycodone or a pharmaceutically acceptable salt thereof in an amount corresponding to 20 mg anhydrous oxycodone hydrochloride, and naloxone or a pharmaceutically acceptable salt thereof in an amount corresponding to 10 mg anhydrous naloxone hydrochloride; or (iii) oxycodone or a pharmaceutically acceptable salt thereof in an amount corresponding to 10 mg anhydrous oxycodone hydrochloride, and naloxone or a pharmaceutically acceptable salt thereof in an amount corresponding to 5 mg naloxone hydrochloride.

2. A sustained release oral dosage form comprising oxycodone or a pharmaceutically acceptable salt thereof and naloxone or a pharmaceutically acceptable salt thereof for use in treating moderate to severe pain and constipation as side effect occurring during pain therapy, wherein the dosage form comprises (i) oxycodone or a pharmaceutically acceptable salt thereof in an amount corresponding to 40 mg anhydrous oxycodone hydrochloride, and naloxone or a pharmaceutically acceptable salt thereof in an amount corresponding to 20 mg anhydrous naloxone hydrochloride; or (ii) oxycodone or a pharmaceutically acceptable salt thereof in an amount corresponding to 20 mg anhydrous oxycodone hydrochloride, and naloxone or a pharmaceutically acceptable salt thereof in an amount corresponding to 10 mg anhydrous naloxone hydrochloride; or (iii) oxycodone or a pharmaceutically acceptable salt thereof in an amount corresponding to 10 mg anhydrous oxycodone hydrochloride, and naloxone or a pharmaceutically acceptable salt thereof in an amount corresponding to 5 mg naloxone hydrochloride.

3. A sustained release oral dosage form comprising oxycodone or a pharmaceutically acceptable salt thereof and naloxone or a pharmaceutically acceptable salt thereof for use in the prevention and/or treatment of constipation as side effect during pain therapy without reducing the analgesic effect of the oxycodone, wherein the dosage form comprises (i) oxycodone or a pharmaceutically acceptable salt thereof in an amount corresponding to 40 mg anhydrous oxycodone hydrochloride, and naloxone or a pharmaceutically acceptable salt thereof in an amount corresponding to 20 mg anhydrous naloxone hydrochloride; or (ii) oxycodone or a pharmaceutically acceptable salt thereof in an amount corresponding to 20 mg anhydrous oxycodone hydrochloride, and naloxone or a pharmaceutically acceptable salt thereof in an amount corresponding to 10 mg anhydrous naloxone hydrochloride; or (iii) oxycodone or a pharmaceutically acceptable salt thereof in an amount corresponding to 10 mg anhydrous oxycodone hydrochloride, and naloxone or a pharmaceutically acceptable salt thereof in an amount corresponding to 5 mg naloxone hydrochloride.

4. A sustained release dosage form for use according to any one of claims 1 to 3, wherein the dosage form is a matrix-based retardation formulation.

5. A sustained release dosage form for use according to any one of claims 1 to 4, wherein said pharmaceutically acceptable salt is the hydrochloride, sulphate, bisulphate, tartrate, nitrate, citrate, bitartrate, phosphate, malate, maleate, hydrobromide, hydroiodide, fumarate or succinate.

**Patentansprüche**

1. Eine verzögernd freisetzende orale Dosierungsform umfassend Oxycodon oder ein pharmazeutisch verträgliches Salz davon und Naloxon oder ein pharmazeutisch verträgliches Salz davon zur Verwendung beim Bereitstellen einer Verbesserung der Darmfunktion während einer Schmerztherapie, wobei die Verbesserung der Darmfunktion verglichen wird mit der Verabreichung von Oxycodon alleine; und wobei die Dosierungsform umfasst (i) Oxycodon oder ein pharmazeutisch verträgliches Salz davon in einer Menge entsprechend 40 mg wasserfreiem Oxycodon-Hydrochlorid, und Naloxon oder ein pharmazeutisch verträgliches Salz davon in einer Menge entsprechend 20 mg wasserfreiem Naloxon-Hydrochlorid; oder (ii) Oxycodon oder ein pharmazeutisch verträgliches Salz davon in einer Menge entsprechend 20 mg wasserfreiem Oxycodon-Hydrochlorid, und Naloxon oder ein pharmazeutisch verträgliches Salz davon in einer Menge entsprechend 10 mg wasserfreiem Naloxon-Hydrochlorid; oder (iii) Oxycodon oder ein pharmazeutisch verträgliches Salz davon in einer Menge entsprechend 10 mg wasserfreiem Oxycodon-Hydrochlorid, und Naloxon oder ein pharmazeutisch verträgliches Salz davon in einer Menge entsprechend 5 mg Naloxon-Hydrochlorid.

2. Eine verzögernd freisetzende orale Dosierungsform umfassend Oxycodon oder ein pharmazeutisch verträgliches Salz davon und Naloxon oder ein pharmazeutisch verträgliches Salz davon zur Verwendung bei der Behandlung von moderaten bis starken Schmerzen und Verstopfung als Nebenwirkung, die während einer Schmerztherapie auftritt, wobei die Dosierungsform umfasst (i) Oxycodon oder ein pharmazeutisch verträgliches Salz davon in einer Menge entsprechend 40 mg wasserfreiem Oxycodon-Hydrochlorid, und Naloxon oder ein pharmazeutisch verträgliches Salz davon in einer Menge entsprechend 20 mg wasserfreiem Naloxon-Hydrochlorid; oder (ii) Oxycodon oder ein pharmazeutisch verträgliches Salz davon in einer Menge entsprechend 20 mg wasserfreiem Oxycodon-Hydrochlorid, und Naloxon oder ein pharmazeutisch verträgliches Salz davon in einer Menge entsprechend 10 mg wasserfreiem Naloxon-Hydrochlorid; oder (iii) Oxycodon oder ein pharmazeutisch verträgliches Salz davon in einer Menge entsprechend 10 mg wasserfreiem Oxycodon-Hydrochlorid, und Naloxon oder ein pharmazeutisch verträgliches Salz davon in einer Menge entsprechend 5 mg Naloxon-Hydrochlorid.

3. Eine verzögernd freisetzende orale Dosierungsform umfassend Oxycodon oder ein pharmazeutisch verträgliches Salz davon und Naloxon oder ein pharmazeutisch verträgliches Salz davon zur Verwendung bei der Prävention und/oder Behandlung von Verstopfung als Nebenwirkung während einer Schmerztherapie ohne dass der analgetische Effekt des Oxycodons reduziert wird, wobei die Dosierungsform umfasst (i) Oxycodon oder ein pharmazeutisch verträgliches Salz davon in einer Menge entsprechend 40 mg wasserfreiem Oxycodon-Hydrochlorid, und Naloxon oder ein pharmazeutisch verträgliches Salz davon in einer Menge entsprechend 20 mg wasserfreiem Naloxon-Hydrochlorid; oder (ii) Oxycodon oder ein pharmazeutisch verträgliches Salz davon in einer Menge entsprechend 20 mg wasserfreiem Oxycodon-Hydrochlorid, und Naloxon oder ein pharmazeutisch verträgliches Salz davon in einer Menge entsprechend 10 mg wasserfreiem Naloxon-Hydrochlorid; oder (iii) Oxycodon oder ein pharmazeutisch verträgliches Salz davon in einer Menge entsprechend 10 mg wasserfreiem Oxycodon-Hydrochlorid, und Naloxon oder ein pharmazeutisch verträgliches Salz davon in einer Menge entsprechend 5 mg Naloxon-Hydrochlorid.

4. Eine verzögernd freisetzende Dosierungsform zur Verwendung gemäß einem der Ansprüche 1 bis 3, wobei die Dosierungsform eine Matrix-basierte Retard-Formulierung ist.

5. Eine verzögernd freisetzende Dosierungsform zur Verwendung gemäß einem der Ansprüche 1 bis 4, wobei das pharmazeutisch akzeptable Salz das Hydrochlorid, Sulfat, Bisulfat, Tartrat, Nitrat, Zitrat, Bitartrat, Phosphat, Malat, Maleat, Hydrobromid, Hydroiodid, Fumarat oder Succinat ist.

**Revendications**

1. Forme posologique orale à libération prolongée comprenant de l'oxycodone ou un de ses sels pharmaceutiquement acceptables et de la naloxone ou un de ses sels pharmaceutiquement acceptables destinée à être utilisée dans la fourniture d'une amélioration de la fonction de l'intestin durant une thérapie de douleur, l'amélioration de la fonction de l'intestin étant comparée à l'administration de l'oxycodone seule ; et la forme posologique comprenant (i) de l'oxycodone ou un de ses sels pharmaceutiquement acceptables en une quantité correspondant à 40 mg de chlorhydrate d'oxycodone anhydre et de la naxolone ou un de ses sels pharmaceutiquement acceptables en une quantité correspondant à 20 mg de chlorhydrate de naloxone anhydre ou (ii) de l'oxycodone ou un de ses sels pharmaceutiquement acceptables en une quantité correspondant à 20 mg de chlorhydrate d'oxycodone anhydre et de la naloxone ou un de ses sels pharmaceutiquement acceptables en une quantité correspondant à 10 mg de chlohydrate de naloxone anhydre ; ou (iii) de l'oxycodone ou un de ses sels pharmaceutiquement acceptables en une quantité correspondant à 10 mg de chlorhydrate d'oxycodone anhydre et de la naloxone ou un de ses sels pharmaceutiquement acceptables en une quantité correspondant à 5 mg de chlorhydrate de naloxone.

2. Forme posologique orale à libération prolongée comprenant de l'oxycodone ou un de ses sels pharmaceutiquement acceptables et de la naloxone ou un de ses sels pharmaceutiquement acceptables destinée à être utilisée dans le traitement de la douleur modérée à sévère et de la constipation comme effet secondaire se produisant durant une thérapie de douleur, la forme posologique comprenant (i) de l'oxycodone ou un de ses sels pharmaceutiquement acceptables en une quantité correspondant à 40 mg de chlorhydrate d'oxycodone anhydre et de la naloxone ou un de ses sels pharmaceutiquement acceptables en une quantité correspondant à 20 mg de chlorhydrate de naloxone anhydre ou (ii) de l'oxycodone ou un de ses sels pharmaceutiquement acceptables en une quantité correspondant à 20 mg de chlorhydrate d'oxycodone anhydre et de la naloxone ou un de ses sels pharmaceutiquement acceptables en une quantité correspondant à 10 mg de chlohydrate de naxolone anhydre ou (iii) de l'oxycodone ou un de ses sels pharmaceutiquement acceptables en une quantité correspondant à 10 mg de chlorhydrate d'oxycodone anhydre et de la naxolone ou un de ses sels pharmaceutiquement acceptables en une quantité correspondant à 5 mg de chlorhydrate de naxolone.

3. Forme posologique orale à libération prolongée comprenant de l'oxycodone ou un de ses sels pharmaceutiquement acceptables et de la naxolone ou un de ses sels pharmaceutiquement acceptables destinée à être utilisée dans la prévention et/ou le traitement de la constipation comme effet secondaire durant une thérapie de douleur sans réduire l'effet analgésique de l'oxycodone, la forme posologique comprenant (i) de l'oxycodone ou un de ses sels pharmaceutiquement acceptables en une quantité correspondant à 40 mg de chlorhydrate d'oxycodone anhydre et de la naloxone ou un de ses sels pharmaceutiquement acceptables en une quantité correspondant à 20 mg de chlorhydrate de naloxone anhydre ou (ii) de l'oxycodone ou un de ses sels pharmaceutiquement acceptables en une quantité correspondant à 20 mg de chlorhydrate d'oxycodone anhydre et de la naloxone ou un de ses sels pharmaceutiquement acceptables en une quantité correspondant à 10 mg de chlohydrate de naxolone anhydre ou (iii) de l'oxycodone ou un de ses sels pharmaceutiquement acceptables en une quantité correspondant à 10 mg de chlorhydrate d'oxycodone anhydre et de la naxolone ou un de ses sels pharmaceutiquement acceptables en une quantité correspondant à 5 mg de chlorhydrate de naxolone.

4. Forme posologique à libération prolongée destinée à être utilisée selon l'une quelconque des revendications 1 à 3, dans laquelle la forme posologique est une formulation de retardement à base de matrice.

5. Forme posologique à libération prolongée destinée à être utilisée selon l'une quelconque des revendications 1 à 4, dans laquelle ledit sel pharmaceutiquement acceptable est le chlohydrate, le sulfate, le bisulfate, le tartrate, le nitrate, le citrate, le bitartrate, le phosphate, le malate, le maléate, le bromhydrate, l'iodohydrate, le fumarate ou le succinate.

Figure 1: Paper form

---

**Bowel Function Index (BFI3)**

Please answer the following three questions by making a mark on the line between 0 and 100. Please transfer the results to the free squares on the bottom of the page. Add the three results and divide through three.

**Question 1 (Q1):**
How would you rate the ease of defecation during the last 7 days according to patient assessment?
(0= easy/no difficulty, 100= severe difficulty)

|---------|---------|---------|---------|---------|---------|---------|---------|---------|---------|
0                                         50                                      100

**Question 2 (Q2):**
Does your patient feel that her/his bowel evacuation has been incomplete during the last 7 days? (0= not at all, 100= very strongly)

|---------|---------|---------|---------|---------|---------|---------|---------|---------|---------|
0                                         50                                      100

**Question 3 (Q3):**
How would you judge your patients constipation throughout the last 7 days?
(0= no constipation at all, 100= very heavily constipated)

|---------|---------|---------|---------|---------|---------|---------|---------|---------|---------|
0                                         50                                      100

$$\boxed{\phantom{Q1}} + \boxed{\phantom{Q2}} \quad \boxed{\phantom{Q3}} \Big/3 = \boxed{\phantom{}}$$

**Q1**          **Q2**          **Q3**

---

Figure 2

## Figure 3: Patient Demographics and Other Baseline Characteristics by Absolute Dose of Naloxone

### Absolute Dose of Naloxone

| Characteristic | Naloxone Placebo N=50 (100%) | Naloxone 10 mg N=51 (100%) | Naloxone 20 mg N=51 (100%) | Naloxone 40 mg N=50 (100%) | Total N=202 (100%) |
|---|---|---|---|---|---|
| **Sex n (%)** | | | | | |
| Male | 19 (38.0) | 8 (35.3) | 17 (33.3) | 21 (42.0) | 75 (37.1) |
| Female | 31 (62.0) | 33 (64.7) | 34 (66.7) | 29 (58.0) | 127 (62.9) |
| **Age [years]** | | | | | |
| Mean | 53.8 | 58.4 | 56.0 | 57.0 | 56.3 |
| SD | 12.74 | 12.75 | 12.65 | 14.03 | 13.06 |
| Median | 53.0 | 57.0 | 56.0 | 57.5 | 55.0 |
| Min-Max | 29 – 84 | 28 – 86 | 33 – 80 | 27 – 78 | 27 – 86 |
| **Race n (%)** | | | | | |
| Caucasian | 50 (100.0) | 51 (100.0) | 51 (100.0) | 50 (100.0) | 202 (100.0) |
| Black | 0 (0.0) | 0 (0.0) | 0 (0.0) | 0 (0.0) | 0 (0.0) |
| Asian | 0 (0.0) | 0 (0.0) | 0 (0.0) | 0 (0.0) | 0 (0.0) |
| Other | 0 (0.0) | 0 (0.0) | 0 (0.0) | 0 (0.0) | 0 (0.0) |
| **Weight (kg)** | | | | | |
| Mean | 75.88 | 77.65 | 81.76 | 80.14 | 78.87 |
| SD | 13.43 | 14.47 | 13.92 | 18.11 | 15.14 |
| Median | 75.0 | 80.0 | 81.0 | 81.0 | 80.0 |
| Min-Max | 46 – 107 | 47 – 105 | 56 – 115 | 45 – 110 | 45 – 115 |
| **Height (cm)** | | | | | |
| Mean | 168.7 | 169.6 | 169.4 | 169.0 | 169.2 |
| SD | 8.29 | 8.00 | 7.97 | 9.12 | 8.30 |
| Median | 168.0 | 168.0 | 170.0 | 168.5 | 169.0 |
| Min-Max | 155 – 190 | 154 – 191 | 155 – 185 | 147 – 189 | 147 – 191 |
| **BMI ($kg/m^2$)** | | | | | |
| Mean | 26.70 | 27.03 | 28.65 | 27.93 | 27.58 |
| SD | 4.58 | 5.11 | 5.50 | 5.57 | 5.22 |
| Median | 26.03 | 25.83 | 27.78 | 28.09 | 26.61 |
| Min-Max | 17.1 – 40.3 | 17.3 – 37.2 | 18.9 – 42.6 | 16.8 – 39.8 | 16.8 – 42.6 |
| **Tumor patient n (%)** | | | | | |
| Yes | 1 (2.0) | 1 (2.0) | 1 (2.0) | 2 (4.0) | 5 (2.5) |
| No | 49 (98.0) | 50 (98.0) | 50 (98.0) | 48 (96.0) | 197 (97.5) |

Figure 4: Patient Demographics and Other Baseline Characteristics by Oxycodone/Naloxone Dose Ratio

Dose Ratios

| Characteristic | 40 mg/ Placebo | 60 mg/ Placebo | 80 mg/ Placebo | 1/1 | 1.5/1 | 2/1 | 3/1 | 4/1 | 6/1 | 8/1 |
|---|---|---|---|---|---|---|---|---|---|---|
| | N=17 (100%) | N=17 (100%) | N=16 (100%) | N=15 (100%) | N=18 (100%) | N=34 (100%) | N=18 (100%) | N=33 (100%) | N=12 (100%) | N=22 (100%) |
| Sex n (%) | | | | | | | | | | |
| Male | 6 (35.3) | 6 (35.3) | 7 (43.8) | 5 (33.3) | 8 (44.4) | 11 (32.4) | 8 (44.4) | 10 (30.3) | 4 (33.3) | 10 (45.5) |
| Female | 11 (64.7) | 11 (64.7) | 9 (56.3) | 10 (66.7) | 10 (55.6) | 23 (67.6) | 10 (55.6) | 23 (69.7) | 8 (66.7) | 12 (54.5) |
| Age [years] | | | | | | | | | | |
| Mean | 51.8 | 52.8 | 56.9 | 59.9 | 55.9 | 56.1 | 58.3 | 57.6 | 57.6 | 55.9 |
| SD | 13.66 | 12.23 | 12.51 | 13.16 | 12.84 | 13.89 | 13.01 | 14.12 | 11.74 | 12.55 |
| Median | 52.0 | 53.0 | 56.5 | 61.0 | 55.5 | 60.0 | 60.5 | 52.0 | 61.0 | 55.0 |
| Min-Max | 37 – 84 | 29 – 75 | 31 – 80 | 34 – 78 | 35 – 72 | 27 – 76 | 39 – 80 | 35 – 86 | 34 – 73 | 28 - 77 |
| Race n (%) | | | | | | | | | | |
| Caucasian | 17 (100.0) | 17 (100.0) | 16 (100.0) | 15 (100.0) | 18 (100.0) | 34 (100.0) | 18 (100.0) | 33 (100.0) | 12 (100.0) | 22 (100.0) |
| Black | 0 (0.0) | 0 (0.0) | 0 (0.0) | 0 (0.0) | 0 (0.0) | 0 (0.0) | 0 (0.0) | 0 (0.0) | 0 (0.0) | 0 (0.0) |
| Asian | 0 (0.0) | 0 (0.0) | 0 (0.0) | 0 (0.0) | 0 (0.0) | 0 (0.0) | 0 (0.0) | 0 (0.0) | 0 (0.0) | 0 (0.0) |
| Other | 0 (0.0) | 0 (0.0) | 0 (0.0) | 0 (0.0) | 0 (0.0) | 0 (0.0) | 0 (0.0) | 0 (0.0) | 0 (0.0) | 0 (0.0) |
| Weight (kg) | | | | | | | | | | |
| Mean | 78.24 | 71.82 | 77.69 | 79.27 | 83.50 | 79.24 | 85.06 | 77.79 | 72.75 | 80.91 |
| SD | 11.00 | 15.33 | 13.45 | 17.71 | 20.36 | 15.78 | 11.93 | 14.34 | 14.23 | 14.26 |
| Median | 80.0 | 70.0 | 75.0 | 78.0 | 85.5 | 80.5 | 82.5 | 78.0 | 69.0 | 82.0 |
| Min-Max | 60 – 98 | 46 – 107 | 57 – 105 | 53 – 110 | 45 – 110 | 50 – 115 | 66 – 110 | 47 – 108 | 50 – 98 | 56 - 105 |
| Height (cm) | | | | | | | | | | |
| Mean | 168.6 | 164.8 | 172.8 | 165.9 | 169.4 | 170.1 | 169.4 | 167.6 | 170.2 | 172.7 |
| SD | 6.56 | 6.52 | 9.87 | 8.62 | 8.87 | 7.80 | 9.19 | 7.98 | 9.29 | 7.22 |
| Median | 169.0 | 164.0 | 173.0 | 165.0 | 169.5 | 169.5 | 170.0 | 165.0 | 168.0 | 172.0 |
| Min-Max | 158 – 178 | 155 – 176 | 158 – 190 | 147 – 178 | 148 – 182 | 158 – 189 | 155 – 182 | 154 – 185 | 155 – 186 | 160- 191 |
| BMI (kg/m$^2$) | | | | | | | | | | |
| Mean | 27.51 | 26.51 | 26.04 | 28.74 | 28.88 | 27.38 | 29.97 | 27.78 | 25.06 | 27.13 |
| SD | 3.64 | 5.77 | 4.18 | 5.94 | 5.96 | 5.19 | 5.83 | 5.41 | 4.36 | 4.73 |
| Median | 26.79 | 25.65 | 25.02 | 28.09 | 30.47 | 26.70 | 28.56 | 26.64 | 24.32 | 26.43 |
| Min-Max | 22.6–34.7 | 17.1–40.3 | 18.8–34.6 | 21.2–39.8 | 16.8–38.6 | 17.0–38.9 | 21.7–42.6 | 17.3–38.3 | 19.9–36.4 | 19.5–37.2 |
| Tumor patient n (%) | | | | | | | | | | |
| Yes | 1 (5.9) | 0 (0.0) | 0 (0.0) | 0 (0.0) | 1 (5.6) | 1 (2.9) | 1 (5.6) | 1 (3.0) | 0 (0.0) | 0 (0.0) |
| No | 16 (94.1) | 17 (100.0) | 16 (100.0) | 15 (100.0) | 17 (94.4) | 33 (97.1) | 17 (94.4) | 32 (97.0) | 12 (100.0) | 22 (100.0) |

# Figure 5

## Study design

Pre-randomization phase                   Maintenance Phase       Follow-up phase

Titration/run-in with oxycodone only

Screening

40 mg oxycodone/day

60 mg oxycodone/day

80 mg oxycodone/day

40-80 mg oxycodone + **placebo** naloxone

40-80 mg oxycodone + **10** mg naloxone

40-80 mg oxycodone + **20** mg naloxone

40-80 mg oxycodone + **40** mg naloxone

40 mg oxycodone

60 mg oxycodone/day

80 mg oxycodone/day

Compulsory telephone contact

| V1 | V2 | V3 | V4 | V5 | V6 |
|----|----|----|----|----|----|

| Week -1 Screening | Week 0 Baseline | Visit 2 + 1-3 weeks End of titration/run-in Randomization | Visit 3 + 1 week | Visit 3 + 4 weeks End of naloxone treatment | Visit 5 + 2 weeks End of Follow-up |

## Figure 6:

Mean Bowel Function at Each Study Visit by Oxycodone/Naloxone Dose Ratio - ITT Population with Non-missing Values

EP 3 578 173 B1

| Average Bowel Function | Dose Ratios | | | | | | | | | |
|---|---|---|---|---|---|---|---|---|---|---|
| | 40 mg/ Placebo | 60 mg/ Placebo | 80 mg/ Placebo | 1/1 | 1.5/1 | 2/1 | 3/1 | 4/1 | 6/1 | 8/1 |
| | N=17 (100%) | N=17 (100%) | N=16 (100%) | N=15 (100%) | N=15 (100%) | N=32 (100%) | N=17 (100%) | N=32 (100%) | N=11 (100%) | N=22 (100%) |
| Visit 2 (Baseline) | | | | | | | | | | |
| N | 17 | 17 | 16 | 15 | 17 | 32 | 17 | 32 | 11 | 22 |
| Mean | 52.2 | 48.7 | 59.3 | 44.7 | 49.7 | 51.6 | 56.2 | 53.4 | 56.7 | 60.3 |
| SD | 21.66 | 28.79 | 17.93 | 15.42 | 18.20 | 21.54 | 19.20 | 22.57 | 19.94 | 18.51 |
| Median | 53.3 | 50.0 | 61.7 | 40.0 | 50.0 | 51.7 | 53.3 | 50.8 | 50.0 | 66.7 |
| Min-Max | 0-83 | 0-93 | 29-83 | 23-77 | 20-83 | 0-87 | 30-93 | 3-100 | 30-100 | 17-87 |
| Visit 3 (Randomization) | | | | | | | | | | |
| N | 17 | 17 | 16 | 15 | 17 | 32 | 17 | 32 | 11 | 22 |
| Mean | 44.9 | 43.0 | 56.5 | 41.6 | 47.9 | 47.4 | 41.4 | 51.4 | 55.5 | 59.7 |
| SD | 25.77 | 26.35 | 17.70 | 20.03 | 21.90 | 20.19 | 21.28 | 23.36 | 23.86 | 22.16 |
| Median | 43.3 | 36.7 | 51.7 | 40.0 | 46.7 | 50.0 | 40.0 | 56.7 | 46.7 | 65.0 |
| Min-Max | 0-100 | 3-87 | 30-83 | 17-90 | 0-87 | 0-87 | 0-80 | 10-100 | 20-100 | 17-100 |
| Visit 4 (Maintenance) | | | | | | | | | | |
| N | 15 | 17 | 16 | 15 | 14 | 28 | 16 | 31 | 11 | 21 |
| Mean | 41.4 | 48.3 | 39.6 | 20.7 | 22.3 | 35.4 | 25.2 | 41.6 | 44.8 | 45.7 |
| SD | 21.83 | 31.24 | 25.56 | 19.24 | 16.97 | 25.19 | 32.79 | 26.51 | 27.66 | 26.86 |
| Median | 43.3 | 50.0 | 45.0 | 20.0 | 20.0 | 33.3 | 11.7 | 40.0 | 43.3 | 40.0 |
| Min-Max | 0-87 | 0-93 | 0-73 | 0-53 | 0-53 | 0-77 | 0-100 | 0-100 | 0-80 | 0-100 |
| Visit 5 (End of Maintenance) | | | | | | | | | | |
| N | 13 | 17 | 15 | 14 | 14 | 27 | 12 | 27 | 10 | 19 |
| Mean | 44.6 | 43.6 | 48.2 | 21.9 | 21.8 | 26.7 | 34.7 | 39.0 | 47.8 | 38.6 |
| SD | 22.42 | 23.75 | 21.71 | 22.25 | 21.35 | 23.98 | 26.99 | 26.24 | 23.20 | 24.68 |
| Median | 43.3 | 40.0 | 53.3 | 20.0 | 16.7 | 23.3 | 31.7 | 33.3 | 51.7 | 33.3 |
| Min-Max | 13-100 | 0-90 | 0-80 | 0-70 | 0-67 | 0-90 | 0-80 | 0-85 | 10-70 | 0-92 |
| Visit 6 (End of Follow-up) | | | | | | | | | | |
| N | 13 | 17 | 15 | 14 | 13 | 26 | 12 | 28 | 10 | 18 |
| Mean | 52.1 | 47.4 | 48.2 | 33.2 | 47.3 | 42.6 | 43.9 | 51.0 | 49.3 | 42.9 |
| SD | 26.79 | 24.25 | 25.82 | 20.76 | 24.32 | 24.37 | 27.99 | 24.16 | 23.92 | 26.41 |
| Median | 50.0 | 50.0 | 50.0 | 35.0 | 46.7 | 43.3 | 41.7 | 50.0 | 50.0 | 45.0 |
| Min-Max | 7-100 | 0-90 | 0-90 | 0-80 | 0-80 | 0-80 | 0-90 | 0-100 | 10-80 | 0-80 |

Note: Average bowel function = average of ease of defecation, feeling of incomplete bowel evacuation and judgment of constipation during the last 7 days according to patient assessment

## Figure 7:

Mean Bowel Function at Each Study Visit by Absolute Dose of Naloxone - ITT Population with Non-missing Values

| | Absolute Dose of Naloxone | | | |
|---|---|---|---|---|
| Average Bowel Function | Naloxone Placebo N=50 (100%) | Naloxone 10 mg N=49 (100%) | Naloxone 20 mg N=49 (100%) | Naloxone 40 mg N=48 (100%) |
| Visit 2 (Baseline) | | | | |
| N | 50 | 49 | 49 | 48 |
| Mean | 53.3 | 55.8 | 56.1 | 47.9 |
| SD | 23.28 | 19.81 | 20.19 | 19.65 |
| Median | 53.3 | 53.3 | 50.0 | 49.2 |
| Min-Max | 0-93 | 7-100 | 3-100 | 0-83 |
| Visit 3 (Randomization) | | | | |
| N | 50 | 49 | 49 | 48 |
| Mean | 48.0 | 52.8 | 49.4 | 46.2 |
| SD | 23.97 | 22.86 | 22.72 | 20.67 |
| Median | 48.3 | 50.0 | 50.0 | 46.7 |
| Min-Max | 0-100 | 10-100 | 0-100 | 0-90 |
| Visit 4 (Maintenance) | | | | |
| N | 48 | 47 | 47 | 42 |
| Mean | 43.3 | 42.1 | 34.2 | 27.9 |
| SD | 26.41 | 25.53 | 30.04 | 22.68 |
| Median | 46.7 | 40.0 | 30.0 | 28.3 |
| Min-Max | 0-93 | 0-100 | 0-100 | 0-73 |
| Visit 5 (End of Maintenance) | | | | |
| N | 45 | 41 | 42 | 40 |
| Mean | 45.4 | 40.3 | 31.3 | 26.1 |
| SD | 22.28 | 23.09 | 25.82 | 25.08 |
| Median | 43.3 | 36.7 | 25.0 | 20.0 |
| Min-Max | 0-100 | 0-92 | 0-85 | 0-90 |
| Visit 6 (End of Follow-up) | | | | |
| N | 45 | 41 | 41 | 39 |
| Mean | 49.0 | 45.1 | 46.4 | 42.4 |
| SD | 25.01 | 23.72 | 26.98 | 23.19 |
| Median | 50.0 | 50.0 | 43.3 | 40.0 |
| Min-Max | 0-100 | 0-80 | 0-100 | 0-80 |

Note: Average bowel function = average of ease of defecation, feeling of incomplete bowel evacuation and judgment of constipation during the last 7 days according to patient assessment

## Figure 8:

Mean Bowel Function at Each Study Visit by Absolute Dose of Naloxone Given the Same Oxycodone/
Naloxone Dose Ratio - ITT Population with Non-missing Values

| | Dose ratio | | | |
|---|---|---|---|---|
| | 4/1 | 4/1 | 2/1 | 2/1 |
| | 10 mg naloxone | 20 mg naloxone | 20 mg naloxone | 40 mg naloxone |
| **Average Bowel Function** | N=16 (100%) | N=16 (100%) | N=16 (100%) | N=16 (100%) |
| Visit 2 (Baseline) | | | | |
| N | 16 | 16 | 16 | 16 |
| Mean | 49.0 | 57.9 | 54.2 | 49.0 |
| SD | 20.76 | 24.06 | 18.04 | 24.88 |
| Median | 50.8 | 55.0 | 48.3 | 56.7 |
| Min-Max | 7-80 | 3-100 | 33-87 | 0-77 |
| Visit 3 (Randomization) | | | | |
| N | 16 | 16 | 16 | 16 |
| Mean | 41.6 | 61.3 | 46.0 | 48.8 |
| SD | 19.90 | 22.73 | 20.44 | 20.51 |
| Median | 41.7 | 63.3 | 45.0 | 51.7 |
| Min-Max | 10-77 | 10-100 | 8-87 | 0-73 |
| Visit 4 (Maintenance) | | | | |
| N | 15 | 16 | 15 | 13 |
| Mean | 35.0 | 47.8 | 29.3 | 42.3 |
| SD | 22.01 | 29.47 | 23.50 | 26.19 |
| Median | 40.0 | 43.3 | 30.0 | 46.7 |
| Min-Max | 0-72 | 0-100 | 0-77 | 0-73 |
| Visit 5 (End of Maintenance) | | | | |
| N | 12 | 15 | 15 | 12 |
| Mean | 36.7 | 40.8 | 19.2 | 36.1 |
| SD | 20.74 | 30.54 | 13.58 | 30.84 |
| Median | 35.0 | 33.3 | 20.0 | 33.3 |
| Min-Max | 3-73 | 0-85 | 0-50 | 0-90 |
| Visit 6 (End of Follow-up) | | | | |
| N | 13 | 15 | 14 | 12 |
| Mean | 45.0 | 56.2 | 38.1 | 47.8 |
| SD | 20.86 | 26.27 | 25.34 | 23.15 |
| Median | 50.0 | 50.0 | 36.7 | 55.0 |
| Min-Max | 0-70 | 23-100 | 0-80 | 0-77 |

Note: Average bowel function = average of ease of defecation, feeling of incomplete bowel evacuation and judgment of constipation during the last 7 days according to patient assessment

## Figure 9:

Mean Bowel Function: Test for Difference Versus Placebo - ITT Population with Non-missing Values

| Category | Absolute Dose of Naloxone | | |
| --- | --- | --- | --- |
| | Naloxone 10 mg vs Placebo | Naloxone 20 mg vs Placebo | Naloxone 40 mg vs Placebo |
| Mean bowel function assessing the last 7 days at Visit 4 | | | |
| N in test group | 47 | 47 | 42 |
| N in placebo group | 48 | 48 | 48 |
| Difference in means* | -1.2 | -9.0 | -15.4 |
| 95% CI | (-11.8, 9.4) | (-20.6, 2.5) | (-25.8, -5.0) |
| P-value** | 0.827 | 0.122 | 0.004 |
| Mean bowel function assessing the last 7 days at Visit 5 (end of maintenance) | | | |
| N in test group | 41 | 42 | 40 |
| N in placebo group | 45 | 45 | 45 |
| Difference in means* | -5.1 | -14.1 | -19.3 |
| 95% CI | (-14.9, 4.6) | (-24.4, -3.8) | (-29.5, -9.1) |
| P-value** | 0.296 | 0.008 | <0.001 |

*Mean in test group minus mean in placebo group; **t-test for difference

**Figure 10:**

Mean Bowel Function: Response Surface Plot - ITT Population with Non-missing Values

## Figure 11:

**Mean Bowel Function: Contour Plot with Granulation 10 - ITT Population with Non-missing Values**

Contour plot of totoxy*treat2.

| Symbol | mconstip | Symbol | mconstip | Symbol | mconstip | Symbol | mconstip | Symbol | mconstip |
|---|---|---|---|---|---|---|---|---|---|
| ..... | 6.777 - 12.192 | ----- | 17.607 - 23.022 | +++++ | 28.437 - 33.851 | XXXXX | 39.266 - 44.681 | ***** | 50.096 - 55.511 |
| ;;;;; | 12.192 - 17.607 | ===== | 23.022 - 28.437 | ooooo | 33.851 - 39.266 | wwwww | 44.681 - 50.096 | ##### | 55.511 - 60.926 |

NOTE: 484 obs hidden. 1 obs out of range.

**Fig. 12**  Global Assessment by Oxycodone/Naloxone Dose Ratio - ITT Population with Non-missing Values

| | Dose Ratios | | | | | | | | | |
|---|---|---|---|---|---|---|---|---|---|---|
| | 40 mg/ Placebo | 60 mg/ Placebo | 80 mg/ Placebo | 1/1 | 1.5/1 | 2/1 | 3/1 | 4/1 | 6/1 | 8/1 |
| Assessment at end of maintenance phase (n, (%)) | N=17 (100%) | N=17 (100%) | N=16 (100%) | N=15 (100%) | N=17 (100%) | N=32 (100%) | N=17 (100%) | N=32 (100%) | N=11 (100%) | N=22 (100%) |
| **Efficacy (investigator)** | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Very good | 2 (11.8) | 2 (11.8) | 2 (12.5) | 2 (13.3) | 3 (17.6) | 4 (12.5) | 2 (11.8) | 5 (15.6) | 0 (0.0) | 2 (9.1) |
| Good | 8 (47.1) | 5 (29.4) | 3 (18.8) | 9 (60.0) | 6 (35.3) | 15 (46.9) | 8 (47.1) | 13 (40.6) | 4 (36.4) | 7 (31.8) |
| Fairly good | 1 (5.9) | 3 (17.6) | 3 (18.8) | 2 (13.3) | 1 (5.9) | 5 (15.6) | 0 (0.0) | 2 (6.3) | 1 (9.1) | 3 (13.6) |
| Moderate | 1 (5.9) | 2 (11.8) | 3 (18.8) | 1 (6.7) | 2 (11.8) | 1 (3.1) | 1 (5.9) | 3 (9.4) | 5 (45.5) | 4(18.2) |
| Slightly poor | 1 (5.9) | 3 (17.6) | 1 (6.3) | 0 (0.0) | 1 (5.9) | 1(3.1) | 0 (0.0) | 1 (3.1) | 0 (0.0) | 1 (4.5) |
| Poor | 0 (0.0) | 1 (5.9) | 2 (12.5) | 0 (0.0) | 0 (0.0) | 1 (3.1) | 1(5.9) | 5 (15.6) | 0 (0.0) | 2 (9.1) |
| Very poor | 1 (5.9) | 1 (5.9) | 1 (6.3) | 0 (0.0) | 1 (5.9) | 0 (0.0) | 0 (0.0) | 0 (0.0) | 0 (0.0) | 0 (0.0) |
| **Efficacy (patient)** | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Very good | 1 (5.9) | 1 (5.9) | 2 (12.5) | 2 (13.3) | 4 (23.5) | 6 (18.8) | 2 (11.8) | 3 (9.4) | 0 (0.0) | 1 (4.5) |
| Good | 7 (41.2) | 7 (41.2) | 2 (12.5) | 8 (53.3) | 7 (41.2) | 13 (40.6) | 9 (52.9) | 14 (43.8) | 3 (27.3) | 7 (31.8) |
| Fairly good | 1 (5.9) | 2 (11.8) | 3 (18.8) | 3 (20.0) | 0 (0.0) | 3 (9.4) | 0 (0.0) | 4 (12.5) | 2 (18.2) | 1 (4.5) |
| Moderate | 3 (17.6) | 2 (11.8) | 4 (25.0) | 1 (6.7) | 2 (11.8) | 3 (9.4) | 1 (5.9) | 2 (6.3) | 4 (36.4) | 5 (22.7) |
| Slightly poor | 0 (0.0) | 3 (17.6) | 1 (6.3) | 0 (0.0) | 0 (0.0) | 1 (3.1) | 0 (0.0) | 2 (6.3) | 1 (9.1) | 3 (13.6) |
| Poor | 1 (5.9) | 0 (0.0) | 3 (18.8) | 0 (0.0) | 0 (0.0) | 1 (3.1) | 0 (0.0) | 4 (12.5) | 0 (0.0) | 2 (9.1) |
| Very poor | 1 (5.9) | 2 (11.8) | 0 (0.0) | 0 (0.0) | 1 (5.9) | 0 (0.0) | 0 (0.0) | 0 (0.0) | 0 (0.0) | 0 (0.0) |
| **Tolerability (investigator)** | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Very good | 2 (11.8) | 5 (29.4) | 2 (12.5) | 3 (20.0) | 4 (23.5) | 9 (28.1) | 4 (23.5) | 5 (15.6) | 1 (9.1) | 5 (22.7) |
| Good | 10 (58.8) | 6 (35.3) | 11 (68.8) | 10 (66.7) | 7 (41.2) | 13 (40.6) | 7 (41.2) | 17 (53.1) | 6 (54.5) | 12 (54.5) |
| Fairly good | 1 (5.9) | 1 (5.9) | 2 (12.5) | 1 (6.7) | 2 (11.8) | 5 (15.6) | 1 (5.9) | 4 (12.5) | 3 (27.3) | 0 (0.0) |
| Moderate | 1 (5.9) | 1 (5.9) | 0 (0.0) | 0 (0.0) | 1 (5.9) | 0 (0.0) | 0 (0.0) | 1 (3.1) | 0 (0.0) | 1 (4.5) |
| Slightly poor | 0 (0.0) | 3 (17.6) | 0 (0.0) | 0 (0.0) | 0 (0.0) | 0 (0.0) | 0 (0.0) | 0 (0.0) | 0 (0.0) | 1 (4.5) |
| Poor | 0 (0.0) | 1 (5.9) | 0 (0.0) | 0 (0.0) | 0 (0.0) | 0 (0.0) | 0 (0.0) | 2 (6.3) | 0 (0.0) | 0 (0.0) |
| Very poor | 0 (0.0) | 0 (0.0) | 0 (0.0) | 0 (0.0) | 0 (0.0) | 0 (0.0) | 0 (0.0) | 0 (0.0) | 0 (0.0) | 0 (0.0) |
| **Tolerability (patient)** | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Very good | 1 (5.9) | 4 (23.5) | 2 (12.5) | 2 (13.3) | 3 (17.6) | 7 (21.9) | 3 (17.6) | 5 (15.6) | 1 (9.1) | 5 (22.7) |
| Good | 10 (58.8) | 7 (41.2) | 9 (56.3) | 10 (66.7) | 8 (47.1) | 15 (46.9) | 8 (47.1) | 17 (53.1) | 6 (54.5) | 12 (54.5) |
| Fairly good | 1 (5.9) | 2 (11.8) | 2 (12.5) | 2 (13.3) | 1 (5.9) | 3 (9.4) | 1 (5.9) | 3 (9.4) | 3 (27.3) | 0 (0.0) |
| Moderate | 1 (5.9) | 1 (5.9) | 2 (12.5) | 0 (0.0) | 2 (11.8) | 2 (6.3) | 0 (0.0) | 2 (6.3) | 0 (0.0) | 1 (4.5) |
| Slightly poor | 0 (0.0) | 2 (11.8) | 0 (0.0) | 0 (0.0) | 0 (0.0) | 0 (0.0) | 0 (0.0) | 1 (3.1) | 0 (0.0) | 1 (4.5) |
| Poor | 1 (5.9) | 0 (0.0) | 0 (0.0) | 0 (0.0) | 0 (0.0) | 0 (0.0) | 0 (0.0) | 0 (0.0) | 0 (0.0) | 0 (0.0) |
| Very poor | 0 (0.0) | 1 (5.9) | 0 (0.0) | 0 (0.0) | 0 (0.0) | 0 (0.0) | 0 (0.0) | 1 (3.1) | 0 (0.0) | 0 (0.0) |

Fig. 12 continued

Global Assessment by Oxycodone/Naloxone Dose Ratio - ITT Population with Non-missing Values

| | | | | Dose Ratios | | | | | | |
|---|---|---|---|---|---|---|---|---|---|---|
| Assessment at end of maintenance phase (n, (%)) | 40 mg/ Placebo N=17 (100%) | 60 mg/ Placebo N=17 (100%) | 80 mg/ Placebo N=16 (100%) | 1/1 N=15 (100%) | 1.5/1 N=17 (100%) | 2/1 N=32 (100%) | 3/1 N=17 (100%) | 4/1 N=32 (100%) | 6/1 N=11 (100%) | 8/1 N=22 (100%) |
| Preference regarding efficacy (investigator) | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Titration phase | 4 (23.5) | 7 (41.2) | 6 (37.5) | 2 (13.3) | 5 (29.4) | 4 (12.5) | 5 (29.4) | 10 (31.3) | 1 (9.1) | 8 (36.4) |
| Maintenance phase | 5 (29.4) | 5 (29.4) | 4 (25.0) | 10 (66.7) | 7 (41.2) | 13 (40.6) | 5 (29.4) | 12 (37.5) | 4 (36.4) | 7 (31.8) |
| No preference | 5 (29.4) | 5 (29.4) | 5 (31.3) | 2 (13.3) | 2 (11.8) | 10 (31.3) | 2 (11.8) | 7 (21.9) | 5 (45.5) | 4 (18.2) |
| Preference regarding efficacy (patient) | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Titration phase | 4 (23.5) | 8 (47.1) | 5 (31.3) | 1 (6.7) | 5 (29.4) | 4 (12.5) | 5 (29.4) | 9 (28.1) | 1 (9.1) | 9 (40.9) |
| Maintenance phase | 6 (35.3) | 5 (29.4) | 5 (31.3) | 10 (66.7) | 7 (41.2) | 14 (43.8) | 6 (35.3) | 13 (40.6) | 4 (36.4) | 7 (31.8) |
| No preference | 4 (23.5) | 4 (23.5) | 5 (31.3) | 3 (20.0) | 2 (11.8) | 9 (28.1) | 1 (5.9) | 7 (21.9) | 5 (45.5) | 3 (13.6) |
| Preference regarding tolerability (investigator) | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Titration phase | 4 (23.5) | 7 (41.2) | 4 (25.0) | 0 (0.0) | 5 (29.4) | 4 (12.5) | 2 (11.8) | 9 (28.1) | 1 (9.1) | 5 (22.7) |
| Maintenance phase | 5 (29.4) | 5 (29.4) | 6 (37.5) | 12 (80.0) | 7 (41.2) | 15 (46.9) | 8 (47.1) | 13 (40.6) | 5 (45.5) | 10 (45.5) |
| No preference | 5 (29.4) | 5 (29.4) | 5 (31.3) | 2 (13.3) | 2 (11.8) | 8 (25.0) | 2 (11.8) | 7 (21.9) | 4 (36.4) | 4 (18.2) |
| Preference regarding tolerability (patient) | | | | | | | | | | |
| N | 14 (82.4) | 17 (100) | 15 (93.8) | 14 (93.3) | 14 (82.4) | 27 (84.4) | 12 (70.6) | 29 (90.6) | 10 (90.9) | 19 (86.4) |
| Titration phase | 4 (23.5) | 7 (41.2) | 4 (25.0) | 0 (0.0) | 5 (29.4) | 4 (12.5) | 2 (11.8) | 8 (25.0) | 1 (9.1) | 5 (22.7) |
| Maintenance phase | 5 (29.4) | 5 (29.4) | 6 (37.5) | 11 (73.3) | 7 (41.2) | 15 (46.9) | 9 (52.9) | 15 (46.9) | 5 (45.5) | 10 (45.5) |
| No preference | 5 (29.4) | 5 (29.4) | 5 (31.3) | 3 (20.0) | 2 (11.8) | 8 (25.0) | 1 (5.9) | 6 (18.8) | 4 (36.4) | 4 (18.2) |

EP 3 578 173 B1

Fig. 13  Global Assessment by Absolute Dose of Naloxone - ITT Population with Non-missing Values

| Assessment at end of maintenance phase (n, (%)) | Absolute Dose of Naloxone | | | |
|---|---|---|---|---|
| | Naloxone Placebo N=50 (100%) | Naloxone 10 mg N=49 (100%) | Naloxone 20 mg N=49 (100%) | Naloxone 40 mg N=48 (100%) |
| **Efficacy (investigator)** | | | | |
| N | 46 (92.0) | 42 (85.7) | 43 (87.8) | 40 (83.3) |
| Very good | 6 (12.0) | 5 (10.2) | 6 (12.2) | 7 (14.6) |
| Good | 16 (32.0) | 18 (36.7) | 23 (46.9) | 21 (43.8) |
| Fairly good | 7 (14.0) | 6 (12.2) | 3 (6.1) | 5 (10.4) |
| Moderate | 6 (12.0) | 10 (20.4) | 4 (8.2) | 3 (6.3) |
| Slightly poor | 5 (10.0) | 1 (2.0) | 1 (2.0) | 2 (4.2) |
| Poor | 3 (6.0) | 2 (4.1) | 6 (12.2) | 1 (2.1) |
| very poor | 3 (6.0) | 0 (0.0) | 0 (0.0) | 1 (2.1) |
| **Efficacy (patient)** | | | | |
| N | 46 (92.0) | 42 (85.7) | 43 (87.8) | 40 (83.3) |
| Very good | 4 (8.0) | 3 (6.1) | 5 (10.2) | 10 (20.8) |
| Good | 16 (32.0) | 18 (36.7) | 24 (49.0) | 19 (39.6) |
| Fairly good | 6 (12.0) | 5 (10.2) | 4 (8.2) | 4 (8.3) |
| Moderate | 9 (18.0) | 10 (20.4) | 4 (8.2) | 4 (8.3) |
| Slightly poor | 4 (8.0) | 4 (8.2) | 2 (4.1) | 1 (2.1) |
| Poor | 4 (8.0) | 2 (4.1) | 4 (8.2) | 1 (2.1) |
| very poor | 3 (6.0) | 0 (0.0) | 0 (0.0) | 1 (2.1) |
| **Tolerability (investigator)** | | | | |
| N | 46 (92.0) | 42 (85.7) | 43 (87.8) | 40 (83.3) |
| Very good | 9 (18.0) | 8 (16.3) | 13 (26.5) | 10 (20.8) |
| Good | 27 (54.0) | 27 (55.1) | 21 (42.9) | 24 (50.0) |
| Fairly good | 4 (8.0) | 5 (10.2) | 6 (12.2) | 5 (10.4) |
| Moderate | 2 (4.0) | 1 (2.0) | 1 (2.0) | 1 (2.1) |
| Slightly poor | 3 (6.0) | 1 (2.0) | 0 (0.0) | 0 (0.0) |
| Poor | 1 (2.0) | 0 (0.0) | 2 (4.1) | 0 (0.0) |
| very poor | 0 (0.0) | 0 (0.0) | 0 (0.0) | 0 (0.0) |
| **Tolerability (patient)** | | | | |
| N | 46 (92.0) | 42 (85.7) | 43 (87.8) | 40 (83.3) |
| Very good | 7 (14.0) | 9 (18.4) | 9 (18.4) | 8 (16.7) |
| Good | 26 (52.0) | 26 (53.1) | 25 (51.0) | 25 (52.1) |
| Fairly good | 5 (10.0) | 5 (10.2) | 3 (6.1) | 5 (10.4) |
| Moderate | 4 (8.0) | 1 (2.0) | 4 (8.2) | 2 (4.2) |
| Slightly poor | 2 (4.0) | 1 (2.0) | 1 (2.0) | 0 (0.0) |
| Poor | 1 (2.0) | 0 (0.0) | 0 (0.0) | 0 (0.0) |
| very poor | 1 (2.0) | 0 (0.0) | 1 (2.0) | 0 (0.0) |

EP 3 578 173 B1

Fig. 13 continued

Global Assessment by Absolute Dose of Naloxone - ITT Population with Non-missing Values

| Assessment at end of maintenance phase (n, (%)) | Absolute Dose of Naloxone | | | |
|---|---|---|---|---|
| | Naloxone Placebo N=50 (100%) | Naloxone 10 mg N=49 (100%) | Naloxone 20 mg N=49 (100%) | Naloxone 40 mg N=48 (100%) |
| Preference regarding efficacy (investigator) | | | | |
| N | 46 (92.0) | 42 (85.7) | 43 (87.8) | 40 (83.3) |
| Titration phase | 17 (34.0) | 11 (22.4) | 15 (30.6) | 9 (18.8) |
| Maintenance phase | 14 (28.0) | 19 (38.8) | 16 (32.7) | 23 (47.9) |
| No preference | 15 (30.0) | 12 (24.5) | 12 (24.5) | 8 (16.7) |
| Preference regarding efficacy (patient) | | | | |
| N | 46 (92.0) | 42 (85.7) | 43 (87.8) | 40 (83.3) |
| Titration phase | 17 (34.0) | 12 (24.5) | 14 (28.6) | 8 (16.7) |
| Maintenance phase | 16 (32.0) | 19 (38.8) | 19 (38.8) | 23 (47.9) |
| No preference | 13 (26.0) | 11 (22.4) | 10 (20.4) | 9 (18.8) |
| Preference regarding tolerability (investigator) | | | | |
| N | 46 (92.0) | 42 (85.7) | 43 (87.8) | 40 (83.3) |
| Titration phase | 15 (30.0) | 8 (16.3) | 10 (20.4) | 8 (16.7) |
| Maintenance phase | 16 (32.0) | 22 (44.9) | 24 (49.0) | 24 (50.0) |
| No preference | 15 (30.0) | 12 (24.5) | 9 (18.4) | 8 (16.7) |
| Preference regarding tolerability (patient) | | | | |
| N | 46 (92.0) | 42 (85.7) | 43 (87.8) | 40 (83.3) |
| Titration phase | 15 (30.0) | 8 (16.3) | 9 (18.4) | 8 (16.7) |
| Maintenance phase | 16 (32.0) | 23 (46.9) | 26 (53.1) | 23 (47.9) |
| No preference | 15 (30.0) | 11 (22.4) | 8 (16.3) | 9 (18.8) |

EP 3 578 173 B1

Figure 14

Global assessment – efficacy patients,
relative frequencies by absolute naloxone dose (ITT)

very good  good  slightly good  □ moderate  slightly poor  □ poor  very poor

Figure 15

Global assessment - preference efficacy patients,
relative frequencies by absolute naloxone dose (ITT)

EP 3 578 173 B1

Fig. 16    Laxative Intake at Each Study Visit by Oxycodone/Naloxone Dose Ratio - ITT Population with Non-missing

| Number of days with Laxative Intake | Dose Ratios | | | | | | | | | |
|---|---|---|---|---|---|---|---|---|---|---|
| | 40 mg/ Placebo | 60 mg/ Placebo | 80 mg/ Placebo | 1/1 | 1.5/1 | 2/1 | 3/1 | 4/1 | 6/1 | 8/1 |
| | N=17 (100%) | N=17 (100%) | N=16 (100%) | N=15 (100%) | N=17 (100%) | N=32 (100%) | N=17 (100%) | N=32 (100%) | N=11 (100%) | N=22 (100%) |
| Visit 3 (Randomization)* | | | | | | | | | | |
| N (n†) | 17 (15) | 15 (16) | 14 (14) | 12 (14) | 16 (16) | 31 (31) | 16 (15) | 29 (30) | 8 (11) | 20 (19) |
| Mean | 4.5 | 4.8 | 4.6 | 5.3 | 5.0 | 5.2 | 4.7 | 4.8 | 6.3 | 4.1 |
| SD | 3.12 | 2.54 | 2.79 | 2.99 | 2.88 | 2.68 | 2.85 | 3.10 | 1.75 | 3.21 |
| Median | 6.0 | 6.0 | 6.0 | 7.0 | 7.0 | 7.0 | 6.0 | 7.0 | 7.0 | 5.5 |
| Min-Max | 0-7 | 0-7 | 0-7 | 0-7 | 0-7 | 0-7 | 0-7 | 0-7 | 2-7 | 0-7 |
| Visit 4 (Maintenance)* | | | | | | | | | | |
| N (n†) | 15 (8) | 16 (13) | 15 (10) | 15 (9) | 14 (6) | 26 (16) | 16 (4) | 31 (21) | 9 (9) | 20 (12) |
| Mean | 1.8 | 2.3 | 2.3 | 2.3 | 1.3 | 1.9 | 1.0 | 2.6 | 3.2 | 1.3 |
| SD | 2.76 | 2.46 | 2.79 | 2.71 | 2.05 | 2.58 | 2.07 | 2.74 | 2.39 | 1.89 |
| Median | 0.0 | 1.0 | 1.0 | 1.0 | 0.0 | 0.0 | 0.0 | 2.0 | 4.0 | 0.5 |
| Min-Max | 0-7 | 0-6 | 0-7 | 0-7 | 0-6 | 0-7 | 0-6 | 0-7 | 0-6 | 0-6 |
| Visit 5 (End of Maintenance)* | | | | | | | | | | |
| N (n†) | 14 (10) | 15 (13) | 14 (12) | 12 (8) | 13 (5) | 26 (13) | 10 (4) | 26 (17) | 7 (8) | 19 (10) |
| Mean | 3.9 | 3.8 | 4.1 | 1.8 | 1.2 | 1.9 | 1.0 | 3.3 | 3.1 | 1.7 |
| SD | 3.30 | 3.55 | 3.52 | 3.17 | 2.61 | 3.15 | 2.31 | 3.53 | 3.63 | 2.94 |
| Median | 5.0 | 7.0 | 7.0 | 0.0 | 0.0 | 0.0 | 0.0 | 0.5 | 1.0 | 0.0 |
| Min-Max | 0-7 | 0-7 | 0-7 | 0-7 | 0-7 | 0-7 | 0-7 | 0-7 | 0-7 | 0-7 |
| Visit 6 (End of Follow-up)* | | | | | | | | | | |
| N (n†) | 13 (10) | 15 (14) | 14 (13) | 12 (11) | 11 (11) | 24 (21) | 11 (8) | 24 (19) | 6 (8) | 13 (14) |
| Mean | 3.8 | 4.0 | 4.5 | 2.8 | 3.4 | 4.0 | 2.3 | 4.1 | 3.5 | 3.9 |
| SD | 3.63 | 3.09 | 3.35 | 3.38 | 3.50 | 3.38 | 3.10 | 3.53 | 3.83 | 3.50 |
| Median | 7.0 | 5.0 | 7.0 | 0.5 | 1.0 | 7.0 | 1.0 | 7.0 | 3.5 | 7.0 |
| Min-Max | 0-7 | 0-7 | 0-7 | 0-7 | 0-7 | 0-7 | 0-7 | 0-7 | 0-7 | 0-7 |
| Entire Maintenance Phase** | | | | | | | | | | |
| N (n†) | 15 (12) | 16 (15) | 16 (14) | 15 (10) | 15 (9) | 26 (24) | 16 (9) | 32 (25) | 9 (9) | 21 (17) |
| Mean | 42.9 | 44.8 | 51.2 | 33.4 | 21.7 | 31.1 | 20.4 | 43.8 | 44.2 | 25.1 |
| SD | 44.60 | 43.84 | 42.37 | 42.87 | 39.71 | 41.47 | 39.80 | 42.57 | 43.13 | 33.54 |
| Median | 25.0 | 31.3 | 60.7 | 15.8 | 0.0 | 7.0 | 0.0 | 24.6 | 39.3 | 7.7 |
| Min-Max | 0-100 | 0-100 | 0-100 | 0-100 | 0-100 | 0-100 | 0-100 | 0-100 | 0-100 | 0-100 |

*Number of days with laxation intake during the last 7 days according to patient diary; **Percentage of days with laxation intake during the maintenance phase according to patient diary   n† Number of patients taking laxatives

EP 3 578 173 B1

Fig. 17 · Laxative Intake at Each Study Visit by Absolute Dose of Naloxone - ITT Population with Non-missing Values

| Number of days with Laxative Intake | Absolute Dose of Naloxone | | | |
|---|---|---|---|---|
| | Naloxone Placebo N=50 (100%) | Naloxone 10 mg N=49 (100%) | Naloxone 20 mg N=49 (100%) | Naloxone 40 mg N=48 (100%) |
| Visit 3 (Randomization)* | | | | |
| N ($n^t$) | 46 (45) | 44 (44) | 45 (47) | 43 (45) |
| Mean | 4.6 | 4.7 | 4.8 | 5.3 |
| SD | 2.78 | 2.99 | 2.91 | 2.73 |
| Median | 6.0 | 7.0 | 7.0 | 7.0 |
| Min-Max | 0-7 | 0-7 | 0-7 | 0-7 |
| Visit 4 (Maintenance)* | | | | |
| N ($n^t$) | 46 (31) | 44 (31) | 47 (24) | 40 (22) |
| Mean | 2.1 | 2.2 | 1.8 | 1.8 |
| SD | 2.62 | 2.46 | 2.52 | 2.42 |
| Median | 1.0 | 1.0 | 0.0 | 0.0 |
| Min-Max | 0-7 | 0-7 | 0-7 | 0-7 |
| Visit 5 (End of Maintenance)* | | | | |
| N ($n^t$) | 43 (35) | 37 (26) | 40 (18) | 36 (21) |
| Mean | 3.9 | 2.6 | 2.0 | 1.6 |
| SD | 3.38 | 3.34 | 3.14 | 2.93 |
| Median | 7.0 | 0.0 | 0.0 | 0.0 |
| Min-Max | 0-7 | 0-7 | 0-7 | 0-7 |
| Visit 6 (End of Follow-up)* | | | | |
| N ($n^t$) | 42 (37) | 32 (29) | 36 (31) | 33 (32) |
| Mean | 4.1 | 3.6 | 3.9 | 3.3 |
| SD | 3.28 | 3.51 | 3.37 | 3.38 |
| Median | 6.5 | 4.5 | 7.0 | 1.0 |
| Min-Max | 0-7 | 0-7 | 0-7 | 0-7 |
| Entire Maintenance Phase** | | | | |
| N ($n^t$) | 47 (41) | 46 (38) | 47 (35) | 41 (30) |
| Mean | 46.4 | 36.5 | 31.3 | 27.8 |
| SD | 42.78 | 39.50 | 41.38 | 41.25 |
| Median | 37.5 | 15.8 | 7.1 | 3.6 |
| Min-Max | 0-100 | 0-100 | 0-100 | 0-100 |

*Number of days with laxation intake during the last 7 days according to patient diary; **Percentage of days with laxation intake during the maintenance phase according to patient diary $n^t$ Number of patients taking laxatives

EP 3 578 173 B1

Figure 18   Overall Summary of Adverse Events During the Maintenance Phase by Oxycodone/Naloxone Dose Ratio - Safety Population

| | Dose Ratios | | | | | | | | | | | | | | | | | | | |
| | 40 mg/ Placebo | | 60 mg/ Placebo | | 80 mg/ Placebo | | 1/1 | | 1.5/1 | | 2/1 | | 3/1 | | 4/1 | | 6/1 | | 8/1 | |
| | N=17 (100%) | | N=17 (100%) | | N=16 (100%) | | N=15 (100%) | | N=18 (100%) | | N=34 (100%) | | N=18 (100%) | | N=33 (100%) | | N=12 (100%) | | N=22 (100%) | |
| Category | E | N (%) | E | N (%) | E | N (%) | E | N (%) | E | N (%) | E | N (%) | E | N (%) | E | N (%) | E | N (%) | E | N (%) |
| Adverse Events | 21 | 8 (47.1) | 54 | 15 (88.2) | 36 | 9 (56.3) | 26 | 8 (53.3) | 61 | 16 (88.9) | 84 | 19 (55.9) | 57 | 11 (61.1) | 73 | 22 (66.7) | 18 | 9 (75.0) | 69 | 17 (77.3) |
| Causally Related# | 12 | 6 (35.3) | 33 | 11 (64.7) | 29 | 8 (50.0) | 23 | 7 (46.7) | 44 | 12 (66.7) | 70 | 19 (55.9) | 40 | 8 (44.4) | 60 | 18 (54.5) | 14 | 6 (50.0) | 51 | 13 (59.1) |
| Leading to discontinuation of study drug | 0 | 0 (0.0) | 0 | 0 (0.0) | 1 | 1 (6.3) | 3 | 1 (6.7) | 14 | 4 (22.2) | 15 | 5 (14.7) | 27 | 5 (27.8) | 2 | 2 (6.1) | 1 | 1 (8.3) | 10 | 2 (9.1) |
| Serious Adverse Events | 0 | 0 (0.0) | 0 | 0 (0.0) | 1 | 1 (6.3) | 0 | 0 (0.0) | 2 | 2 (11.1) | 1 | 1 (2.9) | 1 | 1 (5.6) | 1 | 1 (3.0) | 1 | 1 (8.3) | 3 | 1 (4.5) |
| Causally Related# | 0 | 0 (0.0) | 0 | 0 (0.0) | 0 | 0 (0.0) | 0 | 0 (0.0) | 1 | 1 (5.6) | 1 | 1 (2.9) | 0 | 0 (0.0) | 0 | 0 (0.0) | 0 | 0 (0.0) | 3 | 1 (4.5) |
| Leading to discontinuation of study drug | 0 | 0 (0.0) | 0 | 0 (0.0) | 1 | 1 (6.3) | 0 | 0 (0.0) | 2 | 2 (11.1) | 1 | 1 (2.9) | 1 | 1 (5.6) | 1 | 1 (3.0) | 1 | 1 (8.3) | 3 | 1 (4.5) |
| Deaths | 0 | 0 (0.0) | 0 | 0 (0.0) | 0 | 0 (0.0) | 0 | 0 (0.0) | 0 | 0 (0.0) | 0 | 0 (0.0) | 0 | 0 (0.0) | 0 | 0 (0.0) | 0 | 0 (0.0) | 0 | 0 (0.0) |

E = Number of events
# = Related adverse events include all events listed with a definite, probable, possible or unknown/missing relationship to study drug

EP 3 578 173 B1

Figure 19

Overall Summary of Adverse Events During the Maintenance Phase by Absolute Dose of Naloxone - Safety Population

| | Absolute Dose of Naloxone | | | | | | | |
| | Naloxone Placebo | | Naloxone 10 mg | | Naloxone 20 mg | | Naloxone 40 mg | |
| | N=50 (100%) | | N=51 (100%) | | N=51 (100%) | | N=50 (100%) | |
| Category | E | N (%) | E | N (%) | E | N (%) | E | N (%) |
|---|---|---|---|---|---|---|---|---|
| Adverse Events | 111 | 32 (64.0) | 119 | 35 (68.6) | 129 | 32 (62.7) | 140 | 35 (70.0) |
| Causally Related# | 74 | 25 (50.0) | 93 | 26 (51.0) | 100 | 27 (52.9) | 109 | 30 (60.0) |
| Leading to discontinuation of study drug | 1 | 1 (2.0) | 13 | 5 (9.8) | 29 | 6 (11.8) | 30 | 9 (18.0) |
| Serious Adverse Events | 1 | 1 (2.0) | 5 | 3 (5.9) | 1 | 1 (2.0) | 3 | 3 (6.0) |
| Causally Related# | 0 | 0 (0.0) | 3 | 1 (2.0) | 0 | 0 (0.0) | 2 | 2 (4.0) |
| Leading to discontinuation of study drug | 1 | 1 (2.0) | 5 | 3 (5.9) | 1 | 1 (2.0) | 3 | 3 (6.0) |
| Deaths | 0 | 0 (0.0) | 0 | 0 (0.0) | 0 | 0 (0.0) | 0 | 0 (0.0) |

E = Number of events
# = Related adverse events include all events listed with a definite, probable, possible or unknown/missing relationship to study drug

Fig. 20 Adverse Events During the Maintenance Phase by Oxycodone/Naloxone Dose Ratio (Reported by ≥ 20% of Patients) and System Organ Class - Safety Population

| | Dose Ratios | | | | | | | | | |
|---|---|---|---|---|---|---|---|---|---|---|
| System Organ Class | 40 mg/ Placebo N=17 (100%) | 60 mg/ Placebo N=17 (100%) | 80 mg/ Placebo N=16 (100%) | 1/1 N=15 (100%) | 1.5/1 N=18 (100%) | 2/1 N=34 (100%) | 3/1 N=18 (100%) | 4/1 N=33 (100%) | 6/1 N=12 (100%) | 8/1 N=22 (100%) |
| | N (%) | N (%) | N (%) | N (%) | N (%) | N (%) | N (%) | N (%) | N (%) | N (%) |
| Gastrointestinal Disorders | 5 (29.4) | 10 (58.8) | 4 (25.0) | 7 (46.7) | 11 (61.1) | 16 (47.1) | 8 (44.4) | 16 (48.5) | 6 (50.0) | 9 (40.9) |
| Skin and Subcutaneous Tissue Disorders | 4 (23.5) | 6 (35.3) | 6 (37.5) | 4 (26.7) | 8 (44.4) | 8 (23.5) | 8 (44.4) | 9 (27.3) | 1 (8.3) | 11 (50.0) |
| Nervous System Disorders | 3 (17.6) | 8 (47.1) | 6 (37.5) | 1 (6.7) | 8 (44.4) | 9 (26.5) | 5 (27.8) | 5 (15.2) | 1 (8.3) | 5 (22.7) |
| Musculoskeletal and Connective Tissue Disorders | 3 (17.6) | 3 (17.6) | 1 (6.3) | 3 (20.0) | 3 (16.7) | 7 (20.6) | 4 (22.2) | 9 (27.3) | 2 (16.7) | 5 (22.7) |
| Psychiatric Disorders | 3 (17.6) | 5 (29.4) | 3 (18.8) | 2 (13.3) | 4 (22.2) | 6 (17.6) | 4 (22.2) | 6 (18.2) | 2 (16.7) | 5 (22.7) |
| Ear and Labyrinth Disorders | 0 (0.0) | 6 (35.3) | 2 (12.5) | 1 (6.7) | 3 (16.7) | 4 (11.8) | 2 (11.1) | 2 (6.1) | 1 (8.3) | 3 (13.6) |

EP 3 578 173 B1

Fig. 21 Adverse Events During the Maintenance Phase by Absolute Dose of Naloxone (Reported by ≥ 10% of Patients) and System Organ Class - Safety Population

| System Organ Class | Absolute Dose of Naloxone | | | | | | | |
| --- | --- | --- | --- | --- | --- | --- | --- | --- |
| | Naloxone Placebo | | Naloxone 10 mg | | Naloxone 20 mg | | Naloxone 40 mg | |
| | N=50 (100%) | | N=51 (100%) | | N=51 (100%) | | N=50 (100%) | |
| | N | (%) | N | (%) | N | (%) | N | (%) |
| Gastrointestinal Disorders | 19 | (38.0) | 22 | (43.1) | 24 | (47.1) | 27 | (54.0) |
| Skin and Subcutaneous Tissue Disorders | 16 | (32.0) | 17 | (33.3) | 16 | (31.4) | 16 | (32.0) |
| Nervous System Disorders | 17 | (34.0) | 9 | (17.6) | 11 | (21.6) | 14 | (28.0) |
| Musculoskeletal and Connective Tissue Disorders | 7 | (14.0) | 8 | (15.7) | 13 | (25.5) | 12 | (24.0) |
| Psychiatric Disorders | 11 | (22.0) | 10 | (19.6) | 8 | (15.7) | 11 | (22.0) |
| Ear and Labyrinth Disorders | 8 | (16.0) | 6 | (11.8) | 4 | (7.8) | 6 | (12.0) |
| General disorders and administration site conditions | 0 | (0.0) | 5 | (9.8) | 4 | (7.8) | 5 | (10.0) |

Figure 22.  Mean Plasma Concentration - Time Curves for Oxycodone Over Time by
Treatment - Full Analysis Population for Phamacokinetics

Figure 23.  Mean Plasma Concentration - Time Curves for Naloxone-3-Glucuronide
Over Time by Treatment - Full Analysis Population for Phamacokinetics

Figure 24.    Mean Plasma Concentration - Time Curves for Naloxone Over Time by
Treatment - Full Analysis Population for Phamacokinetics

Figure 25.    Mean Plasma Concentration - Time Curves for Noroxycodone Over
Time by Treatment - Full Analysis Population for Phamacokinetics

Figure 26.    Mean Plasma Concentration - Time Curves for Oxymorphone Over Time by Treatment - Full Analysis Population for Phamacokinetics

Figure 27.    Mean Plasma Concentration - Time Curves for Noroxymorphone Over
Time by Treatment - Full Analysis Population for Phamacokinetics

Figure 28.    Mean Plasma Concentration - Time Curves for 6-$\beta$-Naloxol Over Time
by Treatment - Full Analysis Population for Phamacokinetics

**Study Design**

**PHASE:**

**PERIOD:**

Sd = Study drug according to the RAS
P1-P4= Study Periods 1-4 each with a single dose of study drug according to a RAS, followed
by at least a 7-day washout between doses (periods 1-3 only)

Figure 29

Fig. 30: Mean plasma oxycodone profiles

- 1 tablet of OXN 10/5, fasted
- 1 tablet of OXN 10/5, fed
- 1 tablet of OXN 40/20, fasted
- 1 tablet of OXN 40/20, fed

Plasma concentration (ng/mL)

Time (hr)

Fig. 31: mean plasma naloxone-3-glucuronide profiles

EP 3 578 173 B1

Fig.32 Summary of Oxycodone Pharmacokinetic Parameters by Treatment: Full Analysis Population for Plasma Pharmacokinetic Metrics

| PK Metrics | Statistics | OXN 40/20 Fast | OXN 40/20 Fed | OXN 10/5 Fast | OXN 10/5 Fed |
|---|---|---|---|---|---|
| AUC [0-t] (ng.h/mL) | N | 21 | 23 | 27 | 24 |
| | Mean (SD) | 517.81 (176.87) | 604.34 (198.84) | 136.82 (41.04) | 153.26 (50.97) |
| | Median | 453.96 | 543.65 | 141.87 | 144.00 |
| | Min, Max | 261.26, 840.69 | 296.70, 1021.06 | 65.07, 216.93 | 76.39, 298.17 |
| | Geometric Mean | 489.46 | 574.33 | 130.43 | 145.62 |
| | Exponentiated LS Mean | 506.70 | 594.03 | 130.22 | 148.55 |
| AUC [0-Inf] (ng.h/mL) | N | 21 | 23 | 26 | 23 |
| | Mean (SD) | 519.67 (177.55) | 606.16 (199.48) | 136.11 (40.96) | 155.37 (52.01) |
| | Median | 455.95 | 544.48 | 137.06 | 149.50 |
| | Min, Max | 262.18, 842.79 | 297.35, 1025.03 | 65.71, 217.80 | 77.07, 299.48 |
| | Geometric Mean | 491.20 | 576.06 | 129.81 | 147.45 |
| | Exponentiated LS Mean | 507.78 | 595.06 | 132.15 | 149.72 |
| Cmax (ng/mL) | N | 21 | 23 | 27 | 24 |
| | Mean (SD) | 40.16 (10.42) | 50.29 (12.92) | 12.16 (3.54) | 14.54 (3.27) |
| | Median | 37.60 | 45.10 | 12.50 | 13.80 |
| | Min, Max | 23.90, 61.60 | 31.00, 77.50 | 6.06, 19.20 | 9.37, 23.00 |
| | Geometric Mean | 38.90 | 48.81 | 11.65 | 14.21 |
| | Exponentiated LS Mean | 40.75 | 50.48 | 11.62 | 14.46 |
| Tmax (h) | N | 21 | 23 | 27 | 24 |
| | Mean (SD) | 3.07 (1.519) | 4.35 (3.109) | 3.13 (1.370) | 3.35 (1.463) |
| | Median | 2.50 | 3.00 | 3.00 | 3.50 |
| | Min, Max | 1, 6 | 1, 12 | 1, 5 | 0.5, 5 |
| | LS Mean | 3.05 | 4.31 | 3.13 | 3.35 |
| t1/2 (h) | N | 21 | 23 | 26 | 23 |
| | Mean (SD) | 5.10 (1.275) | 4.85 (1.083) | 4.40 (0.692) | 4.12 (0.783) |
| | Median | 5.36 | 5.04 | 4.39 | 3.93 |
| | Min, Max | 2.88, 7.81 | 2.65, 6.81 | 3.37, 5.94 | 2.84, 6.93 |
| | LS Mean | 5.15 | 4.89 | 4.43 | 4.18 |
| LambdaZ (1/h) | N | 21 | 23 | 26 | 23 |
| | Mean (SD) | 0.146 (0.0423) | 0.151 (0.0388) | 0.161 (0.0251) | 0.173 (0.0278) |
| | Median | 0.129 | 0.138 | 0.158 | 0.177 |
| | Min, Max | 0.089, 0.241 | 0.102, 0.262 | 0.117, 0.206 | 0.100, 0.244 |

Fig.33 Statistics for Oxycodone Pharmacokinetic Metrics by Treatment: Full Analysis Population for Plasma Pharmacokinetic Metrics

| PK Metrics | Treatment Comparison | Exponentiated LS Means Ratio (%) | LS Means Difference | 90% CI - Lower - | - Upper - |
|---|---|---|---|---|---|
| AUC [0-t] | OXN 40/20 Fed vs. OXN 40/20 Fast | 117.23 | | 112.78 | 121.86 |
| | OXN 10/5 Fed vs. OXN 10/5 Fast | 114.07 | | 109.97 | 118.34 |
| AUC [0-Inf] | OXN 40/20 Fed vs. OXN 40/20 Fast | 117.19 | | 112.85 | 121.70 |
| | OXN 10/5 Fed vs. OXN 10/5 Fast | 113.30 | | 109.20 | 117.54 |
| Cmax | OXN 40/20 Fed vs. OXN 40/20 Fast | 123.87 | | 116.16 | 132.10 |
| | OXN 10/5 Fed vs. OXN 10/5 Fast | 124.45 | | 117.12 | 132.24 |
| Tmax (h) | OXN 40/20 Fed vs. OXN 40/20 Fast | | 1.26 | 0.37 | 2.15 |
| | OXN 10/5 Fed vs. OXN 10/5 Fast | | 0.21 | -0.62 | 1.04 |
| t1/2 (h) | OXN 40/20 Fed vs. OXN 40/20 Fast | | -0.26 | -0.65 | 0.14 |
| | OXN 10/5 Fed vs. OXN 10/5 Fast | | -0.25 | -0.63 | 0.13 |

Fig.34    Summary Statistics for Naloxone-3-Glucuronide Pharmacokinetic Parameters
by Treatment: Full Analysis Population for Plasma Pharmacokinetic Metrics

| PK Metrics | Statistics | OXN 40/20 Fast | OXN 40/20 Fed | OXN 10/5 Fast | OXN 10/5 Fed |
|---|---|---|---|---|---|
| AUC [0-t] (ng.h/mL) | n | 21 | 23 | 27 | 24 |
| | Mean (SD) | 638.86 (196.83) | 728.83 (234.65) | 161.18 (55.55) | 176.11 (71.59) |
| | Median | 587.93 | 664.48 | 150.94 | 158.04 |
| | Min, Max | 388.51, 1180.68 | 361.44, 1327.86 | 77.59, 299.63 | 88.38, 394.25 |
| | Geometric Mean | 613.20 | 695.95 | 152.00 | 164.50 |
| | Exponentiated LS Mean | 641.76 | 712.62 | 152.45 | 162.90 |
| AUC [0-inf] (ng.h/mL) | n | 17 | 19 | 19 | 12 |
| | Mean (SD) | 671.14 (203.28) | 703.06 (156.64) | 164.33 (53.42) | 201.64 (72.25) |
| | Median | 607.96 | 673.36 | 153.79 | 176.87 |
| | Min, Max | 410.39, 1181.54 | 485.06, 1049.87 | 80.21, 301.98 | 132.91, 396.40 |
| | Geometric Mean | 645.00 | 687.07 | 156.41 | 192.51 |
| | Exponentiated LS Mean | 653.82 | 718.21 | 156.10 | 169.75 |
| Cmax (ng/mL) | n | 21 | 23 | 27 | 24 |
| | Mean (SD) | 86.05 (24.39) | 84.68 (23.60) | 23.65 (7.01) | 24.30 (9.03) |
| | Median | 84.21 | 86.56 | 23.06 | 24.93 |
| | Min, Max | 49.00, 159.26 | 45.37, 139.09 | 12.08, 43.04 | 10.28, 48.73 |
| | Geometric Mean | 83.08 | 81.48 | 22.65 | 22.74 |
| | Exponentiated LS Mean | 84.54 | 82.31 | 22.67 | 22.41 |
| Tmax (h) | n | 21 | 23 | 27 | 24 |
| | Mean (SD) | 1.07 (1.228) | 3.04 (2.788) | 1.13 (0.957) | 2.50 (1.399) |
| | Median | 0.50 | 2.50 | 1.00 | 2.25 |
| | Min, Max | 0.5, 6 | 0.5, 12 | 0.5, 5 | 0.5, 5 |
| | LS Mean | 1.10 | 3.01 | 1.14 | 2.48 |
| t1/2 (h) | n | 17 | 19 | 19 | 12 |
| | Mean (SD) | 7.73 (1.728) | 7.44 (2.041) | 9.16 (3.865) | 7.70 (1.407) |
| | Median | 7.88 | 7.59 | 8.10 | 7.64 |
| | Min, Max | 5.47, 11.19 | 3.52, 11.19 | 4.12, 21.60 | 5.60, 9.68 |
| | LS Mean | 7.96 | 7.30 | 8.84 | 7.24 |
| LambdaZ (1/h) | n | 17 | 19 | 19 | 12 |
| | Mean (SD) | 0.094 (0.0208) | 0.101 (0.0325) | 0.086 (0.0310) | 0.093 (0.0179) |
| | Median | 0.088 | 0.091 | 0.086 | 0.091 |
| | Min, Max | 0.062, 0.127 | 0.062, 0.197 | 0.032, 0.168 | 0.072, 0.124 |

Fig. 35 Statistics for Naloxone-3-Glucuronide Pharmacokinetic Parameters by
Treatment: Full Analysis Population for Plasma Pharmacokinetic Metrics

| PK Metrics | Treatment Comparison | Exponentiated LS Means Ratio (%) | LS Means Difference | 90% CI - Lower - | 90% CI - Upper - |
|---|---|---|---|---|---|
| AUC [0-t] | OXN 40/20 Fed vs. OXN 40/20 Fast | 111.04 | | 105.79 | 116.56 |
| | OXN 10/5 Fed vs. OXN 10/5 Fast | 106.85 | | 102.05 | 111.87 |
| AUC [0-Inf] | OXN 40/20 Fed vs. OXN 40/20 Fast | 109.85 | | 102.59 | 117.62 |
| | OXN 10/5 Fed vs. OXN 10/5 Fast | 108.74 | | 100.72 | 117.39 |
| Cmax | OXN 40/20 Fed vs. OXN 40/20 Fast | 97.36 | | 88.46 | 107.16 |
| | OXN 10/5 Fed vs. OXN 10/5 Fast | 98.85 | | 90.33 | 108.18 |
| Tmax (h) | OXN 40/20 Fed vs. OXN 40/20 Fast | | 1.91 | 1.12 | 2.71 |
| | OXN 10/5 Fed vs. OXN 10/5 Fast | | 1.34 | 0.60 | 2.08 |
| t1/2 (h) | OXN 40/20 Fed vs. OXN 40/20 Fast | | -0.66 | -1.99 | 0.66 |
| | OXN 10/5 Fed vs. OXN 10/5 Fast | | -1.59 | -3.07 | -0.12 |

Fig.36 **Summary Statistics for Naloxone Pharmacokinetic Parameters by Treatment: Full Analysis Population for Plasma Pharmacokinetic Metrics**

| PK Metrics | Statistics | OXN 40/20 Fast | OXN 40/20 Fed | OXN 10/5 Fast | OXN 10/5 Fed |
|---|---|---|---|---|---|
| AUC [0-t] (pg.h/mL) | n | 21 | 23 | 27 | 24 |
| | Mean (SD) | 953.14 (652.46) | 1089.05 (813.25) | 116.12 (163.04) | 192.26 (266.68) |
| | Median | 800.75 | 870.45 | 45.10 | 99.64 |
| | Min, Max | 164.75, 2737.35 | 320.08, 3350.23 | 0.00, 600.55 | 16.43, 1221.58 |
| | Geometric Mean | 772.36 | 910.17 | 65.59 | 102.37 |
| | Exponentiated LS Mean | 737.78 | 916.77 | 59.48 | 98.34 |
| | | | | | |
| AUC [0-Inf] (pg.h/mL) | n | 3 | 6 | | |
| | Mean (SD) | 1851.24 (638.07) | 1389.93 (1035.49) | | |
| | Median | 1974.87 | 1091.79 | | |
| | Min, Max | 1160.41, 2418.45 | 720.58, 3473.73 | | |
| | Geometric Mean | 1769.68 | 1186.12 | | |
| | Exponentiated LS Mean | | 1109.63 | | |
| | | | | | |
| Cmax (pg/mL) | n | 21 | 23 | 27 | 24 |
| | Mean (SD) | 73.73 (45.14) | 151.90 (202.07) | 24.65 (24.30) | 50.02 (46.88) |
| | Median | 54.10 | 88.80 | 16.20 | 32.20 |
| | Min, Max | 30.30, 177.00 | 50.20, 1034.00 | 0.00, 103.00 | 15.70, 205.00 |
| | Geometric Mean | 62.82 | 108.40 | 23.09 | 37.47 |
| | Exponentiated LS Mean | 63.87 | 111.86 | 21.66 | 37.04 |
| | | | | | |
| Tmax (h) | n | 21 | 23 | 23 | 24 |
| | Mean (SD) | 5.17 (4.613) | 2.00 (2.174) | 5.00 (3.286) | 2.35 (3.184) |
| | Median | 5.00 | 1.00 | 5.00 | 1.00 |
| | Min, Max | 0.5, 16 | 0.5, 8 | 0.5, 12 | 0.5, 12 |
| | LS Mean | 5.06 | 1.93 | 4.77 | 2.32 |
| | | | | | |
| t1/2 (h) | n | 9 | 7 | | |
| | Mean (SD) | 13.90 (5.379) | 9.88 (5.397) | | |
| | Median | 13.08 | 8.39 | | |
| | Min, Max | 6.39, 21.79 | 5.10, 21.54 | | |
| | LS Mean | | 9.32 | | |
| | | | | | |
| LambdaZ (1/h) | n | 9 | 7 | | |
| | Mean (SD) | 0.059 (0.0290) | 0.083 (0.0315) | | |
| | Median | 0.053 | 0.083 | | |
| | Min, Max | 0.032, 0.108 | 0.032, 0.136 | | |

Fig. 37 Statistics for Naloxone Pharmacokinetic Parameters by Treatment: Full Analysis Population for Plasma Pharmacokinetic Metrics

| PK Metrics | Treatment Comparison | Exponentiated LS Means Ratio (%) | LS Means Difference | 90% CI - Lower - | - Upper - |
|---|---|---|---|---|---|
| AUC [0-t] | OXN 40/20 Fed vs. OXN 40/20 Fast | 124.26 | | 92.88 | 166.25 |
| | OXN 10/5 Fed vs. OXN 10/5 Fast | 165.34 | | 123.63 | 221.12 |
| AUC [0-Inf] | OXN 40/20 Fed vs. OXN 40/20 Fast | | | | |
| Cmax | OXN 40/20 Fed vs. OXN 40/20 Fast | 175.13 | | 137.24 | 223.47 |
| | OXN 10/5 Fed vs. OXN 10/5 Fast | 171.03 | | 134.36 | 217.72 |
| Tmax (h) | OXN 40/20 Fed vs. OXN 40/20 Fast | | -3.13 | -4.53 | -1.72 |
| | OXN 10/5 Fed vs. OXN 10/5 Fast | | -2.46 | -3.84 | -1.08 |
| t1/2 (h) | OXN 40/20 Fed vs. OXN 40/20 Fast | | | | |

Fig. 38 Study Diagram

*SD = Study drug according to random allocation schedule*
*P1-P5 = Periods 1-5 each identical with a single dose of study drug according to random allocation schedule, followed by a ≥ 7 day washout (Periods 1, 2, 3 and 4 only)*

Figure 39

**The Experimental Pain Model**

Pain-Ratings                    CSSEP

Figure 40

Fig.41 Pain-related Evoked Potentials, Statistically Significant Total Changes from Baseline in Amplitude P1, Amplitude P1N1, Latency P1, and Latency P2 after Stimulation with 60 and 70% $CO_2$: Safety Population

| | Overall Treatment | Oxycodone PR | Oxy nal 20/5 | Oxy nal 20/15 | Oxy nal 20/45 | Placebo |
|---|---|---|---|---|---|---|
| **Amplitude P1 (in μV)** | | | | | | |
| Cz 70% $CO_2$ | | | | | | |
| Mean | – | -2.25 | -2.51 | -0.31 | -2.58 | 9.27 |
| SD | – | 8.302 | 15.054 | 11.527 | 13.276 | 13.493 |
| p-value Placebo | 0.0460 | 0.0101 | 0.0090 | 0.0326 | 0.0167 | – |
| p-value Oxy PR | – | – | 0.9811 | 0.6414 | 0.8996 | – |
| **Amplitude P1N1 (in μV)** | | | | | | |
| C4 60% $CO_2$ | | | | | | |
| Mean | – | -15.25 | -9.93 | -6.43 | -2.25 | -1.31 |
| SD | – | 16.643 | 15.333 | 11.578 | 10.076 | 8.875 |
| p-value Placebo | 0.0125 | 0.0040 | 0.0841 | 0.3345 | 0.8294 | – |
| p-value Oxy PR | – | – | 0.2184 | 0.0463 | 0.0024 | – |
| **Latency P1 (in ms)** | | | | | | |
| Cz 60% $CO_2$ | | | | | | |
| Mean | – | 97.6 | 83.4 | 50.4 | 36.6 | -16.8 |
| SD | – | 55.02 | 59.81 | 84.22 | 80.26 | 55.22 |
| p-value Placebo | <0.0001 | <0.0001 | <0.0001 | 0.0035 | 0.0313 | – |
| p-value Oxy PR | – | – | 0.5354 | 0.0307 | 0.0049 | – |
| Cz 70% $CO_2$ | | | | | | |
| Mean | – | 85.0 | 101.0 | 32.2 | 41.2 | 8.6 |
| SD | – | 61.84 | 74.97 | 85.16 | 77.73 | 73.51 |
| p-value Placebo | 0.0006 | 0.0010 | 0.0001 | 0.2431 | 0.0543 | – |
| p-value Oxy PR | – | – | 0.5087 | 0.0261 | 0.1684 | – |
| C3 60% $CO_2$ | | | | | | |
| Mean | – | 96.8 | 72.2 | 56.2 | 31.6 | -25.6 |
| SD | – | 57.01 | 67.58 | 76.25 | 105.42 | 71.74 |
| p-value Placebo | 0.0001 | <0.0001 | 0.0003 | 0.0024 | 0.0408 | – |
| p-value Oxy PR | – | – | 0.3701 | 0.1170 | 0.0133 | – |

Fig. 41 continued

**Pain-related Evoked Potentials, Statistically Significant Total Change from Baseline in Amplitude P1, Amplitude P1N1, Latency P1, and Latency P2 after Stimulation with 60 and 70% $CO_2$: Safety Population (continued)**

| | Overall Treatment | Oxycodone PR | Oxy nal 20/5 | Oxy nal 20/15 | Oxy nal 20/45 | Placebo |
|---|---|---|---|---|---|---|
| **C3 70% $CO_2$** | | | | | | |
| Mean | — | 76.0 | 96.0 | 31.6 | 39.4 | -6.4 |
| SD | — | 68.49 | 73.62 | 101.19 | 91.30 | 82.10 |
| p-value Placebo | 0.0012 | 0.0018 | 0.0001 | 0.1167 | 0.0207 | — |
| p-value Oxy PR | — | — | 0.3982 | 0.0966 | 0.4196 | — |
| **C4 60% $CO_2$** | | | | | | |
| Mean | — | 95.0 | 68.6 | 35.0 | 39.4 | -17.2 |
| SD | — | 55.58 | 78.79 | 74.69 | 96.89 | 82.37 |
| p-value Placebo | 0.0011 | 0.0001 | 0.0017 | 0.0542 | 0.0585 | — |
| p-value Oxy PR | — | — | 0.3209 | 0.0242 | 0.0294 | — |
| **C4 70% $CO_2$** | | | | | | |
| Mean | — | 86.6 | 88.4 | 40.4 | 38.0 | -17.2 |
| SD | — | 66.79 | 69.03 | 104.42 | 91.37 | 78.24 |
| p-value Placebo | 0.0004 | 0.0001 | 0.0001 | 0.0265 | 0.0111 | — |
| p-value Oxy PR | — | — | 0.8705 | 0.0773 | 0.1849 | — |
| **Fz 60% $CO_2$** | | | | | | |
| Mean | — | 88.4 | 86.6 | 27.6 | 32.2 | -20.2 |
| SD | — | 59.67 | 68.49 | 86.39 | 57.84 | 72.95 |
| p-value Placebo | <0.0001 | <0.0001 | <0.0001 | 0.0328 | 0.0291 | — |
| p-value Oxy PR | — | — | 0.9806 | 0.0040 | 0.0069 | — |
| **Fz 70% $CO_2$** | | | | | | |
| Mean | — | 56.4 | 82.4 | 27.0 | 28.8 | -23.2 |
| SD | — | 82.34 | 86.69 | 91.18 | 104.66 | 97.31 |
| p-value Placebo | 0.0061 | 0.0077 | 0.0003 | 0.0925 | 0.0406 | — |
| p-value Oxy PR | — | — | 0.3089 | 0.2961 | 0.5532 | — |
| **Pz 60% $CO_2$** | | | | | | |
| Mean | — | 81.8 | 66.0 | 24.2 | 24.8 | -2.2 |
| SD | — | 59.19 | 87.53 | 100.34 | 91.52 | 69.40 |
| p-value Placebo | 0.0144 | 0.0027 | 0.0099 | 0.3523 | 0.2873 | — |
| p-value Oxy PR | — | — | 0.6298 | 0.0308 | 0.0516 | — |

Fig. 41 continued

**Pain-related Evoked Potentials, Statistically Significant Total Change from Baseline in Amplitude P1, Amplitude P1N1, Latency P1, and Latency P2 after Stimulation with 60 and 70% $CO_2$: Safety Population (continued)**

| | Overall Treatment | Oxycodone PR | Oxy nal 20/5 | Oxy nal 20/15 | Oxy nal 20/45 | Placebo |
|---|---|---|---|---|---|---|
| Pz 70% $CO_2$ | | | | | | |
| Mean | – | 75.0 | 80.2 | 38.8 | 26.2 | -1.0 |
| SD | – | 87.62 | 89.76 | 82.47 | 74.80 | 94.46 |
| p-value Placebo | 0.0135 | 0.0041 | 0.0018 | 0.1130 | 0.1271 | – |
| p-value Oxy PR | – | – | 0.7939 | 0.1732 | 0.1788 | – |
| **Latency P2 (In ms)** | | | | | | |
| Cz 70% $CO_2$ | | | | | | |
| Mean | – | 92.8 | 144.8 | 122.4 | 42.2 | -48.0 |
| SD | – | 165.79 | 173.28 | 259.11 | 143.96 | 261.14 |
| p-value Placebo | 0.0293 | 0.0288 | 0.0037 | 0.0073 | 0.1088 | – |
| p-value Oxy PR | – | – | 0.4558 | 0.5928 | 0.5800 | – |
| Fz 60% $CO_2$ | | | | | | |
| Mean | – | 97.8 | 118.2 | 154.8 | -8.6 | -32.4 |
| SD | – | 177.66 | 228.17 | 200.21 | 95.89 | 260.44 |
| p-value Placebo | 0.0175 | 0.0550 | 0.0176 | 0.0054 | 0.7140 | – |
| p-value Oxy PR | – | – | 0.6440 | 0.3564 | 0.1260 | – |
| Pz 70% $CO_2$ | | | | | | |
| Mean | – | 76.2 | 106.4 | 153.6 | 47.4 | -63.8 |
| SD | – | 151.19 | 177.11 | 256.77 | 123.53 | 246.66 |
| p-value Placebo | 0.0117 | 0.0280 | 0.0088 | 0.0006 | 0.0586 | – |
| p-value Oxy PR | – | – | 0.6673 | 0.1813 | 0.7899 | – |

Cross-reference Tables 14.2.1.1-14.2.1.10

Significant changes from baseline in latency P1 over time of treatment could be observed at recording position Cz after stimulation with 60% $CO_2$ and are graphically presented in Figure 5.

Pain-related Evoked Potentials: Mean Changes from Baseline in Latency P1 at Recoiding Position Cz after Stimulation with 60% $CO_2$: Full Analysis for Pharmacokinetics Population

Figure 42

Fig.43 (Mean Tonic Pain Scores in the 2nd Half of the Treatment Period, Change from Baseline over Time of Treatment: Full Analysis for Pharmacokinetics Population

Fig.44   Determination of the Peak Time of Intravenous Oxycodone
Antinociception in Male Sprague Dawley Rats in the 51°C Warm Water Withdrawal
test

* p < 0.05, compared to baseline withdrawal latency
‡ p < 0.05, compared to respective vehicle time-point

Fig. 45  Dose Response of Intravenous Oxycodone Antinociception in Male
Sprague Dawley Rats in the 51°C Warm Water Withdrawal Test

Fig. 46 Global Rating of Withdrawal Precipitation In Oxycodone-Dependent Rats following Intravenous Administration of 2:1 Oxycodone:Naloxone or Vehicle:Naloxone In Oxycodone-Dependent Rats (Oxycodone-P) - A Comparison with Naïve animals (Vehicle-P)

CD Vehicle-P (veh:naloxone 2.4 mg/kg, i.v.)

▆▆ Vehicle-P (oxycodone 4.8 mg/kg:naloxone 2.4 mg/kg, i.v.)

▨▨ Oxycodone-P (veh:naloxone, i.v.)

▨▨ Oxycodone-P (oxycodone:naloxone, i.v.)

‡ p < 0.05, compared to vehicle-P (veh:naloxone 2.4 mg/kg, i.v.)

§ p < 0.05, compared to vehicle-P (oxycodone 4.8 mg/kg:naloxone 2.4 mg/kg, i.v.)

¥ p < 0.05, compared to oxycodone-P (veh:naloxone, i.v.)

Fig.47 Precipitation of Graded Withdrawal Signs of Wet-Dog Shakes and Escape Attempts with Intravenous Oxycodone:Naloxone 2:1 or Vehicle:Naloxone in Oxycodone-Dependent Rats(Oxycodone-P) - A Comparison with Naïve animals (Vehicle-P)

□ Vehicle-P (veh:naloxone 2.4 mg/kg, i.v.)
■ Vehicle-P (oxycodone 4.8 mg/kg:naloxone 2.4 mg/kg, i.v.)
▨ Oxycodone-P (Veh:naloxone, i.v.)
▨ Oxycodone-P (Oxycodone:naloxone, i.v.)

‡ $p < 0.05$, compared to vehicle-P (veh:naloxone 2.4 mg/kg, i.v.)
§ $p < 0.05$, compared to vehicle-P (oxycodone 4.8 mg/kg:naloxone 2.4 mg/kg, i.v.)
¥ $p < 0.05$, compared to oxycodone-P (veh:naloxone, i.v.)

Fig. 48 · Weight Loss Following 2.5 h of Withdrawal Precipitated by Administration of Intravenous Oxycodone:Naloxone 2:1 or Vehicle:Naloxone in Oxycodone-Dependent Rats(Oxycodone-P) - A Comparison with Naïve animals (Vehicle-P)

☐ Vehicle-P (Veh:naloxone 2.4 mg/kg, i.v.)

■ Vehicle-P (Oxycodone 4.8 mg/kg: naloxone 2.4 mg/kg, i.v.)

▨ Oxycodone-P (Veh: naloxone, i.v.)

▨ Oxycodone-P (Oxycodone: naloxone, i.v.)

‡ $p < 0.05$, compared to vehicle-P (veh:naloxone 2.4 mg/kg, i.v.)

§ $p < 0.05$, compared to vehicle-P (oxycodone 4.8 mg/kg:naloxone 2.4 mg/kg, i.v.)

Fig. 49    Sumscores for Elicited Naloxone Typical Adverse events at Each Study Visit by Oxycodone/Naloxone Dose Ratio - ITT Population with Non-missing Values

| Sumscores | Dose Ratios | | | | | | | | | |
|---|---|---|---|---|---|---|---|---|---|---|
| | 40 mg/ Placebo | 60 mg/ Placebo | 80 mg/ Placebo | 1/1 | 1.5/1 | 2/1 | 3/1 | 4/1 | 6/1 | 8/1 |
| | N=17 (100%) | N=17 (100%) | N=16 (100%) | N=15 (100%) | N=17 (100%) | N=32 (100%) | N=17 (100%) | N=32 (100%) | N=11 (100%) | N=22 (100%) |
| Visit 4 (Maintenance)* | | | | | | | | | | |
| N (n†) | 15 (1) | 17 (5) | 16 (0) | 15 (3) | 15 (6) | 28 (6) | 16 (5) | 31 (8) | 11 (2) | 21 (2) |
| Mean | 0.1 | 0.4 | 0.0 | 0.7 | 1.1 | 0.7 | 0.8 | 0.6 | 0.4 | 0.4 |
| SD | 0.26 | 0.71 | 0.0 | 1.71 | 2.12 | 1.44 | 1.47 | 1.38 | 0.81 | 1.43 |
| Median | 0.0 | 0.0 | 0.0 | 0.0 | 0.0 | 0.0 | 0.0 | 0.0 | 0.0 | 0.0 |
| Min-Max | 0-1 | 0-2 | 0-0 | 0-6 | 0-8 | 0-4 | 0-4 | 0-6 | 0-2 | 0-6 |
| Visit 5 (End of Maintenance)* | | | | | | | | | | |
| N (n†) | 14 (1) | 17 (4) | 15 (1) | 14 (0) | 14 (4) | 28 (4) | 12 (0) | 29 (3) | 10 (1) | 19 (1) |
| Mean | 0.1 | 0.3 | 0.1 | 0.0 | 0.4 | 0.4 | 0.0 | 0.1 | 0.2 | 0.1 |
| SD | 0.27 | 0.59 | 0.26 | 0.0 | 0.85 | 0.99 | 0.0 | 0.31 | 0.63 | 0.23 |
| Median | 0.0 | 0.0 | 0.0 | 0.0 | 0.0 | 0.0 | 0.0 | 0.0 | 0.0 | 0.0 |
| Min-Max | 0-1 | 0-2 | 0-1 | 0-0 | 0-3 | 0-4 | 0-0 | 0-1 | 0-2 | 0-1 |
| Entire Maintenance Phase** | | | | | | | | | | |
| N (n†) | 15 (3) | 17 (9) | 16 (3) | 15 (5) | 15 (9) | 29 (10) | 16 (6) | 31 (12) | 11 (4) | 21 (9) |
| Mean | 0.4 | 0.9 | 0.3 | 1.1 | 1.5 | 1.2 | 1.1 | 1.3 | 0.8 | 1.0 |
| SD | 1.06 | 1.11 | 0.58 | 1.96 | 2.20 | 1.93 | 1.81 | 1.88 | 1.33 | 1.66 |
| Median | 0.0 | 1.0 | 0.0 | 0.0 | 1.0 | 0.0 | 0.0 | 0.0 | 0.0 | 0.0 |
| Min-Max | 0-4 | 0-4 | 0-2 | 0-6 | 0-8 | 0-6 | 0-6 | 0-6 | 0-4 | 0-7 |

*Sumscores for elicited adverse events during the last 7 days; **Sumscores for elicited adverse events during the entire maintenance phase
n† Number of patients with at least one elicited naloxone typical side effect

EP 3 578 173 B1

Fig. 50　Sumscores for Elicited Naloxone Typical Adverse events at Each Study Visit by Absolute Dose of Naloxone - ITT Population with Non-missing Values

| Sumscores | Absolute Dose of Naloxone | | | |
| | Naloxone Placebo N=50 (100%) | Naloxone 10 mg N=49 (100%) | Naloxone 20 mg N=49 (100%) | Naloxone 40 mg N=48 (100%) |
|---|---|---|---|---|
| Visit 4 (Maintenance)* | | | | |
| N (n†) | 48 (6) | 47 (6) | 47 (14) | 43 (12) |
| Mean | 0.2 | 0.3 | 0.8 | 0.9 |
| SD | 0.48 | 1.07 | 1.47 | 1.84 |
| Median | 0.0 | 0.0 | 0.0 | 0.0 |
| Min-Max | 0-2 | 0-6 | 0-6 | 0-8 |
| Visit 5 (End of Maintenance)* | | | | |
| N (n†) | 46 (6) | 42 (4) | 43 (4) | 41 (5) |
| Mean | 0.2 | 0.1 | 0.2 | 0.2 |
| SD | 0.42 | 0.40 | 0.57 | 0.80 |
| Median | 0.0 | 0.0 | 0.0 | 0.0 |
| Min-Max | 0-2 | 0-2 | 0-3 | 0-4 |
| Entire Maintenance Phase** | | | | |
| N (n†) | 48 (15) | 47 (17) | 47 (18) | 44 (20) |
| Mean | 0.5 | 0.8 | 1.2 | 1.4 |
| SD | 0.97 | 1.43 | 1.94 | 2.06 |
| Median | 0.0 | 0.0 | 0.0 | 0.0 |
| Min-Max | 0-4 | 0-7 | 0-6 | 0-8 |

*Sumscores for elicited adverse events during the last 7 days; **Sumscores for elicited adverse events during the entire maintenance phase
n† Number of patients with at least one elicited naloxone typical side effect

Fig. 51 Sumscores for Elicited Opioid Typical Adverse events at Each Study Visit by Oxycodone/Naloxone Dose Ratio - ITT Population with Non-missing Values

| | Dose Ratios | | | | | | | | | |
|---|---|---|---|---|---|---|---|---|---|---|
| Sumscores | 40 mg/ Placebo N=17 (100%) | 60 mg/ Placebo N=17 (100%) | 80 mg/ Placebo N=16 (100%) | 1/1 N=15 (100%) | 1.5/1 N=17 (100%) | 2/1 N=32 (100%) | 3/1 N=17 (100%) | 4/1 N=32 (100%) | 6/1 N=11 (100%) | 8/1 N=22 (100%) |
| Visit 3 (Randomization)* | | | | | | | | | | |
| N (n†) | 17 (2) | 17 (5) | 16 (3) | 15 (3) | 17 (6) | 32 (3) | 17 (10) | 32 (10) | 11 (1) | 22 (8) |
| Mean | 0.4 | 1.0 | 0.4 | 0.2 | 0.7 | 0.3 | 1.5 | 1.0 | 0.5 | 0.9 |
| SD | 1.22 | 1.77 | 1.02 | 0.41 | 1.10 | 0.95 | 1.66 | 1.82 | 1.51 | 1.36 |
| Median | 0.0 | 0.0 | - 0.0 | 0.0 | 0.0 | 0.0 | 1.0 | 0.0 | 0.0 | 0.0 |
| Min-Max | 0-5 | 0-5 | 0-4 | 0-1 | 0-3 | 0-5 | 0-5 | 0-7 | 0-5 | 0-4 |
| Visit 4 (Maintenance)* | | | | | | | | | | |
| N (n†) | 15 (4) | 17 (10) | 16 (4) | 15 (5) | 15 (7) | 28 (6) | 16 (8) | 31 (10) | 11 (1) | 21(10) |
| Mean | 0.7 | 1.0 | 0.5 | 0.5 | 1.7 | 0.5 | 1.4 | 0.6 | 0.4 | 0.9 |
| SD | 1.40 | 1.27 | 1.03 | 0.83 | 2.96 | 1.29 | 2.22 | 1.09 | 1.21 | 1.18 |
| Median | 0.0 | 1.0 | 0.0 | 0.0 | 0.0 | 0.0 | 0.5 | 0.0 | 0.0 | 0.0 |
| Min-Max | 0-5 | 0-5 | 0-3 | 0-2 | 0-10 | 0-5 | 0-8 | 0-5 | 0-4 | 0-4 |
| Visit 5 (End of Maintenance)* | | | | | | | | | | |
| N (n†) | 14 (2) | 17 (7) | 15 (2) | 14 (0) | 14 (2) | 28 (0) | 12 (1) | 29 (4) | 10 (1) | 19(4) |
| Mean | 0.6 | 1.1 | 0.3 | 0.0 | 0.3 | 0.0 | 0.1 | 0.3 | 0.2 | 0.3 |
| SD | 1.50 | 1.56 | 0.70 | 0.0 | 0.83 | 0.0 | 0.29 | 0.75 | 0.63 | 0.56 |
| Median | 0.0 | 0.0 | 0.0 | 0.0 | 0.0 | 0.0 | 0.0 | 0.0 | 0.0 | 0.0 |
| Min-Max | 0-5 | 0-5 | 0-2 | 0-0 | 0-3 | 0-0 | 0-1 | 0-3 | 0-2 | 0-2 |
| Visit 6 (End of Follow-up)* | | | | | | | | | | |
| N (n†) | 13 (1) | 17 (5) | 15 (0) | 14 (1) | 13 (4) | 26 (0) | 12 (0) | 28 (2) | 10 (0) | 18 (2) |
| Mean | 0.1 | 0.5 | 0.0 | 0.1 | 0.5 | 0.0 | 0.0 | 0.1 | 0.0 | 0.1 |
| SD | 0.28 | 1.07 | 0.0 | 0.27 | 0.78 | 0.0 | 0.0 | 0.59 | 0.0 | 0.32 |
| Median | 0.0 | 0.0 | 0.0 | 0.0 | 0.0 | 0.0 | 0.0 | 0.0 | 0.0 | 0.0 |
| Min-Max | 0-1 | 0-4 | 0-0 | 0-1 | 0-2 | 0-0 | 0-0 | 0-3 | 0-0 | 0-1 |
| Entire Maintenance Phase** | | | | | | | | | | |
| N (n†) | 15 (5) | 17 (13) | 16 (7) | 15 (7) | 15 (9) | 29 (11) | 16 (10) | 31 (14) | 11 (4) | 21 (11) |
| Mean | 1.0 | 2.1 | 0.9 | 0.8 | 2.2 | 1.1 | 1.9 | 1.0 | 0.8 | 1.3 |
| SD | 1.69 | 1.82 | 1.26 | 0.94 | 2.91 | 2.01 | 2.42 | 1.43 | 1.33 | 1.83 |
| Median | 0.0 | 2.0 | 0.0 | 0.0 | 2.0 | 0.0 | 1.0 | 0.0 | 0.0 | 1.0 |
| Min-Max | 0-5 | 0-6 | 0-4 | 0-2 | 0-10 | 0-8 | 0-8 | 0-5 | 0-4 | 0-6 |

*Sumscores for elicited adverse events during the last 7 days; **Sumscores for elicited adverse events during the entire maintenance phase
n† Number of patients with at least one elicited opioid typical side effect

EP 3 578 173 B1

146

Fig. 52 Sumscores for Elicited Opioid Typical Adverse events at Each Study Visit by Absolute Dose of Naloxone - ITT Population with Non-missing Values

| Sumscores | Absolute Dose of Naloxone | | | |
| | Naloxone Placebo N=50 (100%) | Naloxone 10 mg N=49 (100%) | Naloxone 20 mg N=49 (100%) | Naloxone 40 mg N=48 (100%) |
|---|---|---|---|---|
| Visit 3 (Randomization)* | | | | |
| N (n†) | 50 (10) | 49 (15) | 49 (16) | 48 (10) |
| Mean | 0.6 | 0.9 | 0.9 | 0.3 |
| SD | 1.39 | 1.67 | 1.53 | 0.75 |
| Median | 0.0 | 0.0 | 0.0 | 0.0 |
| Min-Max | 0-5 | 0-7 | 0-5 | 0-3 |
| Visit 4 (Maintenance)* | | | | |
| N (n†) | 48 (18) | 47 (16) | 47 (17) | 43 (14) |
| Mean | 0.7 | 0.7 | 0.8 | 0.9 |
| SD | 1.23 | 1.26 | 1.63 | 1.94 |
| Median | 0.0 | 0.0 | 0.0 | 0.0 |
| Min-Max | 0-5 | 0-5 | 0-8 | 0-10 |
| Visit 5 (End of Maintenance)* | | | | |
| N (n†) | 46 (11) | 42 (7) | 43 (3) | 41 (2) |
| Mean | 0.7 | 0.3 | 0.1 | 0.1 |
| SD | 1.34 | 0.63 | 0.50 | 0.49 |
| Median | 0.0 | 0.0 | 0.0 | 0.0 |
| Min-Max | 0-5 | 0-2 | 0-3 | 0-3 |
| Visit 6 (End of Follow-up)* | | | | |
| N (n†) | 45 (6) | 41 (2) | 41 (2) | 39 (5) |
| Mean | 0.2 | 0.0 | 0.1 | 0.2 |
| SD | 0.70 | 0.22 | 0.49 | 0.51 |
| Median | 0.0 | 0.0 | 0.0 | 0.0 |
| Min-Max | 0-4 | 0-1 | 0-3 | 0-2 |
| Entire Maintenance Phase** | | | | |
| N (n†) | 48 (25) | 47 (23) | 47 (23) | 44 (20) |
| Mean | 1.3 | 1.2 | 1.2 | 1.4 |
| SD | 1.67 | 1.61 | 1.88 | 2.27 |
| Median | 1.0 | 0.0 | 0.0 | 0.0 |
| Min-Max | 0-6 | 0-6 | 0-8 | 0-10 |

*Sumscores for elicited adverse events during the last 7 days; **Sumscores for elicited adverse events during the entire maintenance phase
n† Number of patients with at least one elicited opioid typical side effect

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3773955 A
- US 3966940 A
- US 4457933 A
- US 4582835 A
- EP 0352361 A1
- DE 4325465 A1
- WO 03084520 A
- WO 03084504 A
- US 6258042 B1
- WO 03073937 A1

### Non-patent literature cited in the description

- Supportive Maßnahmen in der Onkologie. Die Schmerzbehandlung von Tumorpatienten. Thieme, 1994, vol. 3
- **ZECH, D. ; GROND, S. ; LYNCH, J. ; HERTEL, D. ; LEHMANN, K.** Validation of World Health Organisation Guidelines for Cancer Pain Relief: a 10-year prospective study. *Pain,* 1995, vol. 63, 65-76
- **FORTH W. ; HENSCHLER, D. ; RUMMEL W. ; STARKE, K.** Allgemeine und Spezielle Pharmakologie und Toxikologie. Spektrum Akademischer Verlag, 1996